(19) Europäisches Patentamt
European Patent Office
Office européen des brevets

(11) Publication number: **0 209 230 B1**

# (12) EUROPEAN PATENT SPECIFICATION

(45) Date of publication of patent specification: **27.02.91**

(51) Int. Cl.5: **C07D 251/16**, C07D 491/048, C07D 491/052, C07C 49/613, A01N 47/36

(21) Application number: **86304077.0**

(22) Date of filing: **29.05.86**

The file contains technical information submitted after the application was filed and not included in this specification

## (54) Herbicidal sulfonamides.

(30) Priority: **30.05.85 US 739215**
**27.03.86 US 842791**
**30.05.85 US 739232**
**26.03.86 US 842295**

(43) Date of publication of application:
**21.01.87 Bulletin 87/04**

(45) Publication of the grant of the patent:
**27.02.91 Bulletin 91/09**

(84) Designated Contracting States:
**BE CH DE FR GB IT LI LU NL SE**

(56) References cited:
**EP-A- 0 096 593**
**EP-A- 0 101 670**
**EP-A- 0 161 211**
**EP-A- 0 178 101**
**EP-A-00 839 75**
**EP-A-00 850 28**
**EP-A-00 854 76**
**EP-A-01 114 42**
**EP-A-01 165 18**
**US-A- 4 475 944**
**US-A- 4 492 599**

**CHEMICAL ABSTRACTS, vol. 100, no. 25, June 18, 1984, Columbus, Ohio, USA; NIHON NOHYAKU CO., LTD. "Sulfonamide derivatives", page 604, column 2, abstract no. 209882s & Jpn. Kokai Tokkyo Koho JP 59 13,778 (84 13,778)**

(73) Proprietor: **E.I. DU PONT DE NEMOURS AND COMPANY**
**1007 Market Street**
**Wilmington Delaware 19898(US)**

(72) Inventor: **Christensen, Joel Robert**
**316 Shipley Street**
**Wilmington Delaware 19809(US)**
Inventor: **Rorer, Morris Padgett**
**64 Lower Valley Lane Green Valley**
**Newark Delaware 19711(US)**

(74) Representative: **Hildyard, Edward Martin et al**
**Frank B. Dehn & Co. Imperial House 15-19 Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

Background of the Invention

European Patent Application (EP-A) No. 83,975, published July 20, 1983, discloses herbicidal benzenesulfonamides of formula

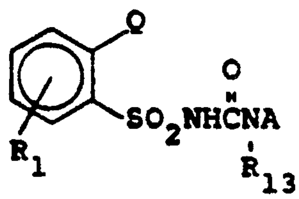

wherein
Q is selected from various five or six-membered aromatic or partially unsaturated heterocyclic rings containing 2 or 3 heteroatoms selected from O, S or NR.

European Patent Application (EP-A) No. 85,476, published August 10, 1983, discloses herbicidal benzenesulfonamides of formulae

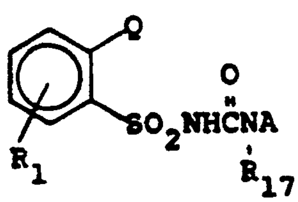

and

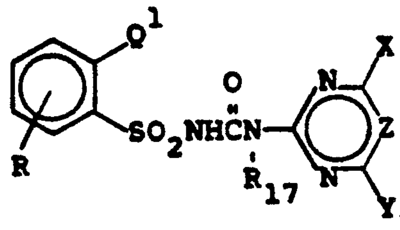

wherein
Q is selected from various 5-membered aromatic heterocycles, and their dihydro and tetrahydro analogs, which contain one heteroatom selected from O, S or NR, or Q is a saturated or partially unsaturated 6-membered ring containing one heteroatom selected from O or S; and
$Q^1$ is a 6-membered aromatic heterocycle containing one to three N atoms.

South African Patent Application 83/8416 (published May 12, 1984) discloses herbicidal benzenesulfonamides of formula

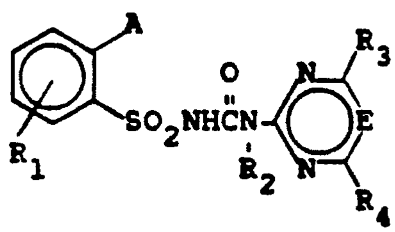

wherein
A is an unsaturated or only partially saturated 5- or 6-membered heterocyclic ring system which is bonded through a carbon atom and contains 1, 2 or 3 heteroatoms.

European Patent Application 116, 518 (Swiss priority 2/4/83, published 9/22/84) discloses herbicidal sulfonamides of formula

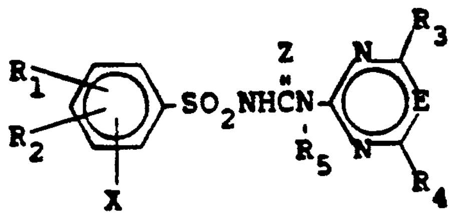

wherein

X is $NR_6R_7$, $N(SO_2R_9)_2$ or

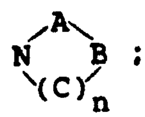

A is CO, $SO_2$, $CONR_{23}$ or $CO_2$;

B is $C_1$-$C_4$ alkyl or $C_2$-$C_4$ alkenyl; and

C is CO, $CR_{21}R_{22}$ or $SO_2$.

U.S. 4,475,944 discloses herbicidal sulfamates, possessing an ortho -heterocyclic ring, such as those of formula

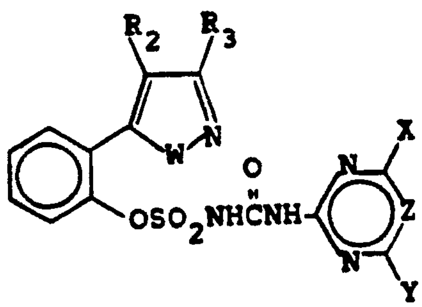

wherein

W is O, S or $NR_1$.

European Patent Application (EP-A) No. 141,777 (Swiss priority 9/9/83, published 6/15/85) discloses herbicidal sulfonamides of formula

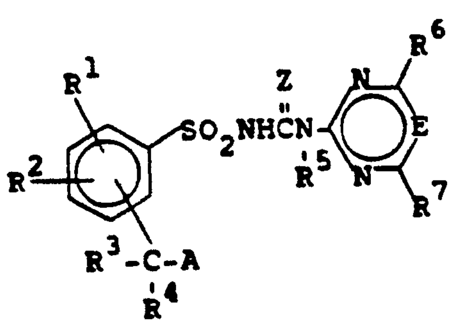

wherein

$R^3$ is H, $C_1$-$C_4$ alkyl, $C_1$-$C_4$ haloalkyl or CN;

$R^4$ is H or $C_1$-$C_4$ alkyl;

A is $Y(CH_2)_nR^{17}$ or

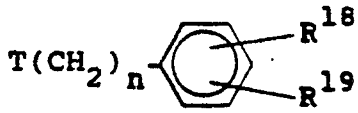 ;

$R^{17}$ is a 5- to 6-membered heterocyclic radical;
Y is O, S or a direct bond; and
n is 0 or 1.

South African Patent Application 83/0441 (Swiss priority 1/25/82) discloses herbicidal benzenesulfonamides of formula

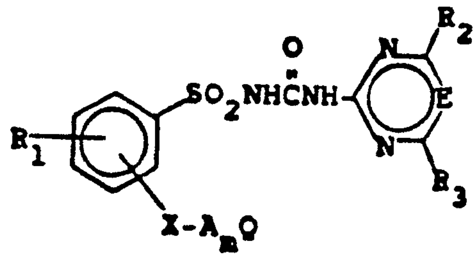

wherein

$R_1$ is H, haLogen. $NO_2$, $C_1$-$C_4$ haloalkyl. $C_1$-$C_4$ alkyl, $C_1$-$C_4$ alkoxy, $C_2$-$C_5$ alkenyl or $C_1$-$C_4$ alkoxycarbonyl;
$R_2$ is $C_1$-$C_3$ alkyl or $C_1$-$C_3$ alkyl, each unsubstituted or substituted by 1 to 3 halogen atoms or $C_1$-$C_4$ alkoxy, or is $C_1$-$C_3$ alkoxy, unsubstituted or substituted by methoxy, ethoxy, or 1 to 3 halogen atoms;
$R_3$ is halogen, H, $NR_4R_5$, $C_1$-$C_3$ alkyl, unsubstituted or substituted by 1 to 3 halogen atoms or $C_1$-$C_4$ alkoxy, or is $C_1$-$C_3$ alkoxy, unsubstituted or substituted by methoxy, ethoxy, or 1 to 3 halogen atoms;
A is $C_1$-$C_4$ alkylene or $C_2$-$C_4$ alkenylene, each unsubstituted or substituted by $C_1$-$C_4$ alkyl;
m is 0 or 1;
E is N or CH;
X is oxygen, sulfur, SO or $SO_2$; and
Q is, in part, a 5- or 6-membered heterocyclic ring or a fused homologue thereof, each linked through a carbon atom to the bridge $-X-A_m-$ or, if the heterocyclic ring contains nitrogen, is also bound through a nitrogen atom, and which is unsubstituted or monoto trisubstituted by halogen, cyano, nitro, $C_1$-$C_4$ alkyl, $C_1$-$C_4$ alkoxy, $C_1$-$C_4$ alkylthio, $C_2$-$C_5$ alkenyl, $C_1$-$C_4$ haloalkyl, $C_1$-$C_4$ alkoxycarbonyl, $-NR_{15}R_{16}$ or $-SO-NR_{17}R_{18}$.

Still further herbicidal sulfonamides are disclosed in EP-A 96,593, US-A -4,492,599 and EP-A -178,101.

## Summary of the Invention

This invention relates to novel compounds of Formula 1, agriculturally suitable compositions containing them, and their method-of-use as preemergent or postemergent herbicides or as plant growth regulants.

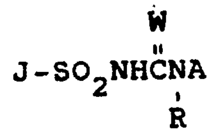

I

wherein

J is 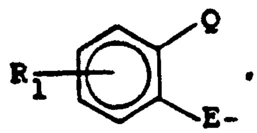 , 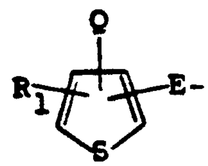 , 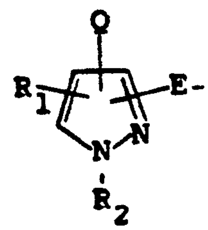 ,

J-1 J-2 J-3

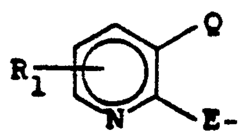 , 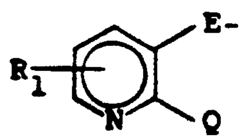

J-4 J-5

W is O or S;
R is H or $CH_3$;
E is a single bond, $CH_2$ or O;
Q is $Q_1$ or $E_1X_aQ_2$;
$Q_1$ is a saturated 5- or 6-membered heterocyclic ring, bonded through carbon or nitrogen, containing a carbonyl group and 2-3 heteroatoms selected from the group consisting of 0-2 oxygen, 0-2 sulfur or 0-2 nitrogen; a 5-membered heterocyclic ring, bonded through carbon or nitrogen, containing a carbonyl group and 2-3 heteroatoms selected from the group consisting of 0-2 oxygen, 0-2 sulfur or 1-3 nitrogen and containing one endocyclic double bond; a 6-membered heterocyclic ring, bonded through carbon or nitrogen, containing a carbonyl group and 2-3 heteroatoms selected from the group consisting of 0-2 oxygen, 0-2 sulfur or 1-3 nitrogen and containing one or two endocyclic double bonds; or a 5-membered heterocyclic ring, bonded through carbon or nitrogen, containing two adjacent carbonyl groups and 2 heteroatoms selected from the group consisting of 0-1 oxygen, 0-1 sulfur, or 1-2 nitrogen and containing one endocyclic double bond, said Q value may be substituted or unsubstituted wherein the substituent groups are selected from the group consisting of $C_1$-$C_4$ alkyl, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl, $C_1$-$C_3$ cyanoalkyl, $C_2$-$C_4$ alkoxyalkyl, $C_2$-$C_4$ alkylthioalkyl, $C_2$-$C_4$ alkylcarbonyl, $C_3$-$C_4$ alkylcarbonylalkyl, $C_1$-$C_4$ alkyl substituted with OH or $NH_2$, $C_2$-$C_4$ alkylaminoalkyl, $C_3$-$C_4$ dialkylaminoalkyl, $CH_2CH(OCH_3)_2$,

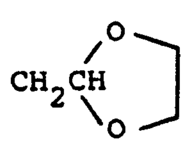 ,

$C(O)N(CH_3)_2$, $P(O)(OC_1$-$C_2$ alkyl$)_2$, $P(S)(OC_1$-$C_2$ alkyl$)_2$ or $C_1$-$C_2$ alkyl substituted with $C_1$-$C_2$ alkoxycarbonyl;
$E_1$ is O, S, SO, $SO_2$ or a single bond;
$Q_2$ is a 5- or 6-membered carbocyclic ring containing either one or two carbonyl groups and 0-1 endocyclic double bonds; a 5-membered heterocyclic ring, containing 2-4 atoms of carbon and 1-3 heteroatoms selected from the group consisting of 0-2 oxygen, 0-2 sulfur or 0-3 nitrogen, wherein sulfur

may take the form of S, SO, or $SO_2$, and containing one or two carbonyl or sulfonyl ($SO_2$) groups, or one carbonyl and one sulfonyl group and 0-1 endocyclic double bonds; or a 6-membered heterocyclic ring, containing 2-5 atoms of carbon and 1-3 heteroatoms selected from the group consisting of 0-2 oxygen, 0-2 sulfur or 0-3 nitrogen, wherein sulfur may take the form of S, SO or $SO_2$, and containing one or two carbonyl or sulfonyl ($SO_2$) groups, or one carbonyl and one sulfonyl group and 0-2 endocyclic double bonds; said Q value may further be optionally substituted with 1-2 substituent groups: substituents on carbon may be selected from the group consisting of halogen, $C_1$-$C_4$ alkyl, $C_1$-$C_4$ haloalkyl, $CH_2(C_2$-$C_3$ alkenyl), $CH_2(C_2$-$C_3$ alkynyl), $C_2$-$C_4$ alkoxycarbonyl, CN, OH, $C_1$-$C_3$ alkoxy, $C_1$-$C_3$ alkylthio, $C_1$-$C_3$ alkylsulfinyl, $C_1$-$C_3$ alkylsulfonyl or $C_2$-$C_4$ alkylcarbonyl; substituents on nitrogen may be selected from the group consisting of $C_1$-$C_4$ alkyl, $C_1$-$C_4$ haloalkyl, $CH_2(C_2$-$C_3$ alkenyl), $CH_2(C_2$-$C_3$ alkynyl), $C_2$-$C_4$ alkoxycarbonyl or $C_2$-$C_4$ alkylcarbonyl;

$X_a$ is $CH_2$, $CH(CH_3)$, $CH_2CH_2$, $CH_2CH_2CH_2$ or CO; $R_1$ is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, $CH_2CN$, CN, $CO_2R_c$, $C_1$-$C_3$ haloalkoxy, $C_1$-$C_3$ haloalkylthio, $C_2$-$C_4$ alkoxyalkyl, $C_2$-$C_4$ alkylthioalkyl, $CH_2N_3$ or $NR_dR_e$;

$R_a$ is H, $C_1$-$C_4$ alkyl, $C_2$-$C_3$ cyanoalkyl, methoxy or ethoxy;

$R_b$ is H, $C_1$-$C_4$ alkyl or $C_3$-$C_4$ alkenyl; or

$R_a$ and $R_b$ may be taken together as $-(CH_2)_3-$, $-(CH_2)_4-$, $-(CH_2)_5-$ or $-CH_2CH_2OCH_2CH_2-$;

$R_c$ is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, $C_5$-$C_6$ cycloalkyl, $C_4$-$C_7$ cycloalkylalkyl or $C_2$-$C_4$ alkoxyalkyl;

$R_d$ and $R_e$ are independently H or $C_1$-$C_2$ alkyl;

A is 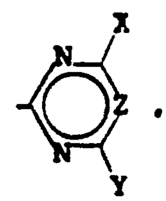 , 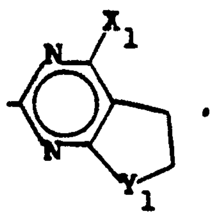 , 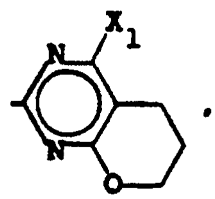 ,

A-1 A-2 A-3

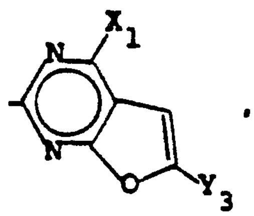 , 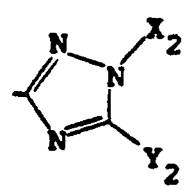 , 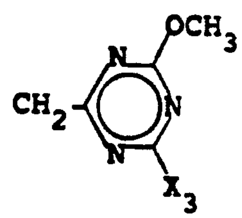 ;

A-4 A-5 A-6

or 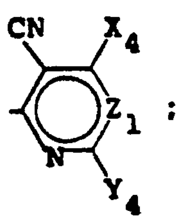 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$

alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$-$C_4$ haloalkyl, $C_2$-$C_4$ alkynyl, azido, cyano, $C_2$-$C_5$ alkylsulfinylalkyl, $C_2$-$C_5$ alkylsulfonylalkyl,

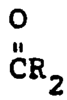, 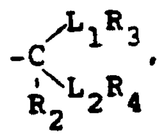, 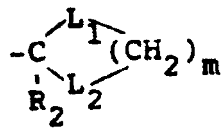, 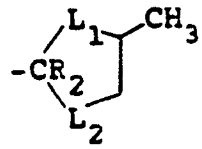

or $N(OCH_3)CH_3$;

m is 2 or 3;
$L_1$ and $L_2$ are independently O or S;
$R_2$ is H or $C_1$-$C_3$ alkyl;
$R_3$ and $R_4$ are independently $C_1$-$C_3$ alkyl;
Z is CH, N, $CCH_3$, $CC_2H_5$, CCl or CBr;
$Z_1$ is CH or N;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl; and
$Y_4$ is $CH_3$, $OCH_3$, $OC_2H_5$ or Cl;

and their agriculturally suitable salts;
provided that

a) when Q contains 2 heteroatoms selected from 0-2 oxygen and 0-2 sulfur, said heteroatoms are not bonded directly to one another, and when Q contains 3 nitrogen heteroatoms, only two of these may be bonded directly together;
b) when X is Cl, F, Br or I, then Z is CH and Y is $OCH_3$, $OC_2H_5$, $N(OCH_3)CH_3$, $NHCH_3$, $N(CH_3)_2$ or $OCF_2H$;
c) when X or Y is $C_1$ haloalkoxy, then Z is CH;
d) when J is J-2 or J-3, the substituent Q and the sulfonylurea bridge are on adjacent carbon atoms;
e) when E is O, then J is J-1 and W is O;
f) when W is S, then R is H, A is A-1, Z is CH or N and Y is $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$ or 1,3-dioxolan-2-yl;
g) when the total number of carbons of X and Y is greater than four, then the number of carbons of $R_1$ must be less than or equal to two, and the number of carbons of the substituent on Q must be less than or equal to two;
h) $X_4$ and $Y_4$ are not simultaneously Cl;
i) when $Q_1$ is bound through nitrogen and contains 2-heteroatoms and one carbonyl group, and said heteroatoms are bound through the carbonyl, then J is other than J-1;
j) when A is A-1 and J is J-1 wherein E is a single bond, $X_a$ is $CH_2$, $CH(CH_3)$ or $CH_2CH_2$ and $Q_2$ is a 5-membered heterocyclic ring containing one endocyclic double bond or a 6-membered heterocyclic ring containing 1 or 2 endocyclic double bonds which is unsubstituted or substituted by one or more $C_1$-$C_4$ alkyl groups then said heterocycle must contain at least one nitrogen and be bound to $X_a$ through nitrogen;
k) when $X_a$ is CO, then $E_1$ is a single bond; and
l) when J is J-1, then $E_1$ is a single bond.

Exemplary values of $Q_1$ include:

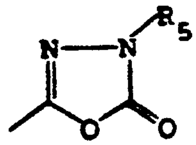 , 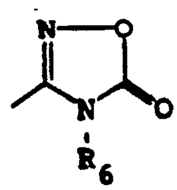 , 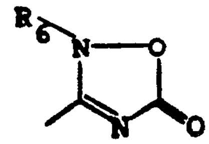 ,

$Q_1$-1 $Q_1$-2 $Q_1$-3

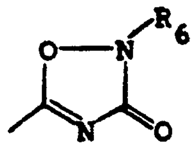 , 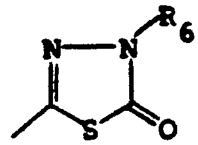 , 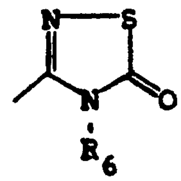 ,

$Q_1$-4 $Q_1$-5 $Q_1$-6

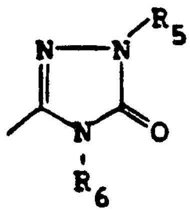 , 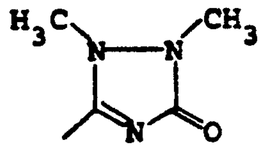 , 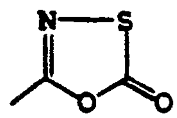 ,

$Q_1$-7 $Q_1$-8 $Q_1$-9

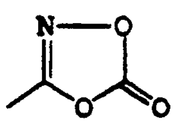 , 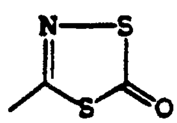 , 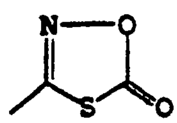 ,

$Q_1$-10 $Q_1$-11 $Q_1$-12

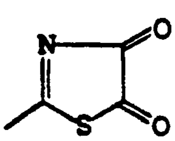 , 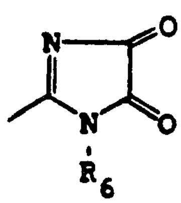 , 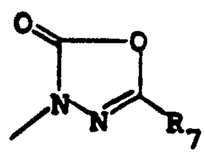 ,

$Q_1$-13 $Q_1$-14 $Q_1$-15

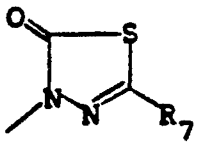 , 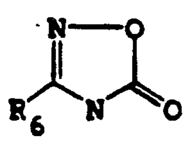 , 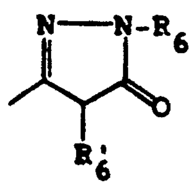 ,

$Q_1$-16 $Q_1$-17 $Q_1$-18

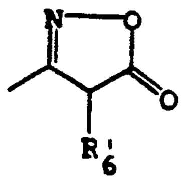 , 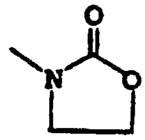 , 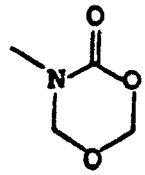 ,

$Q_1$-19 $Q_1$-20 $Q_1$-21

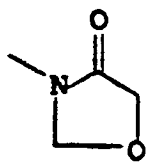 , 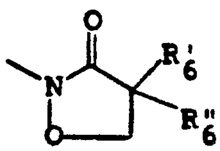 , 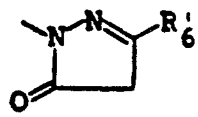

$Q_1$-22 $Q_1$-23 $Q_1$-24

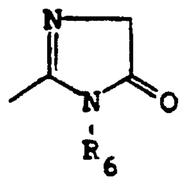 , 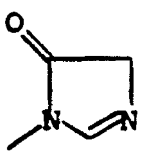 , 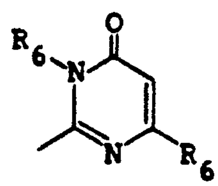 ,

$Q_1$-25 $Q_1$-26 $Q_1$-27

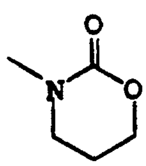 , 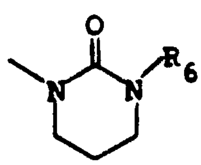 or 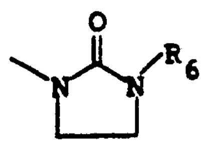 ;

$Q_1$-28 $Q_1$-29 $Q_1$-30

wherein

$R_5$ is H, $C_1$-$C_4$ alkyl, $CH_2CH=CH_2$, $CH_2CH=CHCH_3$, $CH_2C{\equiv}CH$, $CH_2C{\equiv}CCH_3$, $CH_2CN$, $CH_2CO_2$($C_1$-$C_2$ alkyl), $CH(CH_3)CO_2$($C_1$-$C_2$ alkyl), $CF_2H$, $C_2$-$C_3$ alkyl substituted with 1-3 atoms of F or Cl, $CH_2OCH_3$, $CH_2OCH_2CH_3$, $CH_2CH_2OCH_3$, or $CH_2CH_2OCH_2CH_3$;

$R_6$ is $C_1$-$C_3$ alkyl;

$R_6'$ and $R_6''$ are independently H or $C_1$-$C_2$ alkyl; and

$R_7$ is H, $C_1$-$C_4$ alkyl, $C_1$-$C_3$ haloalkyl or $CH_2CH=CH_2$.

Representative examples of preferred $Q_2$ include:

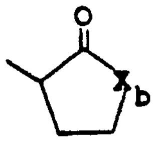 .

$Q_2$-1

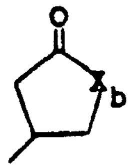 .

$Q_2$-2

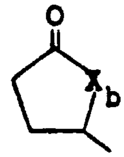 .

$Q_2$-3

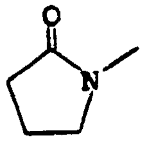 .

$Q_2$-4

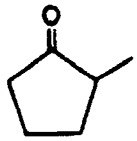 .

$Q_2$-5

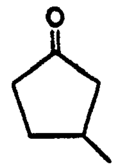 .

$Q_2$-6

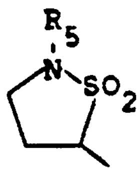 .

$Q_2$-7

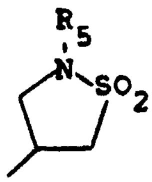 .

$Q_2$-8

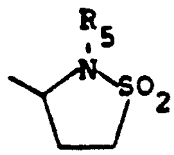 .

$Q_2$-9

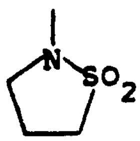 .

$Q_2$-10

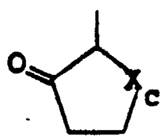 .

$Q_2$-11

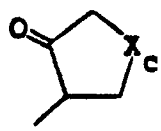 .

$Q_2$-12

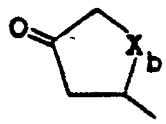,

$Q_2$-13

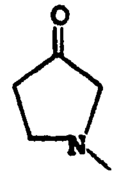,

$Q_2$-14

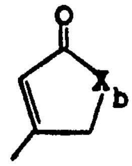,

$Q_2$-15

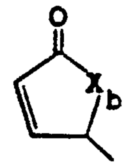,

$Q_2$-16

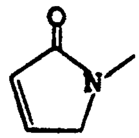,

$Q_2$-17

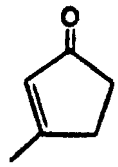,

$Q_2$-18

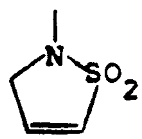,

$Q_2$-19

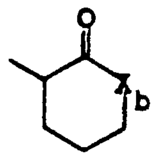,

$Q_2$-20

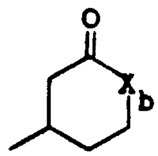,

$Q_2$-21

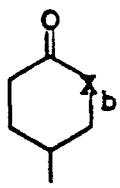,

$Q_2$-22

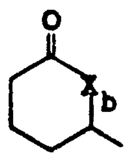,

$Q_2$-23

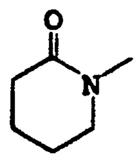,

$Q_2$-24

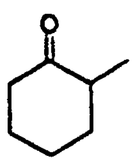,

$Q_2$-25

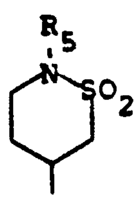,

$Q_2$-26

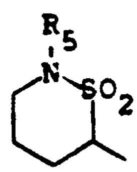,

$Q_2$-27

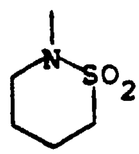 ,

$Q_2$-28

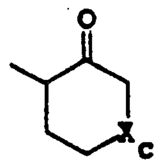 ,

$Q_2$-29

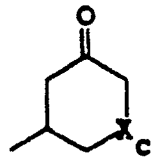 ,

$Q_2$-30

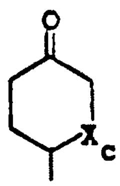 ,

$Q_2$-31

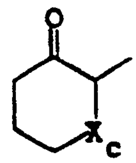 ,

$Q_2$-32

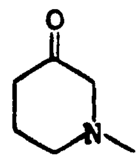 ,

$Q_2$-33

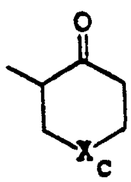 ,

$Q_2$-34

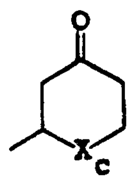 ,

$Q_2$-35

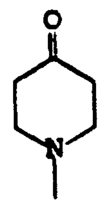 ,

$Q_2$-36

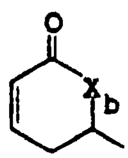 ,

$Q_2$-37

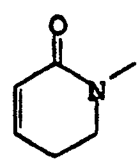 ,

$Q_2$-38

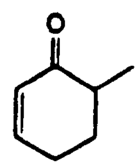 ,

$Q_2$-39

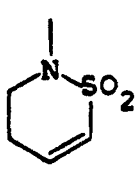 ,

$Q_2$-40

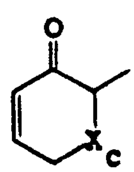 ,

$Q_2$-41

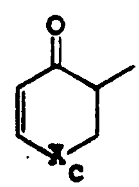 ,

$Q_2$-42

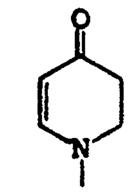,

$Q_2$-43

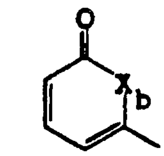,

$Q_2$-44

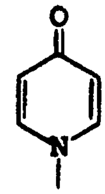,

$Q_2$-45

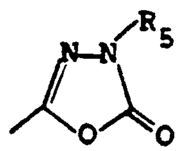,

$Q_2$-46

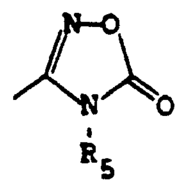,

$Q_2$-47

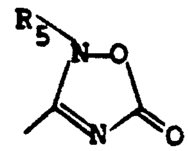,

$Q_2$-48

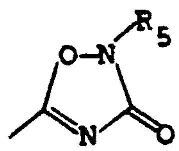,

$Q_2$-49

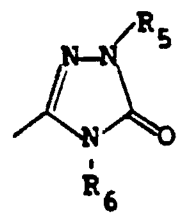,

$Q_2$-50

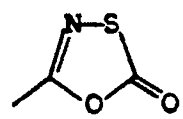,

$Q_2$-51

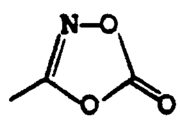,

$Q_2$-52

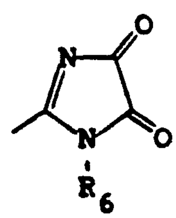,

$Q_2$-53

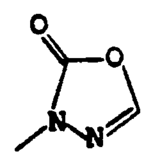,

$Q_2$-54

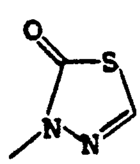,

$Q_2$-55

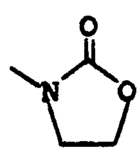,

$Q_2$-56

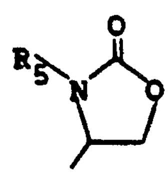,

$Q_2$-57

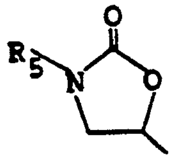 ,

Q2-58

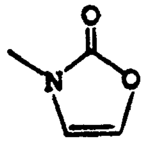 ,

Q2-59

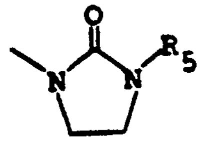 ,

Q2-60

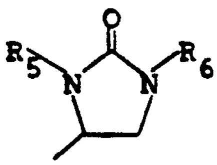 ,

Q2-61

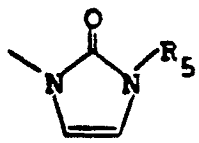 ,

Q2-62

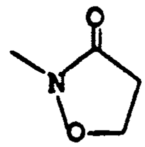 ,

Q2-63

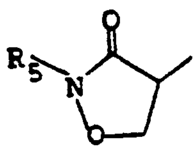 ,

Q2-64

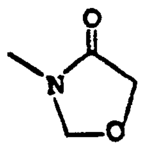 ,

Q2-65

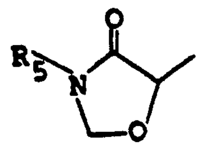 ,

Q2-66

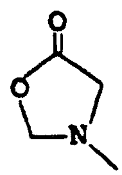 ,

Q2-67

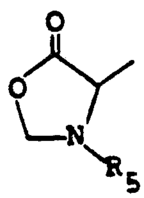 ,

Q2-68

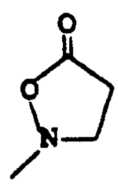 ,

Q2-69

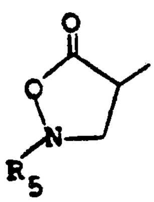 ,

Q2-70

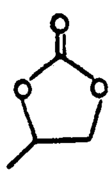 ,

Q2-71

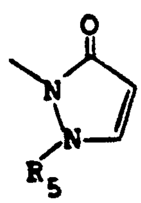 ,

Q2-72

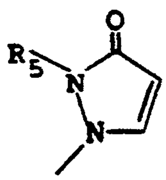 , 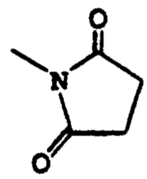 , 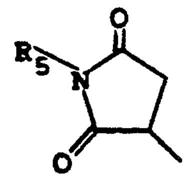 ,

Q2-73 Q2-74 Q2-75

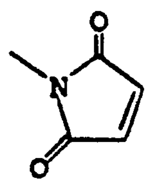 , 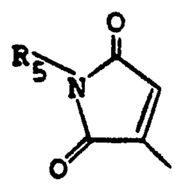 , 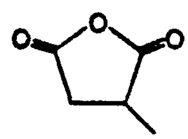 ,

Q2-76 Q2-77 Q2-78

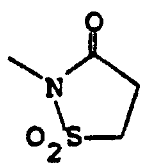 , 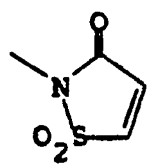 , 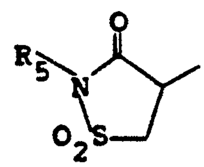 ,

Q2-79 Q2-80 Q2-81

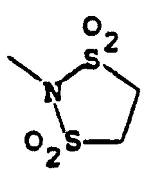 , 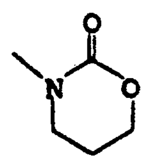 , 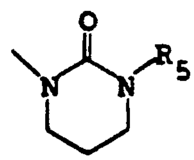 ,

Q2-82 Q2-83 Q2-84

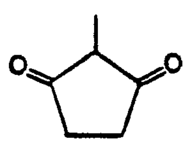 , 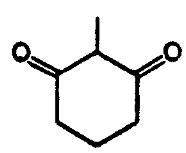 and 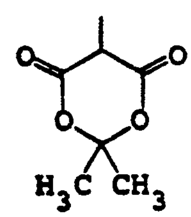 ;

Q2-85 Q2-86 Q2-87

wherein

$Q_2$-1 through $Q_2$-87 may be optionally substituted with 1 or 2 groups selected from $C_1$-$C_2$ alkyl or $C_1$-$C_2$ haloalkyl;

$R_5$ and $R_6$ are independently H or $C_1$-$C_3$ alkyl;

$X_b$ is O or $NR_5$; and

$X_c$ is O, S, SO, $SO_2$ or $NR_5$.

In the above definitions, the term "alkyl", used either alone or in compound words such as "alkylthio" or "haloalkyl", denotes straight chain or branched alkyl, e.g. methyl, ethyl, n -propyl, isopropyl or the different butyl, pentyl, hexyl, heptyl, octyl, nonyl or decyl isomers.

Alkoxy denotes methoxy, ethoxy, n -propoxy, isopropyloxy and the different butyl isomers.

Alkenyl denotes straight chain or branched alkenes, e.g. vinyl, 1-propenyl, 2-propenyl, 3-propenyl and the different butenyl isomers.

Alkynyl denotes straight chain or branch alkynes, e.g., ethynyl, 1-propynyl, 2-propynyl and the different butynyl isomers.

Alkylsulfonyl denotes methylsulfonyl, ethylsulfonyl and the different propylsulfonyl isomers.

Alkylthio, alkylsulfinyl, alkylamino, alkylsulfamoyl, etc. are defined in an analogous manner.

Cycloalkyl denotes cyclopropyl, cyclobutyl, cyclopentyl and cyclohexyl.

The term "halogen", either alone or in compound words such as "haloalkyl", denotes fluorine, chlorine, bromine or iodine. Further, when used in compound words such as "haloalkyl" said alkyl may be partially halogenated or fully substituted with halogen atoms which may be the same or different. Examples of haloalkyl include $CH_2CH_2F$, $CF_2CF_3$ and $CH_2CHFCl$.

Alkylcarbonyl denotes acetyl, propionyl, and the different butyryl isomers.

Alkoxycarbonyl denotes methoxycarbonyl, ethoxycarbonyl, propoxycarbonyl and isopropoxycarbonyl.

The total number of carbon atoms in a substituent group is indicated by the $C_i$-$C_j$ prefix where i and j are numbers from 1 to 10. For example, $C_2$ cyanoalkyl would designate $CH_2CN$, $C_3$ cyanoalkyl would designate $CH_2CH_2CN$ and $CH(CN)CH_3$, and $C_2$-$C_3$ alkylthioaklyl would designate $CH_2SCH_3$, $CH_2SC_2H_5$, $CH_2CH_2SCH_3$ or $CH(CH_3)SCH_3$, and $C_2$-$C_5$ alkoxyalkoxy would represent $OCH_2OCH_3$ through $O(CH_2)_4OCH_3$ or $OCH_2O(CH_2)_3CH_3$ and the various structural isomers embraced therein.

Preferred for reasons of increased ease of synthesis and/or greater herbicidal efficacy are:

1. Compounds of Formula I where
   Q is $Q_1$;
   E is a single bond; and
   Z is CH or N;
2. Compounds of Formula I where
   Q is $Q_1$;
   E is $CH_2$;
   W is O; and
   Z is CH or N;
3. Compounds of Formula I where
   Q is $Q_1$;
   E is O; and
   Z is CH or N;
4. Compounds of Preferred 1 where $Q_1$ is $Q_1$-1 to $Q_1$-30;
5. Compounds of Preferred 4 where
   W is O;
   R is H;
   $R_1$ is H, F, Cl. Br, $C_1$-$C_2$ alkyl, $C_1$-$C_3$ alkoxy, $C_1$-$C_3$ alkylthio, or $C_1$-$C_2$ alkyl, $C_1$-$C_3$ alkoxy or $C_1$-$C_3$ alkylthio substituted with 1-3 atoms of F, Cl or Br;
   X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
   Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C≡CH$, $OCH_2CH_2OCH_3$, $CH_2SCH_3$,

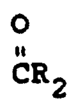, 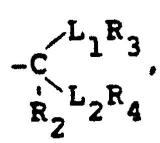, 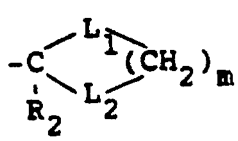, 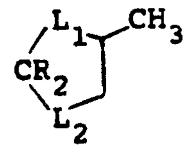.

$OCF_2H$, $SCF_2H$, cyclopropyl, $C≡CH$ or $C≡CCH_3$;

6. Compounds of Preferred 5 where A is A-1;
7. Compounds of Preferred 6 where J is J-1;

8. Compounds of Preferred 6 where J is J-2;
9. Compounds of Preferred 6 where J is J-3;
10. Compounds of Preferred 6 where J is J-4;
11. Compounds of Preferred 6 where J is J-5;
12. Compounds of Preferred 6 where
 J is J-1;
 $R_1$ is H, Cl, $CH_3$ or $OCH_3$ and is not para to the sulfonylurea bridge;
 X is $CH_3$, $OCH_3$, Cl or $OCF_2H$; and
 Y is $CH_3$, $OCH_3$, $C_2H_5$, $CH_2OCH_3$, $NHCH_3$, $CH(OCH_3)_2$ or cyclopropyl;
13. Compounds of Preferred 12 where $Q_1$ is $Q_1$-1;
14. Compounds of Preferred 12 where $Q_1$ is $Q_1$-2;
15. Compounds of Preferred 12 where $Q_1$ is $Q_1$-3;
16. Compounds of Preferred 12 where $Q_1$ is $Q_1$-4;
17. Compounds of Preferred 12 where $Q_1$ is $Q_1$-5;
18. Compounds of Preferred 12 where $Q_1$ is $Q_1$-6;
19. Compounds of Preferred 12 where $Q_1$ is $Q_1$-7;
20. Compounds of Preferred 12 where $Q_1$ is $Q_1$-8;
21. Compounds of Preferred 12 where $Q_1$ is $Q_1$-9;
22. Compounds of Preferred 12 where $Q_1$ is $Q_1$-10;
23. Compounds of Preferred 12 where $Q_1$ is $Q_1$-11;
24. Compounds of Preferred 12 where $Q_1$ is $Q_1$-12;
25. Compounds of Preferred 12 where $Q_1$ is $Q_1$-13;
26. Compounds of Preferred 12 where $Q_1$ is $Q_1$-14;
27. Compounds of Preferred 12 where $Q_1$ is $Q_1$-15;
28. Compounds of Preferred 12 where $Q_1$ is $Q_1$-16;
29. Compounds of Preferred 12 where $Q_1$ is $Q_1$-17;
30. Compounds of Preferred 12 where $Q_1$ is $Q_1$-18;
31. Compounds of Preferred 12 where $Q_1$ is $Q_1$-19;
32. Compounds of Preferred 12 where $Q_1$ is $Q_1$-21;
33. Compounds of Preferred 12 where $Q_1$ is $Q_1$-22;
34. Compounds of Preferred 12 where $Q_1$ is $Q_1$-23;
35. Compounds of Preferred 12 where $Q_1$ is $Q_1$-24;
36. Compounds of Preferred 12 where $Q_1$ is $Q_1$-25;
37. Compounds of Preferred 12 where $Q_1$ is $Q_1$-26;
38. Compounds of Preferred 12 where $Q_1$ is $Q_1$-27;
39. Compounds of Preferred 2 where
 R is H;
 J is J-1;
 $R_1$ is H;
 A is A-1;
 X is $CH_3$, $OCH_3$, $OCH_2CH_3$, Cl or $OCF_2H$;
 Y is $CH_3$, $OCH_3$, $C_2H_5$, $CH_2OCH_3$, $NHCH_3$, $CH(OCH_3)_2$ or cyclopropyl and $Q_1$ is $Q_1$-1, $Q_1$-7, $Q_1$-10 or $Q_1$-15;
40. Compounds of Preferred 3 where
 R is H;
 J is J-1;
 $R_1$ is H;
 A is A-1;
 X is $CH_3$, $OCH_3$, $OCH_2CH_3$, Cl or $OCF_2H$;
 Y is $CH_3$, $OCH_3$, $C_2H_5$, $CH_2OCH_3$, $NHCH_3$, $CH(OCH_3)_2$ or cyclopropyl and $Q_1$ is $Q_1$-1, $Q_1$-7, $Q_1$-10 or $Q_1$-15;
41. Compounds of Formula I where
 Q is $E_1X_aQ_2$;
 E is a single bond; and
 Z is CH or N;
42. Compounds of Formula I where
 Q is $E_1X_aQ_2$;
 E is $CH_2$;

W is O;
Z is CH or N; and
$E_1$ is a single bond;

43. Compounds of Formula I where
Q is $E_1X_aQ_2$;
E is O;
Z is CH or N; and
$E_1$ is a single bond;

44. Compounds of Preferred 1 where
$Q_2$ is $Q_2$-1 to $Q_2$-87; wherein $Q_2$-1 through $Q_2$-87 may be optionally substituted with 1 or 2 groups selected from $C_1$-$C_2$ alkyl or $C_1$-$C_2$ haloalkyl;
$R_5$ and $R_6$ are independently H or $C_1$-$C_3$ alkyl;
$X_b$ is O or $NR_5$;
$X_c$ is O, S, SO, $SO_2$ or $NR_5$;
E is a single bond; and
Z is CH or N;

45. Compounds of Preferred 44 where
W is O;
$E_1$ is a single bond;
$X_a$ is $CH_2$ or $CH_2CH_2$;
R is H;
$R_1$ is H, F, Cl, Br, $C_1$-$C_2$ alkyl, $C_1$-$C_3$ alkoxy, $C_1$-$C_3$ alkylthio, or $C_1$-$C_2$ alkyl, $C_1$-$C_3$ alkoxy or $C_1$-$C_3$ alkylthio substituted with 1-3 atoms of F, Cl or Br;
X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$,

$CH_2SCH_3$, 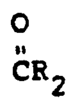, 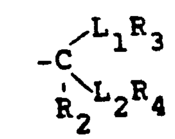, 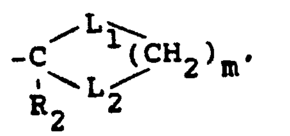,

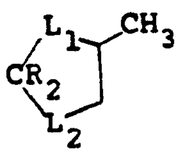, $OCF_2H$, $SCF_2H$, cyclopropyl, $C\equiv CH$

or $C\equiv CCH_3$:

46. Compounds of Preferred 45 where A is A-1;
47. Compounds of Preferred 46 where J is J-1;
48. Compounds of Preferred 46 where J is J-2;
49. Compounds of Preferred 46 where J is J-3;
50. Compounds of Preferred 46 where J is J-4;
51. Compounds of Preferred 46 where J is J-5;
52. Compounds of Preferred 46 where
J is J-1;
$R_1$ is H, Cl, $CH_3$ or $OCH_3$ and is not para to the sulfonylurea bridge;
X is $CH_3$, $OCH_3$, Cl or $OCF_2H$; and
Y is $CH_3$, $OCH_3$, $C_2H_5$, $CH_2OCH_3$, $NHCH_3$, $CH(OCH_3)_2$ or cyclopropyl;
53. Compounds of Preferred 52 where $Q_2$ is $Q_2$-1;
54. Compounds of Preferred 52 where $Q_2$ is $Q_2$-2;
55. Compounds of Preferred 52 where $Q_2$ is $Q_2$-3;
56. Compounds of Preferred 52 where $Q_2$ is $Q_2$-4;
57. Compounds of Preferred 52 where $Q_2$ is $Q_2$-5;
58. Compounds of Preferred 52 where $Q_2$ is $Q_2$-6;
59. Compounds of Preferred 52 where $Q_2$ is $Q_2$-7;
60. Compounds of Preferred 52 where $Q_2$ is $Q_2$-8;

61. Compounds of Preferred 52 where $Q_2$ is $Q_2$-9;
62. Compounds of Preferred 52 where $Q_2$ is $Q_2$-10;
63. Compounds of Preferred 52 where $Q_2$ is $Q_2$-11;
64. Compounds of Preferred 52 where $Q_2$ is $Q_2$-12;
65. Compounds of Preferred 52 where $Q_2$ is $Q_2$-13;
66. Compounds of Preferred 52 where $Q_2$ is $Q_2$-14;
67. Compounds of Preferred 52 where $Q_2$ is $Q_2$-15;
68. Compounds of Preferred 52 where $Q_2$ is $Q_2$-16;
69. Compounds of Preferred 52 where $Q_2$ is $Q_2$-17;
70. Compounds of Preferred 52 where $Q_2$ is $Q_2$-18;
71. Compounds of Preferred 52 where $Q_2$ is $Q_2$-19;
72. Compounds of Preferred 52 where $Q_2$ is $Q_2$-20;
73. Compounds of Preferred 52 where $Q_2$ is $Q_2$-21;
74. Compounds of Preferred 52 where $Q_2$ is $Q_2$-22;
75. Compounds of Preferred 52 where $Q_2$ is $Q_2$-23;
76. Compounds of Preferred 52 where $Q_2$ is $Q_2$-24;
77. Compounds of Preferred 52 where $Q_2$ is $Q_2$-25;
78. Compounds of Preferred 52 where $Q_2$ is $Q_2$-26;
79. Compounds of Preferred 52 where $Q_2$ is $Q_2$-27;
80. Compounds of Preferred 52 where $Q_2$ is $Q_2$-28;
81. Compounds of Preferred 52 where $Q_2$ is $Q_2$-29;
82. Compounds of Preferred 52 where $Q_2$ is $Q_2$-30;
83. Compounds of Preferred 52 where $Q_2$ is $Q_2$-31;
84. Compounds of Preferred 52 where $Q_2$ is $Q_2$-32;
85. Compounds of Preferred 52 where $Q_2$ is $Q_2$-33;
86. Compounds of Preferred 52 where $Q_2$ is $Q_2$-34;
87. Compounds of Preferred 52 where $Q_2$ is $Q_2$-35;
88. Compounds of Preferred 52 where $Q_2$ is $Q_2$-36;
89. Compounds of Preferred 52 where $Q_2$ is $Q_2$-37;
90. Compounds of Preferred 52 where $Q_2$ is $Q_2$-38;
91. Compounds of Preferred 52 where $Q_2$ is $Q_2$-39;
92. Compounds of Preferred 52 where $Q_2$ is $Q_2$-40;
93. Compounds of Preferred 52 where $Q_2$ is $Q_2$-41;
94. Compounds of Preferred 52 where $Q_2$ is $Q_2$-42;
95. Compounds of Preferred 52 where $Q_2$ is $Q_2$-43;
96. Compounds of Preferred 52 where $Q_2$ is $Q_2$-44;
97. Compounds of Preferred 52 where $Q_2$ is $Q_2$-45;
98. Compounds of Preferred 52 where $Q_2$ is $Q_2$-46;
99. Compounds of Preferred 52 where $Q_2$ is $Q_2$-47;
100. Compounds of Preferred 52 where $Q_2$ is $Q_2$-48;
101. Compounds of Preferred 52 where $Q_2$ is $Q_2$-49;
102. Compounds of Preferred 52 where $Q_2$ is $Q_2$-50;
103. Compounds of Preferred 52 where $Q_2$ is $Q_2$-51;
104. Compounds of Preferred 52 where $Q_2$ is $Q_2$-52;
105. Compounds of Preferred 52 where $Q_2$ is $Q_2$-53;
106. Compounds of Preferred 52 where $Q_2$ is $Q_2$-54;
107. Compounds of Preferred 52 where $Q_2$ is $Q_2$-55;
108. Compounds of Preferred 52 where $Q_2$ is $Q_2$-56;
109. Compounds of Preferred 52 where $Q_2$ is $Q_2$-57;
110. Compounds of Preferred 52 where $Q_2$ is $Q_2$-58;
111. Compounds of Preferred 52 where $Q_2$ is $Q_2$-59;
112. Compounds of Preferred 52 where $Q_2$ is $Q_2$-60;
113. Compounds of Preferred 52 where $Q_2$ is $Q_2$-61;
114. Compounds of Preferred 52 where $Q_2$ is $Q_2$-62;
115. Compounds of Preferred 52 where $Q_2$ is $Q_2$-63;
116. Compounds of Preferred 52 where $Q_2$ is $Q_2$-64;
117. Compounds of Preferred 52 where $Q_2$ is $Q_2$-65;
118. Compounds of Preferred 52 where $Q_2$ is $Q_2$-66;

119. Compounds of Preferred 52 where $Q_2$ is $Q_2$-67;
120. Compounds of Preferred 52 where $Q_2$ is $Q_2$-68;
121. Compounds of Preferred 52 where $Q_2$ is $Q_2$-69;
122. Compounds of Preferred 52 where $Q_2$ is $Q_2$-70;
123. Compounds of Preferred 52 where $Q_2$ is $Q_2$-71;
124. Compounds of Preferred 52 where $Q_2$ is $Q_2$-72;
125. Compounds of Preferred 52 where $Q_2$ is $Q_2$-73;
126. Compounds of Preferred 52 where $Q_2$ is $Q_2$-74;
127. Compounds of Preferred 52 where $Q_2$ is $Q_2$-75;
128. Compounds of Preferred 52 where $Q_2$ is $Q_2$-76;
129. Compounds of Preferred 52 where $Q_2$ is $Q_2$-77;
130. Compounds of Preferred 52 where $Q_2$ is $Q_2$-78;
131. Compounds of Preferred 52 where $Q_2$ is $Q_2$-79;
132. Compounds of Preferred 52 where $Q_2$ is $Q_2$-80;
133. Compounds of Preferred 52 where $Q_2$ is $Q_2$-81;
134. Compounds of Preferred 52 where $Q_2$ is $Q_2$-82;
135. Compounds of Preferred 52 where $Q_2$ is $Q_2$-83;
136. Compounds of Preferred 52 where $Q_2$ is $Q_2$-84;
137. Compounds of Preferred 52 where $Q_2$ is $Q_2$-85;
138. Compounds of Preferred 52 where $Q_2$ is $Q_2$-86;
139. Compounds of Preferred 52 where $Q_2$ is $Q_2$-87;
140. Compounds of Preferred 42 where
   R is H;
   J is J-1;
   $R_1$ is H;
   A is A-1;
   X is $CH_3$, $OCH_3$, $OCH_2CH_3$, Cl or $OCF_2H$;
   Y is $CH_3$, $OCH_3$, $C_2H_5$, $CH_2OCH_3$, $NHCH_3$, $CH(OCH_3)_2$ or cyclopropyl;
   Z is CH or N; and
   $Q_2$ is $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24, $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$-60, $Q_2$-63, $Q_2$-71, $Q_2$-74, $Q_2$-76, $Q_2$-78 and $Q_2$-79;
141. Compounds of Preferred 43 where
   R is H;
   $R_1$ is H;
   A is A-1;
   X is $CH_3$, $OCH_3$, $OCH_2CH_3$, Cl or $OCF_2H$;
   Y is $CH_3$, $OCH_3$, $C_2H_5$, $CH_2OCH_3$, $NHCH_3$, $CH(OCH_3)_2$ or cyclopropyl;
   Z is CH or N; and
   $Q_2$ is $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24, $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$60, $Q_2$-63, $Q_2$-71, $Q_2$-74, $Q_2$-76, $Q_2$-78, and $Q_2$-79;
142. Compounds of Formula I wherein
   Q is $E_1X_aQ_2$;
   $E_1$ is a single bond;
   $X_a$ is $CH_2$, $CH(CH_3)$, $CH_2CH_2$ or $CH_2CH_2CH_2$;
   $Q_2$ is a saturated or partially saturated 5-or 6-membered carbocyclic ring, containing either one or two carbonyl groups, or a saturated or partially saturated 5- or 6-membered heterocyclic ring, containing 2-5 atoms of carbon and 1-3 heteroatoms selected from the group consisting of 0-2 oxygen, 0-2 sulfur or 0-3 nitrogen, wherein sulfur may take the form of S, SO or $SO_2$, and containing one or two carbonyl or sulfonyl ($SO_2$) groups, or one carbonyl and one sulfonyl group; Q may further be optionally substituted with 1-2 substituent groups; substituents on carbon may be selected from the group consisting of halogen, $C_1$-$C_4$ alkyl, $C_1$-$C_4$ haloalkyl, $CH_2$($C_2$-$C_3$ alkenyl), $CH_2$($C_2$-$C_3$ alkynyl), $C_2$-$C_4$ alkoxycarbonyl, CN, OH, $C_1$-$C_3$ alkoxy, $C_1$-$C_3$ alkylthio, $C_1$-$C_3$ alkylsulfinyl, $C_1$-$C_3$ alkylsulfonyl or $C_2$-$C_4$ alkylcarbonyl, substituents on nitrogen may be selected from the group consisting of $C_1$-$C_4$ alkyl, $C_1$-$C_4$ haloalkyl, $CH_2$($C_2$-$C_3$ alkenyl), $CH_2$($C_2$-$C_3$ alkynyl), $C_2$-$C_4$ alkoxycarbonyl or $C_2$-$C_4$ alkylcarbonyl;
   $R_1$ is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$

alkylsulfinyl, $C_1$-$C_3$ alkylsulfonyl, $CH_2CN$, CN, $CO_2R_c$, $C_1$-$C_3$ haloalkoxy or $C_1$-$C_3$ haloalkylthio;

A is 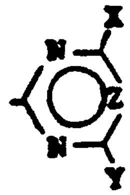 , 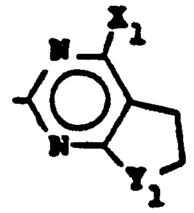 , 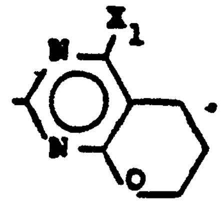 ,

A-1 A-2 A-3

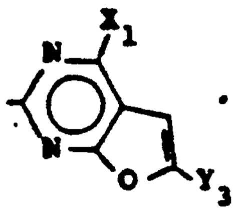 , 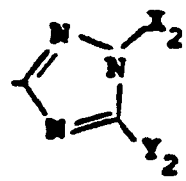 or 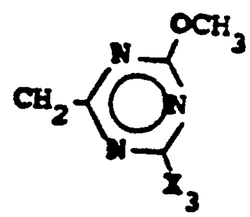 ;

A-4 A-5 A-6

Groups of compounds within the scope of formula (I) inolude those disclosed in our copending US Patent Applications Serial Nos. 739,215, 739,232, 842,295 and 842,791, copies of which are available for inspection on the file of the present Application.

Specifically preferred for reasons of greatest ease of synthesis and/or greatest herbicidal efficaoy are:

- o 2-(4,5-dihydro-4-methyl-5-oxo -1,3,4-oxadiazol-2-yl)-N-[(4,6-dimethoxypyrimidin-2-yl)aminocarbonyl]-benzenesulfonamide, m.p. 208-214 ° C;
- o 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide, m.p. 198-200 ° C;
- o N-[(4,6-dimethoxypyrimidin-2-yl)aminocarbonyl]-2-(2-oxo-1-pyrrolidinylmethyl)benzenesulfonamide, m.p. 185-187 ° C;
- o N-[(4,6-dimethoxy-1,3,5-triazin-2-yl)aminocarbonyl]-2-(2-oxo-1-pyrrolidinylmethyl)benzenesulfonamide, m.p. 194-195 ° C;
- o N-[(4,6-dimethoxypyrimidin-2-yl)aminocarbonyl]-2-(2-oxo-3-oxazolidinylmethyl)-3-thiophenesulfonamide, m.p. 157-160 ° C; and
- o N-[(4-methoxy-6-methyl-1,3,5-triazin-2-yl)aminocarbonyl]-2-(2-oxo-3-oxazolidinylmethyl)-3-thiophenesulfonamide, m.p. 155-161 ° C.

## Detailed Description of the Invention

### Synthesis

In the following discussion, reagents and reaction conditions are given by way of illustration.

Compounds of Formula I, wherein E is $CH_2$ or a single bond, can be synthesized by one or more of the procedures outlined in Equation 1.

Equation 1

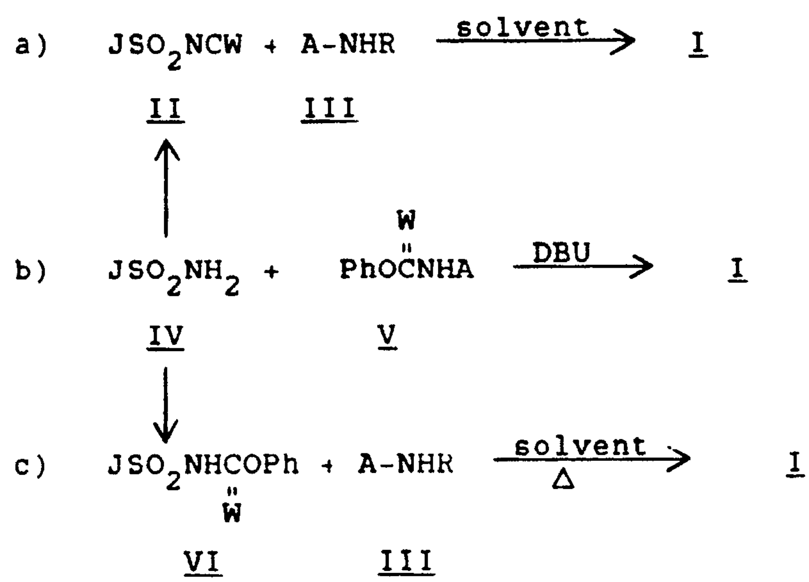


wherein

J, R, and A are as previously defined, provided

E is $CH_2$ or a single bond.

The reaction of Equation 1a can be carried out according to procedures described in U.S. 4,127,405.

The sulfonyl isocyanates II are prepared from the corresponding sulfonamides of Formula IV according to procedures described in United States Patent 4,238,621 or by the procedure of H. Ulrich, B. Tucker, and A. Sayigh, J. Org. Chem. , 34 3200 (1969).

Sulfonyl isothiocyanates (II, W is S) are known in the art and are prepared from the corresponding sulfonamides (IV) by reaction with carbon disulfide and potassium hydroxide followed by treatment of the resulting dipotassium salt VI with phosgene. Such a procedure is described in Arch. Pharm. 299 , 174 (1966).

Alternatively compounds of Formula I can be prepared according to Equation 1b or by the reaction of Equation 1c as described in United States Patent 4,443,243.

The sulfonylureas of Formula I, wherein E is O, can be prepared by one or both of the procedures described in Equation 2.

Equation 2

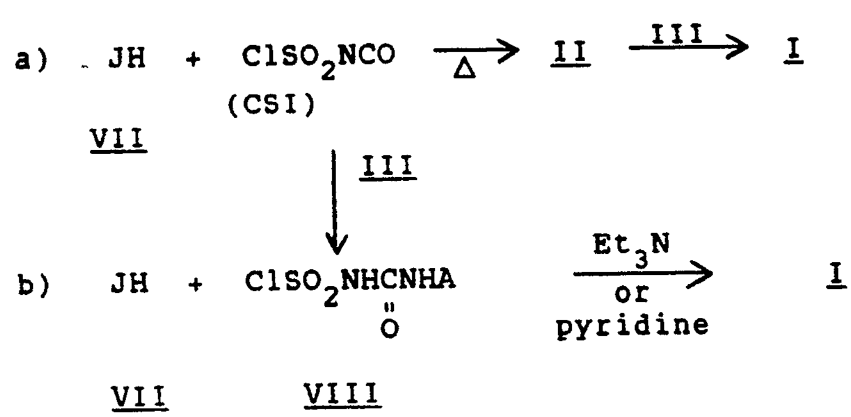


wherein

J is as previously defined, provided

E is O.

Phenols of Formula VII react with chlorsulfonylisocyanate (CSI) under elevated temperatures (Equation 2a) to provide the sulfonyl isocyanates II, which react with heterocyclic amines of Formula III to yield the sulfonylureas I according to the procedure in United States Patent 4,475,944. Alternatively, the reaction of Equation 2b may be employed according to the procedure described in United States Patent 4,391,976.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide or carbonate). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The synthesis of heterocyclic amines such as those represented by Formula III has been reviewed in "The Chemistry of Heterocyclic Compounds," a series published by Interscience Publ., New York and London. Aminopyrimidines are described by D. J. Brown in "The Pyrimidines," Vol. XVI of the series mentioned above which is herein incorporated by reference. The 2-amino-1,3,5-triazines of Formula III, where A is A-1 and Z is N, can be prepared according to methods described by E. M. Smolin and L. Rapaport in "s-Triazines and Derivatives," Vol. XIII.

Pyrimidines of Formula III, where A is A-1 and Y is an acetal or thioacetal substituent, can be prepared by methods taught in European Patent Application No. 84,224 (published July 27, 1983).

Pyrimidines of Formula III, where A is A-1 and Y is cyclopropyl or $OCF_2H$ can be synthesized according to the methods taught in United States Patent 4,515,626 and United States Patent 4,540,782, respectively.

Compounds of Formula III, where A is A-2 or A-3, can be prepared by procedures disclosed in United States Patent 4,339,267.

Compounds of Formula III, where A is A-4, can be prepared by methods taught in United States Patent 4,487,626.

Additional references dealing with the synthesis of bicyclic pyrimidines of Formula III, where A is A-2, A-3, or A-4 are Braker, Sheehan, Spitzmiller and Lott, J. Am. Chem. Soc. , 69 , 3072 (1947); Mitler and Bhattachanya, Quart. J. Indian Chem. Soc. , 4 , 152 (1927); Shrage and Hitchings, J. Org. Chem. , 16 , 1153 (1951); Caldwell, Kornfeld and Donnell, J. Am. Chem. Soc. , 63 , 2188 (1941); and Fissekis, Myles and Brown, J. Org. Chem. , 29 , 2670 (1964).

Compounds of Formula III, where A is A-5, can be prepared by methods taught in United States Patent 4,421,550.

Compounds of Formula III, where A is A-6, can be prepared by methods taught in the United States Patent 4,496,392.

The required sulfonamides of Formula IV, provided E is not oxygen, can be conveniently prepared by amination of the corresponding sulfonyl chlorides with ammonia or ammonium hydroxide by methods known to those skilled in the art. Alternatively deprotection of N-t-butylsulfonamides with polyphosphoric acid (PPA) or trifluoroacetic acid (TFA) as described by J. D Lombardino, J. Org. Chem. , 36 , 1843 (1971) or J. D. Catt and W. L. Matier, J. Org. Chem. , 39 , 566 (1974), respectively, provides compounds of Formula IV.

In addition, deprotection of N-t-butyldimethylsilylsulfonamides with fluoride ion, provides sulfonamides of Formula IV, wherein E is not oxygen.

The intermediate sulfonyl chlorides of Formula X, as depicted in Equation 3, can be prepared from aromatic amines via a diazotization process, as described in EPO Publication Nos. 83,975 and 85,476; or by oxidative chlorination of thiols or thioethers with chlorine and water as reviewed in Gilbert, "Sulfonation and Related Reactions," pp. 202-214, Interscience Publishers, New York, 1965; when $R_7$ = H or benzyl, the oxidative chlorination may be effected by sodium hypochlorite following procedures described by L. H. McKendry and N. R. Pearson in South African Patent Application No. 84/8845 (November 13, 1984); or by

metal halogen exchange or directed lithiation of appropriately substituted aryl or heterocyclic substrates followed by trapping with sulfuryl chloride. The lithiation can be performed according to the procedure of S. H. Bhattacharya, et al., J. Chem. Soc. (C) , 1265 (1968) or by procedures reviewed by H. Gschwend and H. Rodriquez in Organic Reactions , Vol. 26, Wiley, New York, 1979, and references cited within; or finally, when E is a $CH_2$ moiety, by a two step procedure involving the conversion of aromatic chloromethyl or bromomethyl compounds to isothiouronium salts, as described by Johnson and Spraque, J. Am. Chem. Soc. , 58 , 1348 (1936); 59 , 1837 and 2439 (1937); 61 , 176 (1939), followed by oxidative chlorination by the procedure of Johnson as described in J. Am. Chem. Soc. , 61 , 2548 (1939) to provide the sulfonyl chlorides X.

**Equation 3**

$$J\text{-}G \longrightarrow JSO_2Cl$$

IX　　　　　　X

wherein

J is as previously defined provided

E is not oxygen,

G is H, $NH_2$, $SR_7$, Br, $CH_2Cl$, $CH_2Br$, and

$R_7$ is H, $C_1$-$C_3$ alkyl, benzyl.

Amines of the Formula IX, wherein G is $NH_2$, can be prepared from the corresponding nitro compounds by various reduction procedures as described in United States Patents 3,846,440 and 3,846,439 and in EP-A No. 83,975 and references cited therein.

Phenols of Formula VII can be prepared from amines of Formula IX ($G = NH_2$) via a diazotization process, as described in A. I. Vogel, "Practical Organic Chemistry," P. 595 (1956), 3rd Ed; U.S. 3,270,029; J. H. Finley, et al., J. Het. Chem. , 6 , 841 (1969); and M. Ohta, et al., J. Pharm. Soc. Japan , 73 , 701 (1953).

Compounds of the Formula XII, which serve as intermediates to compounds of the Formula I, wherein Q is $E_1X_aQ_2$, as illustrated in Equations 1-3, can be prepared from precursors of the Formula XI by one or more of the procedures outlined below.

**Equation 4**

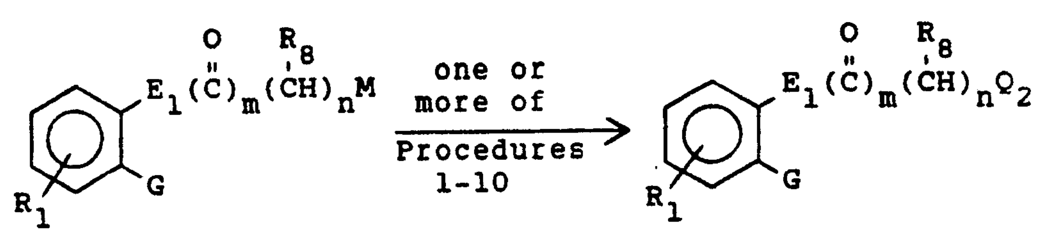


XI　　　　　　XII

wherein

$Q_2$ and $R_1$ are as originally defined;

$R_8$ is H or $CH_3$, provided that when n is 2 or 3,

$R_8$ is H;

m is 0 or 1, provided when m = 1, n must be 0;

n is 0, 1, 2, or 3, provided that m and n cannot both be 0;

G is Cl, Br, $CH_2Cl$, $CH_2Br$, OH, $NH_2$, $NO_2$, $SR_7$, $SO_2NH_2$, $SO_2NH$-t -butyl or $SO_2NHSi(CH_3)_2$-t -butyl;

M is Cl, Br, I, $NH_2$, NHOH, $NHNH_2$, $COOCH_3$, $CONHNH_2$, CN, COCl, CHO, or H or suitable leaving group, provided that when m = 1, M is not Br or I

$R_7$ is H, $C_1$-$C_3$ alkyl or benzyl. $E_1$ is O, S, SO, $SO_2$ or a single bond provided that when m = 1, $E_1$ is a single bond.

The ten procedures are based on established literature methods precedented by the references cited in Table 2. The references cited have direct applicability to compounds of the Formula XII, wherein J is J-1. However, the procedures and experimental methods described in these references are equally applicable to the synthesis of compounds of Formula XII, wherein J is J-2 through J-5, by analogous procedures or slight modifications thereof. The chemistry of the thiophene, pyridine and pyrazole ring systems has been reviewed in "Chemistry of Heterocyclic Compounds," Volumes 3, 14, and 5, respectively, Wiley, New York 1952 and later.

It should be noted that the chemical compatibility of the wide variety of reactions and reaction conditions described throughout this disclosure with J, $R_1$, $Q_2$, and G must be taken into account and as such requires a judicious choice of the appropriate methods for preparing compounds described within this disclosure. In addition, circumvention of instances of incompatibility may be achieved by the suitable selection of a protecting group, obvious to one skilled in the art. For a compilation of references describing the wide variety of protecting groups available, see T. W. Greene, "Protective Groups in Organic Synthesis," John Wiley and Sons, Inc., New York, 1981.

The synthesis of the starting materials of Formula XI are known in the general literature or can be prepared, by those skilled in the art, by simple modifications of established routes.

Procedure 1 : Direct N-alkylation of intact heterocyclic compounds, containing an N-H moiety, with compound substrates of the Formula XI, wherein m = 0 and M is Cl, Br or Iodine; or N-benzoylation of heterocyclic, containing an N-H moiety, with benzoyl chlorides of the form XI, wherein m = 1, M is Cl, and G is Cl, Br, $CH_2Cl$, $CH_2Br$, $NO_2$ or $SR_7$.

Procedure 2 : C-alkylation of heterocyclic compounds containing an acidic C-H moiety, i.e., activated by a carbonyl or sulfonyl group, by substrates of the Formula XI wherein M is Cl, Br, or iodine; or C-benzoylation with benzoyl chlorides of the Formula XI, wherein m = 1, M is Cl, and G is Cl, Br, $CH_2Cl$, $CH_2Br$, $NO_2$ or $SR_7$.

Procedure 3 : Reactions of derivatives of Formula XI, wherein M is $NH_2$, NHOH, or $NHNH_2$, as nucleophiles with bifunctional acyclic and cyclic reagents, which ultimately are converted to various Q values.

Procedure 4 : Use of acid-derivatives of Formula XI, wherein M is $COOCH_3$, $COHNNH_2$, CN, or COCl; see Example 4.

Procedure 5 : Synthesis from dianions derived from N-protected-( o -methyl aromatic sulfonamides) of Formula XI, wherein M is H, n=1, $R_8$=H, and G is $SO_2NH$-t -butyl or $SO_2NHSi(CH_3)_2$-t -butyl. These benzyl or benzyl-like dianions can be prepared by reaction of the appropriate sulfonamide, as defined above, with two equivalents of n -butyllithium at low temperatures in an inert solvent. In some instances, conversion of the lithium dianions to copper-lithium species is dictated by the literature and can be accomplished by known procedures.

Procedure 6 : Reactions of acyclic anions with aldehydes of the Formula XI, wherein M is CHO (m = 0). Further transformations, as described in the cited literature (Table 2) provide compounds of the Formula XII.

Procedure 7 : Reactions of anions derived from aromatic heterocycles such as thiophene, furan, pyrrole, and pyridine (or simple substituted analogues) which act as "masked" heterocycles of the form $Q_2$. For example, alkylation reactions of such anions with compounds of the Formula XI, wherein M is Cl, Br, or iodine, provide intermediates which upon reduction (see for example conversions B and K in Table 1) yield compounds of the Formula XII.

Procedure 8 : Synthesis of compounds of the Formula XII wherein M is a vinyl group (m = 0): see reference 37.

Procedure 9 : This procedure involves the use of compounds which are related to XI, but are outside the defined limits of XI. These compounds may be prepared by literature procedures from compounds of the Formula XI as defined. Additional functional group manipulation is then required, the procedures of which are described in the references cited in Table 2, to convert these compounds to compounds of the Formula XII.

Procedure 10 : This procedure is the conversion of the $Q_2$ value, contained in the Formula XII to a different $Q_2$ value. These conversion procedures are summarized in Table 1. When applicable, the conversion procedures of Table 1 are cited by their letter designations in Table 2.

Table 1

CONVERSION PROCEDURES

| Designation | Conversion From | Conversion To | References |
|---|---|---|---|
| A | lactone | lactam | 1-3 |
| B | thiophene furan pyrrole | tetrahydro derivative | 4-6 |
| C | sulfide | sulfoxide or sulfone | 7 |
| D | lactone, lactam, sulfone or sultam | α-unsaturated derivative | 8, 84 |
| E | lactam | cyclic amine | 9-11 |
| F | cycloketone | lactone | 12-14 |
| G | dihydro-γ-pyrone | tetrahydro-γ-pyrone | 15-19 |
| H | dihydro-γ-pyridone | tetrahydro-γ-pyridone | 20-22 |
| I | γ-pyrone | γ-pyridone | 23-25 |
| J | lactone | tetrahydrofuran or tetrahydropyran | 26 |
| K | pyridine (including quaternary salts) | piperidine or N-substituted piperidines | 27-28 |
| L | anhydride | succinimide | |

Table 2 sumarizes selected synthetic procedures viable for the synthesis of compounds of Formula XII. Table 2 is not meant to be all inclusive, but does provide synthetic routes, which are well established and precedented in the literature, through the known chemical procedures (1-10), conversions (A-L), and methods described in the references (1-84) or slight modifications thereof.

For example, the preparation of a compound of Formula XII, where $Q_2$ is Q-1 and $X_b$ is $NR_5$ can be carried out according to the procedure outlined in line number 2 of Table 2. Thus alkylation (procedure 2) of butyrolactone (QH = Q-1 where $X_b$ = 0) with an appropriately substituted alkyl halide as described in references 29-31, followed by conversion of the lactone moiety to a lactam (conversion procedure A) provides the desired compound of Formula XII. Finally, compounds of Formula XII are converted to the desired sulfonylureas of Formula I via one or more of the procedures outlined in Equation 1 through 3.

Table 2

Preparative Schemes for Compounds of the Formula XII

| No. | $Q_2$ | $X_b$ | $X_c$ | Proc.[1] | $QH(X_{b(c)})$[2] | Conv. Proc.[3] | Ref. | Comments |
|---|---|---|---|---|---|---|---|---|
| 1 | Q-1 | O | - | 2 | 1(O) | - | 29-31 | |
| 2 | Q-1 | $NR_5$ | - | 2 | 1(O) | A | 29-31 | |
| 3 | Q-1 | $NR_5$ | - | 2 | $1(NR_5)$ | | 32-35 | |
| 4 | Q-2 | O | - | 5 | | | 36 | |
| 5 | Q-2 | $NR_5$ | - | 5 | | A | 36 | |
| 6 | Q-3 | O | | 8 | | | 37 | |
| 7 | Q-3 | $NR_5$ | | 8 | | A | 37 | |
| 8 | Q-4 | - | - | 1 | 4 | - | | See Example 1 |
| 9 | Q-5 | - | - | 2 | 5 | | 38,39 | |
| 10 | Q-6 | | | 5 | 18 | | 40-42 | 1,4-Addition |
| 11 | Q-7 | - | - | 2 | | | 43-45 38 | |
| 12 | Q-8 | | | 9 | | | | Cyclize a halo-sulfonamide with base |
| 13 | Q-9 | | | 9 | - | | | Cyclize a halo-sulfonamide with base |
| 14 | Q-10 | - | - | 1 | - | | 46 | Analogous to Example 1 |
| 15 | Q-11 | | $NR_5$ | 2 | | | 49 | |
| 16 | Q-11 | | S | 2 | | | 38,50 | |
| 17 | Q-11 | | SO | 2 | | C | 38,50 | |
| 18 | Q-11 | | $SO_2$ | 2 | | C | 38,50 | |
| 19 | Q-11 | - | O | 6 | | | 51,52 | |
| 20 | Q-12 | - | $NR_5$ | 2 | | | 49 | |
| 21 | Q-12 | - | S | 9 | | | 53,54 | See also ref 50 |
| 22 | Q-12 | - | SO | 9 | | C | 53,54 | See also ref 50 |
| 23 | Q-12 | - | $SO_2$ | 9 | | C | 53,54 | See also ref 50 |
| 24 | Q-12 | - | O | 9 | | | 55 | |

Table 2 (Continued)

| No. | $Q_2$ | $X_b$ | $X_c$ | Proc.[1] | QH($X_{b(c)}$)[2] | Conv. Proc.[3] | Ref. | Comments |
|---|---|---|---|---|---|---|---|---|
| 25 | Q-13 | | O | 9 | | | 55 | |
| 26 | Q-13 | | S | 9 | | | 50, 52-54 | |
| 27 | Q-13 | | SO | 9 | | C | 50, 52-54 | |
| 28 | Q-13 | | $SO_2$ | 9 | | C | 50, 52-54 | |
| 29 | Q-13 | | $NR_5$ | 5 | | | | In addition to to appropriate pyrrolidinone See also ref. 56 |
| 30 | Q-14 | - | - | 1 | | | | Protection of co may be necessary |
| 31 | Q-15 | O | - | 5 | | D | 36 | |
| 32 | Q-15 | $NR_5$ | - | 5 | | D,A | 36 | Conversion D then A |
| 33 | Q-16 | O | | 8 | | D | 37 | |
| 34 | Q-16 | | $NR_5$ | | 8 | D,A | 37 | Conversion D then A |
| 35 | Q-17 | - | - | 1 | 4 | D | | See example 1 |
| 36 | Q-17 | - | - | 1 | 17 | | | See example 1 |
| 37 | Q-18 | - | - | 5 | 20 | D | 58-60 | 1,4 Addition to QH-18 followed by in situ conversion D |
| 38 | Q-19 | - | - | 1 | | | | Use α, β unsaturated propane sultam |
| 39 | Q-20 | O | | 2 | | | 29-31 | |
| 40 | Q-20 | $NR_5$ | | 2 | | | 32-35 | See ref 35, R=H |
| 41 | Q-21 | O | | 9 | | | 57 | Ref 57 discusses lactonization of hydroxy-acids |

Table 2 (Continued)

| No. | $Q_2$ | $X_b$ | $X_c$ | Proc.[1] | $QH(X_{b(c)})$[2] | Conv. Proc.[3] | Ref. | Comments |
|---|---|---|---|---|---|---|---|---|
| 42 | Q-21 | $NR_5$ | | 9 | | D | 57 | |
| 43 | Q-22 | O | | 9 | | | 57 | |
| 44 | Q-22 | $NR_5$ | | 9 | | A | 57 | |
| 45 | Q-23 | O | | 2 | 5 | F | 38,39 | |
| 46 | Q-23 | - | - | 2 | 5 | F,A | | Procedure E then A |
| 47 | Q-24 | - | - | 1 | 24 | | | Modification of example 1 |
| 48 | Q-25 | - | - | 2 | 25 | | 37,39 | |
| 49 | Q-26 | | | 5 | | | 40-42 | 1,4 Addition |
| 50 | Q-26 | - | - | 9 | | | | Cyclization of halo-sulfonamides with base |
| 51 | Q-27 | - | - | 2 | 27 | | 43-45 | |
| 52 | Q-28 | - | - | 1 | | | | Modification of Example 1 |
| 53 | Q-29 | | $NR_5$ | 2 | $29(NR_5)$ | | 49 | |
| 54 | Q-29 | | S | 2,9 | | | 53,54, 58 | |
| 55 | Q-29 | | SO | 2,9 | | C | 38,53, 54 | |
| 56 | Q-29 | | $SO_2$ | 2,9 | | C | 38,53 54 | |
| 57 | Q-29 | | O | 2,9 | | | 38 | |
| 58 | Q-30 | - | O | 5 | 41(O) | | 40-42 | 1,4 Addition |
| 59 | Q-30 | - | $NR_5$ | 5 | $41(NR_5)$ | | 40-42 | 1,4 Addition |
| 60 | Q-30 | - | S | 5 | 41(S) | | 40-42 | 1,4 Addition |
| 61 | Q-30 | - | SO | 5 | 41(S) | C | 40-42 | 1,4 Addition |
| 62 | Q-30 | - | $SO_2$ | 5 | 41(S) | C | 40-42 | 1,4 Addition |
| 63 | Q-31 | - | S | 9 | - | | 53-54 | |
| 64 | Q-31 | - | SO | 9 | - | C | 53-54 | |

Table 2 (Continued)

| No. | $Q_2$ | $X_b$ | $X_c$ | Proc.[1] | $QH(X_{b(c)})$[2] | Conv. Proc.[3] | Ref. | Comments |
|---|---|---|---|---|---|---|---|---|
| 65 | Q-31 | - | $SO_2$ | 9 | - | C | 53-54 | |
| 66 | Q-31 | - | O | 9 | - | - | - | |
| 67 | Q-31 | - | $NR_5$ | 9 | - | - | - | |
| 68 | Q-32 | - | $NR_5$ | 2 | - | - | 49 | |
| 69 | Q-32 | - | O | 2 | | | 38 | Modification of ref. 49 |
| 70 | Q-32 | - | S | 2 | | | 38 | Modification of ref. 49 |
| 71 | Q-32 | - | SO | 2 | | | 38 | Modification of ref. 49 |
| 72 | Q-32 | - | $SO_2$ | 2 | | | 38 | Modification of ref. 49 |
| 73 | Q-33 | | | 1 | 33 | | | C=O protection may be necessary |
| 74 | Q-34 | | O | 2 | 34(O) | | 38 | |
| 75 | Q-34 | | S | 2 | 34(S) | | 38 | |
| 76 | Q-34 | | SO | 2 | 34(S) | C | 38 | |
| 77 | Q-34 | | $SO_2$ | 2 | 34(S) | C | 38 | |
| 78 | Q-34 | | $NR_5$ | 2 | 34($NR_5$) | | 38 | |
| 79 | Q-35 | | O | 4 | | G | 59 | |
| 80 | Q-35 | | $NR_5$ | 9 | | H | 61 | |
| 81 | Q-35 | | S | 9 | | | 62 | |
| 82 | Q-35 | | SO | 9 | | C | 62 | |
| 83 | Q-35 | | $SO_2$ | 9 | | C | 62 | |
| 84 | Q-36 | | | 1 | 36 | | | C=O protection may be necessary |
| 85 | Q-37 | O | | 2 | 5 | F,D | 38,39 | |
| 86 | Q-37 | $NR_5$ | | 2 | 5 | F,D,A | 38,39 | |

Table 2 (Continued)

| No. | $Q_2$ | $X_b$ | $X_c$ | Proc.[1] | $QH(X_{b(c)})$[2] | Conv.[3] Proc. | Ref. | Comments |
|---|---|---|---|---|---|---|---|---|
| 87 | Q-38 | - | - | 2 | 38 | | | Modification of Example 1 |
| 88 | Q-39 | - | - | 2 | 25 | D | 38,39 | |
| 89 | Q-39 | - | - | 2 | 39 | | 38 | |
| 90 | Q-40 | - | - | 1 | | | | Modification of Example 1 |
| 91 | Q-41 | | $NR_5$ | 2 | $30(NR_5)$ | D | 49 | |
| 92 | Q-41 | | O | | | D | 38 | |
| 93 | Q-41 | | S | | | D | 38 | |
| 94 | Q-41 | | SO | | | D,C | 38 | |
| 95 | Q-41 | | $SO_2$ | | | D,C | 38 | |
| 96 | Q-42 | | S | 9 | | D | 62 | |
| 97 | Q-42 | | $NR_5$ | 9 | | | 61 | |
| 98 | Q-42 | | SO | 9 | | D,C | 62 | |
| 99 | Q-42 | | $SO_2$ | 9 | | D,C | 6 | |
| 100 | Q-42 | - | O | 2 | | | 38 | |
| 101 | Q-43 | - | - | 1 | 35 | D | | |
| 102 | Q-44 | O | - | 9 | | | 63-65 | |
| 103 | Q-44 | $NR_5$ | - | 9 | | | 63-65 | |
| 104 | Q-45 | | | 3 | | | | Reaction of amine with γ-pyrone |
| 105 | Q-46 | | | 4 | | | 66 | |
| 106 | Q-47 | | | 4 | | | 67-69 | Ref 69 contains N-alkylation procedures |
| 107 | Q-48 | | | 4 | | | 70 | |
| 108 | Q-49 | | | 4 | | | 71 | |
| 109 | Q-50 | | | 4 | | | 72-73 | |
| 110 | Q-51 | | | 4 | | | 74 | |

Table 2 (Continued)

| No. | $Q_2$ | $X_b$ | $X_c$ | Proc.[1] | $QH(X_{b(c)})$[2] | Conv. Proc.[3] | Ref. | Comments |
|---|---|---|---|---|---|---|---|---|
| 111 | Q-52 | | | 4 | | | 75-77 | |
| 112 | Q-53 | | | 4 | | | 78 | |
| 113 | Q-54 | | | 1 | 54 | | 79,80 | |
| 114 | Q-55 | | | 1 | 55 | | 81 | |
| 115 | Q-56 | | | 1 | 56 | | | |
| 116 | Q-57 | | | 9 | | | 82 | |
| 117 | Q-58 | | | 8 | | | 83 | |
| 118 | Q-59 | | | 1 | 59 | | | |
| 119 | Q-60 | | | 1 | 60 | | | |
| 120 | Q-61 | | | 9 | | | | |
| 121 | Q-62 | | | 1 | 62 | | | |
| 122 | Q-63 | | | 3 | | | | M = NHOH |
| 123 | Q-64 | | | 2 | 64 | | 38 | $R_5$ = 16 |
| 124 | Q-65 | | | 1 | 65 | | | |
| 125 | Q-66 | | | 2 | 66 | | 38 | |
| 126 | Q-67 | | | 1 | 67 | | | |
| 127 | Q-68 | | | 2 | 68 | | 38 | |
| 128 | Q-69 | | | 1 | 69 | | | |
| 129 | Q-69 | | | 3 | | | | M = NHOH |
| 130 | Q-70 | | | 2 | 70 | | 38 | |
| 131 | Q-71 | | | 9 | | | | |
| 132 | Q-72 | | | 1 | 72 | | | |
| 133 | Q-73 | | | 1 | 73 | | | |
| 134 | Q-74 | | | 1 | 74 | | | |
| 135 | Q-75 | | | 6 | | L | | |
| 136 | Q-76 | | | 1 | 76 | | | |
| 137 | Q-77 | | | 6 | | L,D | | |

Table 2 (Continued)

| No. | $Q_2$ | $X_b$ | $X_c$ | Proc.[1] | $QH(X_{b(c)})$[2] | Conv. Proc.[3] | Ref. | Comments |
|---|---|---|---|---|---|---|---|---|
| 138 | Q-78 | | | 6 | | | | Anion from $CH_3CO_2CH_2CH_2$-$CO_2CH_3$ then ring close with acid |
| 139 | Q-79 | | | 1 | 79 | | | |
| 140 | Q-80 | | | 1 | 80 | | | |
| 141 | Q-81 | | | 2 | 81 | | 38 | |
| 142 | Q-82 | | | 1 | 82 | | | |
| 143 | Q-83 | | | 1 | 83 | | | |
| 144 | Q-84 | | | 1 | 84 | | | |
| 145 | Q-85 | | | 2 | 85 | | 38 | |
| 146 | Q-86 | | | 2 | 86 | | 38 | |
| 147 | Q-87 | | | 2 | 87 | | 38 | |

1) procedures of 1-10

2) for example when $QH(X_b) = 1(0)$; QH is Q-1, and $X_b$ is oxygen which suggests butyrolactone would be a viable starting material

3) conversion procedures (A-L) are described in Table I.

The preparation of the compounds of Formula I, wherein Q is $E_1X_aQ_2$, is further illustrated by the following specific examples.

Example 1

1-[(2-(Phenylmethylthio)phenylmethyl]-2-pyrrolidinone

To a solution of 1.35 g of potassium-tert-butoxide in 25 mL of dimethylformamide, cooled to 0 °C, was

added 0.92 mL of 2-pyrrolidinone. As a white precipitate formed, the mixture was stirred for 10 minutes, and 3.0 g of 2-phenylmethylthio chloromethyl benzene was added in one portion. The resulting solution was allowed to warm to room temperature, stirred for 1 hour, poured into water, and extracted with methylene chloride. The organic layer was washed well with water, dried over magnesium sulfate, filtered, and the filtrate evaporated to leave 3.0 g of a yellow oil.

| | | |
|---|---|---|
| NMR ($CDCl_3$) ppm: | 7.3 | (m, 9H, ArH) |
| | 4.5 | (s, 2H, $CH_2$) |
| | 4.0 | (s, 2H $CH_2$) |
| | 3.05 | (t, 2H, $-CH_2-$) |
| | 2.3 | (t, 2H, $-CH_2-$) |
| | 1.7-2.05 | (m, 2H, $CH_2$) |

Example 2

2-(2-Oxo-1-pyrrolidinylmethyl)benzenesulfonamide

To a solution of 2.6 g of the compound of Example 1 dissolved in 100 mL of acetic acid, containing 0.5 mL of water, and cooled to 15°C, was bubbled in chlorine gas for 15 minutes. A slight exotherm of 5°C was noted. The reaction was stirred for an additional 5 minutes, poured into ice water, and extracted with methylene chloride. The organic layer was washed with saturated sodium bicarbonate, dried over magnesium sulfate, filtered, and evaporated to an oil. The oil was immediately dissolved in 100 mL of tetrahydrofuran and treated with 2 mL of concentrated ammonium hydroxide and stirred for 1 hour. The tetrahydrofuran was removed on the rotary-evaporator to give a semisolid. The residue was then triturated with water to form a sticky solid which was filtered off and triturated with ether to provide 1.1g of an off-white solid; m.p. 149-151°C.

IR (Nujol) 1660(C=O) $cm^{-1}$

Example 3

N-[((4,6-dimethoxypyrimidin-2 yl)aminocarbonyl)-2-(2-oxo-1-pyrrolidinylmethyl]-benzenesulfonamide

To a suspension of 254 mg of the product of Example 2 in 10 mL of acetonitrile, containing 275 mg of phenyl (4,6-dimethoxypyrimidin-2-yl)carbamate, was added 0.15 mL of 1,8-diazabicyclo[5.4.0]undec-7-ene (DBU). The resultant solution was stirred for 2 hours, diluted with 20 mL of water and acidified with 5 drops of concentrated hydrochloric acid. n -Butylchloride (10 mL) was added, stirred, and the white precipitate was filtered. The collected white solid was washed with a little water, suction dried and finally dried in vacuo @ 70°C overnight to afford 200 mg of a white solid, m.p. 185-187°C

IR (Nujol) 1730 (C = O)
1668 (C = O) $cm^{-1}$;

| NMR ($CDCl_3$) ppm | | |
|---|---|---|
| | 12.76 | (bs.1H. NH) |
| | 8.21 | (m. 1H. ArH) |
| | 7.36-7.7 | (m. 3H. ArH) |
| | 7.22 | (bs. 1H. NH) |
| | 5.80 | (s. 1H. PyH) |
| | 5.01 | (s. 2H. $CH_2$) |
| | 3.96 | (s. 6H. $OCH_3$) |
| | 3.32 | (t. 2H. $CH_2$) |
| | 2.46 | (t. 2H. $CH_2$) |
| | 2.03 | (m. 2H. $CH_2$) |

3.32 (t. 2H. $CH_2$)
2.46 (t. 2H. $CH_2$)
2.03 (m. 2H. $CH_2$)

Using the techniques described in Equations 1-4 and the synthetic routes summarized in Table 2, which makes use of 10 literature based synthetic procedures and the conversion procedures in Table 1, the following compounds in Tables A, J and Q may be prepared by one skilled in the art.

USE OF TABLES A, J, AND Q

For purposes of expediency and avoidance of voluminous pages of tables each sulfonylurea is divided into three structural pieces as illustrated below.

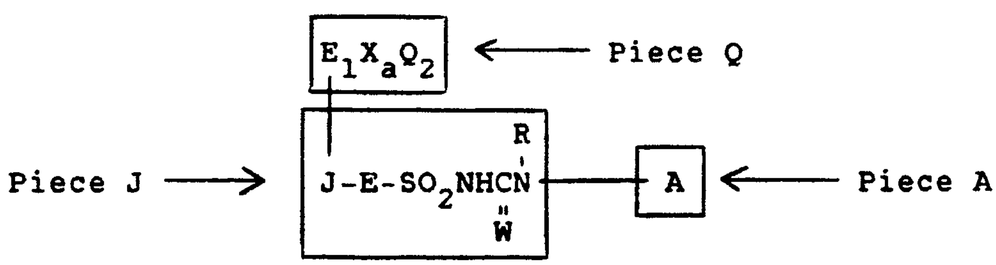


Each structural piece A, J, and Q is fully defined separately in Tables A, J and Q respectively and requires the designation of a particular structure from one of the structure(s) assigned to each of these tables. Thus to fully delineate a complete structure for a unique sulfonylurea it requires the information from one entry in each of the tables A, J, and Q and a designation of one of the structures assigned to that table. Note that only one structure, namely structure A, is assigned to Table A and need not be specifically designated.

The use of Tables A, J, and Q provides an alternative to listing individual compounds line by line as is

done in a conventional table. It is the applicant's intent to specifically disclose each and every compound that can be constructed from Tables A, J and Q using the procedure described above and illustrated below.

The use of Tables A, J, and Q can be illustrated by the following. For example, specifically claimed is compound of Formula XIII and is

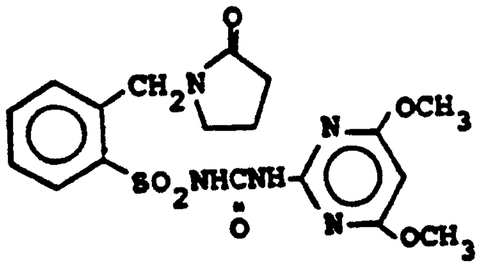

XIII

listed in Tables A, J, and Q as entry number 4 of Table A; entry number one of Table J - Structure B; and entry number seven of Table Q - Structure J. As a shorthand notation, compound of Formula XIII can be listed in a matrix form as:

4; 1-B; 7-J

This provides a convenient method for listing the melting points of compounds as is done in the melting point table (AJQ) (see line number one of Table AJQ). Each compound for which a melting point is available is simply listed in the melting point table (AJQ) as the entry number of Table A; entry number-structure for Table J; and entry number-structure for Table Q in that order.

Also specifically claimed is compound of formula XIV shown below.

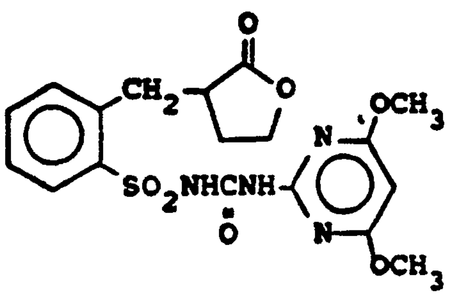

XIV

Compound of Formula XIV is found in Tables A, J, and Q as entry number four of Table A; entry number one of Table J-Structure B; and entry number one of Table Q-Structure J or in the convenient matrix form as:

4; 1-B; 1-J

By analogy compounds of Formula XV and XVI having structures as shown below

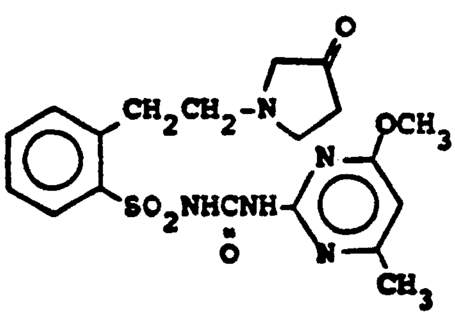

XV

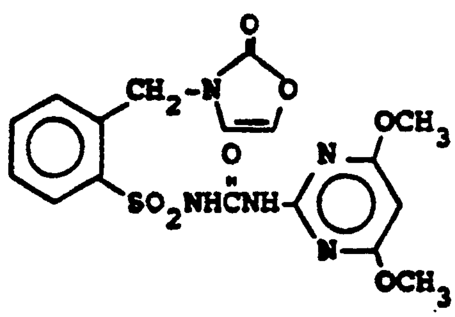

XVI

have matrix designations of 2; 1-B; 19-L and 4; 1-B; 76-J respectively.

The structures assigned to Tables A, J, and Q are shown below followed by Tables A, J, and Q and finally the melting point Table AJQ.

STRUCTURE FOR TABLE A

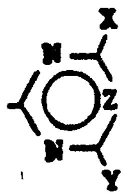

A

STRUCTURES FOR TABLE J

B 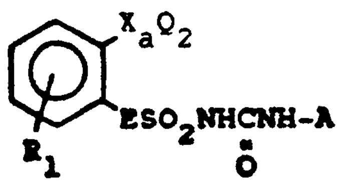

G 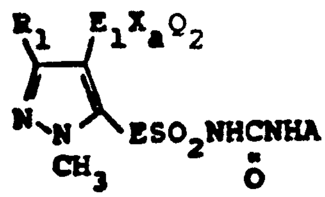

C 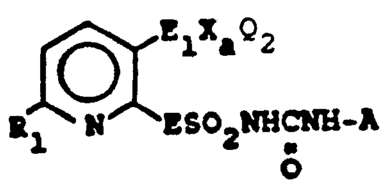

H 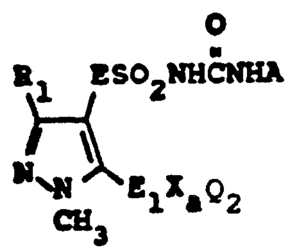

D 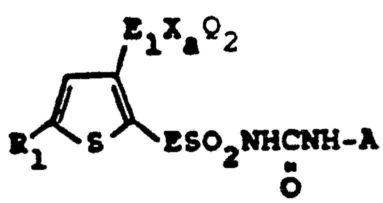

I 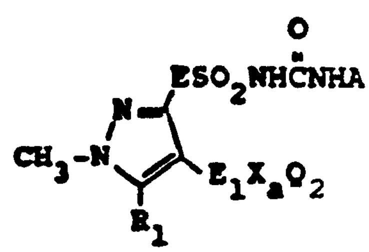

E 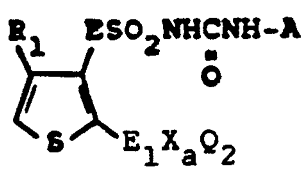

F 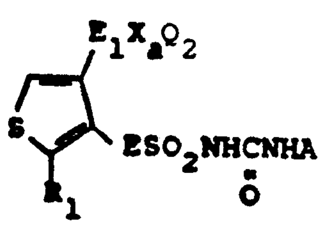

## STRUCTURES FOR TABLE Q

| | |
|---|---|
| **J** | $-CH_2Q_2$ |
| **K** | $CH_3$<br>$\vert$<br>$-CH-Q_2$ |
| **L** | $-CH_2CH_2-Q_2$ |
| **M** | O<br>$\Vert$<br>$-C-Q_2$ |
| **N** | $SCH_2Q_2$ |
| **O** | $SCH_2Q_2$<br>$\Vert$<br>O |
| **P** | $SO_2CH_2Q_2$ |
| **Q** | $OCH_2Q_2$ |

TABLE A FOR STRUCTURE A

| Entry | X | Y | Z |
|---|---|---|---|
| 1 | $CH_3$ | $CH_3$ | CH |
| 2 | $CH_3$ | $OCH_3$ | CH |
| 3 | $CH_3$ | $OCH_3$ | N |
| 4 | $OCH_3$ | $OCH_3$ | CH |
| 5 | $OCH_3$ | $OCH_3$ | N |
| 6 | Cl | $OCH_3$ | CH |
| 7 | $CH_3$ | $C_2H_5$ | CH |
| 8 | $CH_3$ | $CH_2OCH_3$ | CH |
| 9 | $CH_3$ | $CH_2OCH_3$ | N |
| 10 | $CH_3$ | $NHCH_3$ | CH |
| 11 | $CH_3$ | $NHCH_3$ | N |
| 12 | $CH_3$ | $CH(OCH_3)_2$ | CH |
| 13 | $CH_3$ | $CH(OCH_3)_2$ | N |
| 14 | $CH_3$ | Cyclopropyl | CH |
| 15 | $CH_3$ | Cyclopropyl | N |
| 16 | $OCH_3$ | $C_2H_5$ | CH |
| 17 | $OCH_3$ | $C_2H_5$ | N |
| 18 | $OCH_3$ | $CH_2OCH_3$ | CH |
| 19 | $OCH_3$ | $CH_2OCH_3$ | N |
| 20 | $OCH_3$ | $NHCH_3$ | CH |
| 21 | $OCH_3$ | $NHCH_3$ | N |
| 22 | $OCH_3$ | $CH(OCH_3)_2$ | CH |
| 23 | $OCH_3$ | $CH(OCH_3)_2$ | N |
| 24 | $OCH_3$ | Cyclopropyl | CH |
| 25 | $OCH_3$ | Cyclopropyl | N |
| 26 | $OCH_2CH_3$ | $CH_3$ | CH |
| 27 | $OCH_2CH_3$ | $CH_3$ | N |
| 28 | $OCH_2CH_3$ | $OCH_3$ | CH |
| 29 | $OCH_2CH_3$ | $OCH_3$ | N |
| 30 | $OCH_2CH_3$ | $CH_2OCH_3$ | CH |
| 31 | $OCH_2CH_3$ | $CH_2OCH_3$ | N |
| 32 | $OCH_2CH_3$ | $NHCH_3$ | CH |

Table A (Continued)

| Entry | X | Y | Z |
|---|---|---|---|
| 33 | $OCH_2CH_3$ | $NHCH_3$ | N |
| 34 | $OCH_2CH_3$ | $CH(OCH_3)_2$ | CH |
| 35 | $OCH_2CH_3$ | $CH(OCH_3)_2$ | N |
| 36 | Cl | $OCF_2H$ | CH |
| 37 | $OCF_2H$ | $CH_3$ | CH |
| 38 | $OCF_2H$ | $OCH_3$ | CH |
| 39 | $OCF_2H$ | $C_2H_5$ | CH |
| 40 | $OCF_2H$ | $CH_2OCH_3$ | CH |
| 41 | $OCF_2H$ | $NHCH_3$ | CH |
| 42 | $OCF_2H$ | $CH(OCH_3)_2$ | CH |
| 43 | $OCF_2H$ | Cyclopropyl | CH |

TABLE J FOR STRUCTURES B - I[a]

| Entry | E | $R_1$ No. refers to position in Structure B only |
|---|---|---|
| 1 | - (Single bond) | H |
| 2 | - | 5-$CH_3$ |
| 3 | - | 5-$CH_2Cl$ |
| 4 | - | 5-$OCH_3$ |
| 5 | - | 5-$SCH_3$ |
| 6 | - | 5-$SCH_2CH_3$ |
| 7 | - | 5-Cl |
| 8 | - | 6-F |
| 9 | - | 5-$NO_2$ |
| 10 | - | 6-$SO_2N(CH_3)_2$ |
| 11 | - | 5-$SOCH_3$ |
| 12 | - | 3-Cl |
| 13 | - | 6-$SO_2CH_3$ |
| 14 | - | 5-$CH_2CN$ |
| 15 | - | 5-CN |
| 16 | - | 6-CN |
| 17 | - | 5-$CO_2CH_3$ |
| 18 | - | 5-$CF_3$ |
| 19 | $CH_2$ | H |
| 20 | $CH_2$ | 5-CN |
| 21 | $CH_2$ | 5-$SCH_3$ |
| 22 | $CH_2$ | 5-$OCH_3$ |
| 23 | $CH_2$ | 5-Cl |
| 24 | O | H |
| 25 | O | 5-$SCH_3$ |

a) W=O, R=H

TABLE Q FOR STRUCTURES J-Q

| Entry | $Q_2$ | $X_b$ | $X_c$ | $R_5$ | $R_6$ | Proviso (See end of Table) |
|---|---|---|---|---|---|---|
| 1 | Q-1 | O | - | - | - | a |
| 2 | Q-1 | $NR_5$ | - | $CH_3$ | - | - |
| 3 | Q-2 | O | - | - | - | - |
| 4 | Q-2 | $NR_5$ | - | $CH_3$ | - | - |
| 5 | O-3 | O | - | - | - | - |
| 6 | Q-3 | $NR_5$ | - | $CH_3$ | - | - |
| 7 | Q-4 | - | - | - | - | - |
| 8 | Q-5 | - | - | - | - | - |
| 9 | Q-6 | - | - | - | - | - |
| 10 | Q-7 | - | - | $CH_3$ | - | - |
| 11 | Q-8 | - | - | $CH_3$ | - | - |
| 12 | Q-9 | - | - | $CH_3$ | - | - |
| 13 | Q-10 | - | - | - | - | - |
| 14 | Q-11 | - | O | - | - | - |
| 15 | Q-11 | - | $NR_5$ | $CH_2CH_3$ | - | - |
| 16 | Q-12 | - | $NR_5$ | $CH_2CH_3$ | - | - |
| 17 | Q-12 | - | O | - | - | - |
| 18 | Q-13 | - | O | - | - | - |
| 19 | Q-14 | - | - | - | - | - |
| 20 | Q-15 | O | - | - | - | - |
| 21 | Q-15 | $NR_5$ | - | $CH_3$ | - | - |
| 22 | Q-16 | O | - | - | - | b |
| 23 | Q-16 | $NR_5$ | - | $CH_3$ | - | b |
| 24 | Q-17 | - | - | - | - | - |
| 25 | Q-18 | - | - | - | - | - |
| 26 | Q-19 | - | - | - | - | - |
| 27 | Q-20 | O | - | - | - | - |
| 28 | Q-20 | $NR_5$ | - | $CH_3$ | - | - |
| 29 | Q-21 | O | - | - | - | - |
| 30 | Q-22 | O | - | - | - | - |
| 31 | Q-23 | O | - | - | - | - |

TABLE Q (Continued)

| Entry | $Q_2$ | $X_b$ | $X_c$ | $R_5$ | $R_6$ | Proviso (See end of Table) |
|---|---|---|---|---|---|---|
| 32 | Q-23 | $NR_5$ | - | $CH_3$ | - | - |
| 33 | Q-24 | - | - | - | - | - |
| 34 | Q-25 | - | - | - | - | - |
| 35 | Q-26 | - | - | $CH_3$ | - | - |
| 36 | Q-27 | - | - | $CH_3$ | - | - |
| 37 | Q-28 | - | - | - | - | - |
| 38 | Q-29 | - | $NR_5$ | $CH_3$ | - | - |
| 39 | Q-30 | - | O | - | - | - |
| 40 | Q-31 | - | O | - | - | - |
| 41 | Q-32 | - | $NR_5$ | $CH_3$ | - | - |
| 42 | Q-33 | - | - | - | - | - |
| 43 | Q-33 | - | O | - | - | - |
| 44 | Q-34 | - | $NR_5$ | $CH_3$ | - | - |
| 45 | Q-35 | - | O | - | - | - |
| 46 | Q-36 | - | - | - | - | - |
| 47 | Q-37 | O | - | - | - | b |
| 48 | Q-38 | $NR_5$ | - | $CH_3$ | - | - |
| 49 | Q-38 | - | - | - | - | - |
| 50 | Q-39 | - | - | - | - | - |
| 51 | Q-40 | - | - | - | - | - |
| 52 | Q-41 | - | $NR_5$ | $CH_3$ | - | b |
| 53 | Q-41 | - | O | - | - | b |
| 54 | Q-42 | - | O | - | - | b |
| 55 | Q-42 | - | $NR_5$ | - | - | b |
| 56 | Q-43 | - | - | - | - | - |
| 57 | Q-44 | - | O | - | - | b |
| 58 | Q-44 | - | $NR_5$ | $CH_3$ | - | b |
| 59 | Q-45 | - | - | - | - | b |
| 60 | Q-46 | - | - | $CH_3$ | - | b |
| 61 | Q-46 | - | - | H | - | b |
| 62 | Q-47 | - | - | $CH_3$ | - | b |

TABLE Q (Continued)

| Entry | $Q_2$ | $X_b$ | $X_c$ | $R_5$ | $R_6$ | Proviso (See end of Table) |
|---|---|---|---|---|---|---|
| 63 | Q-48 | - | - | $CH_3$ | - | b |
| 64 | Q-49 | - | - | $CH_3$ | - | b |
| 65 | Q-50 | - | - | $CH_3$ | $CH_3$ | b |
| 66 | Q-51 | - | - | - | - | b |
| 67 | Q-52 | - | - | - | - | b |
| 68 | Q-53 | - | - | - | $CH_3$ | b |
| 69 | Q-54 | - | - | - | - | - |
| 70 | Q-54 | - | - | - | - | a |
| 71 | Q-55 | - | - | - | - | a |
| 72 | Q-56 | - | - | - | - | - |
| 73 | Q-57 | - | - | $CH_3$ | - | - |
| 74 | Q-58 | - | - | H | - | - |
| 75 | Q-58 | - | - | $CH_3$ | - | - |
| 76 | Q-59 | - | - | - | - | - |
| 77 | Q-60 | - | - | $CH_3$ | - | - |
| 78 | Q-61 | - | - | $CH_3$ | $CH_3$ | - |
| 79 | Q-62 | - | - | $CH_3$ | - | - |
| 80 | Q-63 | - | - | - | - | - |
| 81 | Q-63 | - | - | - | - | c |
| 82 | Q-64 | - | - | $CH_3$ | - | - |
| 83 | Q-65 | - | - | - | - | - |
| 84 | Q-66 | - | - | $CH_3$ | - | - |
| 85 | Q-67 | - | - | - | - | - |
| 86 | Q-68 | - | - | $CH_3$ | - | - |
| 87 | Q-69 | - | - | - | - | - |
| 88 | Q-70 | - | - | $CH_3$ | - | - |
| 89 | Q-71 | - | - | - | - | - |
| 90 | Q-72 | - | - | $CH_3$ | - | - |
| 91 | Q-73 | - | - | $CH_3$ | - | - |
| 92 | Q-74 | - | - | - | - | - |
| 93 | Q-75 | - | - | $CH_3$ | - | - |

TABLE Q (Continued)

| Entry | $Q_2$ | $X_b$ | $X_c$ | $R_5$ | $R_6$ | Proviso (See end of Table) |
|---|---|---|---|---|---|---|
| 94 | Q-76 | - | - | - | - | - |
| 95 | Q-77 | - | - | $CH_3$ | - | b |
| 96 | Q-78 | - | - | - | - | - |
| 97 | Q-79 | - | - | - | - | - |
| 98 | Q-80 | - | - | - | - | - |
| 99 | Q-81 | - | - | $CH_3$ | - | - |
| 100 | Q-82 | - | - | - | - | - |
| 101 | Q-83 | - | - | - | - | - |
| 102 | Q-84 | - | - | $CH_3$ | - | - |
| 103 | Q-85 | - | - | - | - | - |
| 104 | Q-86 | - | - | - | - | - |
| 105 | Q-87 | - | - | - | - | - |

a) Q-1 is substituted by a 4-methyl group

b) Structure B of Table J is excluded with the use of the Q value designated in this entry

c) Substituted by α,α-dimethyl

## MELTING POINT TABLE (AJQ)

| DESIGNATIONS: | TABLE A; | TABLE J: | TABLE Q |
|---|---|---|---|
| | ENTRY #: | ENTRY # - STRUCTURE | ENTRY # - STRUCTURE |

| No. | Matrix Designation | MP°C |
|---|---|---|
| 1 | 4; 1-B; 7-J | 185-87 |
| 2 | 5; 1-B; 7-J | 194-95 |
| 3 | 1; 1-B; 72-J | 184-85 |
| 4 | 4; 1-B; 72-J | 139-42 |
| 5 | 5; 1-B; 72-J | 184-85 |
| 6 | 1; 1-B; 70-J | 192-94 |
| 7 | 2; 1-B; 70-J | 177-78 |
| 8 | 4; 1-B; 70-J | 183-85 |
| 9 | 6; 1-B; 70-J | 202-04 |
| 10 | 3; 1-B; 70-J | 185-86 |
| 11 | 5; 1-B; 70-J | 195-96 |
| 12 | 2; 1-B; 1-J | 202-04.5 |
| 13 | 6; 1-B; 1-J | 178-82.5 |
| 14 | 1; 1-B; 1-J | 193-96 |
| 15 | 5; 1-B; 1-J | 182-85 |
| 16 | 3; 1-B; 1-J | 188-94 |
| 17 | 4; 1-B; 1-J | 178-82 |
| 18 | 1; 1-E; 7-J | 174-76 |
| 19 | 2; 1-E; 7-J | 163-65 |
| 20 | 4; 1-E; 7-J | 137-39 |
| 21 | 6; 1-E; 7-J | 182-83 |
| 22 | 3; 1-E; 7-J | 171-72 |
| 23 | 5; 1-E; 7-J | 169-71 |
| 24 | 1; 1-E; 72-J | 159-61 |
| 25 | 2; 1-E; 72-J | 146-55 |
| 26 | 4; 1-E; 72-J | 157-60 |
| 27 | 6; 1-E; 72-J | 146-50 |

MELTING POINT TABLE (AJQ)

| DESIGNATIONS: | TABLE A: | TABLE J: | TABLE Q |
|---|---|---|---|
| | ENTRY #: | ENTRY # - STRUCTURE | ENTRY # - STRUCTURE |

| No. | Matrix Designation | MP°C |
|---|---|---|
| 28 | 3; 1-E; 72-J | 155-61 |
| 29 | 1; 1-E; 70-J | 190-91 |
| 30 | 2; 1-E; 70-J | 169-72 |
| 31 | 4; 1-E; 70-J | 129-31 |
| 32 | 6; 1-E; 70-J | 168-75 |
| 33 | 3; 1-E; 70-J | 154-59 |
| 34 | 5; 1-E; 70-J | 170-73 |
| 35 | 4; 1-E; 33-J | 150-60 |
| 36 | 5; 1-E; 33-J | 170-72 |

Intermediate compounds used for preparing compounds of Formula I, wherein Q is $Q_1$, may be prepared as described hereinafter.

Sulfonamides of Formula IV, wherein J is J-1 to J-5, $Q_1$ is Q-20 and E is $CH_2$ or a single bond, may be prepared by reacting a corresponding sulfonamide, wherein $Q_1$ is $NH_2$, with β-chloroethyl chloroformate followed by cyclization of the formed β-chloroethyl carbamate with base such as sodium methoxide. For details, refer to analogous reactions known in the art, for instance, Bull. Chem. Soc. Japan , 35 , 1309 (1962). Similarly, reacting an appropriate sulfonamide ($Q_1$ is $NH_2$) with 3-chloropropyl chloroformate may provide sulfonamides IV, wherein $Q_1$ is Q-28. Also, sulfonamides IV, wherein J is J-1 to J-5, $Q_1$ is Q-24 and E is $CH_2$ or a single bond, may be prepared by reaction of a corresponding sulfonamide, wherein $Q_1$ is $NHNH_2$, with a propiolate ester by obvious methods.

As illustrated in Equation 5, 1,3,4-oxadiazolin-5-ones of Formula XIV are prepared by reaction of hydrazides XIII with trichloromethyl chloroformate to form XIV ($R_5$ = H). Subsequent N-alkylation then provides XIV.

Equation 5

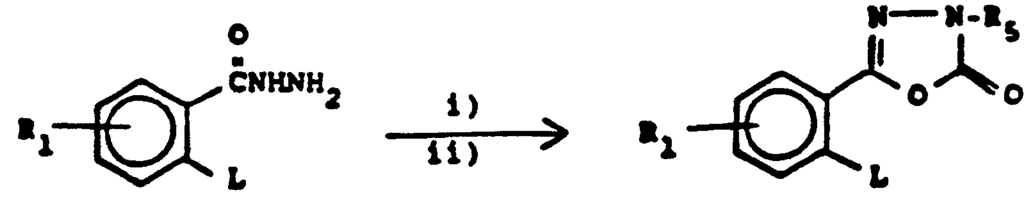


XIII XIV

i) $ClCO_2CCl_3$;
ii) $R_5M$, base; or $R_9SO_2OR_5$, base,
wherein

L is $CH_3$, $SR_8$, $NO_2$, OH, $SO_2NH_2$ or $CH_2SO_2NH_2$;
M is Cl, Br, or I;
$R_1$ and $R_5$ are as originally defined;
$R_9$ is $CH_3$ or p-tolyl; and
$R_8$ is $C_2$-$C_5$ alkyl or benzyl.

The reaction of XIII , wherein L is other than $SO_2NH_2$ or $CH_2SO_2NH_2$, with trichloromethyl chloroformate is best carried out in refluxing dioxane, according to a procedure of N. Chau et al., J. Het. Chem. , 19 , 541 (1982). The preparation of XIV , wherein L is $SO_2NH_2$ or $CH_2SO_2NH_2$ ($R_5$ = H), is preferably carried out by reacting the corresponding XIII with at least one equivalent of trichloromethyl chloroformate or phosgene followed by two equivalents of a suitable base, such as triethylamine, in an inert solvent, such as dioxane, at about 10° to 100° C. Alternatively, XIII may be cyclized to XIV ($R_5$ = H) by reaction with 1,1-carbonyldiimidazole, according to a procedure of E. Tihanyi et al., Heterocycles , 20 , 571 (1983); or, where L is $CH_3$, $NO_2$ or OH, by reaction with excess phosgene in a solvent such as refluxing chloroform or dioxane; for details, see for example, U.S. 3,127,410 ; Chem. Ber. , 98 , 540 (1965); J. Am. Pharm. Assoc. , 47 , 799 (1958); and J. Het. Chem. , 1 , 186 (1964).

The N-alkylation reaction of Equation 5 is best carried out by reacting XIV ($R_5$ = H) with a suitable base followed by an alkylating agent in a solvent such as methanol, tetrahydrofuran or dimethylformamide (DMF) at a temperature between about 0° and 100° C. Suitable bases include sodium hydride, sodium methoxide, potassium carbonate, or preferably, potassium t -butoxide in DMF. The products are isolated by dilution with water and filtration, or extraction with methylene chloride. For analogous reactions see, for example, Heterocycles , 20 , 571 (1983) and Canadian J. Chem. , 43 , 1607 (1965).

As shown in Equation 6, nitrocompounds of Formula XVII may be prepared by reaction of nitrocompounds of Formula XV with the sodium or potassium salts of oxadiazolin-5-ones or thiadiazolin-5-ones of Formula XVI .

**Equation 6**

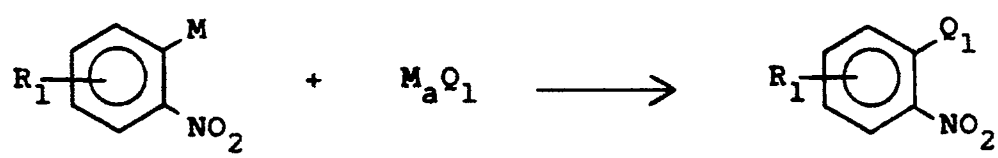

XV XVI XVII

wherein
$M_a$ is $Na^+$ or $K^+$;
$R_1$ and M are as previously defined; and
$Q_1$ is Q-15, Q-16 or Q-17.

The reaction of Equation 6 is best carried out using the analogous N-alkylation procedures described above in Equation 5. For more details, refer to analogous reactions described in J. Het. Chem. , 15 , 1221 (1978); Ibid. , 19 , 823 (1982); U.S. 3,846,439 ; and Heterocycles , 13 , 197 (1979). Compounds XVI ($Q_1$ is Q-15, $M_a$ is H) can be prepared by known methods, such as reaction of hydrazides with phosgene or trichloromethyl chloroformate; see, for examples, Chem. Ber. , 98 , 540 (1965), and J. Het. Chem. , 19 , 541 (1982); or by reaction of trimethyl acetylurea with potassium hypobromite to form XVI , wherein $Q_1$ is Q-15, $R_7$ is t -butyl and $M_a$ is H; see U.S. 3,846,439 . Compounds XVI , wherein $Q_1$ is Q-16 or Q-17 and $M_a$ is H, are known; see respectively, U.S. 4,448,968 and U.S. 3,767,646 .

As shown in Equation 7 below, sulfonamides of Formula IV may be prepared by a sequence of reactions in which hydrazide XVIII is reacted with an alkylisocyanate to form a semicarbazide, which is cyclized to form a 1,3,4-triazolin-5-one of Formula IVa ($R_5$ = H), which is N-alkylated to provide IVa .

Equation 7

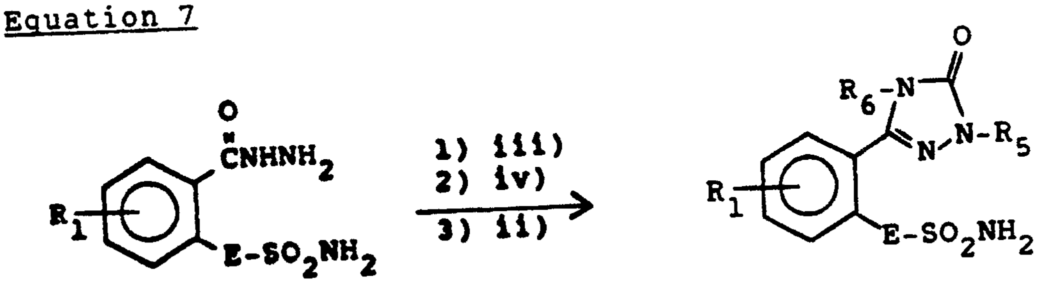


XVIII IVa

iii) is $R_6NCO$;
iv) is NaOH, $NaOCH_3$, or "DBU".
wherein
$R_1$, $R_5$, $R_6$ and ii) are as previously defined; and
E is $CH_2$ or a single bond.

The cyclization reaction of Equation 7 is best carried out according to analogous procedures described in Chem. Pharm. Bull., 24, 1336 (1976) and Ibid., 21, 1342 (1973). Thus, reaction of hydrazide XVIII with an appropriate alkyl isocyanate, in a solvent such as dioxane, by general procedures, provides the corresponding semicarbazide. Subsequent reaction of the semicarbazide with a base, such as aqueous sodium hydroxide in a solvent such as ethanol, or with "DBU" in a solvent such as dioxane, or sodium methoxide in a solvent such as methanol, at about 0° to 80° C can provide IVa ($R_5$ = H). Subsequent N-Alkylation by methods described in Equation 7 provides IVa.

In a similar fashion to reactions described in Equations 5 and 7, sulfonamido -thiophenes, -pyrazoles and -pyridines of Formula IV, wherein J is J-2 to J-5, E is a single bond or $CH_2$ and $Q_1$ is Q-1 or Q-7, may be prepared from appropriate corresponding hydrazido sulfonamides, by those skilled in the art.

Also, t -butylsulfonamido -thiophenes, -pyrazoles and -pyridines of Formula IV, [$SO_2NH_2$ is $SO_2NHC(CH_3)_3$], wherein J is J-2 to J-5, E is a single bond and $Q_1$ is Q-15 or Q-16, may be prepared by reaction of appropriate corresponding t -butylsulfonamides, which contain a o -halogen atom susceptible to nucleophilic displacement reactions, with the sodium or potassium salts of XVI, by those skilled in the art. Similarly, corresponding nitro -thiophenes, -pyrazoles and -pyridines, wherein J is J-2 to J-5, E is a single bond and $Q_1$ is Q-15, Q-16 or Q-17 may be prepared from corresponding nitro compounds containing an appropriate o -halogen atom by those skilled in the art. Preferably, these reactions are run in the presence of a catalyst such as copper (II) oxide.

In addition, as illustrated in Equation 8 below, thioethers of Formula XIX may be prepared from appropriately substituted thioethers of Formula XVIII.

Equation 8

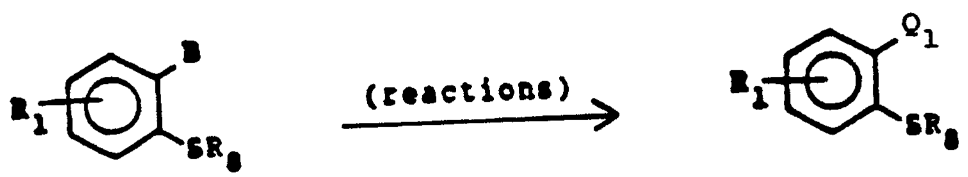


XVIII XIX

wherein
$R_1$ and $R_8$ are as defined in Equation 5;
B represents appropriate functional groups, described hereinafter, which may be reacted to prepare o -groups Q-2 to Q-14 or Q-18 to Q-30; and
$Q_1$ is Q-2 to Q-14 or Q-18 to Q-30.

The reaction of Equation 8 may be run according to analogous methods known in the art, or simple modifications thereof.

For instance, thioethers XIX, wherein $Q_1$ is Q-2 ($R_6$ = H) may be prepared by reaction of XVIII, wherein

B is an amidoxime group, with diketene followed by cyclization of the formed o -acetoacetylamidoxime with base such as sodium methoxide in refluxing toluene, according to Chem. Pharm. Bull. , 30 , 336 (1982); or by reaction of the amidoxime with ethyl chloroformate and a base such as pyridine in refluxing toluene or benzene for a short time, according to U.S. 3,767,646 . Subsequent N-alkylation of XIX (Q-2; $R_6$ is H) provides XIX ($Q_1$ is Q-2); see for example, U.S. 3,767,646 ; Ann. Chim. (Rome) , 53 , 1405 (1963); Tetrahedron , 21 , 1681 (1965); Chem. Ber. , 117 , 2999 (1984); Chem. Ber. , 103 , 2330 (1970); and Equation 5. Alternatively, XIX ($Q_1$ is Q-2) may be prepared by reaction of XVIII , wherein B is a N-alkyl amidoxime, with ethyl chloroformate, according to Chim. Acta. Turc. , 4 , 131 (1976).

Thioethers XIX , wherein $Q_1$ is Q-3, may be prepared by reaction of XVIII , wherein B is a nitrile group, with an N-alkyl-hydroxylamine followed by cyclization of the formed N-alkyl-N-hydroxy amidine with ethyl chloroformate and base such as pyridine, according to J. Chem. Soc. Perkins Trans , I , 85 (1974); by reaction of XVIII , wherein B is a carbonylisocyanate group, with a N-alkyl-hydroxylamine, according to J. Chem. Soc. , ( C ), 2794 (1969); or by reaction of a N-ethoxycarbonylthioamide with N-alkylhydroxylamine, according to J. Org. Chem. , 41 , 3233 (1976).

Thioethers XIX , wherein $Q_1$ is Q-4 ($R_6$ is H), may be prepared by reacting XVIII , wherein B is an acid chloride group, with potassium thiocyanate to form a carbonylthiocyanate, which with methanol gives the corresponding carbamate, which with methyl iodide gives an iminothiolcarbamate, which with hydroxylamine cyclizes to a 3-methoxy-1,2,4-oxadiazol ring, which with an appropriate demethylating agent such as pyridine•HCl is converted to XIX (Q-4, $R_6$ is H), which may be N-alkylated with diazomethane, or other appropriate alkylating agent, to form XIX ($Q_1$ is Q-4), according to Tetrahedron , 21 , 1681 (1965). Alternatively, XVIII , wherein B is 3-methoxy-1,2,4-oxadiazol ring, may be heated with sodium iodide and acetonylacetone to form XIX ($Q_1$ is Q-4, $R_6$ is $CH_3$), according to the above reference.

Thioethers XIX , wherein $Q_1$ is Q-5 ($R_6$ is H), may be prepared by reaction of XVIII , wherein B is a thiohydrazide group, with phosgene, according to Nippon Kagaku Kaishi , 315 (1976); by reacting XVIII , wherein B is a N-carbamoylthiohydrazide, with heat in the presence or absence of additives such as concentrated hydrochloric acid to cause cyclization to XIX (Q-5, $R_6$ is H); or by demethylation of XVIII , wherein B is a 2-methoxy-1,3,4-thiazole ring with hydrogen chloride in a solvent such as dioxane, according to U.S. 4,448,968 . Subsequent N-alkylation by general methods provides XIX ($Q_1$ is Q-5); see, for example, Helv. Chim. Acta , 65 , 2606 (1982) and Equation 5. Thioethers XVIII , wherein B is a 2-methoxy-1,3,4-thiazole ring, may be prepared from XVIII , wherein B is an imidate, ortho-ester or acid chloride, according to U.S. 4,448,968 .

Thioethers XIX , wherein $Q_1$ is Q-6, may be prepared by rearrangement of 3,4-disubstituted-1,2,4-oxadiazoline-5-thiones, according to Tetrahedron , 22 , 1945 (1977), and J. Chem. Soc. Perkin Trans , I , 687 (1983). The 5-thiones may be prepared by reaction of XVIII , wherein B is a N-alkylamidoxime, with thiophosgene, according to Chem. Ber. , 28 , 2227 (1895).

Thioethers XIX , wherein $Q_1$ is Q-7 or Q-8, may be prepared according to the procedures of Chem. Pharm. Bull. , 21 , 1342 (1973) and Chem. Pharm. Bull. , 24 , 1336 (1976). Accordingly, XVIII , wherein B is an alkylsemicarbazide group is reacted with a base such as sodium hydroxide, followed by N-alkylation by general methods to form XIX (Q-7). Reaction of XVIII , wherein B is a 1,2-dimethylsemicarbazide group, with alkaline solutions provides XIX (Q-8).

Thioethers XIX , wherein $Q_1$ is Q-9, are prepared by reaction of XVIII , wherein B is an amide group, with chlorocarbonylsulfenyl chloride in a solvent such as refluxing tetrahydrofuran, according to Phosphorous and Sulfur , 15 , 137 (1983), J. Org. Chem. , 43 , 3736 (1978) and Acta. Chem. Scan. , 21 , 1871 (1967).

Thioethers XIX , wherein $Q_1$ is Q-10, may be prepared by reaction of XVIII , wherein B is a hydroxyoxime group, with phosgene; see, for example, Chem. Ber. , 84 , 688 (1951); Tetrahedron Lett. , 319 , (1968); Ger. Offen. 2,059,990 (1972); and Rocz. Chem. , 45 , 833 (1971).

Thioethers XIX , wherein $Q_1$ is Q-11, may be prepared by reaction of XVIII , wherein B is a thiazolin-4,5-dione group, with sulfur, according to Chem. Ber. , 100 , 1627 (1967).

Thioethers XIX , wherein $Q_1$ is Q-12, may be prepared by reaction of XVIII , wherein B is a thiohydroxamic acid group, with phosgene under alkaline conditions according to conditions obvious to one skilled in the art and found in Belg. 632,072 and Ger. Offen. 2,059,990 .

Thioethers XIX , wherein $Q_1$ is Q-13, may be prepared by reaction of XVIII , wherein B is a thioamide group, with oxalyl chloride, according to Chem. Ber. , 93 , 671 (1960) and Chem. Ber. , 114 , 549 (1981).

Thioethers XIX , wherein $Q_1$ is Q-14, may be prepared by reaction of XVIII , wherein B is a N-alkylamidine group, with oxalyl chloride, according to Chem. Ber. , 100 , 2064 (1967) and Chemistry of Penicillin , 52 (1949).

Thioethers XIX , wherein $Q_1$ is Q-18 or Q-19, may be prepared by reaction of XVIII , wherein B is a α-oxopropanoate ester group, with an alkylhydrazine or hydroxylamine•HCl, respectively, according to methods known in the art; see for example, Tetrahedron , 20 , 299 (1964) and U.S. 4,044,013 for Q-18; and J. Gen. Chem. (U.S.S.R.) , 17 1816 (1947) ( Chem. Abst. 42 : 4170c) for Q-19. One skilled in the art will recognize that in compounds containing the substituents Q-18 or Q-19, these heterocycles may partially exist in their respective tautomeric forms.

Thioethers XIX , wherein $Q_1$ is Q-21 or Q-22, may be prepared by heating XVIII , wherein B is a $NHCOCH_2CH_2OH$ or $NHCOCH_2OH$ group, respectively, with paraformaldehyde and an acid catalyst in a solvent such as toluene, according to analogous methods described in Heterocycles , 7 , 919 (1977) and Japan Kokai Tokyo Koho 79, 24,869 ( Chem. Abst. 91:74630f).

Thioethers XIX , wherein $Q_1$ is Q-23, may be prepared by reaction of XVIII , wherein B is a hydroxylamine group, with an appropriate 3-halopropanoic acid chloride, followed by cyclization of the formed 3-halopropanamide with base. For details refer to analogous methods known in the art, for instance, U.S. 4,405,357.

Thioethers XIX , wherein $Q_1$ is Q-24, may be prepared by reaction of XVIII , wherein B is a hydrazine group, with a propiolate ester or an appropriate derivative.

Thioethers XIX , wherein $Q_1$ is Q-25, may be prepared by reaction of XVIII , wherein B is an amidine group, with chloroacetyl chloride followed by cyclization. Also, reaction of XVIII (B is an amidine group), with an appropriate $\alpha$-oxopropanoate ester may provide XIX ($Q_1$ is Q-27).

Thioethers XIX , wherein $Q_1$ is Q-20 or Q-28, may be prepared by reaction of XVIII wherein B is an amino group, with 2-chloroethyl or 3-chloropropyl chloro- formate followed by cyclization with an appropriate base.

Also, reaction of thioethers XVIII , wherein B is an isocyanate group, with an appropriate 3-chloropropyl or 2-chloroethylamine followed by cyclization of the formed urea may provide thioethers XIX , wherein $Q_1$ is 29 or 30.

In similar fashion, by substituting appropriate nitrobenzenes for thioethers XVIII in Equation 8 above, and carrying out the reactions described therein, those skilled in the art may prepare corresponding nitrobenzenes, wherein J is J-1, E is a single bond and $Q_1$ is as defined in Equation 8.

Also, by substituting appropriate phenols for thioethers XVIII in Equation 8, and carrying out the reactions described therein, or simple modifications thereof, those skilled in the art may prepare corresponding phenols, wherein J is J-1, E is O and $Q_1$ is as defined in Equation 8. In some cases a phenolic protecting group which could be later removed would be desirable. For a review on phenolic protecting groups see, T. W. Greene, Protective Groups in Organic Synthesis , pp. 87-113, John Wiley and Sons, Inc., New York, 1981.

Also, by analogy, by substituting appropriate thiophenes, pyrazoles or pyridines for thioethers XIII and XVIII in Equations 5 and 8 above, respectively, and carrying out the reactions described therein or simple modifications thereof, those skilled in the art may prepare corresponding thioethers, wherein J is J-2 to J-5, E is a single bond and $Q_1$ is as defined in Equations 5 and 8.

The preparation of the compounds of this invention is further illustrated by the following specific examples. Unless otherwise indicated, temperatures are in degrees centigrade.

## Example 4

### 5-{2-[(Phenylmethyl)thio]phenyl}-1,3,4-oxadiazol-2(3H)-one

To a solution containing 12.2 g of trichloromethyl chloroformate in 130 ml of p-dioxane was added portionwise 16 g of 2-[(phenylmethyl)thio]benzoic acid, hydrazide [prepared by the procedure of South African Patent Application 838,416 (1983)] to cause a slight exotherm to 37° C. After stirring at ambient temperature for 0.5 hr., the suspension was refluxed for 6 hours, then cooled to 25° C and poured onto excess ice/water to yield a precipitate. The mixture was filtered, washed with water and the isolated solid was recrystallized from acetonitrile to yield 14 g of the subject compound; m.p. 169-172° C.
IR (Nujol): 1790 $cm^{-1}$ (C=O).

## Example 5

3-Methyl-5-[2-[(phenylmethyl)thio]phenyl]-1,3,4-oxadiazol-2(3H)-one

To a suspension of 2.9 g of potassium t -butoxide in 60 ml of DMF under a $N_2$ atmosphere was added portionwise 7 g of the product of Example 4 while maintaining the reaction temperature at 25-30° C with an ice-water bath. After stirring at 25° C for 1 hour, 7 g of methyl iodide was added dropwise to cause an exotherm to 37° C. The suspension was heated at 40-60° C for 5 hours, then cooled to 25° and poured onto excess ice-water to yield a precipitate. The suspension was filtered, and the isolated solid was dissolved in tetrahydrofuran and dried over $MgSO_4$. The isolated solid was passed through a silica gel column with methylene chloride and, after evaporation of the first eluant, 5 g of the subject compound was obtained; m.p. 137-140° C.

IR (Nujol): 1780 $cm^{-1}$ (C=O)
NMR ($CDCl_3$):ppm 3.4 (s, 3H, $NCH_3$)
4.1 (s, 2H, $CH_2$)
7.2-7.9 (m, 9H, Ar)

Example 6

2-(4,5-Dihydro-4-methyl-5-oxo-1,3,4-oxadiazol-2-yl)benzenesulfonyl chloride

To a suspension of 4 g of the product of Example 5 and 0.72 g of $H_2O$ in 50 ml of propionic acid was added dropwise 3.4 ml of condensed $Cl_2$ while maintaining the reaction temperature at -5° to 0° C with external cooling. The suspension was stirred at 0° for 0.75 hr., then poured onto excess ice-water to yield a precipitate. After filtration the isolated residue was washed with excess $H_2O$ and then about 30 ml of hexane to yield 3.3 g of the subject compound as a crude solid; m.p. 122-125° C.

IR (Njuol): 1755 $cm^{-1}$ (C=O)

Example 7

2-(4,5-Dihydro-4-methyl-5-oxo-1,3,4-oxadiazol-2-yl)benzenesulfonamide

To a suspension of 3.3 g of the product of Example 6 in 50 ml of tetrahydrofuran was added dropwise 3 ml of condensed ammonia while keeping the reaction temperature at -5° C with external cooling. After stirring at 0° for 1.5 hours, the solvent was evaporated in vacuo and water was added to the residue to yield a precipitate. After filtration the isolated residue was washed with water, then dissolved in tetrahydrofuran and dried over $MgSO_4$. After evaporation of the solvent in vacuo , 2.9 g of the subject compound was obtained as a crude solid; m.p. 168-173° C.

IR (Nujol): 1770 $cm^{-1}$ (C=O)
3225 and 3310 $cm^{-1}$ ($SO_2NH_2$)
NMR ($CDCl_3$ + DMSO): ppm 3.5 (s,3H, $NCH_3$)
7.1 (s, 2H, $SO_2NH_2$)
7.7-8.4 (m, 4H, Ar)

Example 8

2-(4,5-Dihydro-4-methyl-5-oxo-1,3,4-oxadiazol-2-yl)-N-[(4,6-dimethoxypyrimidin-2-yl)amino carbonyl]-benzenesulfonamide

To a suspension of 0.4 g of the product of Eample 7 and 0.43 g of phenyl(4,6-dimethoxypyrimidin-2-yl)-carbamate in 10 ml of p-dioxane was added 0.24 g of 1,8-diazabicyclo[5.4.0]undec-7-ene (DBU). The suspension was stirred for 2 hours at room temperature, then diluted with 50 ml of ice-water, and the obtained solution was acidified with ~10 drops of concentrated hydrochloric acid to yield a precipitate. After filtration the residue was washed with excess water, then suction-dried overnight to yield 0.6 g of a white solid. The solid was further purified by slurry-washing with 10 ml of warm ethyl acetate to yield 0.4 g of the subject compound; m.p. 208-214 ° C.

IR (Nujol): 1710; 1770 $cm^{-1}$ (2 x C=O)

NMR ($CDCl_3$): ppm 3.4 (s, 3H, $NCH_3$)
4.0 (s, 6H, 2 x $OCH_3$ )
5.8 (s, 1H, py-H)
7.7-7.9 (m, 3H, Ar)
8.5 (bs, 1H, Ar)

Example 9

2-(4,5-Dihydro-4-methyl-5-oxo-1,3,4-oxadiazol-2-yl-N[(4-methyl-6-methoxy-1,3,5-triazin-2-yl)amino carbonyl]-benzenesulfonamide

By the procedure of Example 8, 0.4 g of the product of Example 7 was reacted with 0.41 g of phenyl-(4-methyl-6-methoxy-1,3,5-triazin-2-yl)carbamate and 0.24 g of "DBU" in 10 ml of p-dioxane. The isolated crude solid was purified by slurry-washing with 10 ml of warm acetonitrile to yield 0.3 g of the subject compound; m.p. 198-200 ° C.

IR (Nujol): 1700 and 1770 $cm^{-1}$ (2 x C=O)

NMR ($CDCl_3$): ppm 2.7 (s, 3H, $CH_3$)
3.5 (s, 3H, $NCH_3$)
4.1 (s, 3H, $OCH_3$)
7.8 (bm, 3H, Ar)
8.5 (bm, 1H, Ar)

Example 10

N-[(Butylamino)carbonyl]-2-(4,5-dihydro-4-methyl-5-oxo-1,3,4-oxadiazol-2-yl)benzenesulfonamide

A suspension of 15 g of the product prepared as in Example 7, 8.2 g of potassium carbonate and 7 g of n -butyl isocyanate in 150 ml of methyl ethyl ketone was refluxed for 6 hours. After concentrating the

suspension in vacuo , ice-water (~300 ml) was added to the residue and the cloudy solution was filtered. The filtrate was acidified with concentrated hydrochloric acid to form a viscous oil. After decanting the water, the residual oil was dissolved in tetrahydrofuran, dried over $MgSO_4$, and the solvent was evaporated in vacuo to yield an oil which slowly solidified to yield 18.5 g of the subject compound as a crude solid; m.p. 110-115 °C.

Example 11

2-(4,5-Dihydro-4-methyl-5-oxo-1,3,4-oxadiazol-2-yl) benzenesulfonyl isocyanate

A suspension of 8 g of the product of Example 10 and 0.4 g of DABCO in 90 ml of xylenes was heated under $N_2$ at 130-135 °C while 1.8 ml of phosgene was added portionwise at a rate to maintain a reflux temperature of about 130-135 °C. The mixture was refluxed an additional two hours, cooled under $N_2$ to room temperature, filtered, and the filtrate was concentrated in vacuo to yield 6.9 g of the subject compound as a crude oil.

IR (neat): 1785 (b) $cm^{-1}$ (C=O)
2240 $cm^{-1}$ (NCO)

Exmple 12

2-(4,5-Dihydro-4-methyl-5-oxo-1,3,4-oxadiazol-2-yl)-N-[(4-methoxypyrimidin-2-yl)aminocarbonyl] benzenesulfonamide

To a solution of 0.18 g of 2-amino-4-methoxypyrimidine in 5 ml of methylene chloride was added a solution of 0.56 g of the product of Example 11 dissolved in 5 ml of methylene chloride. The suspension was stirred at 25 °C for 3 hours, then diluted with 20 ml of 1-chlorobutane and stirred 0.5 hr. After filtration the residue was washed with ether to yield 0.55 g of the subject compound; m.p. 223-224 °C.

IR (Nujol): 1700 and 1780 $cm^{-1}$ (2 x C=O)
NMR ($CDCl_3$):ppm 3.5 (s, 3H, $NCH_3$)
3.9 (s, 3H, $OCH_3$)
6.45 (d, 1H, py H)
8.45 (d, 1H, py H)
7.7 (m, 3H, Ar)
8.5 (m, 1H, Ar)

Example 13

5-(2-Hydroxyphenyl)-1,3,4-oxadiazol-2(3H)-one

To a solution of 10 g of salicylhydrazide in 100 ml of p -dioxane was added dropwise 13.1 g of trichloromethyl chloroformate while maintaining the temperature at 20 °-30 °C with external cooling. After stirring at 25 °C for 0.5 hours, the suspension was refluxed for 4 hours then cooled to 25 °C and poured into

excess ice-water. The mixture was filtered and the residue was recrystallized from acetonitrile to yield 9.0 g of the subject compound; m.p. 198-200 ° C.

Example 14

5-(2-Hydroxyphenyl)-3-methyl-1,3,4-oxadiazol-2(3H)-one

To a suspension of 3.8 g of potassium t -butoxide in 60 ml of dry DMF under a $N_2$ atmosphere was added portionwise 6 g of the product of Example 13 while maintaining the reaction temperature at 10 ° - 20 ° C with external cooling. After stirring at 25 ° C for one hour, 9.6 g of methyl iodide was added dropwise at 10 ° -20 ° C with external cooling. After stirring at room temperature for 16 hours, the suspension was poured into excess ice-water and filtered. The residue was recrystallized from 2-propanol to yield 4.6 g of the subject compound; m.p. 160-162 ° C.

Example

NMR ($CDCl_3$) ppm: 3.5 (s, 3H, $NCH_3$)
6.9-7.7 (m, 4H, Ar)
8.6 (bs, 1H, OH)

15

[2-(4,5-Dihydro-4-methyl-5-oxo-1,3,4-oxadiazol-2-yl-phenoxy]sulfonyl isocyanate

To a suspension of 4 g of the product of Example 14 in 75 ml of dry toluene under a $N_2$atmosphere was added dropwise 3 g of chlorosulfonyl isocyanate at 25 ° -30 ° C. After stirring at 25 ° C for 0.5 hour the suspension was refluxed for one hour, then cooled under $N_2$ to 25 ° . The solvent was evaporated from the solution in vacuo to yield 6 g of the subject compound as a crude oil.

IR (Nujol) $cm^{-1}$ 1770 (C=O)
2245 (NCO)

Example 16

2-(4,5-Dihydro-4-methyl-5-oxo-1,3,4-oxadiazol-2-yl) phenyl [(4-chloro-6-methoxypyrimidin-2-yl) aminocarbonyl]sulfamate

To a suspension of 0.48 g of 2-amino-4-chloro-6-methoxypyrimidine in 5 ml of dry methylene chloride was added a solution of 1.5 g of the crude product of Example 15 in 10 ml of methylene chloride. After stirring at 25 ° C for 16 hours the solvent was evaporated in vacuo . After stirring the residue in about 20 ml of 1-chlorobutane for one hour, the suspension was filtered, and the isolated solid was triturated with about 30 ml of warm ethyl acetate to yield 0.5 g of the subject compound; m.p. 175-178 ° C.

IR (Nujol): 1725; 1780 $cm^{-1}$ (2 x C=O)

NMR ($DCCl_3$ = DMSO): ppm 3.4 (s, 3H, $OCH_3$)
3.7 (s, 3H, $NCH_3$)
6.5 (s, 1H, py H)
7.5-7.9 (m, 5H, Ar + NH)

Using the techniques described in Equations 1-8 and Examples 8, 9, 12 and 16, or simple modifications thereof, the following compounds in Tables 3-11, wherein Q is $Q_1$, may be made by one skilled in the art.

General Formulas for Tables 3-11

General Formula 1

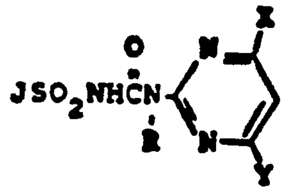

General Formula 2

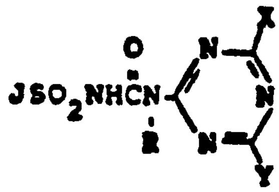

General Formula 3

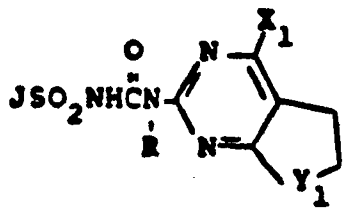

General Formula 4

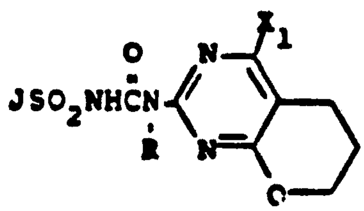

General Formula 5

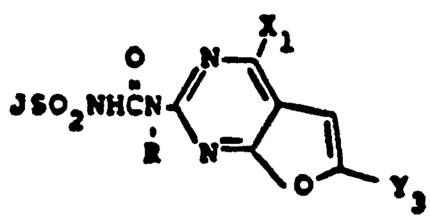

General Formula 6

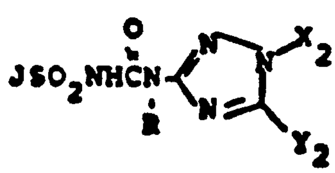

## General Formulas (Continued)

General Formula 7

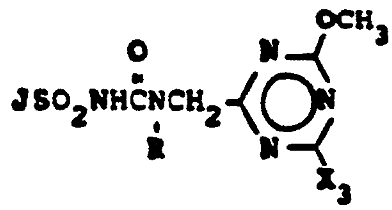

General Formula 8

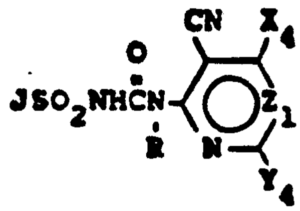

General Formula 9

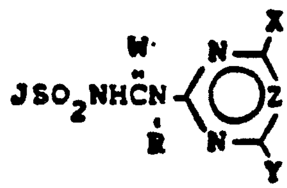

wherein,

for Tables 3 to 11, J-1, J-4 and J-5 represent structures previously described, while J-2a to J-2c and J-3a to J-3d represent the following general structures:

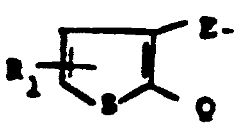 , 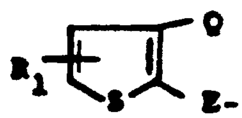 , 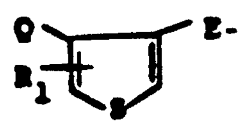

J-2a J-2b J-2c

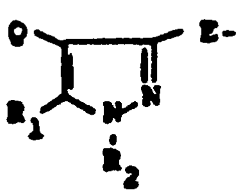 , 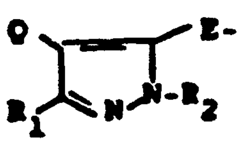 , 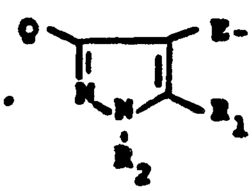 , 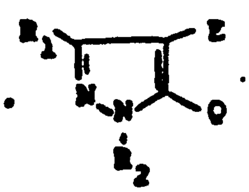

J-3a J-3b J-3c J-3d

Table 3

General Formula 1

| J | $Q_1$ | E | R | $R_1$ | X | Y | m.p.°C |
|---|---|---|---|---|---|---|---|
| 1 | Q-1($R_5$=$CH_3$) | – | H | H | $CH_3$ | $OCH_3$ | 221-222 |
| 1 | Q-1($R_5$=$CH_3$) | – | H | H | $CH_3$ | $CH_3$ | dec. 225 |
| 1 | Q-1($R_5$=$CH_3$) | – | H | H | $OCH_3$ | $OCH_3$ | 208-214 |
| 1 | Q-1($R_5$=$CH_3$) | – | H | H | Cl | $OCH_3$ | 239-242 |
| 1 | Q-1($R_5$=$CH_3$) | – | H | H | Br | $OCH_3$ | 236-240 |
| 1 | Q-1($R_5$=$CH_3$) | – | H | H | $CH_3$ | H | dec. 219 |
| 1 | Q-1($R_5$=$CH_3$) | – | H | H | $OCH_3$ | H | 223-224 |
| 1 | Q-1($R_5$=$CH_3$) | – | H | H | $OCH_3$ | $CH_2OC_2H_5$ | 171-172 |
| 1 | Q-1($R_5$=$CH_3$) | – | H | H | $OCF_2H$ | $OCH_3$ | 174-176 |
| 1 | Q-1($R_5$=$CH_3$) | – | H | H | $OCH_3$ | $CH(OCH_3)_2$ | 142-148 |
| 1 | Q-1($R_5$=$CH_3$) | – | H | H | $CH_3$ | $OC_2H_5$ | 211-213 |
| 1 | Q-1($R_5$=$CH_3$) | – | H | H | $CH_3$ | $CH_2OCH_3$ | 203-205 |
| 1 | Q-1($R_5$=$CH_3$) | – | H | H | $OCH_3$ | $CH_2OCH_3$ | |
| 1 | Q-1($R_5$=$CH_3$) | – | H | H | $OCH_3$ | $C_2H_5$ | |
| 1 | Q-1($R_5$=$CH_3$) | – | H | H | $OC_2H_5$ | $OCH_3$ | |
| 1 | Q-1($R_5$=$CH_3$) | – | H | H | $OCH_2CF_3$ | $OCH_3$ | |
| 1 | Q-1($R_5$=$CH_3$) | – | H | H | $CF_3$ | $OCH_3$ | |
| 1 | Q-1($R_5$=$CH_3$) | – | H | H | $CH_2F$ | $OCH_3$ | |
| 1 | Q-1($R_5$=$CH_3$) | – | H | H | $CH_2Cl$ | $OCH_3$ | |
| 1 | Q-1($R_5$=$CH_3$) | – | H | H | $CH_2Br$ | $OCH_3$ | |
| 1 | Q-1($R_5$=$CH_3$) | – | H | H | F | $OCH_3$ | |
| 1 | Q-1($R_5$=$CH_3$) | – | H | H | I | $OCH_3$ | |
| 1 | Q-1($R_5$=$CH_3$) | – | H | H | $OCH_2CH_2F$ | $OCH_3$ | |
| 1 | Q-1($R_5$=$CH_3$) | – | H | H | $OCH_2CF_3$ | $OCH_3$ | |
| 1 | Q-1($R_5$=$CH_3$) | – | H | H | $OCH_2CHF_2$ | $CH_3$ | |
| 1 | Q-1($R_5$=$CH_3$) | – | H | H | $OCH_2CF_3$ | $CH_3$ | |
| 1 | Q-1($R_5$=$CH_3$) | – | H | H | Cl | $OC_2H_5$ | 179-181 |
| 1 | Q-1($R_5$=$CH_3$) | – | H | H | $OC_2H_5$ | $NHCH_3$ | 183-187 |
| 1 | Q-1($R_5$=$CH_3$) | – | H | H | $OCH_3$ | $CH_2SCH_3$ | 188-193 |
| 1 | Q-1($R_5$=$C_2H_5$) | – | H | H | $OCH_3$ | $OCH_3$ | 210-212 |
| 1 | Q-1($R_5$=$C_2H_5$) | – | H | H | $CH_3$ | $OCH_3$ | 194-196 |

Table 3 (Continued)

| J | $Q_1$ | E | R | $R_1$ | X | Y | m.p.°C |
|---|---|---|---|---|---|---|---|
| 1 | Q-1($R_5$=$C_2H_5$) | - | H | H | $CH_3$ | $CH_3$ | 201-203 |
| 1 | Q-1($R_5$=$C_2H_5$) | - | H | H | Cl | $OCH_3$ | 195-197 |
| 1 | Q-1($R_5$=n-$C_3H_7$) | - | H | H | $OCH_3$ | $OCH_3$ | 185-189 |
| 1 | Q-1($R_5$=n-$C_3H_7$) | - | H | H | $CH_3$ | $OCH_3$ | 163-168 |
| 1 | Q-1($R_5$=n-$C_3H_7$) | - | H | H | $CH_3$ | $CH_3$ | 189-191 |
| 1 | Q-1($R_5$=n-$C_3H_7$) | - | H | H | Cl | $OCH_3$ | 166-169 |
| 1 | Q-1($R_5$=$CH(CH_3)_2$) | - | H | H | $OCH_3$ | $OCH_3$ | 186-188 |
| 1 | Q-1($R_5$=$CH(CH_3)_2$) | - | H | H | $CH_3$ | $OCH_3$ | 191-192 |
| 1 | Q-1($R_5$=$CH(CH_3)_2$) | - | H | H | $CH_3$ | $CH_3$ | 188-189 |
| 1 | Q-1($R_5$=$CH(CH_3)_2$) | - | H | H | Cl | $OCH_3$ | 189-191 |
| 1 | Q-1($R_5$=n-$C_4H_9$) | - | H | H | $OCH_3$ | $OCH_3$ | 182-187 |
| 1 | Q-1($R_5$=t-butyl) | - | H | H | $OCH_3$ | $OCH_3$ | |
| 1 | Q-1($R_5$=$CH_2CH(CH_3)_2$) | - | H | H | $OCH_3$ | $OCH_3$ | |
| 1 | Q-1($R_5$=$CH_2CH{=}CH_2$) | - | H | H | $OCH_3$ | $OCH_3$ | 184-185 |
| 1 | Q-1($R_5$=$CH_2CH{=}CH_2$) | - | H | H | $CH_3$ | $CH_3$ | 189-190 |
| 1 | Q-1($R_5$=$CH_2CH{=}CH_2$) | - | H | H | $OCH_3$ | $CH_3$ | 170-172 |
| 1 | Q-1($R_5$=$CH_2CH{=}CH_2$) | - | H | H | Cl | $OCH_3$ | 164-166 |
| 1 | Q-1($R_5$=$CH_2CH{=}CHCH_3$) | - | H | H | $OCH_3$ | $OCH_3$ | |
| 1 | Q-1($R_5$=$CH_2C{\equiv}CH$) | - | H | H | $OCH_3$ | $OCH_3$ | |
| 1 | Q-1($R_5$=$CH_2C{\equiv}CCH_3$) | - | H | H | $OCH_3$ | $OCH_3$ | |
| 1 | Q-1($R_5$=$CH_2CN$) | - | H | H | $OCH_3$ | $OCH_3$ | 179-183 |
| 1 | Q-1($R_5$=$CH_2CN$) | - | H | H | $CH_3$ | $OCH_3$ | 174-176 |
| 1 | Q-1($R_5$=$CH_2CN$) | - | H | H | $CH_3$ | $CH_3$ | 195-199 |
| 1 | Q-1($R_5$=$CH_2CN$) | - | H | H | Cl | $OCH_3$ | 178-184 |
| 1 | Q-1($R_5$=$CH_2CO_2CH_3$) | - | H | H | $OCH_3$ | $OCH_3$ | 165-170 |
| 1 | Q-1($R_5$=$CH_2CO_2C_2H_5$) | - | H | H | $OCH_3$ | $OCH_3$ | |
| 1 | Q-1($R_5$=$CH(CH_3)CO_2CH_3$) | - | H | H | $OCH_3$ | $OCH_3$ | |
| 1 | Q-1($R_5$=$CH(CH_3)CO_2C_2H_5$) | - | H | H | $OCH_3$ | $OCH_3$ | |
| 1 | Q-1($R_5$=$CF_2H$) | - | H | H | $OCH_3$ | $OCH_3$ | |
| 1 | Q-1($R_5$=$CF_2H$) | - | H | H | $CH_3$ | $OCH_3$ | |
| 1 | Q-1($R_5$=$CF_2H$) | - | H | H | $CH_3$ | $CH_3$ | |
| 1 | Q-1($R_5$=$CF_2H$) | - | H | H | Cl | $OCH_3$ | |

Table 3 (Continued)

| J | $Q_1$ | E | R | $R_1$ | X | Y | m.p.°C |
|---|---|---|---|---|---|---|---|
| 1 | Q-1($R_5$=$CF_2H$) | – | H | H | Br | $OCH_3$ | |
| 1 | Q-1($R_5$=$CF_2H$) | – | H | H | $CH_3$ | H | |
| 1 | Q-1($R_5$=$CF_2H$) | – | H | H | $OCH_3$ | H | |
| 1 | Q-1($R_5$=$CF_2H$) | – | H | H | $OCH_3$ | $CH_2OC_2H_5$ | |
| 1 | Q-1($R_5$=$CF_2H$) | – | H | H | $OCF_2H$ | $OCH_3$ | |
| 1 | Q-1($R_5$=$CF_2H$) | – | H | H | $OCH_3$ | $CH(OCH_3)_2$ | |
| 1 | Q-1($R_5$=$CF_2H$) | – | H | H | $CH_3$ | $OC_2H_5$ | |
| 1 | Q-1($R_5$=$CF_2H$) | – | H | H | $CH_3$ | $CH_2OCH_3$ | |
| 1 | Q-1($R_5$=$CF_2H$) | – | H | H | $OCH_3$ | $CH_2OCH_3$ | |
| 1 | Q-1($R_5$=$CF_2H$) | – | H | H | $OCH_3$ | $C_2H_5$ | |
| 1 | Q-1($R_5$=$CF_2H$) | – | H | H | $OC_2H_5$ | $OCH_3$ | |
| 1 | Q-1($R_5$=$CF_2H$) | – | H | H | $OCH_2CF_3$ | $OCH_3$ | |
| 1 | Q-1($R_5$=$CF_2H$) | – | H | H | $CH_2F$ | $OCH_3$ | |
| 1 | Q-1($R_5$=$CH_2CH_2F$) | – | H | H | $OCH_3$ | $OCH_3$ | |
| 1 | Q-1($R_5$=$CH_2CH_2F$) | – | H | H | $CH_3$ | $OCH_3$ | |
| 1 | Q-1($R_5$=$CH_2CH_2F$) | – | H | H | $CH_3$ | $CH_3$ | |
| 1 | Q-1($R_5$=$CH_2CH_2F$) | – | H | H | Cl | $OCH_3$ | |
| 1 | Q-1($R_5$=$CH_2CH_2Cl$) | – | H | H | $OCH_3$ | $OCH_3$ | |
| 1 | Q-1($R_5$=$CH_2CH_2CH_2F$) | – | H | H | $OCH_3$ | $OCH_3$ | |
| 1 | Q-1($R_5$=$CH_2CHF_2$) | – | H | H | $OCH_3$ | $OCH_3$ | |
| 1 | Q-1($R_5$=$CH_2CF_3$) | – | H | H | $OCH_3$ | $OCH_3$ | |
| 1 | Q-1($R_5$=$CH_2CF_3$) | – | H | H | $CH_3$ | $OCH_3$ | |
| 1 | Q-1($R_5$=$CH_2CF_3$) | – | H | H | $CH_3$ | $CH_3$ | |
| 1 | Q-1($R_5$=$CH_2CF_3$) | – | H | H | Cl | $OCH_3$ | |
| 1 | Q-1($R_5$=$CH_2OCH_3$) | – | H | H | $OCH_3$ | $OCH_3$ | 199-202 |
| 1 | Q-1($R_5$=$CH_2OCH_3$) | – | H | H | $CH_3$ | $OCH_3$ | 209-210 |
| 1 | Q-1($R_5$=$CH_2OCH_3$) | – | H | H | $CH_3$ | $CH_3$ | dec. 217 |
| 1 | Q-1($R_5$=$CH_2OCH_3$) | – | H | H | Cl | $OCH_3$ | 203-206 |
| 1 | Q-1($R_5$=$CH_2OCH_2CH_3$) | – | H | H | $OCH_3$ | $OCH_3$ | |
| 1 | Q-1($R_5$=$CH_2CH_2OCH_3$) | – | H | H | $OCH_3$ | $OCH_3$ | 175-178 |
| 1 | Q-1($R_5$=$CH_2CH_2OC_2H_5$) | – | H | H | $OCH_3$ | $OCH_3$ | |
| 1 | Q-2($R_6$=$CH_3$) | – | H | H | $OCH_3$ | $OCH_3$ | |

Table 3 (Continued)

| J | $Q_1$ | E | R | $R_1$ | X | Y | m.p.°C |
|---|---|---|---|---|---|---|---|
| 1 | Q-2($R_6$=$C_2H_5$) | - | H | H | $OCH_3$ | $OCH_3$ | |
| 1 | Q-2($R_6$=$CH(CH_3)_2$) | - | H | H | $OCH_3$ | $OCH_3$ | |
| 1 | Q-3($R_6$=$CH_3$) | - | H | H | $OCH_3$ | $OCH_3$ | |
| 1 | Q-4($R_6$=$CH_3$) | - | H | H | $OCH_3$ | $OCH_3$ | |
| 1 | Q-5($R_6$=$CH_3$) | - | H | H | $OCH_3$ | $OCH_3$ | |
| 1 | Q-5($R_6$=$CH_3$) | - | H | H | $CH_3$ | $OCH_3$ | |
| 1 | Q-5($R_6$=$CH_3$) | - | H | H | $CH_3$ | $CH_3$ | |
| 1 | Q-5($R_6$=$CH_3$) | - | H | H | Cl | $OCH_3$ | |
| 1 | Q-5($R_6$=n-$C_3H_7$) | - | H | H | $OCH_3$ | $OCH_3$ | |
| 1 | Q-6($R_6$=$CH_3$) | - | H | H | $OCH_3$ | $OCH_3$ | |
| 1 | Q-7($R_5$,$R_6$=$CH_3$) | - | H | H | $OCH_3$ | $OCH_3$ | |
| 1 | Q-7($R_5$,$R_6$=$CH_3$) | - | H | H | $CH_3$ | $OCH_3$ | |
| 1 | Q-7($R_5$,$R_6$=$CH_3$) | - | H | H | $CH_3$ | $CH_3$ | |
| 1 | Q-7($R_5$,$R_6$=$CH_3$) | - | H | H | Cl | $OCH_3$ | |
| 1 | Q-7($R_5$=$C_2H_5$,$R_6$=$CH_3$) | - | H | H | $OCH_3$ | $OCH_3$ | |
| 1 | Q-7($R_5$=$C_2H_5$,$R_6$=$CH_3$) | - | H | H | $CH_3$ | $OCH_3$ | |
| 1 | Q-7($R_5$=$C_2H_5$,$R_6$=$CH_3$) | - | H | H | $CH_3$ | $CH_3$ | |
| 1 | Q-7($R_5$=$C_2H_5$,$R_6$=$CH_3$) | - | H | H | Cl | $OCH_3$ | |
| 1 | Q-8 | - | H | H | $OCH_3$ | $OCH_3$ | |
| 1 | Q-9 | - | H | H | $OCH_3$ | $OCH_3$ | |
| 1 | Q-9 | - | H | H | $CH_3$ | $OCH_3$ | |
| 1 | Q-9 | - | H | H | $CH_3$ | $CH_3$ | dec. 207 |
| 1 | Q-9 | - | H | H | Cl | $OCH_3$ | |
| 1 | Q-10 | - | H | H | $OCH_3$ | $OCH_3$ | |
| 1 | Q-10 | - | H | H | $CH_3$ | $OCH_3$ | |
| 1 | Q-10 | - | H | H | $CH_3$ | $CH_3$ | |
| 1 | Q-10 | - | H | H | Cl | $OCH_3$ | |
| 1 | Q-11 | - | H | H | $OCH_3$ | $OCH_3$ | |
| 1 | Q-12 | - | H | H | $OCH_3$ | $OCH_3$ | |
| 1 | Q-13 | - | H | H | $OCH_3$ | $OCH_3$ | |
| 1 | Q-14($R_6$=$CH_3$) | - | H | H | $OCH_3$ | $OCH_3$ | |
| 1 | Q-15($R_7$=$CH_3$) | - | H | H | $OCH_3$ | $OCH_3$ | |

Table 3 (Continued)

| J | $Q_1$ | E | R | $R_1$ | X | Y | m.p.°C |
|---|---|---|---|---|---|---|---|
| 1 | Q-15($R_7$=$CH_3$) | – | H | H | $CH_3$ | $OCH_3$ | |
| 1 | Q-15($R_7$=$CH_3$) | – | H | H | $CH_3$ | $CH_3$ | |
| 1 | Q-15($R_7$=$CH_3$) | – | H | H | Cl | $OCH_3$ | |
| 1 | Q-15($R_7$=$C_2H_5$) | – | H | H | $OCH_3$ | $OCH_3$ | |
| 1 | Q-15($R_7$=$C_2H_5$) | – | H | H | $CH_3$ | $OCH_3$ | |
| 1 | Q-15($R_7$=$C_2H_5$) | – | H | H | $CH_3$ | $CH_3$ | |
| 1 | Q-15($R_7$=$C_2H_5$) | – | H | H | Cl | $OCH_3$ | |
| 1 | Q-15($R_7$=n-$C_3H_7$) | – | H | H | $OCH_3$ | $OCH_3$ | |
| 1 | Q-15($R_7$=n-$C_3H_7$) | – | H | H | $CH_3$ | $OCH_3$ | |
| 1 | Q-15($R_7$=n-$C_3H_7$) | – | H | H | $CH_3$ | $CH_3$ | |
| 1 | Q-15($R_7$=n-$C_3H_7$) | – | H | H | Cl | $OCH_3$ | |
| 1 | Q-15($R_7$=$CH(CH_3)_2$) | – | H | H | $OCH_3$ | $OCH_3$ | |
| 1 | Q-15($R_7$=$CH(CH_3)_2$) | – | H | H | $CH_3$ | $OCH_3$ | |
| 1 | Q-15($R_7$=$CH(CH_3)_2$) | – | H | H | $CH_3$ | $CH_3$ | |
| 1 | Q-15($R_7$=$CH(CH_3)_2$) | – | H | H | Cl | $OCH_3$ | |
| 1 | Q-15($R_7$=n-$C_4H_9$) | – | H | H | $OCH_3$ | $OCH_3$ | |
| 1 | Q-15($R_7$=t-butyl) | – | H | H | $OCH_3$ | $OCH_3$ | |
| 1 | Q-15($R_7$=t-butyl) | – | H | H | $CH_3$ | $OCH_3$ | |
| 1 | Q-15($R_7$=t-butyl) | – | H | H | $CH_3$ | $CH_3$ | |
| 1 | Q-15($R_7$=t-butyl) | – | H | H | Cl | $OCH_3$ | |
| 1 | Q-16($R_7$=$CH_3$) | – | H | H | $OCH_3$ | $OCH_3$ | |
| 1 | Q-16($R_7$=$CH_3$) | – | H | H | $CH_3$ | $OCH_3$ | |
| 1 | Q-16($R_7$=$CH_3$) | – | H | H | $CH_3$ | $CH_3$ | |
| 1 | Q-16($R_7$=$CH_3$) | – | H | H | Cl | $OCH_3$ | |
| 1 | Q-1($R_5$=$CH_3$) | O | H | H | $OCH_3$ | $OCH_3$ | 153-158 |
| 1 | Q-1($R_5$=$CH_3$) | O | H | H | $CH_3$ | $OCH_3$ | 150-156 |
| 1 | Q-1($R_5$=$CH_3$) | O | H | H | $CH_3$ | $CH_3$ | 99-104 |
| 1 | Q-1($R_5$=$CH_3$) | O | H | H | Cl | $OCH_3$ | 175-178 |
| 1 | Q-1($R_5$=$CH_3$) | O | H | H | Cl | $OC_2H_5$ | |
| 1 | Q-1($R_5$=$CH_3$) | O | H | H | $CH_3$ | H | |
| 1 | Q-1($R_5$=$CH_3$) | O | H | H | $OCH_3$ | H | |
| 1 | Q-1($R_5$=$CH_3$) | O | H | H | $OCH_3$ | $CH_2OCH_3$ | |

Table 3 (Continued)

| J | $Q_1$ | E | R | $R_1$ | X | Y | m.p.°C |
|---|---|---|---|---|---|---|---|
| 1 | Q-1($R_5$=$CH_3$) | O | H | H | $CH_3$ | $CH_2OCH_3$ | |
| 1 | Q-1($R_5$=$CH_3$) | O | H | H | $OCF_2H$ | $OCH_3$ | 129-133 |
| 1 | Q-1($R_5$=$CH_3$) | O | H | H | $OCH_3$ | $CH(OCH_3)_2$ | |
| 1 | Q-1($R_5$=$CH_3$) | O | H | H | $CH_3$ | $OC_2H_5$ | |
| 1 | Q-1($R_5$=$CH_3$) | O | H | H | $OCH_3$ | $CH_2OC_2H_5$ | |
| 1 | Q-1($R_5$=$CH_3$) | O | H | H | $OCH_3$ | $C_2H_5$ | |
| 1 | Q-1($R_5$=$CH_3$) | O | H | H | $OC_2H_5$ | $OCH_3$ | |
| 1 | Q-1($R_5$=$CH_3$) | O | H | H | $OCH_2CF_3$ | $OCH_3$ | |
| 1 | Q-1($R_5$=$CH_3$) | O | H | H | $CH_2F$ | $OCH_3$ | |
| 1 | Q-1($R_5$=$CH_3$) | O | H | H | $OCH_2CF_3$ | $NHCH_3$ | |
| 1 | Q-1($R_5$=$CH_3$) | O | H | H | $OCH_3$ | $CH_2SCH_3$ | |
| 1 | Q-1($R_5$=$CH_3$) | O | H | H | Br | $OCH_3$ | |
| 1 | Q-1($R_5$=$C_2H_5$) | O | H | H | $OCH_3$ | $OCH_3$ | |
| 1 | Q-1($R_5$=$C_2H_5$) | O | H | H | $CH_3$ | $OCH_3$ | |
| 1 | Q-1($R_5$=$C_2H_5$) | O | H | H | $CH_3$ | $CH_3$ | |
| 1 | Q-1($R_5$=$C_2H_5$) | O | H | H | Cl | $OCH_3$ | |
| 1 | Q-1($R_5$=n-$C_3H_7$) | O | H | H | $OCH_3$ | $OCH_3$ | |
| 1 | Q-1($R_5$=n-$C_3H_7$) | O | H | H | $CH_3$ | $OCH_3$ | |
| 1 | Q-1($R_5$=n-$C_3H_7$) | O | H | H | $CH_3$ | $CH_3$ | |
| 1 | Q-1($R_5$=n-$C_3H_7$) | O | H | H | Cl | $OCH_3$ | |
| 1 | Q-1($R_5$=$CH(CH_3)_2$) | O | H | H | $OCH_3$ | $OCH_3$ | |
| 1 | Q-1($R_5$=$CH(CH_3)_2$) | O | H | H | $OCH_3$ | $CH_3$ | |
| 1 | Q-1($R_5$=$CH(CH_3)_2$) | O | H | H | $CH_3$ | $CH_3$ | |
| 1 | Q-1($R_5$=$CH(CH_3)_2$) | O | H | H | Cl | $OCH_3$ | |
| 1 | Q-1($R_5$=$CF_2H$) | O | H | H | $OCH_3$ | $OCH_3$ | |
| 1 | Q-1($R_5$=$CF_2H$) | O | H | H | $OCH_3$ | $CH_3$ | |
| 1 | Q-1($R_5$=$CF_2H$) | O | H | H | $CH_3$ | $OCH_3$ | |
| 1 | Q-1($R_5$=$CF_2H$) | O | H | H | Cl | $OCH_3$ | |
| 1 | Q-1($R_5$=$CH_2CN$) | O | H | H | $OCH_3$ | $OCH_3$ | |
| 1 | Q-1($R_5$=$CH_2CN$) | O | H | H | $CH_3$ | $OCH_3$ | |
| 1 | Q-1($R_5$=$CH_2CN$) | O | H | H | $CH_3$ | $CH_3$ | |
| 1 | Q-1($R_5$=$CH_2CN$) | O | H | H | Cl | $OCH_3$ | |

Table 3 (Continued)

| J | $Q_1$ | E | R | $R_1$ | X | Y | m.p.°C |
|---|---|---|---|---|---|---|---|
| 1 | Q-1($R_5$=$CH_2CH$=$CH_2$) | O | H | H | $OCH_3$ | $OCH_3$ | |
| 1 | Q-1($P_5$=$CH_2CH$=$CH_2$) | O | H | H | $CH_3$ | $OCH_3$ | |
| 1 | Q-1($R_5$=$CH_2CH$=$CH_2$) | O | H | H | $CH_3$ | $CH_3$ | |
| 1 | Q-1($R_5$=$CH_2CH$=$CH_2$) | O | H | H | Cl | $OCH_3$ | |
| 1 | Q-7($R_5$,$R_6$=$CH_3$) | O | H | H | $OCH_3$ | $OCH_3$ | |
| 1 | Q-9 | O | H | H | $OCH_3$ | $OCH_3$ | |
| 1 | Q-10 | O | H | H | $OCH_3$ | $OCH_3$ | 131-135 |
| 1 | Q-10 | O | H | H | $CH_3$ | $OCH_3$ | 175-176 |
| 1 | Q-10 | O | H | H | $CH_3$ | $CH_3$ | 58-72 |
| 1 | Q-10 | O | H | H | Cl | $OCH_3$ | 120-123 |
| 1 | Q-15($R_7$=$CH_3$) | O | H | H | $OCH_3$ | $OCH_3$ | |
| 1 | Q-1($R_5$=$CH_3$) | $CH_2$ | H | H | $OCH_3$ | $OCH_3$ | 195-197 |
| 1 | Q-1($R_5$=$CH_3$) | $CH_2$ | H | H | $CH_3$ | $OCH_3$ | 171-172 |
| 1 | Q-1($R_5$=$CH_3$) | $CH_2$ | H | H | $CH_3$ | $CH_3$ | 194-197 |
| 1 | Q-1($R_5$=$CH_3$) | $CH_2$ | H | H | Cl | $OCH_3$ | 177-180 |
| 1 | Q-2($R_6$=$CH_3$) | $CH_2$ | H | H | $OCH_3$ | $OCH_3$ | |
| 1 | Q-7($R_5$,$R_6$=$CH_3$) | $CH_2$ | H | H | $OCH_3$ | $OCH_3$ | |
| 1 | Q-7($R_5$,$R_6$=$CH_3$) | $CH_2$ | H | H | $CH_3$ | $OCH_3$ | |
| 1 | Q-7($R_5$,$R_6$=$CH_3$) | $CH_2$ | H | H | $CH_3$ | $CH_3$ | |
| 1 | Q-7($R_5$,$R_6$=$CH_3$) | $CH_2$ | H | H | Cl | $OCH_3$ | |
| 1 | Q-9 | $CH_2$ | H | H | $OCH_3$ | $OCH_3$ | |
| 1 | Q-10 | $CH_2$ | H | H | $OCH_3$ | $OCH_3$ | |
| 1 | Q-15 | $CH_2$ | H | H | $OCH_3$ | $OCH_3$ | |
| 1 | Q-1($R_5$=$CH_3$) | - | $CH_3$ | H | $OCH_3$ | $OCH_3$ | |
| 1 | Q-7($R_5$,$R_6$=$CH_3$) | - | $CH_3$ | H | $OCH_3$ | $OCH_3$ | |
| 1 | Q-15($R_7$=$CH_3$) | - | $CH_3$ | H | $OCH_3$ | $OCH_3$ | |
| 1 | Q-1($R_5$=$CH_3$) | - | H | 3-F | $OCH_3$ | $OCH_3$ | |
| 1 | Q-1($R_5$=$CH_3$) | - | H | 5-Cl | $OCH_3$ | $OCH_3$ | 203-206 |
| 1 | Q-1($R_5$=$CH_3$) | - | H | 6-Br | $OCH_3$ | $OCH_3$ | |
| 1 | Q-1($R_5$=$CH_3$) | - | H | 5-$CH_3$ | $OCH_3$ | $OCH_3$ | |
| 1 | Q-1($R_5$=$CH_3$) | - | H | 6-$C_2H_5$ | $OCH_3$ | $OCH_3$ | |
| 1 | Q-1($R_5$=$CH_3$) | - | H | 5-$OCH_3$ | $OCH_3$ | $OCH_3$ | |

Table 3 (Continued)

| J | $Q_1$ | E | R | $R_1$ | X | Y | m.p.°C |
|---|---|---|---|---|---|---|---|
| 1 | Q-1($R_5$=$CH_3$) | - | H | 3-$OC_2H_5$ | $OCH_3$ | $OCH_3$ | |
| 1 | Q-1($R_5$=$CH_3$) | - | H | 6-$OCH(CH_3)_2$ | $OCH_3$ | $OCH_3$ | |
| 1 | Q-1($R_5$=$CH_3$) | - | H | 5-$SCH_3$ | $OCH_3$ | $OCH_3$ | |
| 1 | Q-1($R_5$=$CH_3$) | - | H | 6-$SC_2H_5$ | $OCH_3$ | $OCH_3$ | |
| 1 | Q-1($R_5$=$CH_3$) | - | H | 3-$SCH(CH_3)_2$ | $OCH_3$ | $OCH_3$ | |
| 1 | Q-1($R_5$=$CH_3$) | - | H | 5-$CH_2F$ | $OCH_3$ | $OCH_3$ | |
| 1 | Q-1($R_5$=$CH_3$) | - | H | 5-$CF_3$ | $OCH_3$ | $OCH_3$ | |
| 1 | Q-1($R_5$=$CH_3$) | - | H | 5-$CH_2CH_2Br$ | $OCH_3$ | $OCH_3$ | |
| 1 | Q-1($R_5$=$CH_3$) | - | H | 6-$OCF_2H$ | $OCH_3$ | $OCH_3$ | |
| 1 | Q-1($R_5$=$CH_3$) | - | H | 6-$OCH_2CH_2Br$ | $OCH_3$ | $OCH_3$ | |
| 1 | Q-1($R_5$=$CH_3$) | - | H | 6-$OCH(CH_3)CH_2Cl$ | $OCH_3$ | $OCH_3$ | |
| 1 | Q-1($R_5$=$CH_3$) | - | H | 5-$SCH_2F$ | $OCH_3$ | $OCH_3$ | |
| 1 | Q-1($R_5$=$CH_3$) | - | H | 6-$SCH_2CH_2Br$ | $OCH_3$ | $OCH_3$ | |
| 1 | Q-1($R_5$=$CH_3$) | - | H | 6-$SCH(CH_3)CH_2Cl$ | $OCH_3$ | $OCH_3$ | |
| 1 | Q-1($R_5$=$CH_3$) | - | H | 4-$NO_2$ | $OCH_3$ | $OCH_3$ | |
| 1 | Q-1($R_5$=$CH_3$) | O | H | 5-Cl | $OCH_3$ | $OCH_3$ | |
| 1 | Q-1($R_5$=$CH_3$) | O | H | 5-$CH_3$ | $OCH_3$ | $OCH_3$ | |
| 1 | Q-1($R_5$=$CH_3$) | O | H | 5-$OCH_3$ | $OCH_3$ | $OCH_3$ | |
| 1 | Q-1($R_5$=$CH_3$) | O | H | 5-$SCH_3$ | $OCH_3$ | $OCH_3$ | |
| 1 | Q-1($R_5$=$C_2H_5$) | - | H | H | $C_2H_5$ | $OCH_3$ | |
| 1 | Q-1($R_5$=$C_2H_5$) | - | H | H | $CF_3$ | $OCH_3$ | |
| 1 | Q-1($R_5$=$C_2H_5$) | - | H | H | $OCH_3$ | H | |
| 1 | Q-1($R_5$=$CH_3$) | - | H | H | $NH_2$ | $OCH_3$ | |
| 1 | Q-1($R_5$=$CH_3$) | - | H | H | n-$C_3H_7$ | $OCH_3$ | |
| 1 | Q-1($R_5$=$CH_3$) | - | H | H | $NHCH_3$ | $OCH_3$ | |
| 1 | Q-1($R_5$=$CH_3$) | - | H | H | $OCH_3$ | $SCH_3$ | |
| 1 | Q-1($R_5$=$CH_3$) | - | H | H | $OCH_3$ | $SCF_2H$ | |
| 1 | Q-1($R_5$=$CH_3$) | - | H | H | $OCH_3$ | $OCH_2C\equiv CH$ | |
| 1 | Q-1($R_5$=$CH_3$) | - | H | H | $OCH_3$ | $C\equiv CH$ | |
| 1 | Q-1($R_5$=$CH_3$) | - | H | H | $OCH_3$ | $N(CH_3)_2$ | |
| 1 | Q-1($R_5$=$CH_3$) | - | H | H | $OCH_3$ | cyclopropyl | |
| 1 | Q-1($R_5$=$CH_3$) | - | H | H | $OCH_3$ | $NH_2$ | |

Table 3 (Continued)

| J | $Q_1$ | E | R | $R_1$ | X | Y | m.p.°C |
|---|---|---|---|---|---|---|---|
| 1 | Q-10 | – | H | H | $OCH_3$ | $OCF_2H$ | |
| 1 | Q-1($R_5$=$CH_3$) | – | H | H | $OCH_3$ | $CF_3$ | |
| 1 | Q-1($R_5$=$CH_3$) | – | H | H | $OCH_3$ | $OCH_2CH_2OCH_3$ | |
| 1 | Q-1($R_5$=$CH_3$) | – | H | H | $OCH_3$ | $CH_2SCH_3$ | |
| 1 | Q-1($R_5$=$CH_3$) | – | H | H | $OCH_3$ | CHO | |
| 1 | Q-1($R_5$=$CH_3$) | – | H | H | $OCH_3$ | $COCH_3$ | |
| 1 | Q-1($R_5$=$CH_3$) | – | H | H | $OCH_3$ | $CH(SCH_3)OC_2H_5$ | |
| 1 | Q-1($R_5$=$CH_3$) | – | H | H | $OCH_3$ | $C(CH_3)(SCH_3)_2$ | |
| 1 | Q-1($R_5$=$CH_3$) | – | H | H | $OCH_3$ | $C(SC_2H_5)_2$ | |
| 1 | Q-1($R_5$=$CH_3$) | – | H | H | $OCH_3$ | 1,3-dioxolan-2-yl | |
| 1 | Q-1($R_5$=$CH_3$) | – | H | H | $OCH_3$ | 2-methyl-1,3-oxathiolan-2-yl | |
| 1 | Q-1($R_5$=$CH_3$) | – | H | H | $OCH_3$ | 1,3-oxathian-2-yl | |
| 1 | Q-1($R_5$=$CH_3$) | – | H | H | $OCH_3$ | 2-methyl-1,3-dithian-2-yl | |
| 1 | Q-1($R_5$=$CH_3$) | – | H | H | $OCH_3$ | 4-methyl-1,3-dioxolan-2-yl | |
| 1 | Q-1($R_5$=$CH_3$) | – | H | H | $OCH_3$ | 2,4-dimethyl-1,3-dithiolan-2-yl | |
| 1 | Q-1($R_5$=$CH_3$) | – | H | H | $OCH_3$ | $N(OCH_3)(CH_3)$ dithiolan-2-yl | |
| 1 | Q-1($R_5$=$CH_3$) | – | H | H | $OCH_3$ | $C{\equiv}CCH_3$ | |
| 1 | Q-1($R_5$=$CH_3$) | – | H | H | $OCH_3$ | cyclopropyl | |
| 2a | Q-1($R_5$=$CH_3$) | – | H | H | $OCH_3$ | $OCH_3$ | 150-155 |
| 2a | Q-1($R_5$=$CH_3$) | – | H | H | $CH_3$ | $OCH_3$ | 190-193 |
| 2a | Q-1($R_5$=$CH_3$) | – | H | H | $CH_3$ | $CH_3$ | 164-168 |
| 2a | Q-1($R_5$=$CH_3$) | – | H | H | Cl | $OCH_3$ | 205-207 |
| 2a | Q-1($R_5$=$C_2H_5$) | – | H | H | $OCH_3$ | $OCH_3$ | |
| 2a | Q-1($R_5$=$C_2H_5$) | – | H | H | $CH_3$ | $OCH_3$ | |
| 2a | Q-1($R_5$=$C_2H_5$) | – | H | H | $CH_3$ | $CH_3$ | |
| 2a | Q-1($R_5$=$C_2H_5$) | – | H | H | Cl | $OCH_3$ | |
| 2a | Q-1($R_5$=n-$C_3H_7$) | – | H | H | $OCH_3$ | $OCH_3$ | |
| 2a | Q-1($R_5$=n-$C_3H_7$) | – | H | H | $CH_3$ | $OCH_3$ | |

Table 3 (Continued)

| J | $Q_1$ | E | R | $R_1$ | X | Y | m.p.°C |
|---|---|---|---|---|---|---|---|
| 2a | Q-1($R_5$=n-$C_3H_7$) | – | H | H | $CH_3$ | $CH_3$ | |
| 2a | Q-1($R_5$=n-$C_3H_7$) | – | H | H | Cl | $OCH_3$ | |
| 2a | Q-1($R_5$=$CH(CH_3)_2$ | – | H | H | $OCH_3$ | $OCH_3$ | |
| 2a | Q-1($R_5$=$CH(CH_3)_2$ | – | H | H | $CH_3$ | $OCH_3$ | |
| 2a | Q-1($R_5$=$CH(CH_3)_2$ | – | H | H | $CH_3$ | $CH_3$ | |
| 2a | Q-1($R_5$=$CH(CH_3)_2$ | – | H | H | Cl | $OCH_3$ | |
| 2a | Q-1($R_5$=$CH_2CN$) | – | H | H | $OCH_3$ | $OCH_3$ | 119-122 |
| 2a | Q-1($R_5$=$CH_2CN$) | – | H | H | $CH_3$ | $OCH_3$ | 183-185 |
| 2a | Q-1($R_5$=$CH_2CN$) | – | H | H | $CH_3$ | $CH_3$ | |
| 2a | Q-1($R_5$=$CH_2CN$) | – | H | H | Cl | $OCH_3$ | 172-175 |
| 2a | Q-1($R_5$=$CH_2CH=CH_2$) | – | H | H | $OCH_3$ | $OCH_3$ | 94-97 |
| 2a | Q-1($R_5$=$CH_2CH=CH_2$) | – | H | H | $OCH_3$ | $CH_3$ | 172-174 |
| 2a | Q-1($R_5$=$CH_2CH=CH_2$) | – | H | H | $CH_3$ | $CH_3$ | 193-197 |
| 2a | Q-1($R_5$=$CH_2CH=CH_2$) | – | H | H | Cl | $OCH_3$ | 147-148 |
| 2a | Q-1($R_5$=$CF_2H$) | – | H | H | $OCH_3$ | $OCH_3$ | |
| 2a | Q-1($R_5$=$CF_2H$) | – | H | H | $CH_3$ | $OCH_3$ | |
| 2a | Q-1($R_5$=$CF_2H$) | – | H | H | $CH_3$ | $CH_3$ | |
| 2a | Q-1($R_5$=$CF_2H$) | – | H | H | Cl | $OCH_3$ | |
| 2a | Q-7($R_5$,$R_6$=$CH_3$) | – | H | H | $OCH_3$ | $OCH_3$ | |
| 2a | Q-7($R_5$,$R_6$=$CH_3$) | – | H | H | $CH_3$ | $OCH_3$ | |
| 2a | Q-7($R_5$,$R_6$=$CH_3$) | – | H | H | $CH_3$ | $CH_3$ | |
| 2a | Q-7($R_5$,$R_6$=$CH_3$) | – | H | H | Cl | $OCH_3$ | |
| 2a | Q-15($R_7$=$CH_3$) | – | H | H | $OCH_3$ | $OCH_3$ | |
| 2a | Q-15($R_7$=$CH_3$) | – | H | H | $CH_3$ | $OCH_3$ | |
| 2a | Q-15($R_7$=$CH_3$) | – | H | H | $CH_3$ | $CH_3$ | |
| 2a | Q-15($R_7$=$CH_3$) | – | H | H | Cl | $OCH_3$ | |
| 2b | Q-1($R_5$=$CH_3$) | – | H | H | $OCH_3$ | $OCH_3$ | |
| 2b | Q-1($R_5$=$CH_3$) | – | H | H | $CH_3$ | $OCH_3$ | |
| 2b | Q-1($R_5$=$CH_3$) | – | H | H | $CH_3$ | $CH_3$ | |
| 2b | Q-1($R_5$=$CH_3$) | – | H | H | Cl | $OCH_3$ | |
| 2b | Q-7($R_5$,$R_6$=$CH_3$) | – | H | H | $OCH_3$ | $OCH_3$ | |
| 2b | Q-7($R_5$,$R_6$=$CH_3$) | – | H | H | $CH_3$ | $OCH_3$ | |

Table 3 (Continued)

| J | $Q_1$ | E | R | $R_1$ | X | Y | m.p.°C |
|---|---|---|---|---|---|---|---|
| 2b | Q-1($R_5$,$R_6$=$CH_3$) | - | H | H | $CH_3$ | $CH_3$ | |
| 2b | Q-1($R_5$,$R_6$=$CH_3$) | - | H | H | Cl | $OCH_3$ | |
| 2c | Q-1($R_5$=$CH_3$) | - | H | H | $OCH_3$ | $OCH_3$ | |
| 2c | Q-1($R_5$=$CH_3$) | - | H | H | $CH_3$ | $OCH_3$ | |
| 2c | Q-1($R_5$=$CH_3$) | - | H | H | $CH_3$ | $CH_3$ | |
| 2c | Q-1($R_5$=$CH_3$) | - | H | H | Cl | $OCH_3$ | |
| 3a | ($R_2$=$CH_3$)Q-1($R_5$=$CH_3$) | - | H | H | $OCH_3$ | $OCH_3$ | |
| 3a | ($R_2$=$CH_3$)Q-1($R_5$=$CH_3$) | - | H | H | $CH_3$ | $OCH_3$ | |
| 3a | ($R_2$=$CH_3$)Q-1($R_5$=$CH_3$) | - | H | H | $CH_3$ | $CH_3$ | |
| 3a | ($R_2$=$CH_3$)Q-1($R_5$=$CH_3$) | - | H | H | Cl | $OCH_3$ | |
| 3b | ($R_2$=$C_2H_5$)Q-1($R_5$=$CH_3$) | - | H | H | $OCH_3$ | $OCH_3$ | |
| 3c | ($R_2$=$CH_3$)Q-1($R_5$=$CH_3$) | - | H | H | $OCH_3$ | $OCH_3$ | |
| 3c | ($R_2$=$CH_3$)Q-1($R_5$=$CH_3$) | - | H | H | $CH_3$ | $OCH_3$ | |
| 3c | ($R_2$=$CH_3$)Q-1($R_5$=$CH_3$) | - | H | H | $CH_3$ | $CH_3$ | |
| 3c | ($R_2$=$CH_3$)Q-1($R_5$=$CH_3$) | - | H | H | Cl | $OCH_3$ | |
| 3c | ($R_2$=$CH_3$)Q-7($R_5$,$R_6$=$CH_3$) | - | H | H | $OCH_3$ | $OCH_3$ | |
| 3c | ($R_2$=$CH_3$)Q-7($R_5$,$R_6$=$CH_3$) | - | H | H | $CH_3$ | $OCH_3$ | |
| 3c | ($R_2$=$CH_3$)Q-7($R_5$,$R_6$=$CH_3$) | - | H | H | $CH_3$ | $CH_3$ | |
| 3c | ($R_2$=$CH_3$)Q-7($R_5$,$R_6$=$CH_3$) | - | H | H | Cl | $OCH_3$ | |
| 3d | ($R_2$=$CH_3$)Q-1($R_5$=$CH_3$) | - | H | H | $OCH_3$ | $OCH_3$ | |
| 3d | ($R_2$=$CH_3$)Q-1($R_5$=$CH_3$) | - | H | H | $CH_3$ | $OCH_3$ | |
| 3d | ($R_2$=$CH_3$)Q-1($R_5$=$CH_3$) | - | H | H | $CH_3$ | $CH_3$ | |
| 3d | ($R_2$=$CH_3$)Q-1($R_5$=$CH_3$) | - | H | H | Cl | $OCH_3$ | |
| 3d | ($R_2$=$CH_3$)Q-7($R_5$,$R_6$=$CH_3$) | - | H | H | $OCH_3$ | $OCH_3$ | |
| 3d | ($R_2$=$CH_3$)Q-7($R_5$,$R_6$=$CH_3$) | - | H | H | $CH_3$ | $OCH_3$ | |
| 3d | ($R_2$=$CH_3$)Q-7($R_5$,$R_6$=$CH_3$) | - | H | H | $CH_3$ | $CH_3$ | |
| 3d | ($R_2$=$CH_3$)Q-7($R_5$,$R_6$=$CH_3$) | - | H | H | Cl | $OCH_3$ | |
| 3d | ($R_2$=$C_2H_5$)Q-7($R_5$=$CH_3$) | - | H | H | $OCH_3$ | $OCH_3$ | |
| 3d | ($R_2$=$C_2H_5$)Q-7($R_5$=$CH_3$) | - | H | H | $CH_3$ | $OCH_3$ | |
| 3d | ($R_2$=$C_2H_5$)Q-7($R_5$=$CH_3$) | - | H | H | $CH_3$ | $CH_3$ | |
| 3d | ($R_2$=$C_2H_5$)Q-7($R_5$=$CH_3$) | - | H | H | Cl | $OCH_3$ | |
| 3d | ($R_2$=n-$C_3H_7$)Q-1($R_5$=$CH_3$) | - | H | H | $OCH_3$ | $OCH_3$ | |

Table 3 (Continued)

| J | $Q_1$ | E | R | $R_1$ | X | Y | m.p.°C |
|---|---|---|---|---|---|---|---|
| 3d | $(R_2$=n-$C_3H_7)$Q-1$(R_5=CH_3)$ | - | H | H | $CH_3$ | $OCH_3$ | |
| 3d | $(R_2$=n-$C_3H_7)$Q-1$(R_5=CH_3)$ | - | H | H | $CH_3$ | $CH_3$ | |
| 3d | $(R_2$=n-$C_3H_7)$Q-1$(R_5=CH_3)$ | - | H | H | Cl | $OCH_3$ | |
| 3d | $(R_2=CH(CH_3)_2)$Q-1$(R_5=CH_3)$ | - | H | H | $OCH_3$ | $OCH_3$ | |
| 3d | $(R_2=CH(CH_3)_2)$Q-1$(R_5=CH_3)$ | - | H | H | $CH_3$ | $OCH_3$ | |
| 3d | $(R_2=CH(CH_3)_2)$Q-1$(R_5=CH_3)$ | - | H | H | $CH_3$ | $CH_3$ | |
| 3d | $(R_2=CH(CH_3)_2)$Q-1$(R_5=CH_3)$ | - | H | H | Cl | $OCH_3$ | |
| 4 | Q-1$(R_5=CH_3)$ | - | H | H | $OCH_3$ | $OCH_3$ | dec. 187 |
| 4 | Q-1$(R_5=CH_3)$ | - | H | H | $CH_3$ | $OCH_3$ | dec. 148 |
| 4 | Q-1$(R_5=CH_3)$ | - | H | H | $CH_3$ | $CH_3$ | dec. 146 |
| 4 | Q-1$(R_5=CH_3)$ | - | H | H | Cl | $OCH_3$ | dec. 185 |
| 4 | Q-7$(R_5,R_6=CH_3)$ | - | H | H | $OCH_3$ | $OCH_3$ | |
| 4 | Q-7$(R_5,R_6=CH_3)$ | - | H | H | $CH_3$ | $OCH_3$ | |
| 4 | Q-7$(R_5,R_6=CH_3)$ | - | H | H | $CH_3$ | $CH_3$ | |
| 4 | Q-7$(R_5,R_6=CH_3)$ | - | H | H | Cl | $OCH_3$ | |
| 5 | Q-15$(R_7=CH_3)$ | - | H | H | $OCH_3$ | $OCH_3$ | |
| 5 | Q-15$(R_7=CH_3)$ | - | H | H | $CH_3$ | $OCH_3$ | |
| 5 | Q-15$(R_7=CH_3)$ | - | H | H | $CH_3$ | $CH_3$ | |
| 5 | Q-15$(R_7=CH_3)$ | - | H | H | Cl | $OCH_3$ | |
| 1 | Q-1$(R_5=CH_3)$ | - | H | H | $OCH_3$ | $OCH_2CH=CH_2$ | |
| 1 | Q-1$(R_5=H)$ | - | H | H | $OCH_3$ | $OCH_3$ | |
| 1 | Q-1$(R_5=H)$ | - | H | H | $CH_3$ | $OCH_3$ | |
| 1 | Q-1$(R_5=H)$ | - | H | H | $CH_3$ | $CH_3$ | |
| 1 | Q-1$(R_5=H)$ | - | H | H | Cl | $OCH_3$ | |
| 1 | Q-15$(R_7=H)$ | - | H | H | $OCH_3$ | $OCH_3$ | |
| 1 | Q-18$(R_6=CH_3)$ | - | H | H | $OCH_3$ | $OCH_3$ | |
| 1 | Q-18$(R_6=CH_3)$ | - | H | H | $CH_3$ | $OCH_3$ | |
| 1 | Q-18$(R_6=CH_3)$ | - | H | H | $CH_3$ | $CH_3$ | |
| 1 | Q-18$(R_6=CH_3)$ | - | H | H | Cl | $OCH_3$ | |
| 1 | Q-19 | - | H | H | $OCH_3$ | $OCH_3$ | |
| 1 | Q-19 | - | H | H | $CH_3$ | $OCH_3$ | |
| 1 | Q-19 | - | H | H | $CH_3$ | $CH_3$ | |

Table 3 (Continued)

| J | $Q_1$ | E | R | $R_1$ | X | Y | m.p.°C |
|---|---|---|---|---|---|---|---|
| 1 | Q-19 | – | H | H | Cl | $OCH_3$ | |
| 1 | Q-15 ($R_7$=$CH_2CH$=$CH_2$) | – | H | H | $OCH_3$ | $OCH_3$ | |
| 1 | Q-15($R_7$=$CF_3$) | – | H | H | $OCH_3$ | $OCH_3$ | |
| 1 | Q-15($R_7$=$CF_2CF_2CF_3$) | – | H | H | $OCH_3$ | $OCH_3$ | |
| 1 | Q-21 | – | H | H | $OCH_3$ | $OCH_3$ | |
| 1 | Q-21 | – | H | H | $CH_3$ | $OCH_3$ | |
| 1 | Q-21 | – | H | H | $CH_3$ | $CH_3$ | |
| 1 | Q-21 | – | H | H | Cl | $OCH_3$ | |
| 1 | Q-22 | – | H | H | $OCH_3$ | $OCH_3$ | |
| 1 | Q-22 | – | H | H | $CH_3$ | $OCH_3$ | |
| 1 | Q-22 | – | H | H | $CH_3$ | $CH_3$ | |
| 1 | Q-22 | – | H | H | Cl | $OCH_3$ | |
| 1 | Q-23($R_6'$,$R_6''$=$CH_3$) | – | H | H | $OCH_3$ | $OCH_3$ | |
| 1 | Q-23($R_6'$,$R_6''$=$CH_3$) | – | H | H | $CH_3$ | $OCH_3$ | |
| 1 | Q-23($R_6'$,$R_6''$=$CH_3$) | – | H | H | $CH_3$ | $CH_3$ | |
| 1 | Q-23($R_6'$,$R_6''$=$CH_3$) | – | H | H | Cl | $OCH_3$ | |
| 1 | Q-23($R_6'$=H) | – | H | H | $OCH_3$ | $OCH_3$ | |
| 1 | Q-23($R_6'$=H) | – | H | H | $CH_3$ | $OCH_3$ | |
| 1 | Q-23($R_6'$=H) | – | H | H | $CH_3$ | $CH_3$ | |
| 1 | Q-23($R_6'$=H) | – | H | H | Cl | $OCH_3$ | |
| 1 | Q-1($R_5$=$CH_3$) | – | H | 5-$CH_2CN$ | $OCH_3$ | $OCH_3$ | |
| 1 | Q-1($R_5$=$CH_3$) | – | H | 5-$CH_2CN$ | $OCH_3$ | $CH_3$ | |
| 1 | Q-1($R_5$=$CH_3$) | – | H | 5-$CH_2CN$ | Cl | $OCH_3$ | |
| 1 | Q-1($R_5$=$CH_3$) | – | H | 5-$CH_2CN$ | $CH_3$ | $CH_3$ | |
| 1 | Q-1($R_5$=$CH_3$) | – | H | 5-$CH_2OCH_3$ | $OCH_3$ | $OCH_3$ | |
| 1 | Q-1($R_5$=$CH_3$) | – | H | 5-$CH_2SCH_3$ | $OCH_3$ | $OCH_3$ | |
| 1 | Q-17($R_6$=$CH_3$) | – | H | H | $OCH_3$ | $OCH_3$ | |
| 1 | Q-1($R_5$=$C_2H_5$) | – | H | 5-$CH_2SCH_3$ | $OCH_3$ | $OCH_3$ | |
| 1 | Q-1($R_5$=n-$C_3H_7$) | – | H | 5-$CH_2SCH_3$ | $OCH_3$ | $OCH_3$ | |
| 2a | Q-20 | – | H | H | $OCH_3$ | $OCH_3$ | |
| 2a | Q-20 | – | H | H | $CH_3$ | $OCH_3$ | |
| 2a | Q-20 | – | H | H | $CH_3$ | $CH_3$ | |

Table 3 (Continued)

| J | $Q_1$ | E | R | $R_1$ | X | Y | m.p.°C |
|---|---|---|---|---|---|---|---|
| 2a | Q-20 | - | H | H | Cl | $OCH_3$ | |
| 2b | Q-20 | - | H | H | $OCH_3$ | $OCH_3$ | |
| 2b | Q-20 | - | H | H | $CH_3$ | $OCH_3$ | |
| 2b | Q-20 | - | H | H | $CH_3$ | $CH_3$ | |
| 2b | Q-20 | - | H | H | Cl | $OCH_3$ | |
| 2c | Q-20 | - | H | H | $OCH_3$ | $OCH_3$ | |
| 3a | Q-20 | - | H | H | $OCH_3$ | $OCH_3$ | |
| 3b | Q-20 | - | H | H | $OCH_3$ | $OCH_3$ | |
| 3c | Q-20 | - | H | H | $OCH_3$ | $OCH_3$ | |
| 5 | Q-20 | - | H | H | $OCH_3$ | $OCH_3$ | |
| 1 | Q-10 | O | H | H | $OCF_2H$ | $OCH_3$ | 164-166 |
| 1 | Q-17($R_6$=$CH_3$) | - | H | H | $CH_3$ | $OCH_3$ | |
| 5 | Q-24($R_6'$=H) | - | H | H | $OCH_3$ | $OCH_3$ | 124-128 |
| 5 | Q-24($R_6'$=H) | - | H | H | $CH_3$ | $OCH_3$ | 110-115 |
| 5 | Q-24($R_6'$=H) | - | H | H | $CH_3$ | $CH_3$ | 95-100 |
| 5 | Q-24($R_6'$=$CH_3$) | - | H | H | $OCH_3$ | $OCH_3$ | 220-230 |
| 5 | Q-24($R_6'$=$CH_3$) | - | H | H | $OCH_3$ | $CH_3$ | 118-120 |
| 5 | Q-24($R_6'$=$CH_3$) | - | H | H | $CH_3$ | $CH_3$ | >240 |
| 5 | Q-24($R_6'$=$CH_3$) | - | H | H | Cl | $OCH_3$ | 236-240 |
| 2a | Q-1($R_5$=H) | - | H | H | $CH_3$ | $CH_3$ | 198-201 |
| 2a | Q-1($R_5$=H) | - | H | H | $CH_3$ | $OCH_3$ | 229-231 |
| 2a | Q-1($R_5$=H) | - | H | H | $OCH_3$ | $OCH_3$ | 215-220 |
| 2a | Q-1($R_5$=H) | - | H | H | Cl | $OCH_3$ | 212-215 |
| 1 | Q-1($R_5$=$CH_2CH(Cl)CH_2Cl$) | - | H | H | $OCH_3$ | $OCH_3$ | 75-79 |
| 1 | Q-1($R_5$=$CH_2CH_2CH_2CH_3$) | - | H | H | $OCH_3$ | $CH_3$ | 168-173 |
| 1 | Q-1($R_5$=$CH_2CH_2CH_2CH_3$) | - | H | H | $CH_3$ | $CH_3$ | 163-166 |
| 1 | Q-1($R_5$=$CH_2CH_2CH_2CH_3$) | - | H | H | Cl | $OCH_3$ | 162-170 |
| 1 | Q-1($R_5$=$CH_2CO_2CH_3$) | - | H | H | $CH_3$ | $CH_3$ | 186-191 |
| 1 | Q-1($R_5$=$CH_2CO_2CH_3$) | - | H | H | Cl | $OCH_3$ | 78-97 |
| 1 | Q-1($R_5$=$CH_2CO_2CH_3$) | - | H | H | $CH_3$ | $OCH_3$ | 168-171 |
| 1 | Q-1($R_5$=$CH_2CH_2OCH_3$) | - | H | H | $CH_3$ | $OCH_3$ | 166-170 |
| 1 | Q-1($R_5$=$CH_2CH_2OCH_3$) | - | H | H | $CH_3$ | $CH_3$ | 189-191 |

Table 3 (Continued)

| J | $Q_1$ | E | R | $R_1$ | X | Y | m.p.°C |
|---|---|---|---|---|---|---|---|
| 1 | Q-1($R_5$=$CH_2CH_2OCH_3$) | - | H | H | Cl | $OCH_3$ | 171-173 |
| 1 | Q-1($R_5$=$CH_3$) | - | H | 5-Cl | $CH_3$ | $OCH_3$ | 177-179 |
| 1 | Q-1($R_5$=$CH_3$) | - | H | 5-Cl | $CH_3$ | $CH_3$ | 196-199 |
| 1 | Q-1($R_5$=$CH_3$) | - | H | 5-Cl | Cl | $OCH_3$ | 198-204 |
| 1 | Q-1($R_5$=$CH_2OCH_3$) | - | H | H | $OCF_2H$ | $CH_3$ | 160-165 |
| 1 | Q-1($R_5$=$CH_2OCH_3$) | - | H | H | $OCF_2H$ | $OCH_3$ | 135-140 |
| 1 | Q-1($R_5$=$CH_2OCH_3$) | - | H | H | Cl | $OCF_2H$ | 140-146 |
| 1 | Q-1($R_5$=$CH_3$) | - | H | H | $OCF_2H$ | $CH_3$ | 166-170 |
| 1 | Q-1($R_5$=$CH_3$) | - | H | 6-$CH_3$ | $OCH_3$ | $OCH_3$ | |
| 1 | Q-1($R_5$=$CH_3$) | - | H | 6-$CH_3$ | $CH_3$ | $OCH_3$ | |
| 1 | Q-1($R_5$=$CH_3$) | - | H | 6-$CH_3$ | $CH_3$ | $CH_3$ | |
| 1 | Q-1($R_5$=$CH_3$) | - | H | 6-$CH_3$ | Cl | $OCH_3$ | |
| 1 | Q-1($R_5$=$CH_3$) | - | H | 6-Cl | $OCH_3$ | $OCH_3$ | |
| 1 | Q-1($R_5$=$CH_3$) | - | H | 6-Cl | $CH_3$ | $OCH_3$ | |
| 1 | Q-1($R_5$=$CH_3$) | - | H | 6-Cl | $CH_3$ | $CH_3$ | |
| 1 | Q-1($R_5$=$CH_3$) | - | H | 6-Cl | Cl | $OCH_3$ | |
| 1 | Q-1($R_5$=$CH_3$) | - | H | 5-$SCH_3$ | $CH_3$ | $OCH_3$ | |
| 1 | Q-1($R_5$=$CH_3$) | - | H | 5-$SCH_3$ | $CH_3$ | $CH_3$ | |
| 1 | Q-1($R_5$=$CH_3$) | - | H | 5-$SCH_3$ | Cl | $OCH_3$ | |
| 1 | Q-1($R_5$=$CH_3$) | - | H | 5-$OCH_3$ | $CH_3$ | $OCH_3$ | |
| 1 | Q-1($R_5$=$CH_3$) | - | H | 5-$OCH_3$ | $CH_3$ | $CH_3$ | |
| 1 | Q-1($R_5$=$CH_3$) | - | H | 5-$OCH_3$ | Cl | $OCH_3$ | |
| 1 | Q-1($R_5$=$CH_3$) | - | H | 5-$OCH_2CF_3$ | $OCH_3$ | $OCH_3$ | |
| 1 | Q-1($R_5$=$CH_3$) | - | H | 5-$OCH_2CF_3$ | $OCH_3$ | $CH_3$ | |
| 1 | Q-1($R_5$=$CH_3$) | - | H | 5-$OCH_2CF_3$ | $CH_3$ | $CH_3$ | |
| 1 | Q-1($R_5$=$CH_3$) | - | H | 5-$OCH_2CF_3$ | Cl | $OCH_3$ | |
| 1 | Q-1($R_5$=$CH_3$) | - | H | 5-$CH_2N_3$ | $OCH_3$ | $OCH_3$ | |
| 1 | Q-1($R_5$=$CH_3$) | - | H | 5-$CH_2SO_2CH_3$ | $OCH_3$ | $OCH_3$ | |
| 1 | Q-25($R_6$=$CH_3$) | - | H | H | $OCH_3$ | $OCH_3$ | |
| 1 | Q-26 | - | H | H | $OCH_3$ | $OCH_3$ | |
| 1 | Q-27($R_6$=$CH_3$) | - | H | H | $OCH_3$ | $OCH_3$ | |
| 2a | Q-28 | - | H | H | $OCH_3$ | $OCH_3$ | |

Table 3 (Continued)

| J | $Q_1$ | E | R | $R_1$ | X | Y | m.p.°C |
|---|---|---|---|---|---|---|---|
| 2a | Q-29 | – | H | H | $OCH_3$ | $OCH_3$ | |
| 2a | Q-30 | – | H | H | $OCH_3$ | $OCH_3$ | |
| 2b | Q-1($R_5$=$CH_2CH$=$CH_2$) | – | H | H | $OCH_3$ | $OCH_2CH_2OCH_3$ | |
| 2b | Q-1($R_5$=$CH_2CH$=$CH_2$) | – | H | H | $OCH_3$ | $OCH_3$ | |
| 2b | Q-1($R_5$=$CH_2CH$=$CH_2$) | – | H | H | $CH_3$ | $OCH_3$ | |
| 2b | Q-1($R_5$=$CH_2CH$=$CH_2$) | – | H | H | $CH_3$ | $CH_3$ | |
| 2b | Q-1($R_5$=$CH_2CH$=$CH_2$) | – | H | H | Cl | $OCH_3$ | |

## Table 4

## General Formula 2

| J | $Q_1$ | E | R | $R_1$ | X | Y | m.p.°C |
|---|---|---|---|---|---|---|---|
| 1 | Q-1($R_5$=$CH_3$) | – | H | H | $OCH_3$ | $OCH_3$ | 206-209 |
| 1 | Q-1($R_5$=$CH_3$) | – | H | H | $CH_3$ | $OCH_3$ | 198-200 |
| 1 | Q-1($R_5$=$CH_3$) | – | H | H | $OC_2H_5$ | $NHCH_3$ | 181 dec. |
| 1 | Q-1($R_5$=$CH_3$) | – | H | H | $OCH_3$ | cyclopropyl | 209-211 |
| 1 | Q-1($R_5$=$CH_3$) | – | H | H | $OCH_2CH_2F$ | $CH_3$ | |
| 1 | Q-1($R_5$=$CH_3$) | – | H | H | $OCH_2CH_2F$ | $OCH_3$ | |
| 1 | Q-1($R_5$=$CH_3$) | – | H | H | $OCH_2CF_3$ | $NHCH_3$ | 196 dec. |
| 1 | Q-1($R_5$=$CH_3$) | – | H | H | $OCH_2CF_3$ | $OCH_3$ | 192-194 |
| 1 | Q-1($R_5$=$CH_3$) | – | H | H | $CH_3$ | $OCH_2C\equiv CH$ | 124-150 |
| 1 | Q-1($R_5$=$C_2H_5$) | – | H | H | $OCH_3$ | $CH_3$ | 185-187 |
| 1 | Q-1($R_5$=$C_2H_5$) | – | H | H | $OCH_3$ | $OCH_3$ | 192-194 |
| 1 | Q-1($R_5$=$C_2H_5$) | – | H | H | $OC_2H_5$ | $NHCH_3$ | |
| 1 | Q-1($R_5$=n-$C_3H_7$) | – | H | H | $OCH_3$ | $CH_3$ | 173-175 |
| 1 | Q-1($R_5$=n-$C_3H_7$) | – | H | H | $OCH_3$ | $OCH_3$ | 178-180 |
| 1 | Q-1($R_5$=$CH(CH_3)_2$) | – | H | H | $CH_3$ | $OCH_3$ | 198-200 |
| 1 | Q-1($R_5$=n-$C_4H_9$) | – | H | H | $CH_3$ | $OCH_3$ | 145-149 |
| 1 | Q-1($R_5$=t-butyl) | – | H | H | $OCH_3$ | $CH_3$ | |
| 1 | Q-1($R_5$=$CH_2CH(CH_3)_2$ | – | H | H | $CH_3$ | $OCH_3$ | |
| 1 | Q-1($R_5$=$CH_2CH$=$CH_2$) | – | H | H | $CH_3$ | $OCH_3$ | 173-174 |
| 1 | Q-1($R_5$=$CH_2CH$=$CH_2$) | – | H | H | $OCH_3$ | $OCH_3$ | 174-177 |
| 1 | Q-1($R_5$=$CH_2CH$=$CHCH_3$) | – | H | H | $CH_3$ | $OCH_3$ | |
| 1 | Q-1($R_5$=$CH_2C\equiv CH$) | – | H | H | $CH_3$ | $OCH_3$ | |
| 1 | Q-1($R_5$=$CH_2C\equiv CCH_3$) | – | H | H | $OCH_3$ | $CH_3$ | |
| 1 | Q-1($R_5$=$CH_2CN$) | – | H | H | $OCH_3$ | $CH_3$ | 176-179 |
| 1 | Q-1($R_5$=$CH_2CN$) | – | H | H | $OCH_3$ | $OCH_3$ | 170-175 |
| 1 | Q-1($R_5$=$CH_2CN$) | – | H | H | $OC_2H_5$ | $NHCH_3$ | |
| 1 | Q-1($R_5$=$CH_2CO_2CH_3$) | – | H | H | $OCH_3$ | $CH_3$ | 88-106 |
| 1 | Q-1($R_5$=$CH_2CO_2C_2H_5$) | – | H | H | $CH_3$ | $OCH_3$ | |
| 1 | Q-1($R_5$=$CH(CH_3)CO_2CH_3$) | – | H | H | $OCH_3$ | $OCH_3$ | |
| 1 | Q-1($R_5$=$CH(CH_3)CO_2C_2H_5$) | – | H | H | $CH_3$ | $OCH_3$ | |
| 1 | Q-1($R_5$=$CF_2H$) | – | H | H | $CH_3$ | $OCH_3$ | |

Table 4 (Continued)

| J | $Q_1$ | E | R | $R_1$ | X | Y | m.p.°C |
|---|---|---|---|---|---|---|---|
| 1 | Q-1($R_5$=$CF_2H$) | – | H | H | $OCH_3$ | $OCH_3$ | |
| 1 | Q-1($R_5$=$CF_2H$) | – | H | H | $OC_2H_5$ | $NHCH_3$ | |
| 1 | Q-1($R_5$=$CF_2H$) | – | H | H | $OCH_3$ | cyclopropyl | |
| 1 | Q-1($R_5$=$CF_2H$) | – | H | H | $OCH_2CF_3$ | $OCH_3$ | |
| 1 | Q-1($R_5$=$CF_2H$) | – | H | H | $OCH_2CH_2F$ | $CH_3$ | |
| 1 | Q-1($R_5$=$CH_2CH_2F$) | – | H | H | $CH_3$ | $OCH_3$ | |
| 1 | Q-1($R_5$=$CH_2CH_2Cl$) | – | H | H | $CH_3$ | $OCH_3$ | |
| 1 | Q-1($R_5$=$CH_2CH_2CH_2F$) | – | H | H | $CH_3$ | $OCH_3$ | |
| 1 | Q-1($R_5$=$CH_2CHF_2$) | – | H | H | $CH_3$ | $OCH_3$ | |
| 1 | Q-1($R_5$=$CH_2CF_3$) | – | H | H | $CH_3$ | $OCH_3$ | |
| 1 | Q-1($R_5$=$CH_2OCH_3$) | – | H | H | $OCH_3$ | $CH_3$ | 180-182 |
| 1 | Q-1($R_5$=$CH_2OCH_3$) | – | H | H | $OCH_3$ | $OCH_3$ | 173-178 |
| 1 | Q-1($R_5$=$CH_2OCH_3$) | – | H | H | $OC_2H_5$ | $NHCH_3$ | |
| 1 | Q-1($R_5$=$CH_2OC_2H_5$) | – | H | H | $CH_3$ | $OCH_3$ | |
| 1 | Q-1($R_5$=$CH_2CH_2OCH_3$) | – | H | H | $CH_3$ | $OCH_3$ | 158-161 |
| 1 | Q-1($R_5$=$CH_2CH_2OC_2H_5$) | – | H | H | $CH_3$ | $OCH_3$ | |
| 1 | Q-2($R_6$=$CH_3$) | – | H | H | $CH_3$ | $OCH_3$ | |
| 1 | Q-2($R_6$=$C_2H_5$) | – | H | H | $CH_3$ | $OCH_3$ | |
| 1 | Q-2($R_6$=$CH(CH_3)_2$) | – | H | H | $CH_3$ | $OCH_3$ | |
| 1 | Q-3($R_6$=$CH_3$) | – | H | H | $CH_3$ | $OCH_3$ | |
| 1 | Q-4($R_6$=$CH_3$) | – | H | H | $CH_3$ | $OCH_3$ | |
| 1 | Q-5($R_6$=$CH_3$) | – | H | H | $CH_3$ | $OCH_3$ | |
| 1 | Q-5($R_6$=n-$C_3H_7$) | – | H | H | $CH_3$ | $OCH_3$ | |
| 1 | Q-6($R_6$=$CH_3$) | – | H | H | $CH_3$ | $OCH_3$ | |
| 1 | Q-7($R_5$,$R_6$=$CH_3$) | – | H | H | $CH_3$ | $OCH_3$ | |
| 1 | Q-7($R_5$,$R_6$=$CH_3$) | – | H | H | $OCH_3$ | $OCH_3$ | |
| 1 | Q-7($R_5$,$R_6$=$CH_3$) | – | H | H | $OC_2H_5$ | $NHCH_3$ | |
| 1 | Q-8 | – | H | H | $CH_3$ | $OCH_3$ | |
| 1 | Q-9 | – | H | H | $CH_3$ | $OCH_3$ | |
| 1 | Q-9 | – | H | H | $OCH_3$ | $OCH_3$ | |
| 1 | Q-9 | – | H | H | $OC_2H_5$ | $NHCH_3$ | |
| 1 | Q-9 | – | H | H | $OCH_2CF_3$ | $OCH_3$ | |

Table 4 (Continued)

| J | $Q_1$ | E | R | $R_1$ | X | Y | m.p.°C |
|---|---|---|---|---|---|---|---|
| 1 | Q-9 | – | H | H | $OCH_2CH_2F$ | $CH_3$ | |
| 1 | Q-10 | – | H | H | $CH_3$ | $OCH_3$ | |
| 1 | Q-10 | – | H | H | $OCH_3$ | $OCH_3$ | |
| 1 | Q-10 | – | H | H | $OC_2H_5$ | $NHCH_3$ | |
| 1 | Q-10 | – | H | H | $OCH_2CF_3$ | $OCH_3$ | |
| 1 | Q-10 | – | H | H | $OCH_2CH_2F$ | $CH_3$ | |
| 1 | Q-11 | – | H | H | $CH_3$ | $OCH_3$ | |
| 1 | Q-12 | – | H | H | $CH_3$ | $OCH_3$ | |
| 1 | Q-13 | – | H | H | $CH_3$ | $OCH_3$ | |
| 1 | Q-14($R_6$=$CH_3$) | – | H | H | $CH_3$ | $OCH_3$ | |
| 1 | Q-15($R_7$=$CH_3$) | – | H | H | $CH_3$ | $OCH_3$ | |
| 1 | Q-15($R_7$=$CH_3$) | – | H | H | $OCH_3$ | $OCH_3$ | |
| 1 | Q-15($R_7$=$CH_3$) | – | H | H | $OC_2H_5$ | $NHCH_3$ | |
| 1 | Q-15($R_7$=$CH_3$) | – | H | H | $OCH_2CF_3$ | $OCH_3$ | |
| 1 | Q-15($R_7$=$CH_3$) | – | H | H | $OCH_2CH_2F$ | $CH_3$ | |
| 1 | Q-15($R_7$=$C_2H_5$) | – | H | H | $CH_3$ | $OCH_3$ | |
| 1 | Q-15($R_7$=$C_2H_5$) | – | H | H | $OCH_3$ | $OCH_3$ | |
| 1 | Q-15($R_7$=$C_2H_5$) | – | H | H | $OCH_2CF_3$ | $OCH_3$ | |
| 1 | Q-15($R_7$=$C_2H_5$) | – | H | H | $OC_2H_5$ | $NHCH_3$ | |
| 1 | Q-15($R_7$=$C_2H_5$) | – | H | H | $OCH_2CH_2F$ | $CH_3$ | |
| 1 | Q-15($R_7$=n-$C_3H_7$) | – | H | H | $CH_3$ | $OCH_3$ | |
| 1 | Q-15($R_7$=$CH(CH_3)_2$) | – | H | H | $CH_3$ | $OCH_3$ | |
| 1 | Q-15($R_7$=n-$C_4H_9$) | – | H | H | $CH_3$ | $OCH_3$ | |
| 1 | Q-15($R_7$=t-butyl) | – | H | H | $CH_3$ | $OCH_3$ | |
| 1 | Q-15($R_7$=t-butyl) | – | H | H | $OCH_3$ | $OCH_3$ | |
| 1 | Q-15($R_7$=t-butyl) | – | H | H | $OC_2H_5$ | $NHCH_3$ | |
| 1 | Q-15($R_7$=t-butyl) | – | H | H | $OCH_2CF_3$ | $NHCH_3$ | |
| 1 | Q-15($R_7$=t-butyl) | – | H | H | $OCH_2CH_2F$ | $CH_3$ | |
| 1 | Q-16($R_7$=$CH_3$) | – | H | H | $CH_3$ | $OCH_3$ | |
| 1 | Q-1($R_5$=$CH_3$) | O | H | H | $CH_3$ | $OCH_3$ | 68-73 |
| 1 | Q-1($R_5$=$CH_3$) | O | H | H | $OCH_3$ | $OCH_3$ | 160-164 |
| 1 | Q-1($R_5$=$CH_3$) | O | H | H | $OC_2H_5$ | $NHCH_3$ | |

Table 4 (Continued)

| J | $Q_1$ | E | R | $R_1$ | X | Y | m.p.°C |
|---|---|---|---|---|---|---|---|
| 1 | Q-1($R_5$=$CH_3$) | O | H | H | $OCH_2CF_3$ | $OCH_3$ | |
| 1 | Q-1($R_5$=$CH_3$) | O | H | H | $OCH_2CH_2Cl$ | $CH_3$ | |
| 1 | Q-1($R_5$=$C_2H_5$) | O | H | H | $CH_3$ | $OCH_3$ | |
| 1 | Q-1($R_5$=n-$C_3H_7$) | O | H | H | $CH_3$ | $OCH_3$ | |
| 1 | Q-1($R_5$=$CH(CH_3)_2$) | O | H | H | $CH_3$ | $OCH_3$ | |
| 1 | Q-1($R_5$=$CF_2H$) | O | H | H | $CH_3$ | $OCH_3$ | |
| 1 | Q-1($R_5$=$CF_2H$) | O | H | H | $OCH_3$ | $OCH_3$ | |
| 1 | Q-1($R_5$=$CF_2H$) | O | H | H | $OC_2H_5$ | $NHCH_3$ | |
| 1 | Q-1($R_5$=$CF_2H$) | O | H | H | $OCH_2CF_3$ | $OCH_3$ | |
| 1 | Q-1($R_5$=$CF_2H$) | O | H | H | $CH_2CH_2F$ | $CH_3$ | |
| 1 | Q-1($R_5$=$CH_2CN$) | O | H | H | $CH_3$ | $OCH_3$ | |
| 1 | Q-1($R_5$=$CH_2CH$=$CH_2$) | O | H | H | $CH_3$ | $OCH_3$ | |
| 1 | Q-7($R_5$,$R_6$=$CH_3$) | O | H | H | $CH_3$ | $OCH_3$ | |
| 1 | Q-9 | O | H | H | $CH_3$ | $OCH_3$ | |
| 1 | Q-10 | O | H | H | $OCH_3$ | $CH_3$ | 76-84 |
| 1 | Q-10 | O | H | H | $OCH_3$ | $OCH_3$ | |
| 1 | Q-10 | O | H | H | $OC_2H_5$ | $NHCH_3$ | |
| 1 | Q-10 | O | H | H | $OCH_2CF_3$ | $OCH_3$ | |
| 1 | Q-15($R_7$=$CH_3$) | O | H | H | $CH_3$ | $OCH_3$ | |
| 1 | Q-1($R_5$=$CH_3$) | $CH_2$ | H | H | $CH_3$ | $OCH_3$ | 108-114 |
| 1 | Q-7($R_5$,$R_6$=$CH_3$) | $CH_2$ | H | H | $CH_3$ | $OCH_3$ | |
| 1 | Q-9 | $CH_2$ | H | H | $CH_3$ | $OCH_3$ | |
| 1 | Q-10 | $CH_2$ | H | H | $CH_3$ | $OCH_3$ | |
| 1 | Q-15 | $CH_2$ | H | H | $CH_3$ | $OCH_3$ | |
| 1 | Q-1($R_5$=$CH_3$) | - | $CH_3$ | H | $CH_3$ | $OCH_3$ | |
| 1 | Q-1($R_5$=$CH_3$) | - | H | 3-F | $CH_3$ | $OCH_3$ | |
| 1 | Q-1($R_5$=$CH_3$) | - | H | 5-Cl | $CH_3$ | $OCH_3$ | 195-198 |
| 1 | Q-1($R_5$=$CH_3$) | - | H | 6-Br | $CH_3$ | $OCH_3$ | |
| 1 | Q-1($R_5$=$CH_3$) | - | H | 5-$CH_3$ | $CH_3$ | $OCH_3$ | |
| 1 | Q-1($R_5$=$CH_3$) | - | H | 6-$C_2H_5$ | $CH_3$ | $OCH_3$ | |
| 1 | Q-1($R_5$=$CH_3$) | - | H | 5-$OCH_3$ | $CH_3$ | $OCH_3$ | |
| 1 | Q-1($R_5$=$CH_3$) | - | H | 3-$OC_2H_5$ | $CH_3$ | $OCH_3$ | |

Table 4 (Continued)

| J | $Q_1$ | E | R | $R_1$ | X | Y | m.p.°C |
|---|---|---|---|---|---|---|---|
| 1 | Q-1($R_5$=$CH_3$) | - | H | 6-$OCH(CH_3)_2$ | $CH_3$ | $OCH_3$ | |
| 1 | Q-1($R_5$=$CH_3$) | - | H | 5-$SCH_3$ | $CH_3$ | $OCH_3$ | |
| 1 | Q-1($R_5$=$CH_3$) | - | H | 6-$SC_2H_5$ | $CH_3$ | $OCH_3$ | |
| 1 | Q-1($R_5$=$CH_3$) | - | H | 3-$SCH(CH_3)_2$ | $CH_3$ | $OCH_3$ | |
| 1 | Q-1($R_5$=$CH_3$) | - | H | 5-$CH_2F$ | $CH_3$ | $OCH_3$ | |
| 1 | Q-1($R_5$=$CH_3$) | - | H | 5-$CH_2CH_2Br$ | $CH_3$ | $OCH_3$ | |
| 1 | Q-1($R_5$=$CH_3$) | - | H | 6-$OCF_2H$ | $CH_3$ | $OCH_3$ | |
| 1 | Q-1($R_5$=$CH_3$) | - | H | 6-$OCH_2CH_2Br$ | $CH_3$ | $OCH_3$ | |
| 1 | Q-1($R_5$=$CH_3$) | - | H | 6-$OCH(CH_3)CH_2Cl$ | $CH_3$ | $OCH_3$ | |
| 1 | Q-1($R_5$=$CH_3$) | - | H | 5-$SCH_2F$ | $CH_3$ | $OCH_3$ | |
| 1 | Q-1($R_5$=$CH_3$) | - | H | 6-$SCH_2CH_2Br$ | $CH_3$ | $OCH_3$ | |
| 1 | Q-1($R_5$=$CH_3$) | - | H | 6-$SCH(CH_3)CH_2Cl$ | $CH_3$ | $OCH_3$ | |
| 1 | Q-1($R_5$=$CH_3$) | - | H | H | $C_2H_5$ | $OCH_3$ | |
| 1 | Q-1($R_5$=$CH_3$) | - | H | H | $CH_2F$ | $OCH_3$ | |
| 1 | Q-1($R_5$=$CH_3$) | - | H | H | $CF_3$ | $OCH_3$ | |
| 1 | Q-1($R_5$=$CH_3$) | - | H | H | $OCH_2CHF_2$ | $CH_3$ | |
| 1 | Q-1($R_5$=$CH_3$) | - | H | H | $CH_2Cl$ | $OCH_3$ | |
| 1 | Q-1($R_5$=$CH_3$) | - | H | H | $CH_2Br$ | $OCH_3$ | |
| 1 | Q-1($R_5$=$CH_3$) | - | H | H | H | $OCH_3$ | |
| 1 | Q-1($R_5$=$CH_3$) | - | H | H | $OCH_3$ | $SCH_3$ | |
| 1 | Q-1($R_5$=$CH_3$) | - | H | H | $OCH_3$ | C≡CH | |
| 1 | Q-1($R_5$=$CH_3$) | - | H | H | $OCH_3$ | $N(CH_3)_2$ | |
| 1 | Q-1($R_5$=$CH_3$) | - | H | H | $OCH_3$ | $C_2H_5$ | |
| 1 | Q-1($R_5$=$CH_3$) | - | H | H | $OCH_3$ | $CH_2OCH_3$ | |
| 1 | Q-1($R_5$=$CH_3$) | - | H | H | $OCH_3$ | $CH(OCH_3)_2$ | |
| 2a | Q-1($R_5$=$CH_3$) | - | H | H | $CH_3$ | $OCH_3$ | 188-190 |
| 2a | Q-1($R_5$=$CH_3$) | - | H | H | $OCH_3$ | $OCH_3$ | 168-171 |
| 2a | Q-1($R_5$=$CH_3$) | - | H | H | $OC_2H_5$ | $NHCH_3$ | |
| 2a | Q-1($R_5$=$CH_3$) | - | H | H | $OCH_3$ | cyclopropyl | |
| 2a | Q-1($R_5$=$C_2H_5$) | - | H | H | $CH_3$ | $OCH_3$ | |
| 2a | Q-1($R_5$=n-$C_3H_7$) | - | H | H | $CH_3$ | $OCH_3$ | |
| 2a | Q-1($R_5$=$CH_2CN$) | - | H | H | $CH_3$ | $OCH_3$ | 190-193 |

Table 4 (Continued)

| J | $Q_1$ | E | R | $R_1$ | X | Y | m.p.°C |
|---|---|---|---|---|---|---|---|
| 2a | Q-1($R_5$=$CH_2CH$=$CH_2$) | - | H | H | $CH_3$ | $OCH_3$ | 181-183 |
| 2a | Q-1($R_5$=$CF_2H$) | - | H | H | $CH_3$ | $OCH_3$ | |
| 2a | Q-1($R_5$=$CF_2H$) | - | H | H | $OCH_3$ | $OCH_3$ | |
| 2a | Q-1($R_5$=$CF_2H$) | - | H | H | $OC_2H_5$ | $NHCH_3$ | |
| 2a | Q-7($R_5$,$R_6$=$CH_3$) | - | H | H | $OCH_3$ | $CH_3$ | |
| 2a | Q-15($R_7$=$CH_3$) | - | H | H | $CH_3$ | $OCH_3$ | |
| 2a | Q-15($R_7$=$CH_3$) | - | H | H | $OCH_3$ | $OCH_3$ | |
| 2a | Q-15($R_7$=$CH_3$) | - | H | H | $OC_2H_5$ | $NHCH_3$ | |
| 2a | Q-15($R_7$=$CH_3$) | - | H | H | $OCH_2CF_3$ | $OCH_3$ | |
| 2a | Q-15($R_7$=$CH_3$) | - | H | H | $OCH_2CH_2F$ | $CH_3$ | |
| 2b | Q-1($R_5$=$CH_3$) | - | H | H | $CH_3$ | $OCH_3$ | |
| 2b | Q-7($R_5$,$R_6$=$CH_3$) | - | H | H | $CH_3$ | $OCH_3$ | |
| 2c | Q-1($R_5$=$CH_3$) | - | H | H | $CH_3$ | $OCH_3$ | |
| 3a | ($R_2$=$CH_3$)Q-1($R_5$=$CH_3$) | - | H | H | $CH_3$ | $OCH_3$ | |
| 3b | ($R_2$=$C_2H_5$)Q-1($R_5$=$CH_3$) | - | H | H | $CH_3$ | $OCH_3$ | |
| 3c | ($R_2$=$CH_3$)Q-1($R_5$=$CH_3$) | - | H | H | $CH_3$ | $OCH_3$ | |
| 3d | ($R_2$=$CH_3$)Q-1($R_5$=$CH_3$) | - | H | H | $CH_3$ | $OCH_3$ | |
| 3d | ($R_2$=$C_3H_7$)Q-1($R_5$=$CH_3$) | - | H | H | $CH_3$ | $OCH_3$ | |
| 3d | ($R_2$=$CH(CH_3)_2$)Q-1($R_5$=$CH_3$) | - | H | H | $CH_3$ | $OCH_3$ | |
| 4 | Q-1($R_5$=$CH_3$) | - | H | H | $CH_3$ | $OCH_3$ | dec. 168 |
| 5 | Q-15($R_7$=$CH_3$) | - | H | H | $CH_3$ | $OCH_3$ | |
| 1 | Q-18($R_6$=$CH_3$) | - | H | H | $CH_3$ | $OCH_3$ | |
| 1 | Q-18($R_6$=$CH_3$) | - | H | H | $OCH_3$ | $OCH_3$ | |
| 1 | Q-19 | - | H | H | $CH_3$ | $OCH_3$ | |
| 1 | Q-19 | - | H | H | $OCH_3$ | $OCH_3$ | |
| 1 | Q-15($R_7$=H) | - | H | H | $CH_3$ | $OCH_3$ | |
| 1 | Q-1($R_5$=H) | - | H | H | $CH_3$ | $OCH_3$ | |
| 2a | Q-20 | - | H | H | $CH_3$ | $OCH_3$ | |
| 2a | Q-20 | - | H | H | $OCH_3$ | $OCH_3$ | |
| 1 | Q-21 | - | H | H | $OCH_3$ | $OCH_3$ | |
| 1 | Q-22 | - | H | H | $OCH_3$ | $CH_3$ | |
| 1 | Q-23($R_6'$,$R_6''$=$CH_3$) | - | H | H | $OCH_3$ | $CH_3$ | |

Table 4 (Continued)

| J | $Q_1$ | E | R | $R_1$ | X | Y | m.p.°C |
|---|---|---|---|---|---|---|---|
| 1 | Q-1($R_5$=$CH_3$) | - | H | 5-$CH_2CN$ | $CH_3$ | $OCH_3$ | |
| 1 | Q-1($R_5$=$CH_3$) | - | H | 5-$CH_2OCH_3$ | $CH_3$ | $OCH_3$ | |
| 1 | Q-1($R_5$=$CH_3$) | - | H | 5-$CH_2SCH_3$ | $CH_3$ | $OCH_3$ | |
| 1 | Q-1($R_5$=$CH(CH_3)_2$) | - | H | H | $OCH_3$ | $OCH_3$ | 190-193 |
| 1 | Q-17($R_6$=$CH_3$) | - | H | H | $CH_3$ | $OCH_3$ | |
| 2a | Q-1($R_5$=H) | - | H | H | $CH_3$ | $OCH_3$ | 134-136 |
| 2a | Q-1($R_5$=H) | - | H | H | $OCH_3$ | $OCH_3$ | 137-138 |
| 2a | Q-1($R_5$=$CH_2CN$) | - | H | H | $OCH_3$ | $OCH_3$ | 182-185 |
| 2a | Q-1($R_5$=$C(O)CH_3$) | - | H | H | $OCH_3$ | $CH_3$ | 129-130 |
| 2a | Q-1($R_5$=$CH_2CH$=$CH_2$) | - | H | H | $OCH_3$ | $OCH_3$ | 156-159 |
| 1 | Q-1($R_5$=$CH(CH_3)_2$) | - | H | H | $OCH_3$ | $OCH_3$ | 190-193 |
| 1 | Q-1($R_5$=$CH_2CH_2CH_2CH_3$) | - | H | H | $OCH_3$ | $OCH_3$ | 156-162 |
| 1 | Q-1($R_5$=$CH_2CO_2CH_3$) | - | H | H | $OCH_3$ | $OCH_3$ | 163-173 |
| 1 | Q-1($R_5$=$CH_3$) | - | H | H | $CH_3$ | $CH_3$ | 219-221 |
| 1 | Q-1($R_5$=$CH_2CH_2OCH_3$) | - | H | H | $OCH_3$ | $OCH_3$ | 143-151 |
| 1 | Q-1($R_5$=$CH_3$) | $CH_2$ | H | H | $OCH_3$ | $OCH_3$ | 169-173 |
| 1 | Q-1($R_5$=$CH_3$) | - | H | 5-Cl | $OCH_3$ | $OCH_3$ | 198-200 |
| 4 | Q-1($R_5$=$CH_3$) | - | H | H | $OCH_3$ | $OCH_3$ | dec. 162 |
| 5 | Q-24($R_6'$=$CH_3$) | - | H | H | $OCH_3$ | $CH_3$ | 230-240 |
| 1 | Q-1($R_5$=$CH_3$) | - | H | 6-$CH_3$ | $OCH_3$ | $CH_3$ | |
| 1 | Q-1($R_5$=$CH_3$) | - | H | 6-$CH_3$ | $OCH_3$ | $OCH_3$ | |
| 1 | Q-1($R_5$=$CH_3$) | - | H | 6-Cl | $OCH_3$ | $CH_3$ | |
| 1 | Q-1($R_5$=$CH_3$) | - | H | 6-Cl | $OCH_3$ | $OCH_3$ | |
| 1 | Q-1($R_5$=$CH_3$) | - | H | 5-$SCH_3$ | $OCH_3$ | $OCH_3$ | |
| 1 | Q-1($R_5$=$CH_3$) | - | H | 5-$OCH_3$ | $OCH_3$ | $OCH_3$ | |
| 1 | Q-1($R_5$=$CH_3$) | - | H | 5-$OCH_2CF_3$ | $CH_3$ | $OCH_3$ | |
| 1 | Q-1($R_5$=$CH_3$) | - | H | 5-$OCH_2CF_3$ | $OCH_3$ | $OCH_3$ | |
| 1 | Q-1($R_5$=$CH_3$) | - | H | 5-$OCH_2N_3$ | $OCH_3$ | $CH_3$ | |
| 1 | Q-1($R_5$=$CH_3$) | - | H | 5-$CH_2SO_2CH_3$ | $OCH_3$ | $CH_3$ | |
| 1 | Q-25($R_6$=$CH_3$) | - | H | H | $CH_3$ | $OCH_3$ | |
| 1 | Q-26($R_6$=$CH_3$) | - | H | H | $CH_3$ | $OCH_3$ | |
| 1 | Q-27($R_6$=$CH_3$) | - | H | H | $CH_3$ | $OCH_3$ | |

Table 4 (Continued)

| J | $Q_1$ | E | R | $R_1$ | X | Y | m.p.°C |
|---|---|---|---|---|---|---|---|
| 2a | Q-28 | - | H | H | $CH_3$ | $OCH_3$ | |
| 2a | Q-29 | - | H | H | $CH_3$ | $OCH_3$ | |
| 2a | Q-30 | - | H | H | $CH_3$ | $OCH_3$ | |
| 2b | Q-1($R_5$=$CH_3$) | - | H | H | $OCH_3$ | $OCH_3$ | |
| 2b | Q-1($R_5$=$CH_2CH$=$CH_2$) | - | H | H | $CH_3$ | $OCH_3$ | |
| 2b | Q-1($R_5$=$CH_2CH$=$CH_2$) | - | H | H | $OCH_3$ | $OCH_3$ | |

Table 5

General Formula 3

| J | $Q_1$ | E | $R_1$ | R | $X_1$ | $Y_1$ | m.p.°C |
|---|---|---|---|---|---|---|---|
| 1 | Q-1($R_5$=$CH_3$) | - | H | H | $CH_3$ | O | 222-224 |
| 1 | Q-1($R_5$=$CH_3$) | - | H | H | $OCH_3$ | O | |
| 1 | Q-1($R_5$=$CH_3$) | - | H | H | $OC_2H_5$ | O | |
| 1 | Q-1($R_5$=$CH_3$) | - | H | H | $OCF_2H$ | O | |
| 1 | Q-1($R_5$=$CH_3$) | - | H | H | $OCH_3$ | $CH_2$ | |
| 1 | Q-7($R_5$,$R_6$=$CH_3$) | - | H | H | $CH_3$ | O | |
| 1 | Q-9 | - | H | H | $CH_3$ | O | |
| 1 | Q-10 | - | H | H | $CH_3$ | O | |
| 1 | Q-15($R_7$=$CH_3$) | - | H | H | $CH_3$ | O | |
| 1 | Q-1($R_5$=$CH_3$) | O | H | H | $CH_3$ | O | |
| 1 | Q-1($R_5$=$CH_3$) | $CH_2$ | H | H | $CH_3$ | O | |
| 1 | Q-1($R_5$=$CH_3$) | - | 5-Cl | H | $CH_3$ | O | |
| 1 | Q-1($R_5$=$CH_3$) | - | 5-$CH_3$ | H | $CH_3$ | O | |
| 1 | Q-1($R_5$=$CH_3$) | - | 5-$OCH_3$ | H | $CH_3$ | O | |
| 2a | Q-1($R_5$=$CH_3$) | - | H | H | $CH_3$ | O | |
| 2b | Q-1($R_5$=$CH_3$) | - | H | H | $CH_3$ | O | |
| 3a | Q-1($R_5$=$CH_3$) | - | H | H | $CH_3$ | O | |
| 3c | Q-1($R_5$=$CH_3$) | - | H | H | $CH_3$ | O | |
| 3d | Q-1($R_5$=$CH_3$) | - | H | H | $CH_3$ | O | |
| 5 | Q-15($R_7$=$CH_3$) | - | H | H | $CH_3$ | O | |
| 1 | Q-1($R_5$=$CH_3$) | - | H | $CH_3$ | $OCH_3$ | O | |
| 1 | Q-18($R_6$=$CH_3$) | - | H | H | $CH_3$ | O | |
| 1 | Q-23($R_6'$,$R_6''$=$CH_3$) | - | H | H | $CH_3$ | O | |

## Table 6

## General Formula 4

| J | $Q_1$ | E | R | $R_1$ | $X_1$ | m.p.°C |
|---|---|---|---|---|---|---|
| 1 | Q-1($R_5$=$CH_3$) | - | H | H | $CH_3$ | |
| 1 | Q-1($R_5$=$CH_3$) | - | H | H | $OCH_3$ | |
| 1 | Q-1($R_5$=$CH_3$) | - | H | H | $OC_2H_5$ | |
| 1 | Q-1($R_5$=$CH_3$) | - | H | H | $OCF_2H$ | |
| 1 | Q-7($R_5$,$R_6$=$CH_3$) | - | H | H | $CH_3$ | |
| 1 | Q-9 | - | H | H | $CH_3$ | |
| 1 | Q-10 | - | H | H | $CH_3$ | |
| 1 | Q-15($R_7$=$CH_3$) | - | H | H | $CH_3$ | |
| 1 | Q-1($R_5$=$CH_3$) | O | H | H | $OCH_3$ | |
| 1 | Q-1($R_5$=$CH_3$) | O | H | H | $CH_3$ | |
| 1 | Q-1($R_5$=$CH_3$) | $CH_2$ | H | H | $CH_3$ | |
| 1 | Q-1($R_5$=$CH_3$) | - | H | 5-Cl | $OCH_3$ | |
| 1 | Q-1($R_5$=$CH_3$) | - | H | 5-$CH_3$ | $OCH_3$ | |
| 1 | Q-1($R_5$=$CH_3$) | - | H | 5-$OCH_3$ | $OCH_3$ | |
| 2a | Q-1($R_5$=$CH_3$) | - | H | H | $CH_3$ | |
| 2b | Q-1($R_5$=$CH_3$) | - | H | H | $CH_3$ | |
| 3a | Q-1($R_5$=$CH_3$) | - | H | H | $CH_3$ | |
| 3c | Q-1($R_5$=$CH_3$) | - | H | H | $CH_3$ | |
| 3d | Q-1($R_5$=$CH_3$) | - | H | H | $CH_3$ | |
| 5 | Q-15($R_7$=$CH_3$) | - | H | H | $CH_3$ | |
| 1 | Q-1($R_5$=$CH_3$) | - | $CH_3$ | H | $OCH_3$ | |
| 1 | Q-18($R_6$=$CH_3$) | - | H | H | $CH_3$ | |
| 1 | Q-23($R_6'$,$R_6''$=$CH_3$) | - | H | H | $CH_3$ | |

**Table 7**

**General Formula 5**

| J | $Q_1$ | E | R | $R_1$ | $X_1$ | $Y_3$ | m.p.°C |
|---|---|---|---|---|---|---|---|
| 1 | Q-1($R_5$=$CH_3$) | - | H | H | $CH_3$ | $CH_3$ | |
| 1 | Q-1($R_5$=$CH_3$) | - | H | H | $OCH_3$ | $CH_3$ | |
| 1 | Q-1($R_5$=$CH_3$) | - | H | H | $OC_2H_5$ | $CH_3$ | |
| 1 | Q-1($R_5$=$CH_3$) | - | H | H | $OCF_2H$ | $CH_3$ | |
| 1 | Q-1($R_5$=$CH_3$) | - | H | H | $OCH_3$ | H | |
| 1 | Q-1($R_5$=$CH_3$) | - | H | H | $CH_3$ | H | |
| 1 | Q-7($R_5$,$R_6$=$CH_3$) | - | H | H | $OCH_3$ | $CH_3$ | |
| 1 | Q-9 | - | H | H | $OCH_3$ | $CH_3$ | |
| 1 | Q-10 | - | H | H | $OCH_3$ | $CH_3$ | |
| 1 | Q-15($R_7$=$CH_3$) | - | H | H | $OCH_3$ | $CH_3$ | |
| 1 | Q-1($R_5$=$CH_3$) | O | H | H | $OCH_3$ | H | |
| 1 | Q-1($R_5$=$CH_3$) | $CH_2$ | H | H | $OCH_3$ | H | |
| 1 | Q-1($R_5$=$CH_3$) | - | 5-Cl | H | $OCH_3$ | H | |
| 1 | Q-1($R_5$=$CH_3$) | - | 5-$CH_3$ | H | $OCH_3$ | H | |
| 1 | Q-1($R_5$=$CH_3$) | - | 5-$OCH_3$ | H | $OCH_3$ | H | |
| 2a | Q-1($R_5$=$CH_3$) | - | H | H | $CH_3$ | H | |
| 2b | Q-1($R_5$=$CH_3$) | - | H | H | $OCH_3$ | H | |
| 3a | Q-1($R_5$=$CH_3$) | - | H | H | $CH_3$ | H | |
| 3c | Q-1($R_5$=$CH_3$) | - | H | H | $CH_3$ | H | |
| 3d | Q-1($R_5$=$CH_3$) | - | H | H | $CH_3$ | H | |
| 5 | Q-15($R_7$=$CH_3$) | - | H | H | $OCH_3$ | H | |
| 1 | Q-1($R_5$=$CH_3$) | - | $CH_3$ | H | $OCH_3$ | H | |
| 1 | Q-18($R_6$=$CH_3$) | - | H | H | $CH_3$ | H | |
| 1 | Q-23($R_6'$,$R_6''$=$CH_3$) | - | H | H | $CH_3$ | H | |

Table 8

General Formula 6

| J | $Q_1$ | E | R | $R_1$ | $X_2$ | $Y_2$ | m.p.°C |
|---|---|---|---|---|---|---|---|
| 1 | Q-1($R_5$=$CH_3$) | - | H | H | $CH_3$ | $OCH_3$ | |
| 1 | Q-1($R_5$=$CH_3$) | - | H | H | $CH_3$ | $OC_2H_5$ | |
| 1 | Q-1($R_5$=$CH_3$) | - | H | H | $CH_3$ | $SCH_3$ | |
| 1 | Q-1($R_5$=$CH_3$) | - | H | H | $CH_3$ | $SC_2H_5$ | |
| 1 | Q-1($R_5$=$CH_3$) | - | H | H | $CH_3$ | $CH_3$ | |
| 1 | Q-1($R_5$=$CH_3$) | - | H | H | $CH_3$ | $CH_2CH_3$ | |
| 1 | Q-1($R_5$=$CH_3$) | - | H | H | $C_2H_5$ | $OCH_3$ | |
| 1 | Q-1($R_5$=$CH_3$) | - | H | H | $CH_2CF_3$ | $OCH_3$ | |
| 1 | Q-7($R_5$,$R_6$=$CH_3$) | - | H | H | $CH_3$ | $OCH_3$ | |
| 1 | Q-9 | - | H | H | $CH_3$ | $OCH_3$ | |
| 1 | Q-10 | - | H | H | $CH_3$ | $OCH_3$ | |
| 1 | Q-15($R_7$=$CH_3$) | - | H | H | $CH_3$ | $SCH_3$ | |
| 1 | Q-1($R_5$=$CH_3$) | O | H | H | $CH_3$ | $OCH_3$ | |
| 1 | Q-1($R_5$=$CH_3$) | $CH_2$ | H | H | $CH_3$ | $OCH_3$ | |
| 1 | Q-1($R_5$=$CH_3$) | - | H | 5-Cl | $CH_3$ | $OCH_3$ | |
| 1 | Q-1($R_5$=$CH_3$) | - | H | $5CH_3$ | $CH_3$ | $OCH_3$ | |
| 1 | Q-1($R_5$=$CH_3$) | - | H | 5-$OCH_3$ | $CH_3$ | $OCH_3$ | |
| 2a | Q-1($R_5$=$CH_3$) | - | H | H | $CH_3$ | $OCH_3$ | |
| 2b | Q-1($R_5$=$CH_3$) | - | H | H | $CH_3$ | $OCH_3$ | |
| 3a | Q-1($R_5$=$CH_3$) | - | H | H | $CH_3$ | $SCH_3$ | |
| 3c | Q-1($R_5$=$CH_3$) | - | H | H | $CH_3$ | $OCH_3$ | |
| 3d | Q-1($R_5$=$CH_3$) | - | H | H | $CH_3$ | $OCH_3$ | |
| 5 | Q-15($R_7$=$CH_3$) | - | H | H | $CH_3$ | $OCH_3$ | |
| 1 | Q-1($R_5$=$CH_3$) | - | $CH_3$ | H | $CH_3$ | $OCH_3$ | |
| 1 | Q-18($R_6$=$CH_3$) | - | H | H | $CH_3$ | $OCH_3$ | |
| 1 | Q-23($R_6'$,$R_6''$=$CH_3$) | - | H | H | $CH_3$ | $OCH_3$ | |

Table 9

General Formula 7

| J | $Q_1$ | E | R | $R_1$ | $X_3$ | m.p.°C |
|---|---|---|---|---|---|---|
| 1 | Q-1($R_5$=$CH_3$) | - | H | H | $CH_3$ | |
| 1 | Q-1($R_5$=$CH_3$) | - | H | H | $OCH_3$ | |
| 1 | Q-7($R_5$,$R_6$=$CH_3$) | - | H | H | $CH_3$ | |
| 1 | Q-9 | - | H | H | $CH_3$ | |
| 1 | Q-10 | - | H | H | $OCH_3$ | |
| 1 | Q-15($R_7$=$CH_3$) | - | H | H | $OCH_3$ | |
| 1 | Q-1($R_5$=$CH_3$) | O | H | H | $CH_3$ | |
| 1 | Q-1($R_5$=$CH_3$) | $CH_2$ | H | H | $CH_3$ | |
| 1 | Q-1($R_5$=$CH_3$) | - | H | 5-Cl | $CH_3$ | |
| 1 | Q-1($R_5$=$CH_3$) | - | H | 5-$CH_3$ | $CH_3$ | |
| 1 | Q-1($R_5$=$CH_3$) | - | H | 5-$OCH_3$ | $CH_3$ | |
| 2a | Q-1($R_5$=$CH_3$) | - | H | H | $OCH_3$ | |
| 2b | Q-1($R_5$=$CH_3$) | - | H | H | $CH_3$ | |
| 3a | Q-1($R_5$=$CH_3$) | - | H | H | $CH_3$ | |
| 3c | Q-1($R_5$=$CH_3$) | - | H | H | $OCH_3$ | |
| 3d | Q-1($R_5$=$CH_3$) | - | H | H | $OCH_3$ | |
| 5 | Q-15($R_7$=$CH_3$) | - | H | H | $OCH_3$ | |
| 1 | Q-1($R_5$=$CH_3$) | - | $CH_3$ | H | $OCH_3$ | |

Table 10

General Formula 8

| J | $Q_1$ | E | R | $R_1$ | $X_4$ | $Y_4$ | $Z_1$ | m.p.(°C) |
|---|---|---|---|---|---|---|---|---|
| 1 | Q-1($R_5$=$CH_3$) | - | H | H | $CH_3$ | $CH_3$ | CH | 144-155 |
| 1 | Q-1($R_5$=$CH_3$) | - | H | H | $CH_3$ | $CH_3$ | N | |
| 1 | Q-1($R_5$=$CH_3$) | - | H | H | $OCH_3$ | $CH_3$ | CH | |
| 1 | Q-1($R_5$=$CH_3$) | - | H | H | $OCH_3$ | $CH_3$ | N | |
| 1 | Q-1($R_5$=$CH_3$) | - | H | H | $OCH_3$ | $OCH_3$ | CH | |
| 1 | Q-1($R_5$=$CH_3$) | - | H | H | $OCH_3$ | $OCH_3$ | N | |
| 1 | Q-1($R_5$=$CH_3$) | - | H | H | Cl | $CH_3$ | CH | |
| 1 | Q-1($R_5$=$CH_3$) | - | H | H | $OCH_3$ | Cl | CH | |
| 1 | Q-1($R_5$=$CH_3$) | - | H | H | $OC_2H_5$ | $CH_3$ | CH | |
| 1 | Q-1($R_5$=$CH_3$) | - | H | H | $CH_2OCH_3$ | $CH_3$ | N | |
| 1 | Q-1($R_5$=$CH_3$) | - | H | H | $CH_2OCH_3$ | $OCH_3$ | CH | |
| 1 | Q-1($R_5$=$CH_3$) | - | H | H | $OC_2H_5$ | $OC_2H_5$ | N | |
| 1 | Q-1($R_5$=$CH_3$) | O | H | H | $CH_3$ | $CH_3$ | CH | |
| 1 | Q-7($R_5$,$R_6$=$CH_3$) | $CH_2$ | H | H | $CH_3$ | $CH_3$ | CH | |
| 1 | Q-9 | - | H | H | $CH_3$ | $CH_3$ | CH | |
| 1 | Q-9 | - | H | H | $OCH_3$ | $OCH_3$ | N | |
| 1 | Q-10 | - | H | H | $CH_3$ | $CH_3$ | CH | |
| 1 | Q-1($R_5$=$C_2H_5$) | - | H | H | $CH_3$ | $CH_3$ | CH | |
| 1 | Q-1($R_5$=n-$C_3H_7$) | - | H | H | $CH_3$ | $CH_3$ | CH | |
| 2a | Q-1($R_5$=$CH_3$) | - | H | H | $CH_3$ | $CH_3$ | CH | |
| 2b | Q-1($R_5$=$CH_3$) | - | H | H | $CH_3$ | $CH_3$ | CH | |
| 3a | Q-1($R_5$=$CH_3$) | - | H | H | $CH_3$ | $CH_3$ | CH | |
| 3c | Q-1($R_5$=$CH_3$) | - | H | H | $CH_3$ | $CH_3$ | CH | |
| 3d | Q-1($R_5$=$CH_3$) | - | H | H | $CH_3$ | $CH_3$ | CH | |
| 1 | Q-18($R_6$=$CH_3$) | - | H | H | $CH_3$ | $CH_3$ | CH | |
| 1 | Q-23($R_6'$,$R_6''$=$CH_3$) | - | H | H | $CH_3$ | $CH_3$ | CH | |
| 2a | Q-20 | - | H | H | $CH_3$ | $CH_3$ | CH | |

**Table 11**

**General Formula 9**

| J | $Q_1$ | E | R | $R_1$ | W | X | Y | Z | m.p.(°C) |
|---|---|---|---|---|---|---|---|---|---|
| 1 | Q-1($P_5$=$CH_3$) | - | H | H | O | $OCH_3$ | $OCH_3$ | $CCH_3$ | |
| 1 | Q-1($R_5$=$C_2H_5$) | - | H | H | O | $OCH_3$ | $OCH_3$ | $CCH_3$ | |
| 1 | Q-1($R_5$=$\underline{n}$-$C_3H_7$) | - | H | H | O | $OCH_3$ | $OCH_3$ | $CCH_3$ | |
| 1 | Q-1($R_5$=$CH_3$) | - | H | H | O | $OCH_3$ | $OCH_3$ | $CC_2H_5$ | |
| 1 | Q-1($R_5$=$CH_3$) | - | H | H | O | $OCH_3$ | $OCH_3$ | CCl | |
| 1 | Q-1($R_5$=$CH_3$) | - | H | H | O | $OCH_3$ | $OCH_3$ | CBr | |
| 1 | Q-9 | - | H | H | O | $OCH_3$ | $OCH_3$ | $CCH_3$ | |
| 2a | Q-1($R_5$=$CH_3$) | - | H | H | O | $OCH_3$ | $OCH_3$ | $CCH_3$ | |
| 2b | Q-1($R_5$=$CH_3$) | - | H | H | O | $OCH_3$ | $OCH_3$ | $CCH_3$ | |
| 3c | Q-1($R_5$=$CH_3$) | - | H | H | O | $OCH_3$ | $OCH_3$ | $CCH_3$ | |
| 3d | Q-1($R_5$=$CH_3$) | - | H | H | O | $OCH_3$ | $OCH_3$ | $CCH_3$ | |
| 1 | Q-1($R_5$=$CH_3$) | - | H | H | S | $OCH_3$ | $OCH_3$ | CH | |
| 1 | Q-1($R_5$=$CH_3$) | - | H | H | S | $OCH_3$ | $CH_3$ | N | |
| 1 | Q-1($R_5$=$C_2H_5$) | - | H | H | S | $OCH_3$ | $OCH_3$ | CH | |
| 1 | Q-1($R_5$=$\underline{n}$-$C_3H_7$) | - | H | H | S | $OCH_3$ | $OCH_3$ | CH | |
| 1 | Q-9 | - | H | H | S | $OCH_3$ | $CH_3$ | N | |
| 1 | Q-9 | - | H | H | S | $OCH_3$ | $CH_3$ | CH | |
| 1 | Q-7($R_5$,$R_6$=$CH_3$) | - | H | H | S | $OCH_3$ | $OCH_3$ | CH | |
| 1 | Q-10 | - | H | H | S | $OCH_3$ | $OCH_3$ | CH | |
| 2a | Q-1($R_5$=$CH_3$) | - | H | H | S | $OCH_3$ | $OCH_3$ | CH | |
| 2b | Q-1($R_5$=$CH_3$) | - | H | H | S | $OCH_3$ | $OCH_3$ | CH | |
| 2c | Q-1($R_5$=$CH_3$) | - | H | H | S | $OCH_3$ | $OCH_3$ | CH | |
| 3d | Q-1($R_5$=$CH_3$) | - | H | H | S | $OCH_3$ | $OCH_3$ | CH | |
| 1 | Q-18($R_6$=$CH_3$) | - | H | H | S | $OCH_3$ | $OCH_3$ | CH | |
| 1 | Q-20 | - | H | H | S | $OCH_3$ | $OCH_3$ | CH | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table 12

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspension | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook or Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 ° C. "McCutcheon"s Detergents and Emulsifiers Annual", MC Publishing Corp., Ridgewood, New Jersey, as well as Sisely and Wood, "Encylopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering , December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 17

**Aqueous Suspension**

| | |
|---|---|
| 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide | 25% |
| hydrated attapulgite | 3% |
| crude calcium ligninsulfonate | 10% |
| sodium dihydrogen phospate | 0.5% |
| water | 61.5% |

The ingredients are ground together in a ball or roller mill until the solid particles have been reduced to diameters under 10 microns.

Example 18

**Wettable Powder**

| | |
|---|---|
| 2-(4,5-dihydro-4-methyl-5-oxo-1,3,4-oxadiazol-2-yl)-N-[(4,6-dimethoxypyrimidin-2-yl)aminocarbonyl]benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended and then ground in a hammermill to produce particles with an average particle size less than 25 microns in diameter. The material is reblended and sifted through a U.S.S. No. 50 sieve (0.3 mm opening) before being packaged.

Example 19

Extruded Pellet

| | |
|---|---|
| 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 20

High Strength Concentrate

| | |
|---|---|
| 2-(4,5-dihydro-4-methyl-5-oxo-1,3,4-oxadiazol-2-yl)-N-[(4,6-dimethoxypyrimidin-2-yl)amino-carbonyl]benzenesulfonamide | 98.5% |
| silica aerogel | 0.5% |
| synthetic amorphous silica | 1.0% |

The ingredients are blended and ground in a hammer-mill to produce a material essentially all passing a U.S.S. No. 50 screen (0.3 mm opening). The concentrate may then be formulated in a variety of ways.

Example 21

Wettable Powder

| | |
|---|---|
| 2-(4,5-dihydro-4-methyl-5-oxo-1,3,4-oxadiazol-2-yl)-N-[(4,6-dimethoxypyrimidin-2-yl)amino-carbonyl]benzenesulfonamide | 65% |
| dodecylphenol polyethylene glycol ether | 4% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammer-mill to produce particles essentially all below 100

microns, the material is reblended and sifted through a U.S.S. No. 50 sieve (0.3 mm opening) and packaged.

Example 22

**Wettable Powder**

| | |
|---|---|
| 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 23

**Wettable Powder**

| | |
|---|---|
| 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended, and sifted through a U.S.S. No. 50 sieve (0.3 mm opening) before packaging.

All compounds of the invention may be formulated in the same manner.

Example 24

**Granule**

| | |
|---|---|
| wettable powder of Example 23 | 15% |
| gypsum | 69% |
| potassium sulfate | 16% |

The ingredients are blended in a rotating mixer and water sprayed on to accomplish granulation. When most of the material has reached the desired range of 1.0 to 0.42 mm (U.S.S. #18 to 40 sieves), the granules are removed, dried and screened. Oversize material is crushed to produce additional material in

the desired range. These granules contain 6% active ingredient.

Example 25

**Oil Suspension**

| | |
|---|---|
| 2-(4,5-dihydro-4-methyl-5-oxo-1,3,4-oxadiazol-2-yl)-N-[(4,6-dimethoxypyrimidin-2-yl)aminocarbonyl]benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 26

**Oil Suspension**

| | |
|---|---|
| 2-(4,5-dihydro-4-methyl-5-oxo-1,3,4-oxadiazol-2-yl)-N-[(4,6-dimethoxypyrimidin-2-yl)aminocarbonyl]benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 27

**Wettable Powder**

| | |
|---|---|
| N-[[(4,6-dimethoxypyrimidin-2-yl)aminocarbonyl]-2-(2-oxo-1-pyrrolidinylmethyl)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns,

reblended, and packaged.

Example 28

**Wettable Powder**

| | |
|---|---|
| N-[[4-methoxy-6-methyl-1,3,5-triazin-2-yl)-aminocarbonyl]-2-(2-oxo-1-pyrrolidinylmethyl)-benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 29

**Granule**

| | |
|---|---|
| Wettable Powder of Example 28 | 5% |
| attapulgite granules (U.S.S. 20-40 mesh; 0.84-0.42 mm) | 95% |

A slurry of wettable powder containing 25% solids is sprayed on the surface of attapulgite granules while tumbling in a double-cone blender. The granules are dried and packaged.

Example 30

**Extruded Pellet**

| | |
|---|---|
| N-[[(4,6-dimethoxypyrimidin-2-yl)aminocarbonyl]-2-(2-oxo-1-pyrrolidinylmethyl)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 31

Oil Suspension

| | |
|---|---|
| N-[[(4-methoxy-6-methyl-1,3,5-triazin-2-yl)aminocarbonyl]-2-(2-oxo-1-pyrrolidinylmethyl)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 32

Wettable Powder

| | |
|---|---|
| N-[[(4-methoxy-6-methyl-1,3,5-triazin-2-yl)aminocarbonyl]-2-(2-oxo-1-pyrrolidinylmethyl)-benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 33

Low Strength Granule

| | |
|---|---|
| N-[[(4,6-dimethoxypyrimidin-2-yl)aminocarbonyl]-2-(2-oxo-1-pyrrolidinylmethyl)benzenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 34

**Aqueous Suspension**

| | |
|---|---|
| N-[[(4-methoxy-6-methyl-1,3,5-triazin-2-yl)aminocarbonyl]-2-(2-oxo-1-pyrrolidinylmethyl)benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1% |
| water | 56.7% |

The ingredients are blended and ground together in a sand mill to produce particles essentially all under 5 microns in size.

Example 35

**Solution**

| | |
|---|---|
| N-[[(4,6-dimethoxypyrimidin-2-yl)aminocarbonyl]-2-(2-oxo-1-pyrrolidinylmethyl)benzenesulfonamide, sodium salt | 5% |
| water | 95% |

The salt is added directly to the water with stirring to produce the solution, which may then be packaged for use.

Example 36

**Low Strength Granule**

| | |
|---|---|
| N-[[(4-methoxy-6-methyl-1,3,5-triazin-2-yl)aminocarbonyl]-2-(2-oxo-1-pyrrolidinylmethyl)benzenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 37

Granule

| | |
|---|---|
| N-[[(4,6-dimethoxypyrimidin-2-yl)aminocarbonyl]-2-(2-oxo-1-pyrrolidinylmethyl)benezenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until the granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 38

High Strength Concentrate

| | |
|---|---|
| N-[[(4,6-dimethoxypyrimidin-2-yl)aminocarbonyl]-2-(2-oxo-1-pyrrolidinylmethyl)benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 39

Wettable Powder

| | |
|---|---|
| N-[[(4,6-dimethoxypyrimidin-2-yl)aminocarbonyl]-2-(2-oxo-1-pyrrolidinylmethyl)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 40

Wettable Powder

| | |
|---|---|
| N-[[(4-methoxy-6-methyl-1,3,5-triazin-2-yl)aminocarbonyl]-2-(2-oxo-1-pyrrolidinylmethyl)-benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 41

Oil Suspension

| | |
|---|---|
| N-[[(4,6-dimethoxypyrimidin-2-yl)aminocarbonyl]-2-(2-oxo-1-pyrrolidinylmethyl)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 42

Dust

| | |
|---|---|
| N-[[(4-methoxy-6-methyl-1,3,4-triazin-2-yl)aminocarbonyl]-2-(2-oxo-1-pyrrolidinylmethyl)-benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

Test results indicate that the compounds of the present invention are highly active pre-emergent or post-emergent herbicides or plant growth regulants. Many of them have utility for broad-spectrum preand/or post-emergence weed control in areas where complete control of all vegetation is desired, such as around fuel storage tanks, ammunition depots, industrial storage areas, parking lots, drive-in theaters, around

billboards, highway and railroad structures. Some of the compounds have utility for selective weed control in crops such as wheat and barley. Alternatively, the subject compounds are useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.005 to 10 kg/ha, the lower rates being suggested for use on lighter soils and/or those having a low organic matter content, for plant growth modifications or for situations where only short-term persistence is required.

The compounds of the invention may be used in combination with any other commercial herbicide; examples of which are those of the triazine, triazole, uracil, urea, amide, diphenylether, carbamate and bipyridylium types.

The herbicidal properties of the subject compounds were discovered in a number of greenhouse tests. The test procedures and results follow.

Compounds

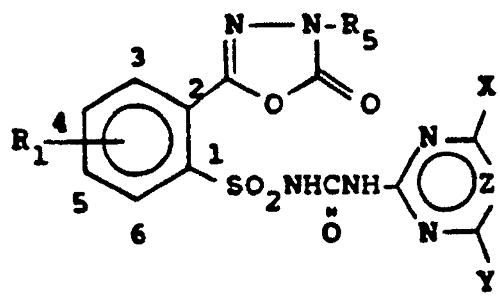

| Compound | $R_1$ | $R_5$ | X | Y | Z |
|---|---|---|---|---|---|
| 1 | H | $CH_3$ | $OCH_3$ | $OCH_3$ | CH |
| 2 | H | $CH_3$ | $OCH_3$ | $CH_3$ | CH |
| 3 | H | $CH_3$ | $CH_3$ | $CH_3$ | CH |
| 4 | H | $CH_3$ | Cl | $OCH_3$ | CH |
| 5 | H | $CH_3$ | $OCH_3$ | $CH_3$ | N |
| 6 | H | $CH_3$ | $OCH_3$ | $OCH_3$ | N |
| 7 | H | $CH_3$ | Cl | $OCH_2CH_3$ | CH |
| 8 | H | $CH_3$ | $OC_2H_5$ | $NHCH_3$ | CH |
| 9 | H | $CH_3$ | $OCH_3$ | $CH_2SCH_3$ | CH |
| 10 | H | $CH_3$ | $OCH_3$ | H | CH |
| 11 | H | $CH_3$ | $CH_3$ | H | CH |
| 12 | H | $CH_3$ | $OCH_3$ | $CH_2OCH_2CH_3$ | CH |
| 13 | H | $CH_3$ | $OCF_2H$ | $OCH_3$ | CH |
| 14 | H | $CH_3$ | Br | $OCH_3$ | CH |
| 15 | H | $CH_3$ | $OCH_3$ | $CH(OCH_3)_2$ | CH |
| 16 | H | $CH_3$ | $CH_3$ | $OC_2H_5$ | CH |
| 17 | H | $CH_3$ | $CH_3$ | $CH_2OCH_3$ | CH |
| 18 | H | $CH_3$ | cyclopropyl | $OCH_3$ | N |
| 19 | H | $C_2H_5$ | $OCH_3$ | $OCH_3$ | CH |
| 20 | H | $C_2H_5$ | $CH_3$ | $OCH_3$ | CH |
| 21 | H | $C_2H_5$ | $CH_3$ | $CH_3$ | CH |
| 22 | H | $C_2H_5$ | Cl | $OCH_3$ | CH |

Compounds (continued)

| Compound | $R_1$ | $R_5$ | X | Y | Z |
|---|---|---|---|---|---|
| 23 | H | $C_2H_5$ | $CH_3$ | $OCH_3$ | N |
| 24 | H | $C_2H_5$ | $OCH_3$ | $OCH_3$ | N |
| 25 | H | n-$C_3H_7$ | $OCH_3$ | $OCH_3$ | CH |
| 26 | H | n-$C_3H_7$ | $CH_3$ | $OCH_3$ | CH |
| 27 | H | n-$C_3H_7$ | $CH_3$ | $OCH_3$ | N |
| 28 | H | $CH_2CH(Cl)CH_2Cl$ | $OCH_3$ | $OCH_3$ | CH |
| 29 | H | i-$C_3H_7$ | $OCH_3$ | $OCH_3$ | CH |
| 30 | H | i-$C_3H_7$ | $CH_3$ | $OCH_3$ | CH |
| 31 | H | i-$C_3H_7$ | $CH_3$ | $CH_3$ | CH |
| 32 | H | n-$C_4H_9$ | $CH_3$ | $OCH_3$ | CH |
| 33 | H | $CH_2OCH_3$ | $OCH_3$ | $OCH_3$ | CH |
| 34 | H | $CH_2OCH_3$ | $CH_3$ | $OCH_3$ | CH |
| 35 | H | $CH_2OCH_3$ | $CH_3$ | $CH_3$ | CH |
| 36 | H | $CH_2OCH_3$ | Cl | $OCH_3$ | CH |
| 37 | H | $CH_2OCH_3$ | $CH_3$ | $OCH_3$ | N |
| 38 | H | $CH_2OCH_3$ | $OCH_3$ | $OCH_3$ | N |
| 39 | H | $CH_2CO_2CH_3$ | $OCH_3$ | $OCH_3$ | CH |
| 40 | H | $CH_2CH_2OCH_3$ | $OCH_3$ | $OCH_3$ | CH |
| 41 | H | $CH_2CH_2OCH_3$ | $CH_3$ | $OCH_3$ | CH |
| 42 | H | $CH_2CN$ | $OCH_3$ | $OCH_3$ | CH |
| 43 | H | $CH_2CN$ | $CH_3$ | $OCH_3$ | CH |
| 44 | H | $CH_2CH=CH_2$ | $OCH_3$ | $OCH_3$ | CH |
| 45 | H | $CH_2CH=CH_2$ | $CH_3$ | $OCH_3$ | CH |
| 46 | H | $CH_2CH=CH_2$ | $CH_3$ | $CH_3$ | CH |
| 47 | H | $CH_2CH=CH_2$ | Cl | $OCH_3$ | CH |
| 48 | H | $CH_2CH=CH_2$ | $CH_3$ | $OCH_3$ | N |
| 49 | H | $CH_2CH=CH_2$ | $OCH_3$ | $OCH_3$ | N |
| 50 | 5-Cl | $CH_3$ | $OCH_3$ | $OCH_3$ | CH |
| 51 | 5-Cl | $CH_3$ | $CH_3$ | $OCH_3$ | CH |
| 52 | 5-Cl | $CH_3$ | $CH_3$ | $CH_3$ | CH |
| 53 | 5-Cl | $CH_3$ | Cl | $OCH_3$ | CH |
| 54 | 5-Cl | $CH_3$ | $CH_3$ | $OCH_3$ | N |

Compounds (continued)

| Compound | $R_1$ | $R_5$ | X | Y | Z |
|---|---|---|---|---|---|
| 55 | 5-Cl | $CH_3$ | $OCH_3$ | $OCH_3$ | N |
| 56 | H | $CH_3$ | $OC_2H_5$ | $NHCH_3$ | N |
| 57 | H | $CH_3$ | $OCH_2CF_3$ | $NHCH_3$ | N |
| 58 | H | $CH_2OCH_3$ | $OCF_2H$ | $OCH_3$ | CH |

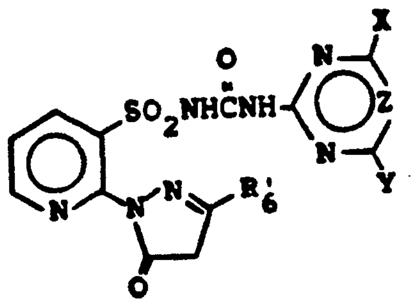

| Compound | $R'_6$ | X | Y | Z |
|---|---|---|---|---|
| 59 | H | $OCH_3$ | $OCH_3$ | CH |
| 60 | H | $CH_3$ | $OCH_3$ | CH |
| 61 | $CH_3$ | $OCH_3$ | $OCH_3$ | CH |

Compounds (continued)

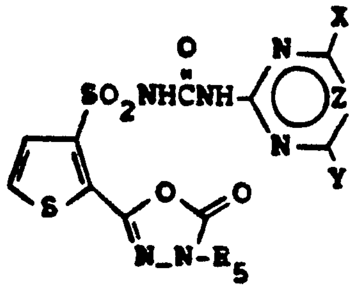

| Compound | $R_5$ | X | Y | Z |
|---|---|---|---|---|
| 62 | $CH_2CH=CH_2$ | $CH_3$ | $OCH_3$ | CH |
| 63 | $CH_2CH=CH_2$ | $OCH_3$ | $OCH_3$ | CH |
| 64 | $CH_2CN$ | $OCH_3$ | $OCH_3$ | CH |
| 65 | $CH_3$ | $CH_3$ | $CH_3$ | CH |
| 66 | $CH_3$ | $CH_3$ | $OCH_3$ | CH |
| 67 | $CH_3$ | $OCH_3$ | $OCH_3$ | CH |

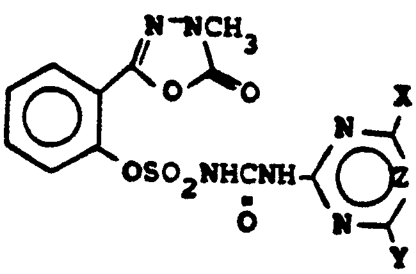

| Compound | X | Y | Z |
|---|---|---|---|
| 68 | $OCH_3$ | $OCH_3$ | CH |
| 69 | $CH_3$ | $OCH_3$ | CH |

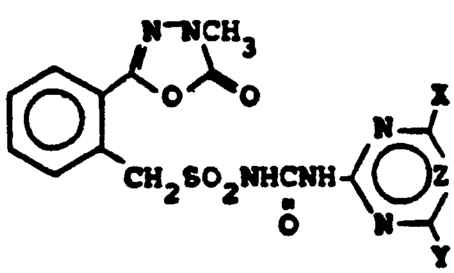

| Compound | X | Y | Z |
|---|---|---|---|
| 70 | $OCH_3$ | $OCH_3$ | CH |

COMPOUNDS

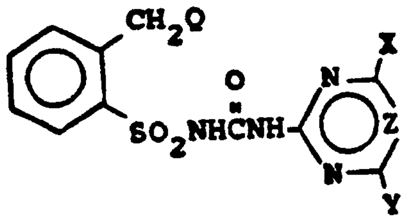

| COMPOUND | Q | X | Y | Z |
|---|---|---|---|---|
| 71 | 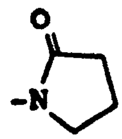 | $OCH_3$ | $OCH_3$ | CH |
| 72 | 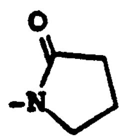 | $OCH_3$ | $OCH_3$ | N |
| 73 | 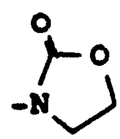 | $CH_3$ | $CH_3$ | CH |
| 74 | 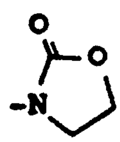 | $OCH_3$ | $OCH_3$ | CH |
| 75 | 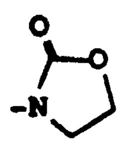 | $OCH_3$ | $OCH_3$ | N |
| 76 | 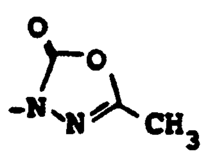 | $CH_3$ | $CH_3$ | CH |
| 77 | 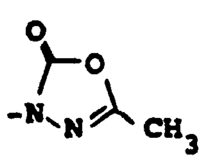 | $CH_3$ | $OCH_3$ | CH |

Compounds (continued)

| COMPOUND | Q | X | Y | Z |
|---|---|---|---|---|
| 78 | 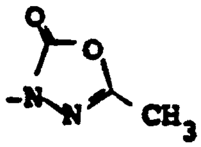 | Cl | $OCH_3$ | CH |
| 79 | 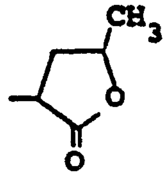 | $CH_3$ | $OCH_3$ | CH |
| 80 | 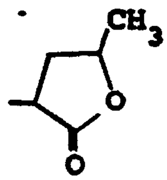 | $OCH_3$ | $OCH_3$ | N |
| 81 | 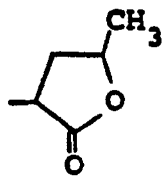 | $CH_3$ | $OCH_3$ | N |
| 82 | 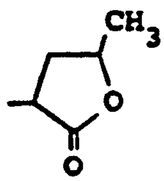 | $OCH_3$ | $OCH_3$ | CH |

Compounds (continued)

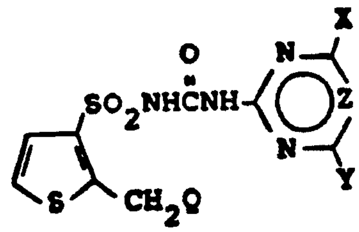

| COMPOUND | Q | X | Y | Z |
|---|---|---|---|---|
| 83 | 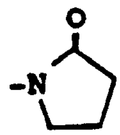 | $CH_3$ | $OCH_3$ | CH |
| 84 | 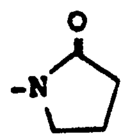 | $OCH_3$ | $OCH_3$ | CH |
| 85 | 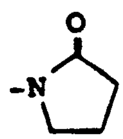 | Cl | $OCH_3$ | CH |
| 86 | 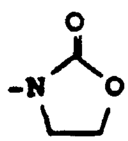 | $CH_3$ | $CH_3$ | CH |
| 87 | 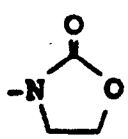 | $CH_3$ | $OCH_3$ | CH |
| 88 | 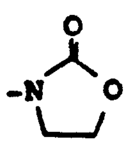 | $OCH_3$ | $OCH_3$ | CH |
| 89 | 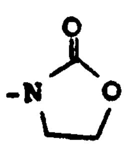 | Cl | $OCH_3$ | CH |

## Compounds (continued)

| COMPOUND | Q | X | Y | Z |
| --- | --- | --- | --- | --- |
| 90 | 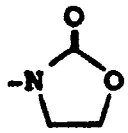 | $CH_3$ | $OCH_3$ | N |
| 91 | 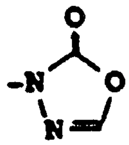 | $CH_3$ | $OCH_3$ | CH |
| 92 | 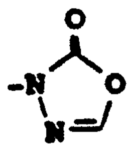 | $OCH_3$ | $OCH_3$ | CH |

Test A

Seeds of crabgrass ( Digitaria sp.), barnyardgrass ( Echinochloa crusgalli ), wild oats ( Avena fatua ), cheatgrass ( Bromus secalinus ), velvetleaf ( Abutilon theophrasti ), morningglory ( Ipomoea spp.), cocklebur ( Xanthium pensylvanicum ), sorghum, corn, soybean, sugarbeet, cotton, rice, wheat and purple nutsedge ( Cyperus rotundus ) tubers were planted and treated pre-emergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis or necrosis;
B = burn;
D = defoliation;
E = emergence inhibition;
G = growth retardation;
H = formative effects;
U = unusual pigmentation;
X = axillary stimulation;
S = albinism; and
6Y = abscised buds or flowers.

Table A

| | Cmpd. 1 | Cmpd. 2 | Cmpd. 3 |
|---|---|---|---|
| Rate kg/ha | 0.05 | 0.05 | 0.05 |
| POST-EMERGENCE | | | |
| Morningglory | 10C | 10C | 10C |
| Cocklebur | 10C | 10C | 10C |
| Velvetleaf | 10C | 10C | 9C |
| Nutsedge | 10C | 9C | 9C |
| Crabgrass | 3C,8G | 4C,9G | 3C,8G |
| Barnyardgrass | 5C,9H | 3C,9H | 5C,9H |
| Cheatgrass | 6C,9G | 9C | 9C |
| Wild Oats | 3C,8G | 5C,9G | 3C,9G |
| Wheat | 3G | 9G | 3C,9G |
| Corn | 3C,9G | 5C,9G | 3C,9G |
| Soybean | 9C | 9C | 5C,9G |
| Rice | 9C | 9C | 9C |
| Sorghum | 2C,9G | 4C,9G | 4C,9G |
| Sugar beet | 9C | 9C | 9C |
| Cotton | 9C | 9C | 9C |
| PRE-EMERGENCE | | | |
| Morningglory | 9G | 9G | 9C |
| Cocklebur | 9H | 9H | 9H |
| Velvetleaf | 9C | 9C | 5C,9G |
| Nutsedge | 10E | 10E | 10E |
| Crabgrass | 7G | 3C,7G | 3C,6G |
| Barnyardgrass | 9H | 5C,9H | 3C,9H |
| Cheatgrass | 10H | 9H | 9H |
| Wild Oats | 2C,7G | 5C,9G | 3C,8H |
| Wheat | 6G | 5C,9H | 5C,9H |
| Corn | 2C,9G | 2U,9G | 9G |
| Soybean | 9H | 9H | 4C,6H |
| Rice | 10E | 10E | 10E |
| Sorghum | 10H | 10H | 5C,9H |
| Sugar beet | 9C | 9C | 4C,9G |
| Cotton | 9G | 9G | 9G |

Table A (Continued)

| | Cmpd. 4 | Cmpd. 5 | Cmpd. 6 |
|---|---|---|---|
| Rate kg/ha | 0.05 | 0.05 | 0.05 |
| POST-EMERGENCE | | | |
| Morningglory | 9C | 9C | 10C |
| Cocklebur | 10C | 10C | 4G |
| Velvetleaf | 9C | 9C | 4C,8G |
| Nutsedge | 9G | 3G | 2G |
| Crabgrass | 5G | 3G | 0 |
| Barnyardgrass | 3C,9H | 2H | 2H |
| Cheatgrass | 7G | 2G | 2G |
| Wild Oats | 3C,8G | 0 | 0 |
| Wheat | 2G | 0 | 0 |
| Corn | 3C,9H | 9H | 7H |
| Soybean | 6H | 4C,9G | 5C,9G |
| Rice | 3C,9G | 8G | 4G |
| Sorghum | 9H | 3C,8G | 2C,5G |
| Sugar beet | 9C | 9C | 9C |
| Cotton | 10C | 9C | 5C,9G |
| PRE-EMERGENCE | | | |
| Morningglory | 9G | 9C | 9G |
| Cocklebur | 9H | 9H | - |
| Velvetleaf | 9G | 5C,9H | 2G |
| Nutsedge | 10E | 5G | 0 |
| Crabgrass | 3G | 3G | 0 |
| Barnyardgrass | 3C,9H | 1C | 0 |
| Cheatgrass | 6G | 2G | 0 |
| Wild Oats | 3C,8G | 0 | 0 |
| Wheat | 7G | 0 | 0 |
| Corn | 2C,8G | 2C,7H | 2C,8H |
| Soybean | 2C,5H | 4C,7H | 3C,7G |
| Rice | 9H | 2G | 5G |
| Sorghum | 3C,9H | 5G | 2C,5G |
| Sugar beet | 4C,9G | 5C,9G | 5C,9G |
| Cotton | 9G | 8G | 9G |

Table A (continued)

| | Cmpd. 7 | Cmpd. 8 | Cmpd. 9 | Cmpd. 10 |
|---|---|---|---|---|
| Rate kg/ha | 0.05 | 0.05 | 0.05 | 0.05 |
| POSTEMERGENCE | | | | |
| Morningglory | 3C,9G | 4G | 3C,8H | 4C,9H |
| Cocklebur | 5C,9G | 2C,8G | 5C,9G | 4C,9G |
| Velvetleaf | 3G | 2C,8G | 4C,9H | 5C,9H |
| Nutsedge | 2C,8G | 2C,8G | 2C,8G | 0 |
| Crabgrass | 2G | 2C,5G | 3G | 0 |
| Giant Foxtail | - | - | - | - |
| Barnyardgrass | 3C,7H | 4C,9H | 5C,9H | 2H |
| Cheatgrass | 8G | 2C,8G | 4C,8G | 2G |
| Wild Oats | 4G | 2C,5G | 3G | 2H |
| Wheat | 3G | 5G | 2G | 0 |
| Corn | 5H | 3C,9G | 3C,9G | 0 |
| Barley | - | - | - | - |
| Soybean | 5C,9G | 5C,9G | 5C,9G | 4C,9G |
| Rice | 2C,8G | 5C,9H | 5C,9G | 2G |
| Sorghum | 3C,9H | 3C,9H | 3C,9G | 2G |
| Sugar beet | 3C,6H | 3C,8H | 5C,9G | 10C |
| Cotton | 2C,8G | 3C,8G | 4C,9G | 4C,9H |
| PREEMERGENCE | | | | |
| Morningglory | 8G | 3G | 2C,2H | 3H |
| Cocklebur | 9H | 9H | 5G | 4G |
| Velvetleaf | 3G | 9G | 3C,7G | 3C,3H |
| Nutsedge | 2C,5G | 3C,9G | 3G | 0 |
| Crabgrass | 0 | 5G | 2G | 0 |
| Giant Foxtail | - | - | - | - |
| Barnyardgrass | 2C,2H | 4C,9H | 3C,9H | 0 |
| Cheatgrass | 7G | 4C,9H | 5C,9H | 0 |
| Wild Oats | 2C | 4C,9H | 7G | 0 |
| Wheat | 3G | 5C,9H | 6G | 0 |
| Corn | 2C,7G | 3C,9H | 3C,8G | 0 |
| Barley | - | - | - | - |
| Soybean | 5H | 3C,3H | 2C,6H | 2C,2H |
| Rice | 2C,8H | 5C,9H | 4C,9H | 0 |
| Sorghum | 3C,5G | 4C,9H | 4C,8H | 0 |
| Sugar beet | 3C,8G | 10E | - | 4C,7G |
| Cotton | 6G | 8G | 5C,9G | 2G |

Table A (continued)

| | Cmpd. 11 | Compound 12 | | Cmpd. 13 |
|---|---|---|---|---|
| Rate kg/ha | 0.05 | 0.05 | 0.01 | 0.05 |
| POSTEMERGENCE | | | | |
| Morningglory | 4C,9H | 2C,7H | 5G | 9C |
| Cocklebur | 2C,9G | 5C,9H | 8G | 10C |
| Velvetleaf | 4C,8G | 3C,6G | 2G | 9C |
| Nutsedge | 0 | 5G | 2G | 6C,9G |
| Crabgrass | 0 | 3C,5G | 0 | 2G |
| Giant Foxtail | - | - | - | - |
| Barnyardgrass | 7H | 4C,9H | 8H | 3C,5H |
| Cheatgrass | 4G | 2C,9G | 4G | 3C,9G |
| Wild Oats | 2C,7G | 3C,7H | 1C | 0 |
| Wheat | 0 | 5G | 1C | 0 |
| Corn | 2H | 9C | 2U,8G | 3C,8H |
| Barley | - | - | - | - |
| Soybean | 5C,9G | 3C,9G | 3C,8G | 9C |
| Rice | 3C,9G | 4C,9G | 4C,9G | 2C,8G |
| Sorghum | 2C,7G | 3C,9H | 4C,9H | 4C,9H |
| Sugar beet | 9C | 4C,7H | 3G | 9C |
| Cotton | 4C,9G | 4C,8H | 5G | 9C |
| PREEMERGENCE | | | | |
| Morningglory | 2C | 7G | 6H | 9G |
| Cocklebur | 2H | - | 3G | 6H |
| Velvetleaf | 2H | 3C | 0 | 8C |
| Nutsedge | 0 | 0 | 0 | 10E |
| Crabgrass | 0 | 3G | 2G | 0 |
| Giant Foxtail | - | - | - | - |
| Barnyardgrass | 0 | 4C,7H | 0 | 3H |
| Cheatgrass | 0 | 7G | 0 | 5G |
| Wild Oats | 0 | 3C,7G | 2C,5G | 0 |
| Wheat | 0 | 7G | 6G | 0 |
| Corn | 0 | 3C,7G | 2C | 0 |
| Barley | - | - | - | - |
| Soybean | 7H | 3C,5H | 0 | 6H |
| Rice | 0 | 4C,8H | 0 | 3G |
| Sorghum | 2G | 4C,8H | 3C,6H | 3C,7G |
| Sugar beet | 2C,6H | 3C,7H | 3C,9G | 4C,9G |
| Cotton | 5G | 2H | - | 4G |

Table A (continued)

| | Cmpd. 14 | Cmpd. 15 | Cmpd. 16 | Cmpd. 17 |
|---|---|---|---|---|
| Rate kg/ha | 0.05 | 0.05 | 0.05 | 0.05 |
| POSTEMERGENCE | | | | |
| Morningglory | 1C,7G | 3C,8H | 10C | 4C,9G |
| Cocklebur | 5C,9G | 5C,9H | 9C | 6C,9G |
| Velvetleaf | 4C,8H | 3C,7G | 9C | 9C |
| Nutsedge | 2C,8G | 3G | 9G | 4C,8G |
| Crabgrass | 0 | 3G | 3C,7H | 4H |
| Giant Foxtail | - | - | - | - |
| Barnyardgrass | 7H | 4C,9H | 5C,9H | 4C,8H |
| Cheatgrass | 4G | 2C,9G | 3C,9G | 4C,9G |
| Wild Oats | 0 | 3C,6G | 3C,7G | 3C,8G |
| Wheat | 0 | 4G | 5G | 2C,7G |
| Corn | 7H | 9C | 5C,9G | 5C,9G |
| Barley | - | - | - | - |
| Soybean | 2C,4G | 4C,9H | 9C | 9C |
| Rice | 3C,8G | 6C,9G | 9C | 9C |
| Sorghum | 4C,9G | 4C,9G | 5C,9H | 3U,9H |
| Sugar beet | 4C,8G | 6C,9H | 9C | 9G |
| Cotton | 5C,9G | 4C,9H | 9C | 5C,8G |
| PREEMERGENCE | | | | |
| Morningglory | 8G | 3H | 8H | 6H |
| Cocklebur | 9G | 4G | 7H | 9H |
| Velvetleaf | 5G | 0 | 10C | 5H |
| Nutsedge | 5G | 0 | 10E | 0 |
| Crabgrass | 0 | 0 | 5G | 2G |
| Giant Foxtail | - | - | - | - |
| Barnyardgrass | 5G | 3C,7H | 4C,9H | 6H |
| Cheatgrass | 4G | 7G | 10H | 6H |
| Wild Oats | 0 | 2C,6G | 5C,9G | 6G |
| Wheat | 0 | 2C,6G | 5C,9G | 6G |
| Corn | 4G | 3C,8H | 4C,9H | 3C,7H |
| Barley | - | - | - | - |
| Soybean | 2G | 0 | 4C,6H | 3C,4H |
| Rice | 7H | 4C,8H | 9H | 9H |
| Sorghum | 5G | 4C,9H | 5C,9H | 4C,8H |
| Sugar beet | 8G | 5H | 5C,9G | 4C,9G |
| Cotton | 4G | 0 | 4C,8G | 4G |

Table A (continued)

| | Cmpd. 18 | Compound 19 | | Cmpd. 20 |
|---|---|---|---|---|
| Rate kg/ha | 0.05 | 0.05 | 0.01 | 0.05 |
| POSTEMERGENCE | | | | |
| Morningglory | 5C,9G | 10C | 4C,8G | 10C |
| Cocklebur | 5C,9G | 10C | 5C,9G | 10C |
| Velvetleaf | 4C,9G | 10C | 3G | 10C |
| Nutsedge | 0 | 10C | 3G | 10C |
| Crabgrass | 0 | 4G | 0 | 5G |
| Giant Foxtail | - | - | - | - |
| Barnyardgrass | 0 | 3C,8G | 2C,7H | 5C,9H |
| Cheatgrass | 0 | 9C | 2G | 9C |
| Wild Oats | 0 | 2C,7G | 0 | 6C,9G |
| Wheat | 0 | 5G | 0 | 9G |
| Corn | 2C,4H | 9C | 5H | 10C |
| Barley | - | - | - | - |
| Soybean | 5C,9G | 9C | 4C,9G | 5C,9G |
| Rice | 0 | 5C,9G | 4G | 6C,9G |
| Sorghum | 2H | 4C,9H | 2G | 3U,9H |
| Sugar beet | 5C,9G | 9C | 9C | 10C |
| Cotton | 4C,9G | 9C | 3G | 9C |
| PREEMERGENCE | | | | |
| Morningglory | 9G | 9G | 2C,4G | 9G |
| Cocklebur | 9H | - | 3H | 9H |
| Velvetleaf | 2C,8G | 9G | 3G | 5C,9G |
| Nutsedge | 0 | 9G | 3G | 10E |
| Crabgrass | 0 | 4G | 0 | 2G |
| Giant Foxtail | - | - | - | - |
| Barnyardgrass | 0 | 2C,5G | 2C | 3C,9H |
| Cheatgrass | 0 | 7G | 0 | 5C,9H |
| Wild Oats | 0 | 2C,5G | 0 | 5C,8G |
| Wheat | 0 | 5G | 0 | 4C,9G |
| Corn | 4C,8G | 5G | 2G | 4C,9H |
| Barley | - | - | - | - |
| Soybean | 4C,5H | 5G | 2G | 3C,7H |
| Rice | 0 | 2C,8G | 0 | 5C,9H |
| Sorghum | 0 | 2C,7G | 0 | 4C,9G |
| Sugar beet | 5C,9G | 5C,9G | 10C | 10C |
| Cotton | 8G | 9G | 5G | 9G |

Table A (continued)

| | Cmpd. 21 | Cmpd. 22 | Cmpd. 23 | Cmpd. 24 |
|---|---|---|---|---|
| Rate kg/ha | 0.05 | 0.05 | 0.05 | 0.05 |
| POSTEMERGENCE | | | | |
| Morningglory | 5C,9G | 10C | 10C | 10C |
| Cocklebur | 9C | 9C | 10C | 10C |
| Velvetleaf | 5C,9G | 10C | 9C | 5C,9G |
| Nutsedge | 4C,9G | 3C,9G | 4G | 2C,5G |
| Crabgrass | 2G | 0 | 0 | 2G |
| Giant Foxtail | - | - | - | - |
| Barnyardgrass | 9H | 4H | 0 | 3H |
| Cheatgrass | 5C,9G | 8G | 2G | 0 |
| Wild Oats | 6C,9G | 7G | 0 | 0 |
| Wheat | 9G | 2G | 0 | 0 |
| Corn | 4C,9G | 3U,9H | 2C,7G | 2C,5H |
| Barley | - | - | - | - |
| Soybean | 9C | 4C,9G | 9C | 9C |
| Rice | 4C,9G | 5C,9G | 2G | 3G |
| Sorghum | 3C,9G | 3C,9H | 2C,3G | 2G |
| Sugar beet | 9C | 9C | 9C | 9C |
| Cotton | 5C,9G | 9C | 5C,9G | 9C |
| PREEMERGENCE | | | | |
| Morningglory | 9G | 9G | 8G | 8G |
| Cocklebur | 8H | 9H | 8H | - |
| Velvetleaf | 8G | 8H | 8G | 7G |
| Nutsedge | 4G | 5G | 2G | 0 |
| Crabgrass | 0 | 4G | 0 | 0 |
| Giant Foxtail | - | - | - | - |
| Barnyardgrass | 3H | 2C,4G | 0 | 3G |
| Cheatgrass | 2C,7H | 2C,6G | 0 | 0 |
| Wild Oats | 2C,8G | 2C,6G | 3G | 3G |
| Wheat | 2C,8G | 6G | 2G | 3G |
| Corn | 2C,8G | 4G | 4G | 5G |
| Barley | - | - | - | - |
| Soybean | 2C,5G | 2C | 4C,6H | 3C,5G |
| Rice | 3C,8G | 2C,6G | 0 | 2G |
| Sorghum | 2C,5G | 3C,5G | 0 | 2G |
| Sugar beet | 10C | 5C,9G | 9C | 10C |
| Cotton | 8G | 7G | 3G | - |

Table A (continued)

| | Cmpd. 25 | Cmpd. 26 | Cmpd. 27 | Cmpd. 28 |
|---|---|---|---|---|
| Rate kg/ha | 0.05 | 0.05 | 0.05 | 0.05 |
| POSTEMERGENCE | | | | |
| Morningglory | 9C | 9C | 9C | 2C,7G |
| Cocklebur | 10C | 9C | 9C | 5C,9G |
| Velvetleaf | 9C | 9C | 4C,9H | 4C,8H |
| Nutsedge | 2C,9G | 9G | 0 | 3G |
| Crabgrass | 2G | 3G | 0 | 2G |
| Giant Foxtail | - | - | - | - |
| Barnyardgrass | 9H | 9H | 2H | 3C,8H |
| Cheatgrass | 8G | 9G | 0 | 2C,5G |
| Wild Oats | 5G | 3C,9G | 0 | 0 |
| Wheat | 0 | 7G | 0 | 0 |
| Corn | 5C,9G | 4U,9C | 0 | 8H |
| Barley | - | - | - | - |
| Soybean | 6C,9G | 6C,9G | 5C,9G | 4C,8G |
| Rice | 4C,9G | 6C,9G | 0 | 4C,8G |
| Sorghum | 3C,7G | 4C,9H | 0 | 1C,3H |
| Sugar beet | 9C | 3C,7G | 9C | 3C,5G |
| Cotton | 10C | 2C,6G | 5C,9G | 4G |
| PREEMERGENCE | | | | |
| Morningglory | 9G | 9G | 8G | 7G |
| Cocklebur | 8H | 9H | 7H | 7G |
| Velvetleaf | 4C,9G | 4C,9G | 5G | 5G |
| Nutsedge | 10E | 10E | 0 | 3G |
| Crabgrass | 0 | 3G | 0 | 3G |
| Giant Foxtail | - | - | - | - |
| Barnyardgrass | 7G | 3C,9H | 0 | 2G |
| Cheatgrass | 6G | 3C,9H | 0 | 4G |
| Wild Oats | 2C,7G | 3C,9G | 0 | 3C,5G |
| Wheat | 5G | 8G | 0 | 5G |
| Corn | 2C,7G | 2C,9G | 0 | 6G |
| Barley | - | - | - | - |
| Soybean | 6H | 3C,6H | 2C,5G | 3C,4G |
| Rice | 2C,8G | 3C,9H | 0 | 3C,5G |
| Sorghum | 2C | 3C,9G | 0 | 5G |
| Sugar beet | 9C | 5C,9G | 3C,9G | 5G |
| Cotton | 7G | 4G | 0 | 5G |

Table A (continued)

| | Cmpd. 29 | Cmpd. 30 | Cmpd. 31 | Cmpd. 32 |
|---|---|---|---|---|
| Rate kg/ha | 0.05 | 0.05 | 0.05 | 0.05 |
| POSTEMERGENCE | | | | |
| Morningglory | 9C | 10C | 3C,9G | 9C |
| Cocklebur | 10C | 9C | 3C,9H | 9C |
| Velvetleaf | 10C | 9C | 3C,9H | 5C,9G |
| Nutsedge | 10C | 2C,8G | 2C,8G | 4C,8G |
| Crabgrass | 3G | 0 | 3G | 0 |
| Giant Foxtail | - | - | - | 2H |
| Barnyardgrass | 4C,9H | 3C,9H | 3C,8H | 3C,7H |
| Cheatgrass | 2C,8G | 3C,9H | 3C,8G | 3G |
| Wild Oats | 6G | 3C,9G | 3C,9G | 2C,5G |
| Wheat | 0 | 7G | 5G | 3G |
| Corn | 3C,9H | 3C,9G | 3C,9H | 4C,9G |
| Barley | - | - | - | 4G |
| Soybean | 4C,9G | 4C,9G | 3C,9G | 3C,8H |
| Rice | 2C,6G | 4C,9G | 5C,9G | 4C,9H |
| Sorghum | 4G | 4C,9H | 3C,8G | 3C,7H |
| Sugar beet | 9C | 3C,9H | 5C,9G | 3G |
| Cotton | 5C,9G | 5C,9G | 4C,9H | 5C,9G |
| PREEMERGENCE | | | | |
| Morningglory | 9C | 9C | 7H | 8G |
| Cocklebur | 9H | 9H | 8G | 7H |
| Velvetleaf | 9C | 9C | 7H | 8G |
| Nutsedge | 10E | 10E | 3G | 5G |
| Crabgrass | 6G | 2C | 0 | 2G |
| Giant Foxtail | - | - | - | 5G |
| Barnyardgrass | 3C,7G | 2C,8H | 2G | 5G |
| Cheatgrass | 5G | 8G | 0 | 2G |
| Wild Oats | 3G | 2C,8G | 2C,4G | 5G |
| Wheat | 0 | 7G | 4G | 3G |
| Corn | 2C,6G | 4C,9H | 2C,5G | 3C,8H |
| Barley | - | - | - | 9G |
| Soybean | 3C,6G | 3C,7H | 2C,3G | 3C,5G |
| Rice | 7G | 3C,9H | 3C,7G | 3C,7H |
| Sorghum | 4G | 3C,9H | 3C,3G | 2C,8H |
| Sugar beet | 10C | 10C | 3C,8G | 8G |
| Cotton | 8G | 9G | 7G | 7H |

Table A (continued)

| | Cmpd. 33 | Cmpd. 34 | Cmpd. 35 | Cmpd. 36 |
|---|---|---|---|---|
| Rate kg/ha | 0.05 | 0.05 | 0.05 | 0.05 |
| POSTEMERGENCE | | | | |
| Morningglory | 10C | 10C | 7G | 9G |
| Cocklebur | 10C | 10C | 9G | 10C |
| Velvetleaf | 10C | 10C | 9G | 9G |
| Nutsedge | 10C | 9G | 2G | 6G |
| Crabgrass | 2G | 3G | 0 | 0 |
| Giant Foxtail | 3G | 7G | 2G | 0 |
| Barnyardgrass | 6G | 4C,9G | 3H,7G | 5G |
| Cheatgrass | 2G | 4C,5G | 4G | 0 |
| Wild Oats | 7G | 8G | 9G | 6G |
| Wheat | 4G | 8G | 8G | 0 |
| Corn | 8G | 5C,9G | 8G | 4H,6G |
| Barley | 0 | 5G | 6G | 0 |
| Soybean | 10C | 9G | 9G | 7G |
| Rice | 6G | 9C | 9G | 7G |
| Sorghum | 8G | 9G | 9G | 9G |
| Sugar beet | 10C | 9G | 10C | 10C |
| Cotton | 10C | 9G | 10C | 9G |
| PREEMERGENCE | | | | |
| Morningglory | 9G | 9G | 9G | 9G |
| Cocklebur | 9G | 9G | 9G | 7G |
| Velvetleaf | 9G | 9G | 9G | 9G |
| Nutsedge | 10E | 10E | 4G | 8G |
| Crabgrass | 6G | 3G | 2G | 3G |
| Giant Foxtail | 5G | 6G | 2C | 3G |
| Barnyardgrass | 3G | 6G | 3C | 2G |
| Cheatgrass | 6G | 10E | 10E | 4G |
| Wild Oats | 4G | 8G | 8G | 5G |
| Wheat | 2G | 8G | 8G | 0 |
| Corn | 8G | 9G | 9G | 2G |
| Barley | 3G | 7G | 8G | 0 |
| Soybean | 6G | 8G | 7G | 2G |
| Rice | 9G | 10E | 9G | 9G |
| Sorghum | 8G | 9G | 9G | 7G |
| Sugar beet | 9G | 9G | 10E | 9G |
| Cotton | 9G | 9G | 9G | 9G |

Table A (continued)

| | Cmpd. 37 | Cmpd. 38 | Cmpd. 39 |
|---|---|---|---|
| Rate kg/ha | 0.05 | 0.05 | 0.05 |
| POSTEMERGENCE | | | |
| Morningglory | 9G | 3C,8G | 2C,5H |
| Cocklebur | 10C | 9G | 3G |
| Velvetleaf | 9G | 9G | 2C,7H |
| Nutsedge | 0 | 0 | 2C |
| Crabgrass | 0 | 0 | 0 |
| Giant Foxtail | 0 | 0 | 2G |
| Barnyardgrass | 3G | 4G | 3C,9H |
| Cheatgrass | 0 | 0 | 0 |
| Wild Oats | 0 | 0 | 1C |
| Wheat | 0 | 0 | 5G |
| Corn | 6G | 4G | 2C,4G |
| Barley | 0 | 0 | - |
| Soybean | 9G | 9G | 2C,6G |
| Rice | 3G | 0 | 3G |
| Sorghum | 2G | 0 | 3C,9G |
| Sugar beet | 10C | 9G | 3C,9H |
| Cotton | 9G | 9G | 4C,9G |
| PREEMERGENCE | | | |
| Morningglory | 9G | 6G | 7H |
| Cocklebur | 9G | 7G | 8H |
| Velvetleaf | 8G | 3G | 8G |
| Nutsedge | 3G | 0 | 0 |
| Crabgrass | 0 | 0 | 0 |
| Giant Foxtail | 0 | 0 | 2C |
| Barnyardgrass | 0 | 0 | 1C |
| Cheatgrass | 0 | 0 | 3G |
| Wild Oats | 0 | 0 | 2C |
| Wheat | 0 | 0 | 2G |
| Corn | 0 | 2C | 4G |
| Barley | 0 | 0 | - |
| Soybean | 3C,7G | 3C,7G | 2C,2H |
| Rice | 2C | 0 | 2C |
| Sorghum | 3G | 3G | 2C |
| Sugar beet | 9G | 9G | 6G |
| Cotton | 9G | 9G | 8G |

Table A (continued)

| | Compound 40 | | Compound 41 | |
|---|---|---|---|---|
| Rate kg/ha | 0.05 | 0.01 | 0.05 | 0.01 |
| POSTEMERGENCE | | | | |
| Morningglory | 5C,9G | 2C,4G | 10C | 5C,9G |
| Cocklebur | 5C,9G | 6C,9G | 5C,9G | 5C,9G |
| Velvetleaf | 9C | 9C | 9C | 3C,9H |
| Nutsedge | 4C,9G | 3C,9G | 9G | 7G |
| Crabgrass | 0 | 0 | 4G | 0 |
| Giant Foxtail | 4C,8G | 2C,7G | 4C,8G | 7G |
| Barnyardgrass | 9C | 5C,9G | 9C | 4C,9H |
| Cheatgrass | 8G | 2C,7G | 3C,8G | 2C,6G |
| Wild Oats | 9G | 2G | 3C,9G | 2C,5G |
| Wheat | 7G | 3G | 9G | 4G |
| Corn | 2C,9H | 3C,9H | 3C,9H | 4C,9H |
| Barley | 9G | 6G | 2C,8G | 2C,5G |
| Soybean | 5C,9G | 3C,9H | 4C,9H | 4C,9G |
| Rice | 4C,9G | 2C,8G | 5C,9G | 4C,9G |
| Sorghum | 2C,9G | 3C,9H | 4C,9H | 4C,8H |
| Sugar beet | 5C,9H | 4C,8H | 9C | 9C |
| Cotton | 9C | 9G | 4C,9H | 9H |
| PREEMERGENCE | | | | |
| Morningglory | 5G | 2C,3H | 9G | 5H |
| Cocklebur | 9H | 3C,7H | 8H | 2C,5H |
| Velvetleaf | 9G | 5G | 10E | 4G |
| Nutsedge | 10E | 5G | 9G | 0 |
| Crabgrass | 2G | 0 | 5G | 0 |
| Giant Foxtail | 8G | 4G | 4C,8H | 5G |
| Barnyardgrass | 3C,9H | 3C,8H | 5C,9H | 2C,7G |
| Cheatgrass | 9H | 3G | 6C,9H | 5G |
| Wild Oats | 7G | 0 | 2C,8G | 2C,5G |
| Wheat | 7G | 0 | 2C,9H | 5G |
| Corn | 4C,9H | 3C,8H | 5C,9H | 4C,9H |
| Barley | 6C,9G | 8G | 2C,9H | 9G |
| Soybean | 3C,7H | 3C,4H | 9H | 3C,6H |
| Rice | 9H | 3C,7H | 10E | 3C,7H |
| Sorghum | 9H | 4C,9H | 5C,9H | 3C,8H |
| Sugar beet | 10E | 8G | 4C,9G | 8H |
| Cotton | 4C,9G | 8G | 2C,9G | 3C,8H |

Table A (continued)

| | Compound 42 | | Compound 43 | |
|---|---|---|---|---|
| Rate kg/ha | 0.05 | 0.01 | 0.05 | 0.01 |
| POSTEMERGENCE | | | | |
| Morningglory | 3G | 6G | 3C,7G | 2C,6G |
| Cocklebur | 8H | 3G | 3C,8H | 5G |
| Velvetleaf | 2C,7G | 2G | 3C,7G | 4G |
| Nutsedge | 8G | 2G | 3C,8G | 3C,7G |
| Crabgrass | 3G | 0 | 5G | 0 |
| Giant Foxtail | 6G | 2G | 7G | 3G |
| Barnyardgrass | 3C,9H | 2C,5H | 3C,7H | 2H |
| Cheatgrass | 3C,9G | 7G | 3C,7G | 5G |
| Wild Oats | 9G | 3G | 3C,9G | 3G |
| Wheat | 9G | 5G | 7G | 2G |
| Corn | 2C,9H | 2C,7G | 3C,9G | 2C,7H |
| Barley | 2C,8G | 0 | 2C,5G | 0 |
| Soybean | 3C,9G | 3C,3H | 3C,7G | 2C,3H |
| Rice | 7G | 3G | 3C,6G | 3G |
| Sorghum | 3C,8H | 2C,5G | 5G | 0 |
| Sugar beet | 9C | 3C,7G | 3C,7G | 5G |
| Cotton | 10C | 5G | 2C,7G | 3G |
| PREEMERGENCE | | | | |
| Morningglory | 6G | 8H | 9G | 7G |
| Cocklebur | 8H | 8H | - | 7H |
| Velvetleaf | 7G | 7G | 7G | 7G |
| Nutsedge | 4G | 0 | 8G | 0 |
| Crabgrass | 0 | 0 | 5G | 5G |
| Giant Foxtail | 5G | 0 | 4G | 4G |
| Barnyardgrass | 5G | 0 | 6G | 3G |
| Cheatgrass | 5G | 0 | 6G | 3G |
| Wild Oats | 6G | 0 | 6G | 2G |
| Wheat | 5G | 0 | 6G | 0 |
| Corn | 7G | 6G | 7G | 4G |
| Barley | 5G | 0 | 7G | 2G |
| Soybean | 0 | 2G | 2C,2H | 0 |
| Rice | 6G | 3G | 8G | 5G |
| Sorghum | 5G | 2G | 3C,8G | 3G |
| Sugar beet | 6G | 6G | 8G | 5G |
| Cotton | 9G | 7G | 9G | 6G |

Table A (continued)

| | Compound 44 | | Compound 45 | |
|---|---|---|---|---|
| Rate kg/ha | 0.05 | 0.01 | 0.05 | 0.01 |
| POSTEMERGENCE | | | | |
| Morningglory | 10C | 10C | 9C | 9C |
| Cocklebur | 10C | 10C | 10C | 10C |
| Velvetleaf | 10C | 9C | 10C | 9C |
| Nutsedge | 3C,7G | 4C,9G | 3C,7G | 8G |
| Crabgrass | 6G | 0 | 7G | 3G |
| Giant Foxtail | 3C,8G | 3G | 2C,8G | 6G |
| Barnyardgrass | 5C,9G | 9H | 5C,9H | 7H |
| Cheatgrass | 4C,9G | 6G | 4C,8G | 3C,6G |
| Wild Oats | 3C,7G | 4G | 2C,8G | 2C,6G |
| Wheat | 2C,7G | 0 | 9G | 3G |
| Corn | 9G | 3C,9H | 9C | 3C,9G |
| Barley | 8G | 0 | 2C,9G | 5G |
| Soybean | 5C,9G | 4C,9G | 4C,9G | 5C,9G |
| Rice | 4C,9G | 5G | 5C,9G | 4C,9G |
| Sorghum | 3C,9H | 3C,7G | 2C,9H | 3C,7G |
| Sugar beet | 9C | 10C | 9C | 9C |
| Cotton | 10C | 10C | 9C | 10C |
| PREEMERGENCE | | | | |
| Morningglory | 9G | 8G | 9G | 7H |
| Cocklebur | 9H | 9H | 9H | 7H |
| Velvetleaf | 9C | 9G | 9C | 3C,7G |
| Nutsedge | 8G | 4G | 8G | 0 |
| Crabgrass | 0 | 3G | 5G | 3G |
| Giant Foxtail | 5G | 3G | 4G | 3G |
| Barnyardgrass | 3C,7G | 3G | 8G | 3C,5G |
| Cheatgrass | 7G | 5G | 9H | 7G |
| Wild Oats | 3C,6G | 0 | 2C,8G | 6G |
| Wheat | 6G | 0 | 9H | 6G |
| Corn | 3C,7G | 3C,7G | 3C,9H | 2C,8G |
| Barley | 8G | 0 | 9G | 7G |
| Soybean | 3C,7G | 6H | 3C,7H | 2C,6H |
| Rice | 4C,9H | 5G | 4C,9H | 8H |
| Sorghum | 4C,9H | 2C,5G | 3C,8H | 3C,8G |
| Sugar beet | 10E | 7G | 5C,9G | 3C,9G |
| Cotton | 9G | 8G | 9G | 9C |

Table A (continued)

| | Compound 46 | | Compound 47 | |
|---|---|---|---|---|
| Rate kg/ha | 0.05 | 0.01 | 0.05 | 0.01 |
| POSTEMERGENCE | | | | |
| Morningglory | 9C | 3C,7G | 2C,7G | 2C,5G |
| Cocklebur | 10C | 3C,9H | 9C | 2C,8G |
| Velvetleaf | 9C | 3C,8H | 2C,8G | 7G |
| Nutsedge | 7G | 0 | 8G | 0 |
| Crabgrass | 3G | 0 | 4G | 0 |
| Giant Foxtail | 3G | 2G | 2C,5G | 0 |
| Barnyardgrass | 0 | 0 | 3C,6H | 0 |
| Cheatgrass | 6G | 5G | 2C,5G | 0 |
| Wild Oats | 2C,9G | 6G | 6G | 0 |
| Wheat | 7G | 3G | 2G | 0 |
| Corn | 9G | 3C,8H | 2C,8H | 5G |
| Barley | 6G | 3G | 3G | 0 |
| Soybean | 4C,9G | 3C,9G | 3C,7G | 1H |
| Rice | 2C,9G | 8G | 7G | 2G |
| Sorghum | 2C,6G | 3C,6G | 7G | 2G |
| Sugar beet | 9C | 3C,6G | 3C,8G | 2C,4G |
| Cotton | 9C | 3C,6G | 4C,9G | 2C,8G |
| PREEMERGENCE | | | | |
| Morningglory | 9C | 2G | 9G | 5G |
| Cocklebur | 9H | 4H | 9H | 8H |
| Velvetleaf | 7H | 2G | 5G | 4G |
| Nutsedge | 0 | 0 | 0 | 0 |
| Crabgrass | 4G | 0 | 5G | 0 |
| Giant Foxtail | 0 | 0 | 4G | 0 |
| Barnyardgrass | 4G | 0 | 5G | 0 |
| Cheatgrass | 3C,8H | 5G | 2G | 0 |
| Wild Oats | 6G | 2G | 0 | 0 |
| Wheat | 5G | 0 | 0 | 0 |
| Corn | 8G | 5G | 2C,5G | 0 |
| Barley | 8G | 4G | 2G | 0 |
| Soybean | 2C,5H | 3G | 2G | 0 |
| Rice | 9H | 6G | 3G | 0 |
| Sorghum | 3C,7G | 2G | 3G | 0 |
| Sugar beet | 8G | 5G | 7G | 5G |
| Cotton | 9G | 5G | 9G | - |

Table A (continued)

| | Compound 48 | | Compound 49 | |
|---|---|---|---|---|
| Rate kg/ha | 0.05 | 0.01 | 0.05 | 0.01 |
| POSTEMERGENCE | | | | |
| Morningglory | 10C | 4C,9H | 5C,9G | 4C,9H |
| Cocklebur | 10C | 5C,9H | 9C | 10C |
| Velvetleaf | 9C | 4C,8H | 9C | 4C,8H |
| Nutsedge | 0 | 0 | 0 | 0 |
| Crabgrass | 2G | 0 | 0 | 0 |
| Giant Foxtail | 2G | 0 | 2G | 0 |
| Barnyardgrass | 2H | 0 | 3H | 0 |
| Cheatgrass | 0 | 0 | 0 | 0 |
| Wild Oats | 2G | 0 | 0 | 0 |
| Wheat | 2G | 0 | 0 | 0 |
| Corn | 6G | 0 | 4G | 0 |
| Barley | 0 | 0 | 0 | 0 |
| Soybean | 4C,9G | 4C,9G | 5C,9G | 4C,9G |
| Rice | 0 | 0 | 0 | 0 |
| Sorghum | 4G | 0 | 4G | 0 |
| Sugar beet | 9C | 9C | 9C | 9C |
| Cotton | 10C | 4C,9H | 10C | 4C,9G |
| PREEMERGENCE | | | | |
| Morningglory | 8G | 7H | 9G | 5H |
| Cocklebur | 9H | 8H | 9H | 4H |
| Velvetleaf | 7H | 2C,2G | 7G | 0 |
| Nutsedge | 0 | 0 | 0 | 0 |
| Crabgrass | 0 | 0 | 0 | 0 |
| Giant Foxtail | 0 | 0 | 2G | 0 |
| Barnyardgrass | 0 | 0 | 2G | 0 |
| Cheatgrass | 0 | 0 | 0 | 0 |
| Wild Oats | 0 | 0 | 0 | 0 |
| Wheat | 0 | 0 | 0 | 0 |
| Corn | 5G | 0 | 3G | 2G |
| Barley | 2G | 0 | 0 | 0 |
| Soybean | 4G | 0 | 3C,7H | 2C |
| Rice | 0 | 0 | 2G | 0 |
| Sorghum | 4G | 0 | 5G | 0 |
| Sugar beet | 8G | 3C,5G | 4C,9G | 5G |
| Cotton | 2C,6G | 3C,5G | 2C,9G | 6G |

Table A (continued)

| | Compound 50 | | Compound 51 | |
|---|---|---|---|---|
| Rate kg/ha | 0.05 | 0.01 | 0.05 | 0.01 |
| POSTEMERGENCE | | | | |
| Morningglory | 10C | 10C | 10C | 10C |
| Cocklebur | 10C | 10C | 10C | 10C |
| Velvetleaf | 10C | 10C | 10C | 10C |
| Nutsedge | 5C,9G | 2C,9G | 9G | 9G |
| Crabgrass | 5G | 0 | 2C,7G | 4G |
| Giant Foxtail | 7G | 3G | 4C,9G | 7G |
| Barnyardgrass | 9H | 8H | 9C | 3C,9H |
| Cheatgrass | 9C | 9G | 6C,9G | 4C,9G |
| Wild Oats | 3C,8G | 1C | 4C,9G | 3C,9G |
| Wheat | 7G | 2G | 9G | 5G |
| Corn | 5C,9G | 3C,7H | 6C,9G | 3C,9H |
| Barley | 8G | 2C,5G | 9H | 8G |
| Soybean | 9C | 9C | 9C | 9C |
| Rice | 4C,9G | 7G | 6C,9G | 3C,8G |
| Sorghum | 3C,9H | 2C,9G | 6C,9G | 2C,9G |
| Sugar beet | 10C | 10C | 10C | 10C |
| Cotton | 9C | 10C | 9C | 9C |
| PREEMERGENCE | | | | |
| Morningglory | 9C | 9G | 9G | 9G |
| Cocklebur | 9H | 9H | 9H | - |
| Velvetleaf | 9C | - | 10C | - |
| Nutsedge | 10E | 10E | 10E | 10E |
| Crabgrass | 5G | 7G | 3C,7G | 4G |
| Giant Foxtail | 2C,8G | 3C,7G | 5C,9H | 3C,8G |
| Barnyardgrass | 3C,9H | 3C,8H | 9H | 3C,9H |
| Cheatgrass | 10E | 9H | 10E | 9C |
| Wild Oats | 3C,8G | 5G | 9C | 9C |
| Wheat | 2C,8G | 3G | 6C,9G | 8G |
| Corn | 9H | 7H | 2C,9G | 2C,9G |
| Barley | 8G | 8G | 9G | 9G |
| Soybean | 8H | 7H | 9H | 3C,8H |
| Rice | 3C,9H | 3C,8H | 5C,9H | 3C,9H |
| Sorghum | 9H | 3C,9H | 5C,9H | 3C,9G |
| Sugar beet | 9C | 9C | 9C | 9C |
| Cotton | 9C | 10C | 9G | 9G |

Table A (continued)

| | Compound 52 | | Compound 53 | |
|---|---|---|---|---|
| Rate kg/ha | 0.05 | 0.01 | 0.05 | 0.01 |
| POSTEMERGENCE | | | | |
| Morningglory | 10C | 10C | 10C | 10C |
| Cocklebur | 10C | 10C | 10C | 2C,9H |
| Velvetleaf | 10C | 10C | 9C | 8H |
| Nutsedge | 5C,9G | 2C,9G | 4C,9G | 2C,5G |
| Crabgrass | 7G | 0 | 3G | 0 |
| Giant Foxtail | 2C,8G | 4G | 2G | 0 |
| Barnyardgrass | 5C,9H | 3C,9H | 3C,7H | 3C,3H |
| Cheatgrass | 4C,9G | 9G | 6G | 2G |
| Wild Oats | 9G | 3C,9G | 2C,5G | 2C |
| Wheat | 2C,9G | 8G | 4G | 0 |
| Corn | 2C,9G | 4C,9H | 3C,6G | 2H |
| Barley | 2C,9G | 7G | 4G | 0 |
| Soybean | 9C | 9C | 9C | 2C,8G |
| Rice | 9C | 5C,9G | 5G | 2G |
| Sorghum | 3C,9G | 9G | 3C,9H | 2C,8H |
| Sugar beet | 10C | 10C | 10C | 9C |
| Cotton | 10C | 10C | 10C | 10C |
| PREEMERGENCE | | | | |
| Morningglory | 9G | 5G | 8G | 7H |
| Cocklebur | 9H | 9H | - | 7H |
| Velvetleaf | 9C | 8G | 7G | 5G |
| Nutsedge | 10E | 9G | 9G | 0 |
| Crabgrass | 3C,7G | 0 | 5G | 0 |
| Giant Foxtail | 3C,7G | 3C,5G | 3C,3G | 2G |
| Barnyardgrass | 3C,9H | 3C,7H | 7G | 7G |
| Cheatgrass | 5C,9H | 8H | 4G | 0 |
| Wild Oats | 3C,7H | 7G | 6G | 1C |
| Wheat | 9H | 8G | 2G | 0 |
| Corn | 9G | 2C,7G | 2C,7G | 5G |
| Barley | 9G | 2C,9G | 6G | 3G |
| Soybean | 9H | 7H | 2C,6H | 5H |
| Rice | 10E | 4C,9H | 7G | 5G |
| Sorghum | 6C,9H | 9H | 9G | 8G |
| Sugar beet | 9C | 9C | 5C,9G | 8G |
| Cotton | 9G | 8G | 9G | 8G |

Table A (continued)

| | Compound 54 | | Compound 55 | |
|---|---|---|---|---|
| Rate kg/ha | 0.05 | 0.01 | 0.05 | 0.01 |
| POSTEMERGENCE | | | | |
| Morningglory | 10C | 10C | 10C | 5C,9G |
| Cocklebur | 10C | 9C | 10C | 5C,9H |
| Velvetleaf | 10C | 9C | 9C | 7G |
| Nutsedge | 9G | 5G | 5G | 0 |
| Crabgrass | 2G | 0 | 4G | 0 |
| Giant Foxtail | 5G | 0 | 4G | 0 |
| Barnyardgrass | 9C | 3C,4H | 9H | 3C,7H |
| Cheatgrass | 5G | 2G | 7G | 0 |
| Wild Oats | 5G | 0 | 3C,3G | 0 |
| Wheat | 3G | 0 | 4G | 0 |
| Corn | 3C,9G | 4G | 2C,5H | 0 |
| Barley | 4G | 2G | 2C | 0 |
| Soybean | 9C | 5C,9G | 5C,9G | 4C,9G |
| Rice | 5C,9G | 6G | 4C,9G | 5G |
| Sorghum | 3C,8G | 3G | 3C,7G | 2G |
| Sugar beet | 10C | 10C | 10C | 9C |
| Cotton | 9C | 4C,9G | 10C | 4C,9G |
| PREEMERGENCE | | | | |
| Morningglory | 9G | 9G | 9H | 8H |
| Cocklebur | 9G | 3C,8H | - | 3C,5H |
| Velvetleaf | 4C,9G | 3C,5H | 6H | - |
| Nutsedge | 10E | 5C,9G | 4C,9G | 0 |
| Crabgrass | 0 | 0 | 7G | 0 |
| Giant Foxtail | 3C,5G | 4G | 2C,7G | 3G |
| Barnyardgrass | 3C,7G | 5G | 5C,9H | 3C,7G |
| Cheatgrass | 6G | 5G | 5C,9H | 3G |
| Wild Oats | 2C,6G | 0 | 2C,2G | 0 |
| Wheat | 5G | 0 | 4G | 3G |
| Corn | 3C,7G | 7G | 2C,7G | 5G |
| Barley | 9G | 3G | 9G | 3G |
| Soybean | 9H | 3C,6G | 9H | 3C,7G |
| Rice | 4C,9H | 7H | 10E | 9H |
| Sorghum | 3C,8G | 3C,7G | 3C,7G | 3C,5G |
| Sugar beet | 9C | 9C | 5C,9G | 4C,8G |
| Cotton | 4C,9G | 4C,8H | 4C,8G | 4C,7G |

Table A (continued)

| | Cmpd. 56 | Cmpd. 57 | Cmpd. 58 | Cmpd. 59 |
|---|---|---|---|---|
| Rate kg/ha | 0.05 | 0.05 | 0.05 | 0.05 |
| POSTEMERGENCE | | | | |
| Morningglory | 5C,9G | 9C | 4C,9G | 3C,7G |
| Cocklebur | 6G | 5C,9G | 10C | 4C,9H |
| Velvetleaf | 4C,9H | 5G | 9C | 4C,9G |
| Nutsedge | 5G | 7G | 5C,9G | 4C,9G |
| Crabgrass | 5G | 0 | 0 | 2C,5G |
| Giant Foxtail | - | - | 4G | 4C,9G |
| Barnyardgrass | 5C,9H | 2C,7H | 4H | 4C,9H |
| Cheatgrass | 2C,5G | 3C,7G | 0 | 6C,9G |
| Wild Oats | 2C,5G | 2C,5G | 2G | 3C,8G |
| Wheat | 0 | 0 | 0 | 8G |
| Corn | 4C,9H | 3C,8H | 8H | 3C,9H |
| Barley | - | - | 0 | 9C |
| Soybean | 3C,6H | 4C,9G | 4C,9G | 3H,6G |
| Rice | 5C,9G | 5C,9G | 3G | 9C |
| Sorghum | 9H | 4C,9H | 3C,4G | 4C,9G |
| Sugar beet | 5C,9G | 9C | 9C | 5C,9G |
| Cotton | 4C,8H | 4C,8H | 5C,9G | 4C,9H |
| PREEMERGENCE | | | | |
| Morningglory | 9G | 8G | 9G | 0 |
| Cocklebur | 5G | 7G | 9H | 0 |
| Velvetleaf | 5C,9G | 7G | 4C,9G | 0 |
| Nutsedge | 5G | 3G | 10E | 0 |
| Crabgrass | 3G | 0 | 0 | 0 |
| Giant Foxtail | - | - | 4G | 0 |
| Barnyardgrass | 9H | 2G | 3C,6G | 0 |
| Cheatgrass | 9G | 2C,6G | 3G | 0 |
| Wild Oats | 3C,7G | 0 | 2C,5G | 0 |
| Wheat | 0 | 0 | 5G | 4G |
| Corn | 3C,8H | 3C,6G | 3C,6G | 0 |
| Barley | - | - | 4G | 8G |
| Soybean | 6H | 3C,4H | 3C,6H | 0 |
| Rice | 10E | 3C,7G | 8G | 0 |
| Sorghum | 3C,8G | 3C,7G | 2C,4G | 0 |
| Sugar beet | 5C,9G | 5C,9G | - | 4H |
| Cotton | 9G | 4G | 9G | 0 |

Table A (continued)

| | Cmpd. 60 | Cmpd. 61 | Compound 62 | |
|---|---|---|---|---|
| Rate kg/ha | 0.05 | 0.05 | 0.05 | 0.01 |
| POSTEMERGENCE | | | | |
| Morningglory | 3C,7H | 1C | 10C | 3C,7G |
| Cocklebur | 4G | 3C,9G | 2C,7H | 1H |
| Velvetleaf | 3C,8H | 2C,8G | 10C | 9C |
| Nutsedge | 2C,5G | 2C,5G | 2C,8G | 4G |
| Crabgrass | 0 | 3C,7G | 0 | 0 |
| Giant Foxtail | 2C,6G | 9C | 3G | 0 |
| Barnyardgrass | 3C,6H | 5C,9G | 9H | 0 |
| Cheatgrass | 3C,7G | 2C,9G | 2C,9G | 2G |
| Wild Oats | 0 | 4C,9G | 2C,7G | 0 |
| Wheat | 3G | 9G | 4G | 0 |
| Corn | 3C,8H | 5C,9G | 4C,9H | 2H |
| Barley | 3G | 3C,9G | 4G | 0 |
| Soybean | 3H | 3C,9G | 2C,5H | 1H |
| Rice | 5C,9G | 9C | 3C,7G | 5G |
| Sorghum | 4C,9G | 3C,9G | 2C,8H | 4G |
| Sugar beet | 9C | 3C,7G | 3C,9H | 9H |
| Cotton | 3C,8H | 3C,6G | 10C | 2C,9G |
| PREEMERGENCE | | | | |
| Morningglory | 0 | 0 | 4G | 5G |
| Cocklebur | 1H | 0 | 7G | 3G |
| Velvetleaf | 4G | 0 | 9G | 3G |
| Nutsedge | 0 | 0 | 0 | 0 |
| Crabgrass | 0 | 0 | 3G | 2G |
| Giant Foxtail | 0 | 2G | 10H | 0 |
| Barnyardgrass | 0 | 3G | 3G | 0 |
| Cheatgrass | 0 | 5G | 5G | 0 |
| Wild Oats | 0 | 3G | 5G | 0 |
| Wheat | 0 | 7G | 3G | 0 |
| Corn | 0 | 2C,4G | 5G | 0 |
| Barley | 0 | 6G | 0 | 0 |
| Soybean | 0 | 2C,2H | 4H | 0 |
| Rice | 0 | 2C,8G | 2C,8G | 4G |
| Sorghum | 0 | 3C,6G | 2C,5G | 0 |
| Sugar beet | 2H | 0 | 8G | 7G |
| Cotton | 0 | 0 | 8G | 4G |

Table A (continued)

| | Compound 63 | | Compound 64 | |
|---|---|---|---|---|
| Rate kg/ha | 0.05 | 0.01 | 0.05 | 0.01 |
| POSTEMERGENCE | | | | |
| Morningglory | 10C | 10C | 2C,7G | 1H |
| Cocklebur | 2C,9H | 3H | 3G | 0 |
| Velvetleaf | 9C | 9C | 10C | 2C,8H |
| Nutsedge | 4G | 0 | 0 | 0 |
| Crabgrass | 2G | 0 | 0 | 0 |
| Giant Foxtail | 5G | 0 | 5G | 2G |
| Barnyardgrass | 8H | 2H | 8G | 0 |
| Cheatgrass | 7G | 3G | 2C,5G | 0 |
| Wild Oats | 2C,5G | 0 | 2C,4G | 0 |
| Wheat | 0 | 0 | 0 | 0 |
| Corn | 5H | 0 | 0 | 2C |
| Barley | 2G | 0 | 0 | 0 |
| Soybean | 3C,7H | 3H | 2C,7G | 3C,4G |
| Rice | 2C,5G | 0 | 0 | 0 |
| Sorghum | 6G | 4G | 5G | 1C,6G |
| Sugar beet | 3C,9H | 3C,8H | 9C | 3C,9G |
| Cotton | 9C | 10C | 2C,9H | 2C,7H |
| PREEMERGENCE | | | | |
| Morningglory | 4G | 3G | 0 | 0 |
| Cocklebur | 3G | 5H | 0 | 0 |
| Velvetleaf | 8G | 0 | 2H | 0 |
| Nutsedge | 3G | 0 | 0 | 0 |
| Crabgrass | 4G | 0 | 0 | 0 |
| Giant Foxtail | 7G | 0 | 2G | 0 |
| Barnyardgrass | 0 | 0 | 0 | 0 |
| Cheatgrass | 5G | 0 | 0 | 0 |
| Wild Oats | 0 | 0 | 0 | 0 |
| Wheat | 2G | 0 | 0 | 0 |
| Corn | 3G | 0 | 0 | 0 |
| Barley | 3G | 0 | 0 | 0 |
| Soybean | 5H | 0 | 0 | 0 |
| Rice | 3C,8G | 0 | 0 | 0 |
| Sorghum | 3C,6G | 0 | 0 | 0 |
| Sugar beet | 8G | 7G | 10E | 0 |
| Cotton | 5G | 5G | 0 | - |

Table A (continued)

| | Compound 65 | | Compound 66 | |
|---|---|---|---|---|
| Rate kg/ha | 0.05 | 0.01 | 0.05 | 0.01 |
| POSTEMERGENCE | | | | |
| Morningglory | 3C,7G | 2G | 10C | 3C,8G |
| Cocklebur | 9C | 4G | 9C | 5G |
| Velvetleaf | 9C | 9C | 10C | 10C |
| Nutsedge | 2G | 0 | 9G | 2C,8G |
| Crabgrass | 2G | 0 | 2C,6G | 3G |
| Giant Foxtail | 2C,4G | 0 | 8G | 2C,4G |
| Barnyardgrass | 3C,9H | 2C,5H | 9H | 3C,8H |
| Cheatgrass | 4C,9G | 9G | 8G | 2C,8G |
| Wild Oats | 3C,9G | 3C,6G | 3C,9G | 2C,3G |
| Wheat | 9G | 4G | 9G | 2G |
| Corn | 4C,9G | 3C,6G | 3C,9H | 3C,9H |
| Barley | 3C,8G | 0 | 3C,9G | 3G |
| Soybean | 9C | 5C,9G | 9C | 9C |
| Rice | 5C,9G | 3C,9G | 4C,9G | 3C,8G |
| Sorghum | 2C,9G | 3C,8G | 9G | 5C,9G |
| Sugar beet | 9C | 9C | 10C | 10C |
| Cotton | 9C | 5C,9G | 10C | 10C |
| PREEMERGENCE | | | | |
| Morningglory | 6G | 2H | 8G | 9G |
| Cocklebur | 9H | 8G | 3H | 9H |
| Velvetleaf | 9G | 3G | 9G | 9G |
| Nutsedge | 5G | 0 | 10E | 6G |
| Crabgrass | 0 | 0 | 1C | 0 |
| Giant Foxtail | 0 | 0 | 3C,7G | 3C,7G |
| Barnyardgrass | 3C,7H | 0 | 9H | 9H |
| Cheatgrass | 9H | 5G | 3C,9H | 9H |
| Wild Oats | 8G | 2C,7G | 3C,8G | 3C,6G |
| Wheat | 8G | 3G | 8G | 6G |
| Corn | 3C,9G | 4G | 2U,9G | 2C,8G |
| Barley | 9G | 8G | 7G | 7G |
| Soybean | 3C,8H | 5G | 3C,8H | 3C,8H |
| Rice | 9H | 2C,8H | 3C,9G | 3C,9H |
| Sorghum | 3C,9H | 2C,3G | 9G | 3C,9G |
| Sugar beet | 9C | 4C,9G | 9C | 5C,9G |
| Cotton | 9G | 8G | 9G | 9G |

Table A (continued)

| | Compound 67 | | Cmpd. 68 | Cmpd. 69 | Compound 70 | |
|---|---|---|---|---|---|---|
| Rate kg/ha | 0.05 | 0.01 | 0.05 | 0.05 | 0.05 | 0.01 |
| POSTEMERGENCE | | | | | | |
| Morningglory | 10C | 2C,8G | 5C,9G | 2C,3G | 3C,7G | 1H |
| Cocklebur | 10C | 7G | 5C,9G | 3C,9H | 2C,5G | 1H |
| Velvetleaf | 10C | 10C | 3C,5G | 2G | 4G | 0 |
| Nutsedge | 3C,9G | 3C,6G | 2C,5G | 0 | 3G | 0 |
| Crabgrass | 4G | 0 | 0 | 2G | 0 | 0 |
| Giant Foxtail | 2C,7G | 3G | - | - | 0 | 0 |
| Barnyardgrass | 3C,8H | 2C,3H | 0 | 2G | 0 | 0 |
| Cheatgrass | 2C,9G | 4G | 0 | 0 | 0 | 0 |
| Wild Oats | 2C,3G | 0 | 0 | 0 | 0 | 0 |
| Wheat | 2G | 0 | 0 | 0 | 0 | 0 |
| Corn | 3C,7H | 3G | 0 | 0 | 0 | 0 |
| Barley | 3G | 0 | - | - | 0 | 0 |
| Soybean | 9C | 5C,9G | 5C,9G | 2H | 3C,8H | 3C,5H |
| Rice | 2C,7G | 2G | 0 | 0 | 0 | 0 |
| Sorghum | 3C,9H | 3C,7G | 0 | 0 | 0 | 0 |
| Sugar beet | 9C | 10C | 2C,4G | 3G | 1H | 0 |
| Cotton | 10C | 10C | 2C,4G | 5G | 1H | 0 |
| PREEMERGENCE | | | | | | |
| Morningglory | 9G | 6G | 6G | 0 | 2H | 0 |
| Cocklebur | 9H | 9H | 0 | - | 3H | - |
| Velvetleaf | 9G | 9G | 3G | 0 | 5G | 0 |
| Nutsedge | 10E | 4G | 0 | 0 | 0 | 0 |
| Crabgrass | 0 | 0 | 0 | 0 | 0 | 0 |
| Giant Foxtail | 3C,7G | 2C,4G | - | - | 2C | 0 |
| Barnyardgrass | 3C,7G | 2C,3G | 0 | 0 | 3G | 0 |
| Cheatgrass | 3C,9H | 6G | 0 | 0 | 0 | 0 |
| Wild Oats | 2C,6G | 0 | 0 | 0 | 0 | 0 |
| Wheat | 3G | 0 | 0 | 0 | 0 | 0 |
| Corn | 2C,7G | 7G | 0 | 0 | 0 | 0 |
| Barley | 4G | 2G | - | - | 0 | 0 |
| Soybean | 3C,8H | 6G | 1C | 0 | 3G | 0 |
| Rice | 7G | 3G | 3C | 0 | 0 | 0 |
| Sorghum | 8G | 3C,7G | 0 | 3G | 3G | 0 |
| Sugar beet | 9C | 9C | 5G | 3G | 6G | 5G |
| Cotton | 9G | 9G | 2G | 0 | - | - |

Table A (continued)

| | Cmpd. 71 | Cmpd. 72 | Cmpd. 73 |
|---|---|---|---|
| Rate (kg/ha) | 0.05 | 0.05 | 0.05 |
| POSTEMERGENCE | | | |
| Morningglory | 10C | 1C,1H | 9C |
| Cocklebur | 10C | 1C | 5C,9G |
| Velvetleaf | 9C | 0 | 2C,6G |
| Nutsedge | 3C,8G,9X | 0 | 5G |
| Crabgrass | 4C,9G | 0 | 5C,9G |
| Barnyardgrass | 9C | 0 | 9C |
| Cheatgrass | 9C | 0 | 8G |
| Wild Oats | 4C,8G | 2C,2H | 9C |
| Wheat | 3G | 0 | 9C |
| Corn | 5U,9G | 8G | 10C |
| Soybean | 9C | 3C | 9C |
| Rice | 9C | 5G | 6C,9G |
| Sorghum | 9C | 4G | 9C |
| Sugar Beets | 9C | 3C,3H | 9C |
| Cotton | 9C | 2C,2H | 5C,9G |
| PREEMERGENCE | | | |
| Morningglory | 9G | 2H | 8H |
| Cocklebur | 9H | 2H | 9H |
| Velvetleaf | 9C | 7G | 7G |
| Nutsedge | 8G | 0 | 4G |
| Crabgrass | 2C,6G | 2G | 2G |
| Barnyardgrass | 4C,9H | 2G | 2C,9H |
| Cheatgrass | 5C,9H | 0 | 7H |
| Wild Oats | 4C,9G | 0 | 9C |
| Wheat | 4C,8G | 0 | 9H |
| Corn | 4C,9H | 2G | 4C,9H |
| Soybean | 3C,7G | 0 | 5G |
| Rice | 10E | 0 | 10E |
| Sorghum | 10E | 0 | 10E |
| Sugar Beets | 5C,9G | | 10E |
| Cotton | 4C,9G | 0 | 8G |

Table A (continued)

| | Cmpd. 74 | Cmpd. 75 | Cmpd. 76 |
|---|---|---|---|
| Rate (kg/ha) | 0.05 | 0.05 | 0.05 |
| POSTEMERGENCE | | | |
| Morningglory | 10C | 2C,6G | 2C,5G |
| Cocklebur | 9C | 2C,3H | 3C,9H |
| Velvetleaf | 10C | 0 | 3G |
| Nutsedge | 3C,9G | 0 | 2C,4G |
| Crabgrass | 3C,9H | 0 | 0 |
| Barnyardgrass | 9C | 3C,8H | 3C,5H |
| Cheatgrass | 7G | 0 | 6G |
| Wild Oats | 4C,8G | 4C,8G | 6G |
| Wheat | 0 | 0 | 4G |
| Corn | 6C,9G | 5C,9G | 3C,7H |
| Soybean | 6C,9G | 4C,8G | 5C,9G |
| Rice | 9C | 4C,8G | 5G |
| Sorghum | 5C,9G | 3C,6H | 4C,8H |
| Sugar Beets | 9C | 4C,7G | 2H |
| Cotton | 9C | 4C,6G | 4C,8H |
| PREEMERGENCE | | | |
| Morningglory | 9G | 3G | 7H |
| Cocklebur | 9H | 3G | 2H |
| Velvetleaf | 8G | 0 | 3G |
| Nutsedge | 9G | 8G | 0 |
| Crabgrass | 9G | 0 | 4G |
| Barnyardgrass | 5C,9H | 2C | 0 |
| Cheatgrass | 2C,7G | 0 | 7G |
| Wild Oats | 3C,8G | 0 | 0 |
| Wheat | 7G | 0 | 0 |
| Corn | 3U,9H | 2C,5G | 0 |
| Soybean | 4C,8H | 2G | 10E |
| Rice | 10E | 7G | 0 |
| Sorghum | 4C,9H | 2C,6G | 3G |
| Sugar Beets | 10C | 10C | 8G |
| Cotton | 5C,9G | 0 | 0 |

Table A (continued)

| | Cmpd. 77 | Cmpd. 78 |
|---|---|---|
| Rate (kg/ha) | 0.05 | 0.05 |
| POSTEMERGENCE | | |
| Morningglory | 5C,9G | 4C,9G |
| Cocklebur | 6C,9G | 5C,9G |
| Velvetleaf | 3C,8H | 5G |
| Nutsedge | 5G | 2G |
| Crabgrass | 3G | 0 |
| Barnyardgrass | 3C,8H | 0 |
| Cheatgrass | 2C,8G | 0 |
| Wild Oats | 2C,5G | 0 |
| Wheat | 2C,5G | 0 |
| Corn | 3C,9G | 0 |
| Soybean | 9C | 3C,8G |
| Rice | 3C,8G | 5G |
| Sorghum | 2C,9G | 3C,7G |
| Sugar Beets | 3C,5G | 2H |
| Cotton | 3C,6G | 5G |
| PREEMERGENCE | | |
| Morningglory | 9G | 3G |
| Cocklebur | 9H | 9G |
| Velvetleaf | 9C | 0 |
| Nutsedge | 3G | 0 |
| Crabgrass | 2G | 0 |
| Barnyardgrass | 2C,5G | 0 |
| Cheatgrass | 8G | 0 |
| Wild Oats | 2C,6G | 0 |
| Wheat | 7G | 2G |
| Corn | 2C,8H | 2G |
| Soybean | 3C,6H | 2H |
| Rice | 3C,7H | 0 |
| Sorghum | 3C,9H | 2C |
| Sugar Beets | 4C,9G | 6G |
| Cotton | 5G | 0 |

Table A (continued)

| | Cmpd. 79 | Cmpd. 80 | Cmpd. 81 |
|---|---|---|---|
| Rate kg/ha | 0.05 | 0.05 | 0.05 |
| POSTEMERGENCE | | | |
| Morningglory | 10C | 4C,8G | 6C,9G |
| Cocklebur | 10C | 4C,9G | 4C,9G |
| Velvetleaf | 4C,9G | 4C,8H | 4C,9G |
| Nutsedge | 0 | 0 | 0 |
| Crabgrass | 3G | 0 | 0 |
| Giant Foxtail | 4G | 0 | 0 |
| Barnyardgrass | 2C,8G | 1C | 0 |
| Cheatgrass | 2C,8G | 0 | 0 |
| Wild Oats | 5G | 0 | 0 |
| Wheat | 4G | 0 | 0 |
| Corn | 3C,7G | 1H | 0 |
| Barley | 2C,7G | 0 | 0 |
| Soybean | 5H | 0 | 0 |
| Rice | 7G | 0 | 2G |
| Sorghum | 2C,9G | 2C,5G | 4G |
| Sugar beet | 3C,7G | 9C | 9C |
| Cotton | 9C | 8G | 2C,9G |
| PREEMERGENCE | | | |
| Morningglory | 9G | 5G | 9C |
| Cocklebur | - | 9H | - |
| Velvetleaf | 10C | 9C | 2G |
| Nutsedge | 0 | 0 | 0 |
| Crabgrass | 7G | 0 | 0 |
| Giant Foxtail | 5G | 0 | 0 |
| Barnyardgrass | 2C,8G | 2C | 0 |
| Cheatgrass | 7G | 0 | 0 |
| Wild Oats | 5G | 0 | 0 |
| Wheat | 5G | 0 | 0 |
| Corn | 2C,7G | 0 | 0 |
| Barley | 8G | 0 | 0 |
| Soybean | 2G | 2G | 2C,5G |
| Rice | 7G | 2G | 0 |
| Sorghum | 2C,8G | 3G | 4G |
| Sugar beet | 8G | 7G | 4C,8G |
| Cotton | 7G | 4G | 5G |

Table A (continued)

| | Compound 82 | | Cmpd. 83 | Cmpd. 84 |
|---|---|---|---|---|
| Rate kg/ha | 0.05 | 0.01 | 0.05 | 0.05 |
| POSTEMERGENCE | | | | |
| Morningglory | 9C | 2G | 10C | 10C |
| Cocklebur | 10C | 2C,9H | 9C | 9C |
| Velvetleaf | 10C | 5C,9G | 4C,8G | 9C |
| Nutsedge | 6G | 2G | 4G | 4C,8G |
| Crabgrass | 0 | 0 | 0 | 0 |
| Giant Foxtail | 2G | 0 | 4G | 4C,9G |
| Barnyardgrass | 7H | 3H | 8H | 10C |
| Cheatgrass | 7G | 6G | 0 | 7G |
| Wild Oats | 0 | 0 | 3C,6G | 0 |
| Wheat | 5G | 0 | 4G | 0 |
| Corn | 1H | 0 | 3C,8H | 9G |
| Barley | 7G | 3G | 0 | 2C |
| Soybean | 2C,8G | 5H | 5C,9G | 9C |
| Rice | 8G | 3G | 2C,6G | 2C,9G |
| Sorghum | 2C,8H | 7G | 3C,8H | 4C,9G |
| Sugar beet | 9C | 5C,9G | 10C | 10C |
| Cotton | 3C,9G | 4C,8G | 5C,9G | 9C |
| PREEMERGENCE | | | | |
| Morningglory | 5G | 5G | 8G | 9G |
| Cocklebur | - | 7G | 9H | 9H |
| Velvetleaf | 7G | 6G | 9G | 9G |
| Nutsedge | 4G | 0 | 0 | 5G |
| Crabgrass | 0 | 0 | 4G | - |
| Giant Foxtail | 4G | 2C | 2G | 6G |
| Barnyardgrass | 2C,7G | 2C | 2G | 8G |
| Cheatgrass | 9H | 7G | 0 | 7G |
| Wild Oats | 0 | 0 | 0 | 0 |
| Wheat | 0 | 0 | 0 | 0 |
| Corn | 6G | 5G | 3C,7H | 3C,8G |
| Barley | 7G | 0 | 0 | 5G |
| Soybean | 0 | 0 | 3C,7H | 9H |
| Rice | 6G | 0 | 3G | 6G |
| Sorghum | 2C,8H | 3G | 4G | 4C,9H |
| Sugar beet | 8G | 5G | 10C | 10C |
| Cotton | 5G | - | 3C,7G | 9G |

Table A (continued)

| | Cmpd. 85 | Cmpd. 86 | Cmpd. 87 | Cmpd. 88 |
|---|---|---|---|---|
| Rate kg/ha | 0.05 | 0.05 | 0.05 | 0.05 |
| POSTEMERGENCE | | | | |
| Morningglory | 5C,9G | 10C | 10C | 10C |
| Cocklebur | 5C,9H | 10C | 10C | 10C |
| Velvetleaf | 2G | 5G | 4C,9G | 9C |
| Nutsedge | 0 | 2C,9G | 3C,7G | 5C,9G |
| Crabgrass | 0 | 6G | 2G | 3G |
| Giant Foxtail | 0 | 5C,9G | 4C,8G | 3C,8G |
| Barnyardgrass | 5H | 10C | 4C,9H | 4C,9H |
| Cheatgrass | 0 | 9G | 7G | 4C,9G |
| Wild Oats | 0 | 3G | 0 | 0 |
| Wheat | 0 | 3G | 3G | 0 |
| Corn | 4G | 9C | 9C | 10C |
| Barley | 0 | 2C,5G | 0 | 0 |
| Soybean | 3C,8G | 4C,9G | 4C,9G | 9C |
| Rice | 6G | 9C | 5C,9G | 8G |
| Sorghum | 4C,9H | 4C,9G | 4C,9H | 9H |
| Sugar beet | 4C,8G | 10C | 10C | 10C |
| Cotton | 3C,7G | 4C,9G | 10C | 10C |
| PREEMERGENCE | | | | |
| Morningglory | 8G | 9G | 9G | 9G |
| Cocklebur | - | 9H | 9H | 9H |
| Velvetleaf | 7G | 9G | 9G | 9G |
| Nutsedge | 0 | 9G | 8G | 10E |
| Crabgrass | - | 6G | 5G | 5G |
| Giant Foxtail | 3G | 3C,7G | 3C,7G | 3C,7G |
| Barnyardgrass | 2G | 3C,9H | 4C,9H | 9H |
| Cheatgrass | 0 | 8G | 8G | 9H |
| Wild Oats | 0 | 5G | 5G | 4G |
| Wheat | 0 | 4G | 2G | 0 |
| Corn | 3G | 9H | 3C,9H | 3C,9H |
| Barley | 0 | 2C,8G | 5G | 6G |
| Soybean | 0 | 7H | 9H | 9H |
| Rice | 3G | 4C,8H | 8G | 8G |
| Sorghum | 0 | 8G | 3C,8G | 7G |
| Sugar beet | 8G | 10E | 10E | 10E |
| Cotton | 8G | 9G | 9G | 9G |

Table A (continued)

| | Cmpd. 89 | Cmpd. 90 | Cmpd. 91 | Cmpd. 92 |
|---|---|---|---|---|
| Rate kg/ha | 0.05 | 0.05 | 0.05 | 0.05 |
| POSTEMERGENCE | | | | |
| Morningglory | 10C | 4C,8H | 10C | 4C,9G |
| Cocklebur | 10C | 4C,9G | 9C | 4C,9H |
| Velvetleaf | 3C,7G | 3C,7H | 4C,8G | 4C,8G |
| Nutsedge | 4G | 0 | 2C | 2G |
| Crabgrass | 3G | 0 | 3G | 2G |
| Giant Foxtail | 3C,5G | 2C,8G | 2G | 0 |
| Barnyardgrass | 3C,8H | 5C,9H | 2C,5H | 0 |
| Cheatgrass | 3C,6G | 0 | 0 | 0 |
| Wild Oats | 0 | 0 | 0 | 0 |
| Wheat | 2G | 0 | 0 | 0 |
| Corn | 9H | 10C | 8H | 3H |
| Barley | 0 | 0 | 0 | 0 |
| Soybean | 4C,9G | 2C,7G | 4C,9G | 4C,9G |
| Rice | 8G | 7G | 5G | 4G |
| Sorghum | 4C,9H | 4C,9H | 3C,8G | 2G |
| Sugar beet | 9C | 4C,8H | 3C,7G | 2C,6G |
| Cotton | 10C | 3C,7G | 4C,8H | 3C,6G |
| PREEMERGENCE | | | | |
| Morningglory | 8G | 7H | 9G | 7H |
| Cocklebur | 8H | 7G | 9H | 7H |
| Velvetleaf | 9G | 0 | 8G | 7G |
| Nutsedge | 9G | 0 | 0 | 0 |
| Crabgrass | 2G | 0 | 3G | - |
| Giant Foxtail | 2C,4G | 0 | 3G | 0 |
| Barnyardgrass | 3C,7G | 0 | 0 | 0 |
| Cheatgrass | 5G | 0 | 0 | 0 |
| Wild Oats | 0 | 0 | 0 | 0 |
| Wheat | 0 | 0 | 0 | 0 |
| Corn | 7G | 7G | 5G | 0 |
| Barley | 0 | 0 | 0 | 0 |
| Soybean | 0 | 0 | 3C,6H | 2C,4H |
| Rice | 5G | 5G | 2C,5G | 0 |
| Sorghum | 6G | 0 | 2C,4G | 2C |
| Sugar beet | 4C,9G | 9C | 8G | 8G |
| Cotton | 2C,8G | 4G | 2C,6G | 7G |

REFERENCES


1. Scott and Kearse, J. Org. Chem., 5, 598 (1940).
2. Jones et. al., J. Am. Chem. Soc., 48, 181 (1926).
3. Jones et. al., IBID., 49, 2528 (1927).
4. Z. N. Parnes et al., Izv. Akad. Nauk SSSR, Ser. Khim., (11) 2526 (1977); CA., 88 62239k.
5. E. Spath and F. Kuffner., Ber., 68, 2238 (1936).
6. D.S. Tarbell and C. Weaver, J. Am. Chem. Soc., 63, 2939 (1941).

7. H. O. House, "Modern Synthetic Reactions," 2nd Ed., W. A. Benjamin, FNC., Menlo Park, 1972, pp. 334-335 and references cited within.
8. B. M. Trost and T. H. Salzmann, J. Am. Chem. Soc. , 95 , 6840 (1973). This procedure can be utilized for the synthesis of $\alpha,\beta$-unsaturated lactams, sulfones and sultams. Literature procedures for the formation of $\alpha$-anions of the above active methylene compounds are incorporated in table 2 where pertinent.
9. C. V. Wilson and J. F. Stenberg, Org. Syn. , Coll. Vol. 4, 564 (1963).
10. T. Satoh, S. Suzuki, Y. Suzuki, Y. Miyaji and Z. Imai, Tet. Lett. , (52) 4555 (1969).
11. A. C. Cope and E. Ciganek, Org. Syn. , Coll. Vol. 4, 339 (1963).
12. S. L. Friess, J. Am Chem. Soc. , 71 , 2571 (1949).
13. S. L. Friess and P. E. Frankenburg, Ibid ., 74 , 2679 (1952).
14. C. H. Hassall, Org. Reactions , 9 , 73 (1957).
15. Borsche, Ber. , 48 , 682 (1915).
16. Ibid ., 56 , 2012, 2132 (1923).
17. Ibid ., 59 , 237 (1926).
18. Cawley and Plant, J. Chem. Soc. , 1214 (1938).
19. Attenburrow, et . al ., Ibid ., 571 (1945).
20. E. Winterfeldt, Ber Deutsch Chem Ges. , 97 2463 (1964).
21. J. Hebky and J. Kejha, CA , 50 , 155326.
22. J. V. Greenhill, Chem. Soc. Rev. , 6 , 277 (1977).
23. J. A. Leben, Ber. , 29, 1673, (1896).
24. Von Pechmann and W. Welsh, Ber. , 17 , 2391 (1884).
25. J. H. Boyer and W. Schoen, Org. Syntheses , Coll. Vol. IV, 532 (1963).
26. H. Hiari and K. Miyata, J. Patent 72, 42,832, Jan. 28, 1970.
27. D. E. Heitmeier, J. T. Hortenstine Jr., and A. P. Gray, J. Org. Chem. , 36 , 1449 (1971).
28. T. S. Hamilton and R. J. Adams, J. Am. Chem. Soc. , 50, 2260 (1928).
29. J. L. Herrmann and R. H. Schlessinger, J. Am. Chem. Soc., Chem. Comm. , 711 (1973).
30. G. H. Posner and G. L. Lomis, Chem. Comm ., 892 (1972).
31. K. Iwai, Chem. Lett. , 385 (1974).
32. P. Hullot et. al., Can. J. Chem. , 54 , 1098 (1976).
33. P. A. Zoretic and F. Barcelos, Tet. Lett. , 529 (1977).
34. B. M. Trost and R. A. Kunz, J. Org. Chem. , 34 (1974).
35. J. P. Depres, A. E. Greene and P. Crabbe, Tet. Lett. , 2191 (1978).
36. J. K. Crandall and A. C. Clark, Tet. Lett. , 325-28 (1969).
37. J. B. Bush and H. Finkbiener, J. Am. Chem. Soc. , 90 , 5903 (1968).
38. H. O. House, "Modern Synthetic Reactions," 2nd Ed., W. A. Benjamin, Inc., Menlo Park, 1972, pp. 492-628 and references cited within.
39. N. C. Corbin, P. Fraher, and J. D. McChesney, J. Pharm. Science , 68 , 1501 (1979).
40. Gor, Harmon, Levisalles and Wagnon, Chem. Comm. , 88 (1973).
41. G. H. Posner, Org. Reactions , 19 1 (1972).
42. H. O. House, Acc. Chem. Res. , 9 (1976).
43. Truce, Hollister, Lindy and Parr, J. Org. Chem. , 33 , 43 (1968).
44. Truce and Vrencur, J. Org. Chem. , 35, 1226 (1970).
45. M. Julia and Arnould, Bull. Soc. Chim. Fr. , 743, 746 (1973).
46. A. D. Bliss, W. K. Cline, C. E. H. Milton and O. J. Sweeting, J. Org. Chem. , 28, 3537 (1963).
47. H. D. Hartough, "Chemistry of Heterocyclic Compounds," 3 , Interscience Publishers, Inc., New York (1952).
48. A. Williams, "Furans Synthesis and Applications," Noyes Data Corp., New Jersey, 1973.
49. M. E. Garst, J. N. Bonfiglio, D. A. Grudoski, and J. Marks, Tet. Lett. , 2671 (1978).
50. Wolf and Folkers, Org. Reactions , 6 , 443-468 (1951).
51. G. Stork and L. Maldonado, J. Am. Chem. Soc. , 73 , 5286 (1971).
52. G. Stork and L. Maldonado, Ibid ., 76 , 5272 (1974).
53. R. Woodward and Eastman, J. Am. Chem. Soc. , 68 , 2229 (1946).
54. R. Woodward and Eastman, Ibid ., 66 , 849 (1944).
55. V. Luhmann and W. Luttke, Chem. Ber. , 105, 1350 (1972).
56. I. J. Borowitz and G. J. Williams, J. Org. Chem. , 32 , 4157 (1967).
57. J. March, "Advanced Organic Chemistry," 2nd Ed., McGraw Hill Book Company, New York, 1977, pp. 363-365.

58. R. Gorski, G. J. Wolber and J. Wemple, Tet. Lett. , 2577 (1976).
59. T. A. Morgan and B. Ganem, Tet. Lett. , 2773 (1980).
60. M. Koreeda and H. Akagi, Tet. Lett. , 1197 (1980).
61. N. Sugiyama, M. Yamamoto and C. Kashima, Bull. Chem. Soc. Japan , 42, 1357 (1969).
62. G. M. Bennett and L. V. D. Scorah, J. Chem. Soc. , 194 (1927).
63. A. Belanger and P. Brassard, Chem. Comm. , 863 (1972).
64. J. Fried, "Heterocyclic Compounds," ed. by R. C. Elderfield, Wiley, New York, 1950, Vol 1, pp. 358-370.
65. L. F. Cavalieri, Chem. Rev. , 41 , (525) (1947).
66. E. Tihanyi, M. Gai, and P. Dvortsak, Heterocycles , 20, 571 (1983).
67. Y. Kurasawa, M. Ichikawa, A. Sakukura and H. Takada, Chem. Pharm. Bull., Tokyo , 32 , 4140 (1984).
68. Ibid ., 30 , 336 (1982).
69. K. Takacs and K. Harsanyi, Chem. Ber. , 103 , 2330 (1970).
70. P. W. Searle and W. K. Warburton, J. Chem. Soc. , Perkins Trans. , 1 , 85 (1974).
71. A. R. Katritzky, B. Wallis, R. T. C. Brownlee, and R. D. Topsom, Tet. , 21 , 1681 (1965).
72. S. Kubota and M. Uda, Chem. Pharm. Bull. , 21 , 1342, (1973).
73. S. Kubuta and M. Uda, Ibid ., 24 , 1336 (1976).
74. R. K. Howe, T. A. Gruner, L. G. Carter, L. L. Black, and J. E. Franz, J. Org. Chem. 43 , 3736 (1978).
75. R. Boyle, F. Eloy and R. Lenaers, Helv. Chim. Acta. , XLVI, 1073 (1973).
76. J. Sauer and K. K. Mayer, Tet. Lett. , 319 (1968).
77. G. Beck, Chem. Ber. , 84 , 688 (1951).
78. J. Goerdeler and R. Sappelt, Chem. Ber. , 100 , 2064 (1967).
79. T. J. Giacobbe, J. Het. Chem. 15 , 1227 (1978).
80. Kurt Pilgram, Ibid., 19 , 823 (1982).
81. M. Kishi, H. Ishitobi, W. Nagata, and T. Tsuji, Heterocycles , 13 197 (1979).
82. A. K. Saund and M. K. Mathur, Int. J. Peptide Protein Res. , 5 , 7, (1964).
83. Y. Fuju Moto et. al., Heterocycles , 6, 1604 (1977).
84. For a discussion of the optimal conditions required for selectively generating thermodynamic or kinetic enolates, see J. C. Stowell, "Carbanions in Organic Synthesis," John Wiley and Sons, Inc., New York, 1979, pp. 8-11 and references cited therein.

## Claims

**1.** A compound selected from

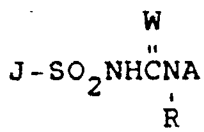

wherein

J is 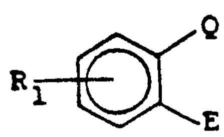 , 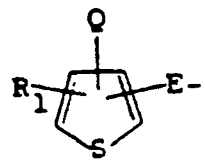 , 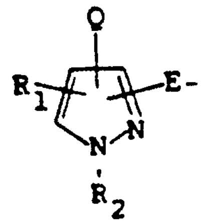 ,

J-1 J-2 J-3

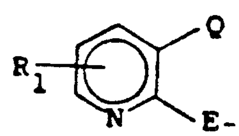 , 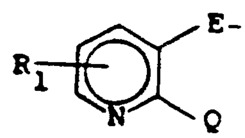

J-4 J-5

W is O or S;
R is H or $CH_3$;
E is a single bond, $CH_2$ or O;
Q is $Q_1$ or $E_1X_aQ_2$;
$Q_1$ is a saturated 5- or 6-membered heterocyclic ring, bonded through carbon or nitrogen, containing a carbonyl group and 2-3 heteroatoms selected from the group consisting of 0-2 oxygen, 0-2 sulfur or 0-2 nitrogen; a 5-membered heterocyclic ring, bonded through carbon or nitrogen, containing a carbonyl group and 2-3 heteroatoms selected from the group consisting of 0-2 oxygen, 0-2 sulfur or 1-3 nitrogen and containing one endocyclic double bond; a 6-membered heterocyclic ring, bonded through carbon or nitrogen, containing a carbonyl group and 2-3 heteroatoms selected from the group consisting of 0-2 oxygen, 0-2 sulfur or 1-3 nitrogen and containing one or two endocyclic double bonds; or a 5-membered heterocyclic ring, bonded through carbon or nitrogen, containing two adjacent carbonyl groups and 2 heteroatoms selected from the group consisting of 0-1 oxygen, 0-1 sulfur, 1-2 nitrogen and containing one endocyclic double bond, said Q value may be substituted or unsubstituted wherein the substituent groups are selected from the group consisting of $C_1$-$C_4$ alkyl, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl, $C_1$-$C_3$ cyanoalkyl, $C_2$-$C_4$ alkoxyalkyl, $C_2$-$C_4$ alkylthioalkyl, $C_2$-$C_4$ alkylcarbonyl, $C_3$-$C_4$ alkylcarbonylalkyl, $C_1$-$C_4$ alkyl substituted with OH or $NH_2$, $C_2$-$C_4$ alkylaminoalkyl, $C_3$-$C_4$ dialkylaminoalkyl, $CH_2CH(OCH_3)_2$,

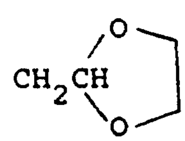 ,

$C(O)N(CH_3)_2$, $P(O)(OC_1$-$C_2$ alkyl$)_2$, $P(S)(OC_1$-$C_2$ alkyl$)_2$ or $C_1$-$C_2$ alkyl substituted with $C_1$-$C_2$ alkoxycarbonyl;
$E_1$ is O, S, SO, $SO_2$ or a single bond;
$Q_2$ is a 5- or 6-membered carbocyclic ring containing either one or two carbonyl groups and 0-1 endocyclic double bonds; a 5-membered heterocyclic ring, containing 2-4 atoms of carbon and 1-3 heteroatoms selected from the group consisting of 0-2 oxygen, 0-2 sulfur or 0-3 nitrogen, wherein sulfur may take the form of S, SO or $SO_2$, and containing one or two carbonyl or sulfonyl ($SO_2$)

groups, or one carbonyl and one sulfonyl group and 0-1 endocyclic double bonds; or a 6-membered heterocyclic ring, containing 2-5 atoms of carbon and 1-3 heteroatoms selected from the group consisting of 0-2 oxygen, 0-2 sulfur or 0-3 nitrogen, wherein sulfur may take the form of S, SO or $SO_2$, and containing one or two carbonyl or sulfonyl ($SO_2$) groups, or one carbonyl and one sulfonyl group and 0-2 endocyclic double bonds; said Q value may further be optionally substituted with 1-2 substituent groups: substituents on carbon may be selected from the group consisting of halogen, $C_1$-$C_4$ alkyl, $C_1$-$C_4$ haloalkyl, $CH_2$($C_2$-$C_3$ alkenyl), $CH_2$($C_2$-$C_3$ alkynyl), $C_2$-$C_4$ alkoxycarbonyl, CN, OH, $C_1$-$C_3$ alkoxy, $C_1$-$C_3$ alkylthio, $C_1$-$C_3$ alkylsulfinyl, $C_1$-$C_3$ alkylsulfonyl or $C_2$-$C_4$ alkylcarbonyl; substituents on nitrogen may be selected from the group consisting of $C_1$-$C_4$ alkyl, $C_1$-$C_4$ haloalkyl, $CH_2$($C_2$-$C_3$ alkenyl), $CH_2$($C_2$-$C_3$ alkynyl), $C_2$-$C_4$ alkoxycarbonyl or $C_2$-$C_4$ alkylcarbonyl;

$X_a$ is $CH_2$, $CH(CH_3)$, $CH_2CH_2$, $CH_2CH_2CH_2$ or CO;

$R_1$ is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, $CH_2CN$, CN, $CO_2R_c$, $C_1$-$C_3$ haloalkoxy, $C_1$-$C_3$ haloalkylthio, $C_2$-$C_4$ alkoxyalkyl, $C_2$-$C_4$ alkylthioalkyl, $CH_2N_3$ or $NR_dR_e$;

$R_a$ is H, $C_1$-$C_4$ alkyl, $C_2$-$C_3$ cyanoalkyl, methoxy or ethoxy;

$R_b$ is H, $C_1$-$C_4$ alkyl or $C_3$-$C_4$ alkenyl; or

$R_a$ and $R_b$ may be taken together as $-(CH_2)_3-$, $-(CH_2)_4-$, $-(CH_2)_5-$ or $-CH_2CH_2OCH_2CH_2-$;

$R_c$ is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, $C_5$-$C_6$ cycloalkyl, $C_4$-$C_7$ cycloalkylalkyl or $C_2$-$C_4$ alkoxyalkyl;

$R_d$ and $R_e$ are independently H or $C_1$-$C_2$ alkyl;

A is 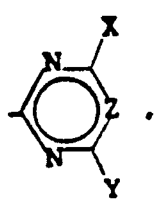 , 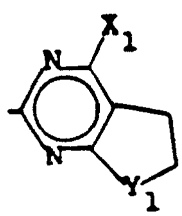 , 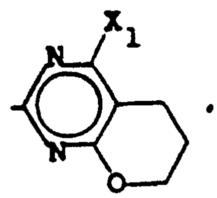 ,

A-1 A-2 A-3

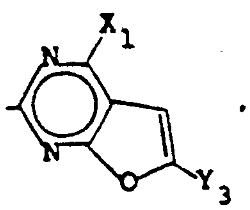 , 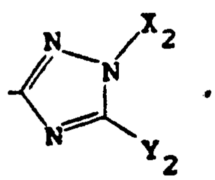 , 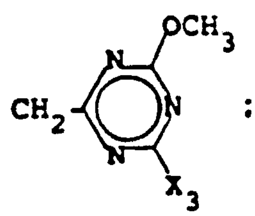 ;

A-4 A-5 A-6

or 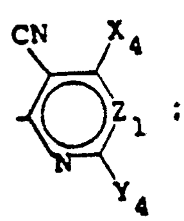 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)-amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$

alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$-$C_4$ haloalkyl, $C_2$-$C_4$ alkynyl, azido, cyano, $C_2$-$C_5$ alkylsulfinylalkyl, $C_2$-$C_5$ alkylsulfonylalkyl,

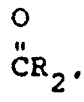, 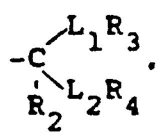, 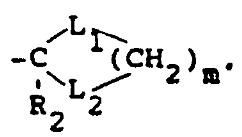, 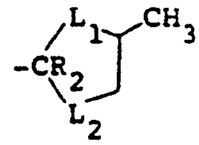

or $N(OCH_3)CH_3$;

m is 2 or 3;
$L_1$ and $L_2$ are independently O or S;
$R_2$ is H or $C_1$-$C_3$ alkyl;
$R_3$ and $R_4$ are independently $C_1$-$C_3$ alkyl;
Z is CH, N, $CCH_3$, $CC_2H_5$, CCl or CBr;
$Z_1$ is CH or N;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl; and
$Y_4$ is $CH_3$, $OCH_3$, $OC_2H_5$ or Cl; and their agriculturally suitable salts;
provided that
a) when Q contains 2 heteroatoms selected from 0-2 oxygen and 0-2 sulfur, said heteroatoms are not bonded directly to one another, and when Q contains 3 nitrogen heteroatoms, only two of these may be bonded directly together;
b) when X is Cl, F, Br of I, then Z is CH and Y is $OCH_3$, $OC_2H_5$, $N(OCH_3)CH_3$, $NHCH_3$, $N(CH_3)_2$ or $OCF_2H$;
c) when X or Y is $C_1$ haloalkoxy, then Z is CH;
d) when J is J-2 or J-3, the substituent Q and the sulfonylurea bridge are on adjacent carbon atoms;
e) when E is O, then J is J-1 and W is O;
f) when W is S, then R is H, A is A-1, Z is CH or N and Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$ or 1,3-dioxolan-2-yl; g) when the total number of carbons of X and Y is greater than four, then the number of carbons of $R_1$ must be less than or equal to two, and the number of carbons of the substituent on Q must be less than or equal to two;
h) $X_4$ and $Y_4$ are not simultaneously Cl;
i) when $Q_1$ is bound through nitrogen and contains 2-heteroatoms and one carbonyl group, and said heteroatoms are bound through the carbonyl, then J is other than J-1;
j) when A is A-1 and J is J-1 wherein E is a single bond, $X_a$ is $CH_2$, $CH(CH_3)$ or $CH_2CH_2$ and $Q_2$ is a 5-membered heterocyclic ring containing one endocyclic double bond or a 6-membered heterocyclic ring containing 1 or 2 endocyclic double bonds which is unsubstituted or substituted by one or more $C_1$-$C_4$ alkyl groups then said heterocycle must contain at least one nitrogen and be bound to $X_a$ through nitrogen;
k) when $X_a$ is CO, then $E_1$ is a single bond; and
l) when J is J-1, then $E_1$ is a single bond.

2. A compound of Claim 1 wherein $Q_1$ is selected from

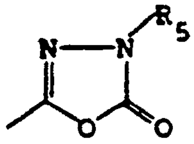 , 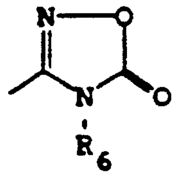 , 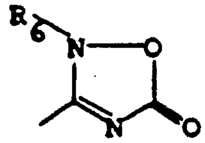 ,

Q1-1 Q1-2 Q1-3

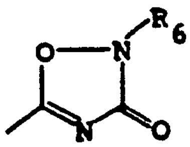 , 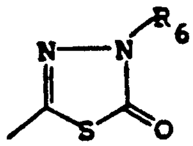 , 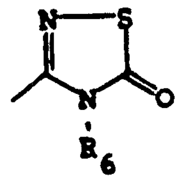 ,

Q1-4 Q1-5 Q1-6

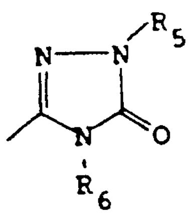 , 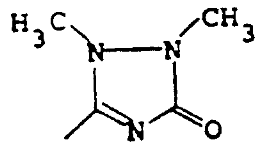 , 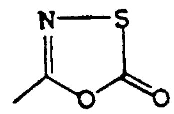 ,

$Q_1$-7 $Q_1$-8 $Q_1$-9

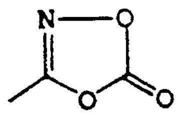 , 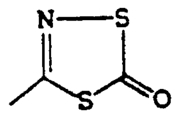 , 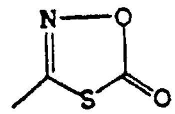 ,

$Q_1$-10 $Q_1$-11 $Q_1$-12

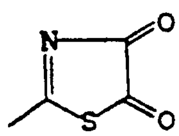 , 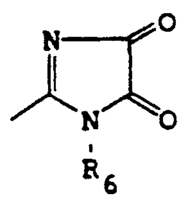 , 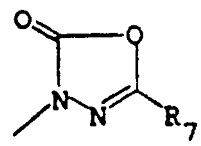 ,

$Q_1$-13 $Q_1$-14 $Q_1$-15

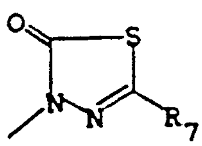 , 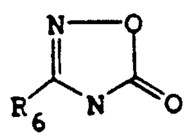 , 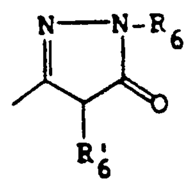 ,

$Q_1$-16 $Q_1$-17 $Q_1$-18

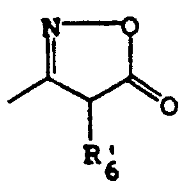 , 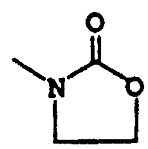 , 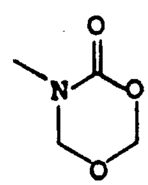 ,

$Q_1$-19 $Q_1$-20 $Q_1$-21

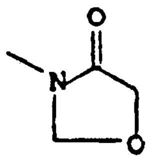

$Q_1$-22

,

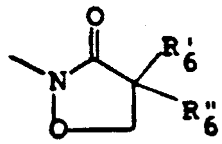

$Q_1$-23

,

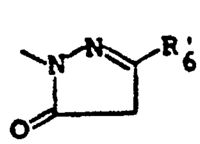

$Q_1$-24

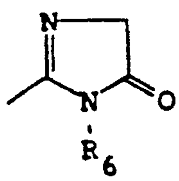

$Q_1$-25

,

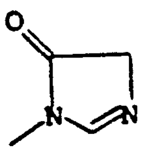

$Q_1$-26

,

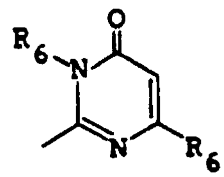

$Q_1$-27

,

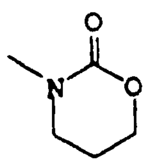

$Q_1$-28

,

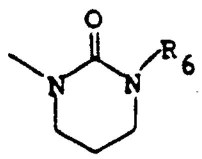

$Q_1$-29

or

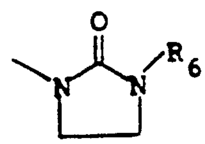

$Q_1$-30

;

wherein

$R_5$ is H, $C_1$-$C_4$ alkyl, $CH_2CH=CH_2$, $CH_2CH=CHCH_3$, $CH_2C{\equiv}CH$, $CH_2C{\equiv}CCH_3$, $CH_2CN$, $CH_2CO_2$-($C_1$-$C_2$ alkyl), $CH(CH_3)CO_2$($C_1$-$C_2$ alkyl), $CF_2H$, $C_2$-$C_3$ alkyl substituted with 1-3 atoms of F or Cl, $CH_2OCH_3$, $CH_2OCH_2CH_3$, $CH_2CH_2OCH_3$, or $CH_2CH_2OCH_2CH_3$;

$R_6$ is $C_1$-$C_3$ alkyl;

$R_6'$ and $R_6''$ are independently H or $C_1$-$C_2$ alkyl; and

$R_7$ is H, $C_1$-$C_4$ alkyl, $C_1$-$C_3$ haloalkyl or $CH_2CH=CH_2$.

**3.** A compound of Claim 1 wherein $Q_2$ is selected from

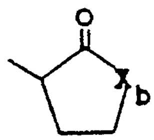 .

$Q_2$-1

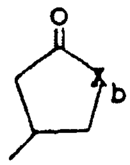 .

$Q_2$-2

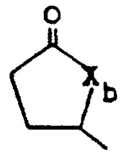 .

$Q_2$-3

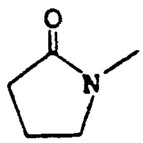 .

$Q_2$-4

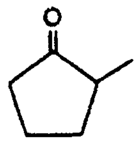 .

$Q_2$-5

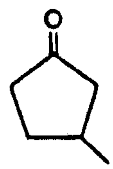 .

$Q_2$-6

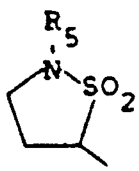 .

$Q_2$-7

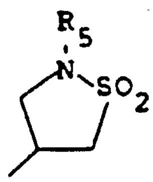 .

$Q_2$-8

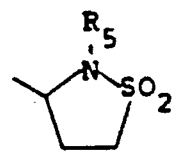 .

$Q_2$-9

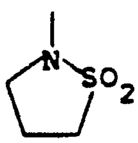 .

$Q_2$-10

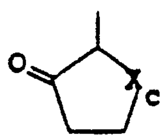 .

$Q_2$-11

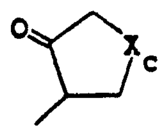 .

$Q_2$-12

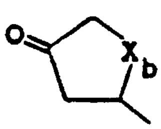 .

$Q_2$-13

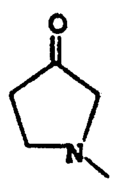 .

$Q_2$-14

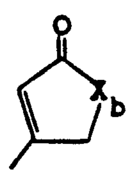 .

$Q_2$-15

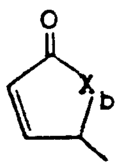 .

Q

$Q_2$-16

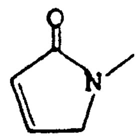 .

$Q_2$-17

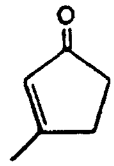 .

$Q_2$-18

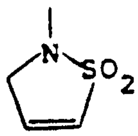 .

$Q_2$-19

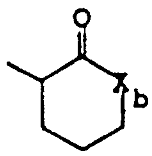 .

$Q_2$-20

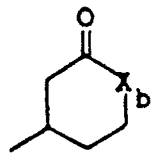 .

$Q_2$-21

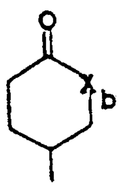 .

$Q_2$-22

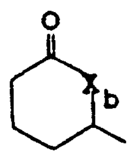 .

$Q_2$-23

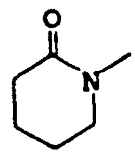 .

$Q_2$-24

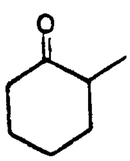 .

$Q_2$-25

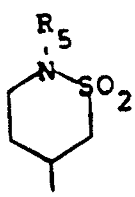 .

$Q_2$-26

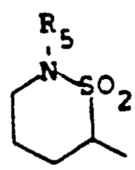 .

$Q_2$-27

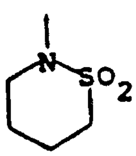 .

$Q_2$-28

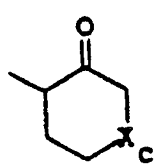 .

$Q_2$-29

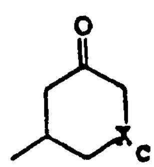 .

$Q_2$-30

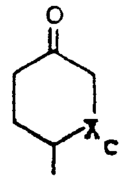
,
$Q_2$-31

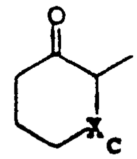
,
$Q_2$-32

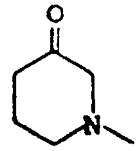
,
$Q_2$-33

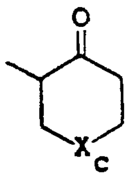
,
$Q_2$-34

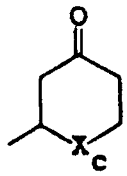
,
$Q_2$-35

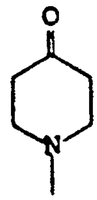
,
$Q_2$-36

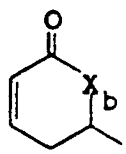
,
$Q_2$-37

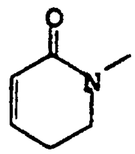
,
$Q_2$-38

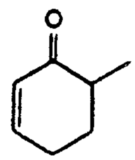
,
$Q_2$-39

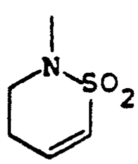
,
$Q_2$-40

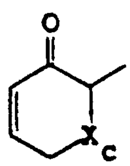
,
$Q_2$-41

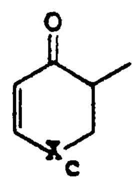
,
$Q_2$-42

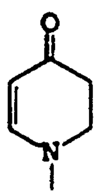
,
$Q_2$-43

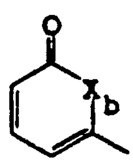
,
$Q_2$-44

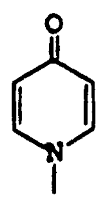
,
$Q_2$-45

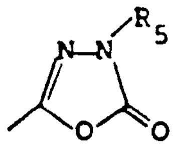 .

$Q_2$-46

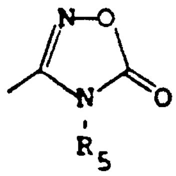 .

$Q_2$-47

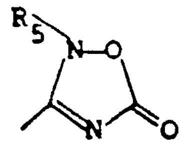 .

$Q_2$-48

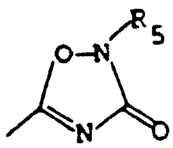 .

$Q_2$-49

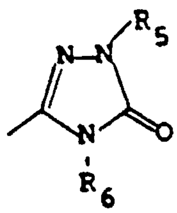 .

$Q_2$-50

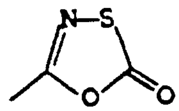 .

$Q_2$-51

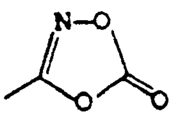 .

$Q_2$-52

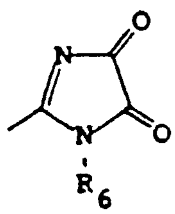 .

$Q_2$-53

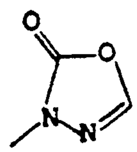 .

$Q_2$-54

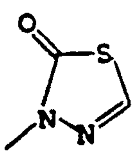 .

$Q_2$-55

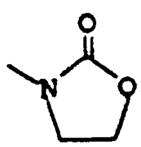 .

$Q_2$-56

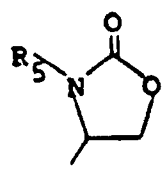 .

$Q_2$-57

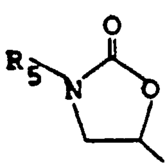 .

$Q_2$-58

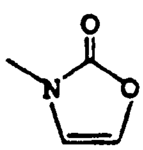 .

$Q_2$-59

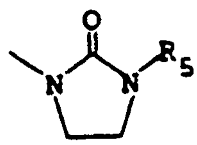 .

$Q_2$-60

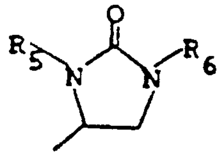 .

$Q_2$-61

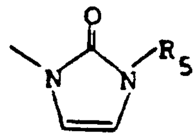 .

$Q_2$-62

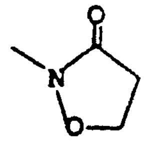 .

$Q_2$-63

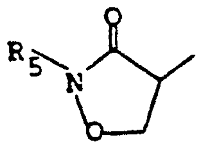 .

$Q_2$-64

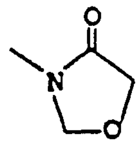 .

$Q_2$-65

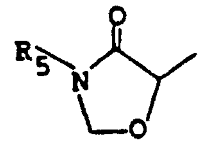 .

$Q_2$-66

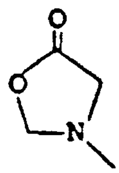 .

$Q_2$-67

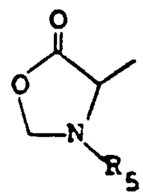 .

$Q_2$-68

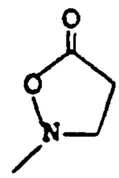 .

$Q_2$-69

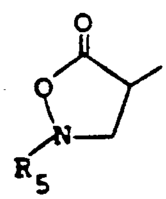 .

$Q_2$-70

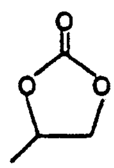 .

$Q_2$-71

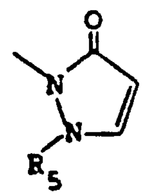 .

$Q_2$-72

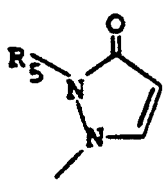 .

$Q_2$-73

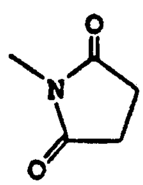 .

$Q_2$-74

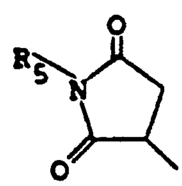 .

$Q_2$-75

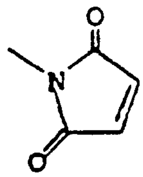 ,

$Q_2$-76

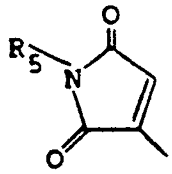 ,

$Q_2$-77

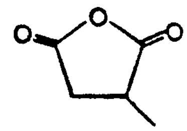 ,

$Q_2$-78

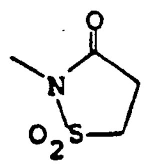 ,

$Q_2$-79

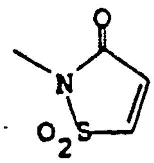 ,

$Q_2$-80

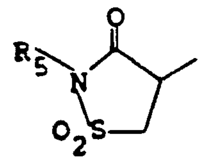 ,

$Q_2$-81

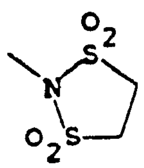 ,

$Q_2$-82

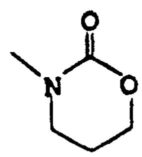 ,

$Q_2$-83

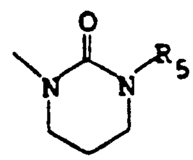 ,

$Q_2$-84

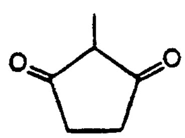 ,

$Q_2$-85

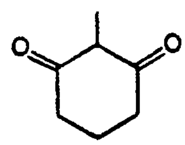 and

$Q_2$-86

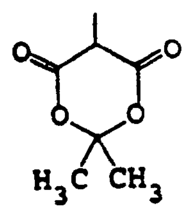 ;

$Q_2$-87

wherein

$Q_2$-1 through $Q_2$-87 may be optionally substituted with 1 or 2 groups selected from $C_1$-$C_2$ alkyl or $C_1$-$C_2$ haloalkyl;
$R_5$ and $R_6$ are independently H or $C_1$-$C_3$ alkyl;
$X_b$ is O or $NR_5$; and
$X_c$ is O, S, SO, $SO_2$ or $NR_5$.

**4.** Compounds of Claim 1 where
Q is $Q_1$;
E is a single bond; and
Z is CH or N.

**5.** Compounds of Claim 1 where
Q is $Q_1$;
E is $CH_2$;
W is O; and
Z is CH or N.

**6.** Compounds of Claim 1 where
Q is $Q_1$;
E is O; and
Z is CH or N.

**7.** Compounds of Claim 2 where E is a single bond and Z is CH or N.

**8.** Compounds of Claim 7 where
W is O;
R is H;
$R_1$ is H, F, Cl, Br, $C_1$-$C_2$ alkyl, $C_1$-$C_3$ alkoxy, $C_1$-$C_3$ alkylthio, or $C_1$-$C_2$ alkyl, $C_1$-$C_3$ alkoxy or $C_1$-$C_3$ alkylthio substituted with 1-3 atoms of F, Cl or Br;
X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$, $CH_2SCH_3$,

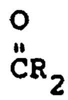, 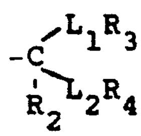, 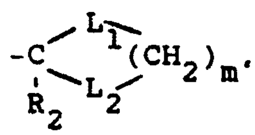, 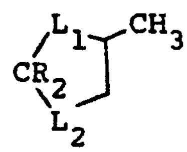,

$OCF_2H$, $SCF_2H$, cyclopropyl, $C\equiv CH$ or $C\equiv CCH_3$.

**9.** Compounds of Claim 8 where A is A-1.

**10.** Compounds of Claim 9 where J is J-1 to J-5.

**11.** Compounds of Claim 9 where
J is J-1;
$R_1$ is H, Cl, $CH_3$ or $OCH_3$ and is not para to the sulfonylurea bridge;
X is $CH_3$, $OCH_3$, Cl or $OCF_2H$; and
Y is $CH_3$, $OCH_3$, $C_2H_5$, $CH_2OCH_3$, $NHCH_3$, $CH(OCH_3)_2$ or cyclopropyl.

**12.** Compounds of Claim 11 where $Q_1$ is $Q_1$-1 to $Q_{27}$.

**13.** Compounds of Claim 2 where
E is $CH_2$;
W is O;
R is H;
J is J-1;
$R_1$ is H;
A is A-1;
X is $CH_3$, $OCH_3$, $OCH_2CH_3$, Cl or $OCF_2H$;
Y is $CH_3$, $OCH_3$, $C_2H_5$, $CH_2OCH_3$, $NHCH_3$, $CH(OCH_3)_2$ or cyclopropyl and $Q_1$ is $Q_1$-1, $Q_1$-7, $Q_1$-10 or $Q_1$-15; and
Z is CH or N.

**14.** Compounds of Claim 2 where
E is O;
R is H;
J is J-1;
$R_1$ is H;
A is A-1;
X is $CH_3$, $OCH_3$, $OCH_2CH_3$, Cl or $OCF_2H$;
Y is $CH_3$, $OCH_3$, $C_2H_5$, $CH_2OCH_3$, $NHCH_3$, $CH(OCH_3)_2$ or cyclopropyl and $Q_1$ is $Q_1$-1, $Q_1$-7, $Q_1$-10 or $Q_1$-15; and

Z is CH or N.

**15.** Compounds of Claim 1 where
Q is $E_1X_aQ_2$;
E is a single bond; and
Z is CH or N.

**16.** Compounds of Claim 1 where
Q is $E_1X_aQ_2$;
E is $CH_2$;
W is O;
Z is CH or N; and
$E_1$ is a single bond.

**17.** Compounds of Claim 1 where
Q is $E_1X_aQ_2$;
E is O;
Z is CH or N; and
$E_1$ is a single bond.

**18.** Compounds of Claim 3 wherein
$Q_2$-1 through $Q_2$-87 may be optionally substituted with 1 or 2 groups selected from $C_1$-$C_2$ alkyl or $C_1$-$C_2$ haloalkyl;
$R_5$ and $R_6$ are independently H or $C_1$-$C_3$ alkyl;
$X_b$ is O or $NR_5$;
$X_c$ is O, S, SO, $SO_2$ or $NR_5$;
E is a single bond; and
Z is CH or N.

**19.** Compounds of Claim 18 where
W is O;
$E_1$ is a single bond;
$X_a$ is $CH_2$ or $CH_2CH_2$;
R is H;
$R_1$ is H, F, Cl, Br, $C_1$-$C_2$ alkyl, $C_1$-$C_3$ alkoxy, $C_1$-$C_3$ alkylthio, or $C_1$-$C_2$ alkyl, $C_1$-$C_3$ alkoxy or $C_1$-$C_3$ alkylthio substituted with 1-3 atoms of F, Cl or Br;
X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$, $CH_2SCH_3$,

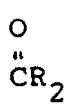,
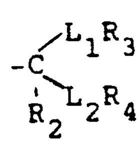,
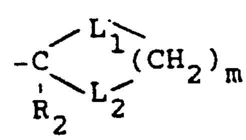,
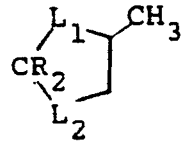

$OCF_2H$, $SCF_2H$, cyclopropyl, $C\equiv CH$ or $C\equiv CCH_3$.

**20.** Compounds of Claim 19 where A is A-1.

**21.** Compounds of Claim 20 where J is J-1 to J-5.

**22.** Compounds of Claim 21 where
J is J-1;
$R_1$ is H, Cl, $CH_3$ or $OCH_3$;
X is $CH_3$, $OCH_3$, Cl or $OCF_2H$; and
Y is $CH_3$, $OCH_3$, $C_2H_5$, $CH_2OCH_3$, $NHCH_3$, $CH(OCH_3)_2$ or cyclopropyl.

**23.** Compounds of Claim 16 where
R is H;
J is J-1;
$R_1$ is H;
A is A-1;
X is $CH_3$, $OCH_3$, $OCH_2CH_3$, Cl or $OCF_2H$;
Y is $CH_3$, $OCH_3$, $C_2H_5$, $CH_2OCH_3$, $NHCH_3$, $CH(OCH_3)_2$ or cyclopropyl;
Z is CH or N; and
$Q_2$ is $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24 $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$-60, $Q_2$-63, $Q_2$-71, $Q_2$-74, $Q_2$-76, $Q_2$-78 and $Q_2$-79.

**24.** Compounds of Claim 17 where
R is H;
$R_1$ is H;
A is A-1;
X is $CH_3$, $OCH_3$, $OCH_2CH_3$, Cl or $OCF_2H$;
Y is $CH_3$, $OCH_3$, $C_2H_5$, $CH_2OCH_3$, $NHCH_3$, $CH(OCH_3)_2$ or cyclopropyl;
Z is CH or N; and
$Q_2$ is $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24 $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$-60, $Q_2$-63, $Q_2$-71, $Q_2$-74, $Q_2$-76, $Q_2$-78 and $Q_2$-79.

**25.** Compounds of Claim 1 wherein
Q is $E_1X_aQ_2$;
$E_1$ is a single bond;
$X_a$ is $CH_2$, $CH(CH_3)$. $CH_2CH_2$ or $CH_2CH_2CH_2$;
$Q_2$ is a saturated or partially saturated 5-or 6-membered carbocyclic ring, containing either one or two carbonyl groups, or a saturated or partially saturated 5- or 6-membered heterocyclic ring, containing 2-5 atoms of carbon and 1-3 heteroatoms selected from the group consisting of 0-2 oxygen, 0-2 sulfur or 0-3 nitrogen, wherein sulfur may take the form of S, SO or $SO_2$, and containing one or two carbonyl or sulfonyl ($SO_2$) groups, or one carbonyl and one sulfonyl group; Q may further be optionally substituted with 1-2 substituent groups: substituents on carbon may be selected from the group consisting of halogen, $C_1$-$C_4$ alkyl, $C_1$-$C_4$ haloalkyl, $CH_2$($C_2$-$C_3$ alkenyl), $CH_2$($C_2$-$C_3$ alkynyl), $C_2$-$C_4$ alkoxycarbonyl, CN, OH, $C_1$-$C_3$ alkoxy, $C_1$-$C_3$ alkylthio, $C_1$-$C_3$ alkylsulfinyl, $C_1$-$C_3$ alkylsulfonyl or $C_2$-$C_4$ alkylcarbonyl, substituents on nitrogen may be selected from the group consisting of $C_1$-$C_4$ alkyl, $C_1$-$C_4$haloalkyl, $CH_2$($C_2$-$C_3$ alkenyl), $CH_2$($C_2$-$C_3$ alkynyl), $C_2$-$C_4$ alkoxycarbonyl or $C_2$-$C_4$ alkylcarbonyl;
$R_1$ is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, $CH_2CN$, CN, $CO_2R_c$, $C_1$-$C_3$ haloalkoxy or $C_1$-$C_3$ haloalkylthio;

A is 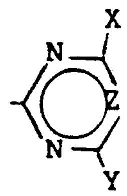 , 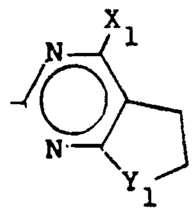 , 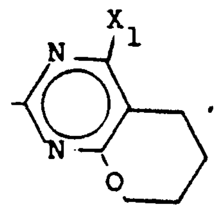 ,

A-1 A-2 A-3

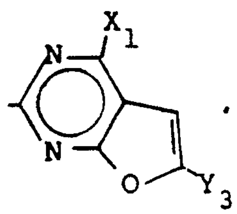 , 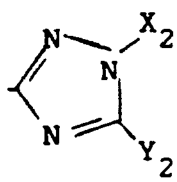 or 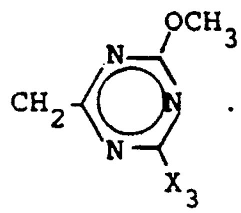 .

A-4 A-5 A-6

**26.** A compound selected from

- o 2-(4,5-dihydro-4-methyl-5-oxo-1,3,4-oxadiazol-2-yl)-N-[(4,6-dimethoxypyrimidin-2-yl)-aminocarbonyl]benzenesulfonamide,
- o 2-(4,5-dihydro-4-methyl-5-oxo-1,3,4-oxadiazol-2-yl)-N-[(4-methoxy-6-methyl-1,3,5-triazin-2-yl)-aminocarbonyl]benzenesulfonamide,
- o N-[(4,6-dimethoxypyrimidin-2-yl)aminocarbonyl]-2-(2-oxo = 1 = pyrrolidinylmethyl)-benzenesulfonamide,
- o N-[(4,6-dimethoxy-1,3,5-triazin-2-yl)aminocarbonyl]-2-(2-oxo-1-pyrrolidinylmethyl)-benzenesulfonamide,
- o N-[(4,6-dimethoxypyrimidin-2-yl)aminocarbonyl]-2-(2-oxo-3-oxazolidinylmethyl)-3-thiophenesulfonamide, and
- o N-[(4-methoxy-6-methyl-1,3,5-triazin-2-yl)aminocarbonyl]-2-(2-oxo-3-oxazolidinylmethyl)-3-thiophenesulfonamide.

**27.** A composition suitable for controlling the growth of undesired vegetation which comprises an effective amount of a compound of any of claims 1 to 26 and at least one of the following: surfactant, solid or liquid diluent.

**28.** A method of controlling the growth of undesired vegetation which comprises applying to the locus to be protected an effective amount of a compound of any of claims 1 to 26.

**29.** A method for regulating the growth of plants which comprises applying to the locus of such plants an effective but substantially non-phytotoxic amount of a plant growth regulant selected from compounds of any of claims 1 to 26.

**30.** A process for the preparation of a compound of claim 1, which comprises

(I) (a) reacting $JSO_2NCW$ with A-NHR

(b) reacting $JSO_2NH_2$ with $PhO\overset{W}{\overset{\|}{C}}NHA$

(c) reacting $JSO_2NH\underset{W}{\underset{\|}{C}}OPh$ with A-NHR

wherein J, R, W and A are as defined in claim 1 provided that E is $CH_2$ or a single bond; or

(II) (a) reacting $JSO_2NCW$ with A-NHR

(b) reacting JH with $ClSO_2NH\underset{O}{\underset{\|}{C}}NHA$

wherein J, W and A are as defined in claim 1, provided that E is O.

**31.** Compounds of formulae:

$JSO_2NCW$

$JSO_2NH_2$

$JSO_2NH\underset{W}{\underset{\|}{C}}OPh$

and JH

as defined in claim 30.

## Revendications

**1.** Un composé choisi parmi

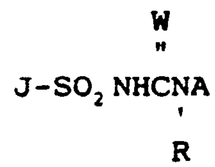

où

J est 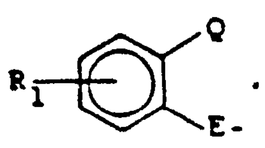 , 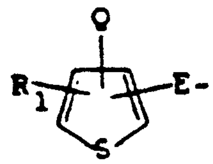 , 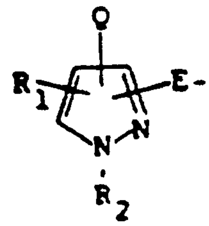 ,

J-1 J-2 J-3

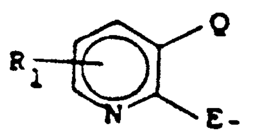 , 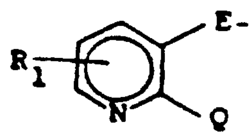

J-4 J-5

W est O ou S ;
R est H ou $CH_3$ ;
E est une liaison simple, $CH_2$ ou O ;
Q est $Q_1$ ou $E_1X_aQ_2$ ;
$Q_1$ est un hétérocycle penta- ou hexagonal saturé, lié par un atome de carbone ou d'azote, oontenant un groupe carbonyle et 2 ou 3 hétéroatomes choisis dans le groupe formé par 0 à 2 atomes d'oxygène, 0 à 2 atomes de soufre ou 0 à 2 atomes d'azote ; un hétérocycle pentagonal, lié par un atome de carbone ou d'azote, contenant un groupe carbonyle et 2 ou 3 hétéroatomes choisis dans le groupe formé par 0 à 2 atomes d'oxygène, 0 à 2 atomes de soufre ou 1 à 3 atomes d'azote et contenant un double liaison endocyclique ; un hétérocycle hexagonal, lié par un atome de carbone ou d'azote, contenant un groupe carbonyle et 2 ou 3 hétéroatomes choisis dans le groupe formé par 0 à 2 atomes d'oxygène, 0 à 2 atomes de soufre ou 1 à 3 atomes d'azote et contenant une ou deux doubles liaisons endocycliques ; ou un hétérocycle pentagonal, lié par un atome de carbone ou d'azote, contenant deux groupes carbonyle adjacents et 2 hétéroatomes choisis dans le groupe formé par 0 ou 1 atome d'oxygéne, 0 ou 1 atome de soufre ou 1 ou 2 atomes d'azote et contenant une double liaison endocyclique, cette valeur de Q pouvant être substituée ou non substituée, les groupes substituants étant choisis dans la classe formée par les groupes alkyles en $C_1$ -$C_4$, halogénalkyles en $C_1$ -$C_4$, alcényles en $C_3$ -$C_4$, halogénalcényles en $C_3$ -$C_4$, alcynyles en $C_3$ -$C_4$, halogénalcynyles en $C_3$ -$C_4$, cyanoalkyles en $C_1$ -$C_3$, alcoxyalkyles en $C_2$-$C_4$, alkylthioalkyles en $C_2$-$C_4$, alkylcarbonyles en $C_2$-$C_4$, alkylcarbonylalkyles en $C_3$ -$C_4$, alkyles en $C_1$ -$C_4$ substitués par OH ou $NH_2$, alkylaminoalkyles en $C_2$ -$C_4$, dialkylaminoalkyles en $C_3$ -$C_4$, $CH_2CH(OCH_3)_2$,

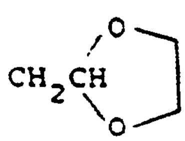 ,

$C(O)N(CH_3)_2$,
p(O) (O-alkyle en $C_1$ -$C_2$ )$_2$, P(S)(O-alkyle en $C_1$ -$C_2$ )$_2$ ou alkyles en $C_1$ -$C_2$ substitués par un groupe alcoxycarbonyle en $C_1$ -$C_2$ ;
$E_1$ est O, S, SO, $SO_2$ ou une liaison simple ;

$Q_2$ est un carbocycle penta- ou hexagonal contenant un ou deux groupes carbonyle et 0 ou 1 double liaison endocyclique ; un hétérocycle pentagonal contenant 2 à 4 atomes de carbone et 1 à 3 hétéroatomes choisis dans le groupe formé par 0 à 2 atomes d'oxygène, 0 à 2 atomes de soufre ou 0 à 3 atomes d'azote, où le soufre peut prendre la forme de S, SO ou $SO_2$, et contenant un ou deux groupes carbonyle ou sulfonyle ($SO_2$ ), ou un groupe carbonyle et un groupe sulfonyle et 0 ou 1 double liaison endocyclique ; ou un hétérocycle hexagonal contenant 2 à 5 atomes de carbone et 1 à 3 hétéroatomes choisis dans le groupe formé par 0 à 2 atomes d'oxygène, 0 à 2 atomes de soufre ou 0 à 3 atomes d'azote, où le soufre peut prendre la forme de S, SO ou $SO_2$, et contenant un ou deux groupes carbonyle ou sulfonyle ($SO_2$ ), ou un groupe carbonyle et un groupe sulfonyle et 0 à 2 doubles liaisons endocycliques ; cette valeur de Q pouvant de plus être facultativement substituée par 1 ou 2 groupes substituants : les substituants fixés sur du carbone peuvent être choisis dans la classe formée par les groupes halogéno, alkyles en $C_1$- $C_4$, halogénalkyles en $C_1$ -$C_4$, $CH_2$ (alcényle en $C_2$ -$C_3$), $CH_2$ (alcynyle en $C_2$ -$C_3$), alcoxycarbonyle en $C_2$ -$C_4$, CN, OH, alcoxy en $C_1$ -$C_3$, alkylthio en $C_1$ -$C_3$, alkylsulfinyles en $C_1$-$C_3$, alkylsulfonyles en $C_1$-$C_3$ ou alkylcarbonyles en $C_2$ -$C_4$ ; les substituants fixés sur de l'azote peuvent être choisis dans la classe formée par les groupes alkyles en $C_1$ -$C_4$, halogénalkyles en $C_1$ -$C_4$, $CH_2$ (alcényle en $C_2$ -$C_3$), $CH_2$ (alcynyle en $C_2$ -$C_3$), alcoxycarbonyles en $C_2$ -$C_4$ ou alkylcarbonyles en $C_2$ -$C_4$ ;

$X_a$ est $CH_2$, $CH(CH_3)$, $CH_2CH_2$, $CH_2CH_2CH_2$ ou CO ;

$R_1$ est H, un groupe alkyle en $C_1$ -$C_3$, halogénalkyle en $C_1$ -$C_3$, halogéno, nitro, alcoxy en $C_1$ -$C_3$, $SO_2NR_aR_b$, alkylthio en $C_1$ -$C_3$, alkylsulfinyle en $C_1$ -$C_3$, alkylsulfonyle en $C_1$ -$C_3$, $CH_2CN$, CN, $CO_2R_c$, halogénalcoxy en $C_1$ -$C_3$, halogénalkylthio en $C_1$ -$C_3$, alcoxyalkyle en $C_2$ -$C_4$, alkylthioalkyle en $C_2$ -$C_4$, $CH_2N_3$ ou $NR_dR_e$ ;

$R_a$ est H, un groupe alkyle en $C_1$ -$C_4$, cyanoalkyle en $C_2$ -$C_3$, méthoxy ou éthoxy ;

$R_b$ est H, un groupe alkyle en $C_1$ -$C_4$ ou alcényle en $C_3$ -$C_4$ ; ou bien

$R_a$ et $R_b$ peuvent être pris ensemble sous la forme $-(CH_2)_3$-, $-(CH_2)_4$ -, $-(CH_2)_5$ - ou $-CH_2CH_2OCH_2CH_2$ - ;

$R_c$ est un groupe alkyle en $C_1$ -$C_4$, alcényle en $C_3$ -$C_4$, alcynyle en $C_3$ -$C_4$, halogénalkyle en $C_2$ -$C_4$, cyanoalkyle en $C_2$ -$C_3$, cycloalkyle en $C_5$ -$C_6$, cycloalkylalkyle en $C_4$ -$C_7$ ou alcoxyalkyle en $C_2$-$C_4$ ;

$R_d$ et $R_e$ sont indépendamment H ou un groupe alkyle en $C_1$ -$C_2$ ;

A est 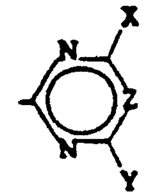 , 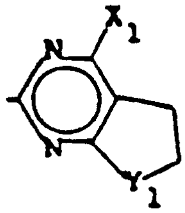 , 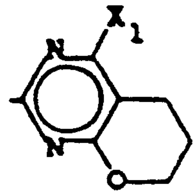 ,

A-1 A-2 A-3

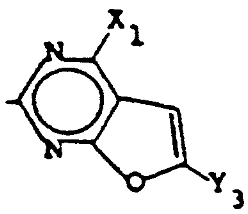 , 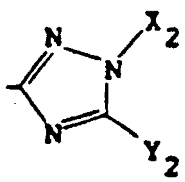 , 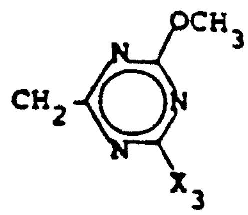 ;

A-4 A-5 A-6

ou 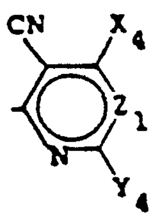 ;

A-7

X est H, un groupe alkyle en $C_1$ -$C_4$, alcoxy en $C_1$ -$C_4$, halogénalcoxy en $C_1$ -$C_4$, halogénalkyle en $C_1$ -$C_4$, halogénalkylthio en $C_1$ -$C_4$, alkylthio en $C_1$ -$C_4$, halogéno, alcoxyalkyle en $C_2$-$C_5$, alcoxyalcoxy en $C_2$ -$C_5$ , amino, alkylamino en $C_1$ -$C_3$, di(alkyle en $C_1$ -$C_3$)amino ou cycloalkyle en $C_3$ -$C_5$ ;
Y est H, un groupe alkyle en $C_1$ -$C_4$, alcoxy en $C_1$ -$C_4$, halogénalcoxy en $C_1$ -$C_4$, halogénalkylthio en $C_1$ -$C_4$, alkylthio en $C_1$ -$C_4$, alcoxyalkyle en $C_2$ -$C_5$, alcoxyalcoxy en $C_2$ -$C_5$, amino, alkylamino en $C_1$ -$C_3$, di(alkyle en $C_1$ -$C_3$ )amino, alcényloxy en $C_3$-$C_4$, alcynyloxy en $C_3$ -$C_4$, alkylthioalkyle en $C_2$ -$C_5$, halogénalkyle en $C_1$ -$C_4$, alcynyle en $C_2$ -$C_4$, azido, cyano, alkylsulfinylalkyle en $C_2$ -$C_5$,

alkylsulfonylalkyle en $C_2$ -$C_5$ , 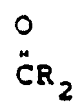 , 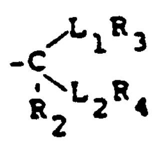 ,

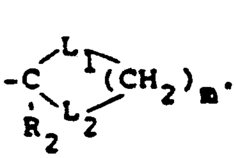 , 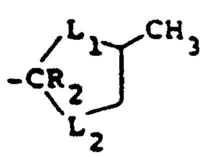 ou $N(OCH_3)CH_3$ ;

m est 2 ou 3 ;
$L_1$ et $L_2$ sont indépendamment O ou S ;
$R_2$ est H ou un groupe alkyle en $C_1$ -$C_3$ ;
$R_1$ et $R_4$ sont indépendamment un groupe alkyle en $C_1$ -$C_3$ ;
Z est CH, N, $CCH_3$, $CC_2H_5$ , CCl ou CBr ;
$Z_1$ est CH ou N ;
$Y_1$ est O ou $CH_2$ ;

$X_1$ est $CH_3$, $OCH_3$, $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $CH_3$ ou $CH_2CH_3$ ;
$X_3$ est $CH_3$ ou $OCH_3$ ;
$Y_3$ est H ou $CH_3$ ;
$X_4$ est $CH_3$, $OCH_3$, $OC_2H_5$, $CH_2OCH_3$ ou Cl ; et
$Y_4$ est $CH_3$, $OCH_3$, $OC_2H_5$ ou Cl ; et ses sels utilisables en agriculture ;
avec les conditions suivantes
a) si Q contient 2 hétéroatomes choisis parmi 0 à 2 atomes d'oxygène et 0 à 2 atomes de soufre, lesdits hétéroatomes ne sont pas liés directement l'un à l'autre, et si Q contient 3 hétéroatomes d'azote, seuls deux d'entre eux peuvent être liés directement ensemble ;
b) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$ ; c) si X ou Y est un groupe halogénalcoxy en $C_1$, alors Z est CH ;
d) si J est J-2 ou J-3, le substituant Q et le pont sulfonylurée se trouvent sur des atomes de carbone adjacents ;
e) si E est O, alors J est J-1 et W est 0 ;
f) si W est S, alors R est H, A est A-1, Z est CH ou N et Y est $CH_3$, $OCH_3$, $OC_2H_5$, $CH_2OCH_3$, $C_2H_5$, $CF_3$, $SCH_3$, $OCH_2$ $CH=CH_2$, $OCH_2C{\equiv}CH$, $OCH_2$ $CH_2$ $OCH_3$, $CH(OCH_3)_2$ ou le groupe 1,3-dioxolanne-2-yle ;
g) si le nombre total d'atomes de carbone de X et Y est supérieur à quatre, alors le nombre d'atomes de carbone de $R_1$ doit être inférieur ou égal à deux, et le nombre d'atomes de carbone du substituant filé sur Q doit être inférieur ou égal à deux ;
h) $X_4$ et $Y_4$ ne sont pas simultanément Cl ;
i) si $Q_1$ est lié par un atome d'azote et contient 2 hétéroatomes et un groupe carbonyle, et lesdits hétéroatomes sont liés par l'intermédiaire du groupe carbonyle, alors J est autre chose que J-1 ;
j) si A est A-1 et J est J-1 où E est une liaison simple, $X_a$ est $CH_2$, $CH(CH_3)$ ou $CH_2CH_2$ et $Q_2$ est un hétérocycle pentagonal contenant une double liaison endocyclique ou un hétérocycle hexagonal contenant 1 ou 2 doubles liaisons endocycliques qui est non substitué ou qui est substitué par un ou plusieurs groupes alkyles en $C_1$ -$C_4$, alors ledit hétérocycle doit contenir au moins un atome d'azote et être lié à $X_a$ par l'atome d'azote ;
k) si $X_a$ est CO, alors $E_1$ est une liaison simple ; et
1) si J est J-1, alors $E_1$ est une liaison simple.

2. Un composé de la revendication 1, dans lequel $Q_1$ est choisi parmi

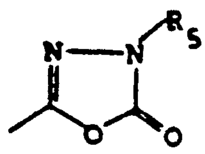 ,

$Q_1$-1

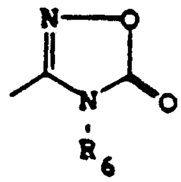 ,

$Q_1$-2

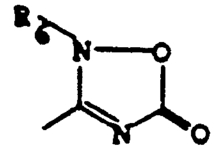 ,

$Q_1$-3

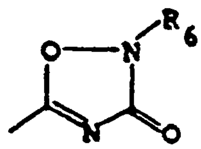 ,

$Q_1$-4

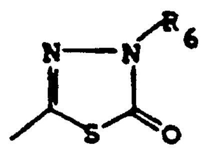 ,

$Q_1$-5

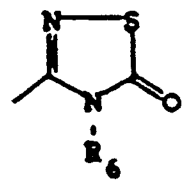 ,

$Q_1$-6

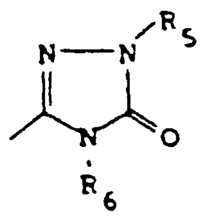 ,

$Q_1$-7

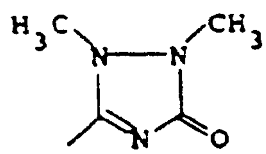 ,

$Q_1$-8

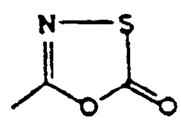 ,

$Q_1$-9

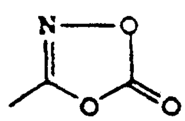 ,

$Q_1$-10

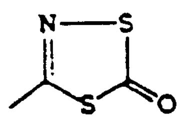 ,

$Q_1$-11

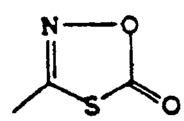 ,

$Q_1$-12

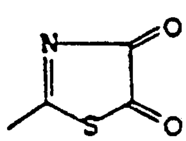 ,

$Q_1$-13

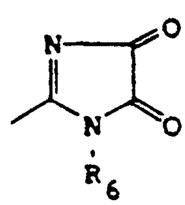 ,

$Q_1$-14

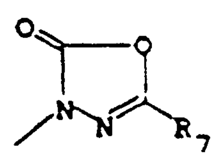 ,

$Q_1$-15

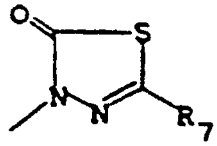
Q1-16 ,

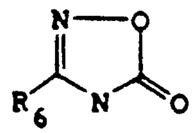
Q1-17 ,

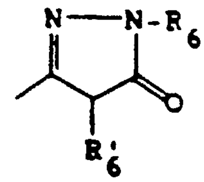
Q1-18 ,

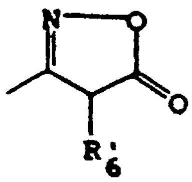
Q1-19 ,

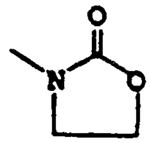
Q1-20 ,

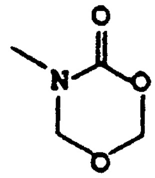
Q1-21 ,

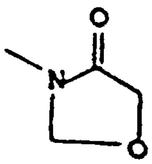
Q1-22 ,

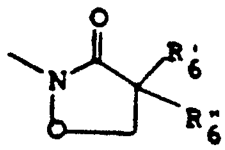
Q1-23 ,

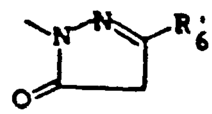
Q1-24 ,

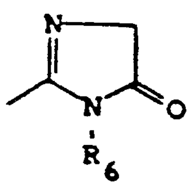
Q1-25 ,

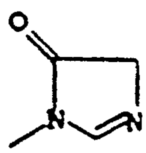
Q1-26 ,

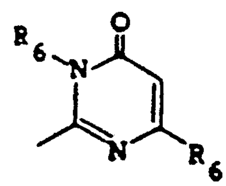
Q1-27 ,

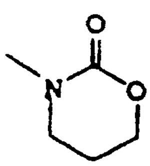
Q1-28 ,

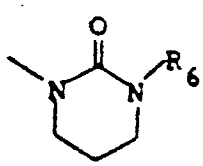
Q1-29 ou

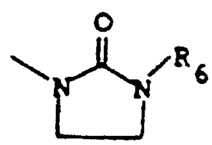
Q1-30 ;

où

$R_5$ est H, un groupe alkyle en $C_1$ -$C_4$, $CH_2CH=CH_2$, $CH_2$ $CH=CHCH_3$, $CH_2$ $C\equiv CH$, $CH_2$ $C\equiv CCH_3$, $CH_2$ CN, $CH_2$ $CO_2$ (alkyle en $C_1$ -$C_2$), $CH(CH_3)CO_2$ (alkyle en $C_1$ -$C_2$), $CF_2H$, alkyle en $C_2$ -$C_3$ substitué par 1 à 3 atomes de F ou Cl, $CH_2$ $OCH_3$, $CH_2$ $OCH_2$ $CH_3$, $CH_2CH_2OCH_3$ ou $CH_2CH_2OCH_2CH_3$ ;

$R_6$ est un groupe alkyle en $C_1$ -$C_3$ ;

$R_6'$ et $R_6''$ sont indépendamment H ou un groupe alkyle en $C_1$ -$C_2$ ; et

$R_7$ est H, un groupe alkyle en $C_1$ -$C_4$, halogénalkyle en $C_1$ -$C_3$ ou $CH_2CH=CH_2$ .

3. Un composé de la revendication 1, dans lequel $Q_2$ est choisi parmi

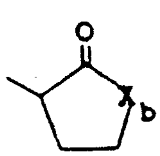

$Q_2$-1

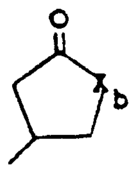

$Q_2$-2

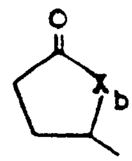

$Q_2$-3

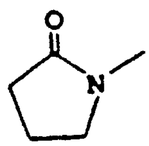

$Q_2$-4

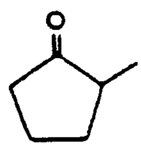

$Q_2$-5

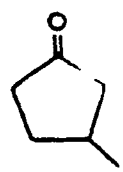

$Q_2$-6

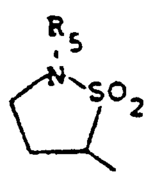

$Q_2$-7

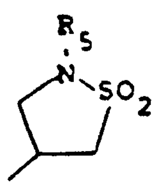

$Q_2$-8

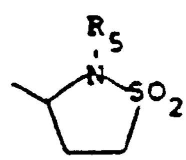

$Q_2$-9

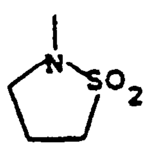

$Q_2$-10

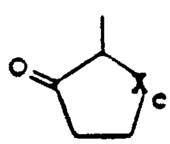

$Q_2$-11

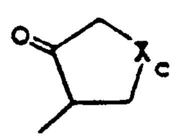

$Q_2$-12

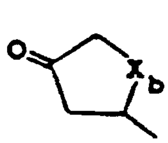 .

$Q_2$-13

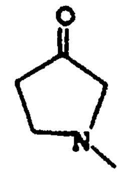 .

$Q_2$-14

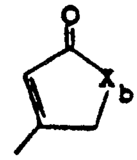 .

$Q_2$-15

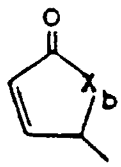 .

$Q_2$-16

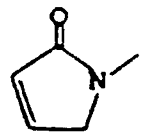 .

$Q_2$-17

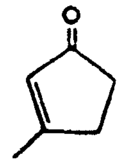 .

$Q_2$-18

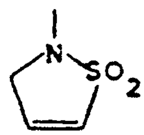 .

$Q_2$-19

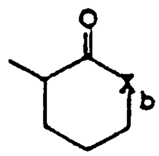 .

$Q_2$-20

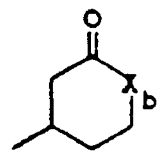 .

$Q_2$-21

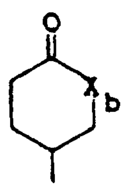 .

$Q_2$-22

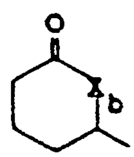 .

$Q_2$-23

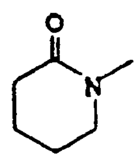 .

$Q_2$-24

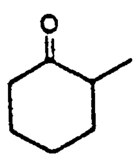 .

$Q_2$-25

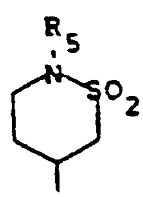 .

$Q_2$-26

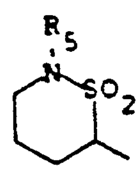 .

$Q_2$-27

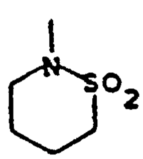 .

$Q_2$-28

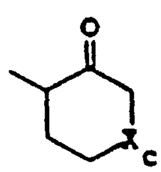 .

$Q_2$-29

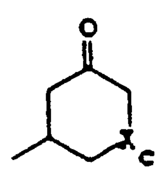 .

$Q_2$-30

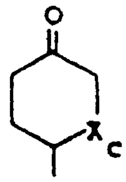 ,

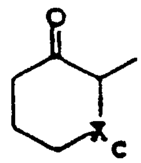 ,

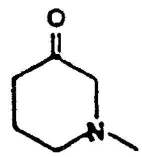 ,

$Q_2$-31 $Q_2$-32 $Q_2$-33

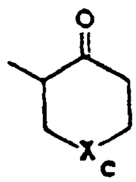 ,

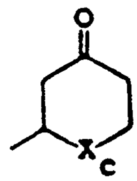 ,

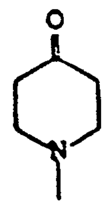 ,

$Q_2$-34 $Q_2$-35 $Q_2$-36

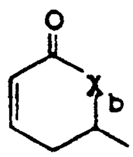 ,

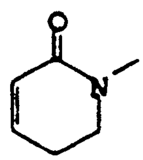 ,

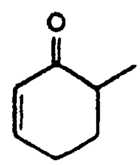 ,

$Q_2$-37 $Q_2$-38 $Q_2$-39

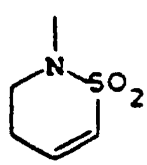 ,

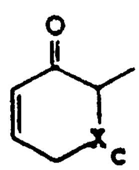 ,

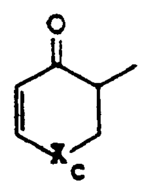 ,

$Q_2$-40 $Q_2$-41 $Q_2$-42

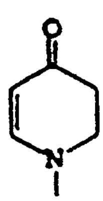 ,

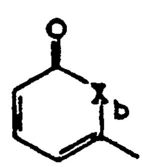 ,

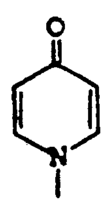 ,

$Q_2$-43 $Q_2$-44 $Q_2$-45

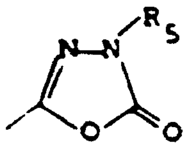 .

Q2-46

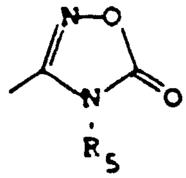 .

Q2-47

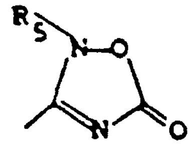 .

Q2-48

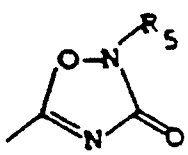 .

Q2-49

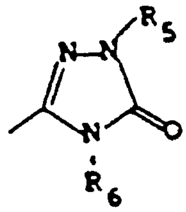 .

Q2-50

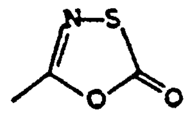 .

Q2-51

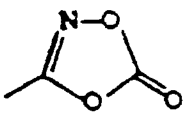 .

Q2-52

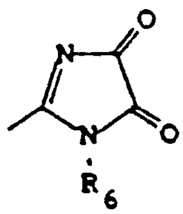 .

Q2-53

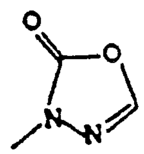 .

Q2-54

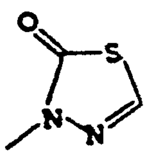 .

Q2-55

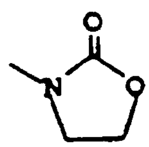 .

Q2-56

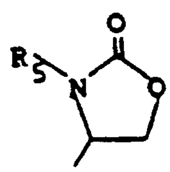 .

Q2-57

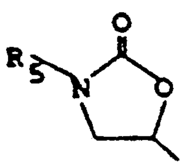 .

Q2-58

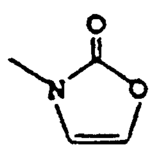 .

Q2-59

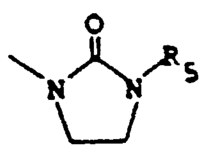 .

Q2-60

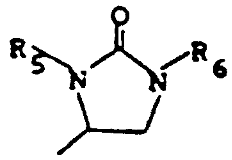 .

$Q_2$-61

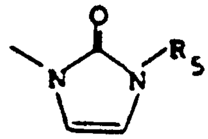 .

$Q_2$-62

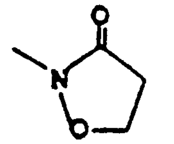 .

$Q_2$-63

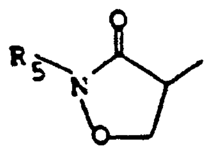 .

$Q_2$-64

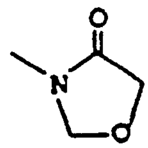 .

$Q_2$-65

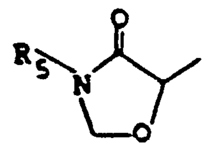 .

$Q_2$-66

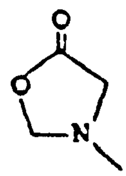 .

$Q_2$-67

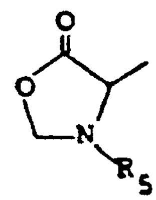 .

$Q_2$-68

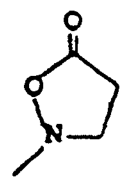 .

$Q_2$-69

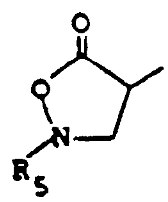 .

$Q_2$-70

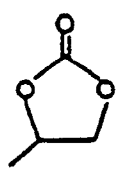 .

$Q_2$-71

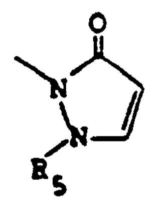 .

$Q_2$-72

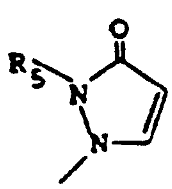 .

$Q_2$-73

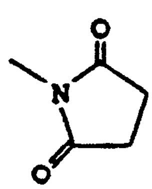 .

$Q_2$-74

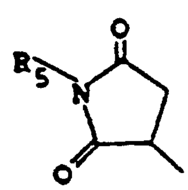 .

$Q_2$-75

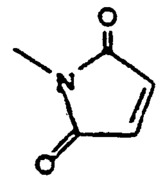 .

$Q_2$-76

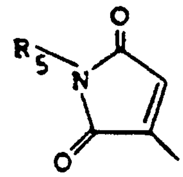 .

$Q_2$-77

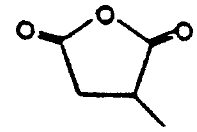 .

$Q_2$-78

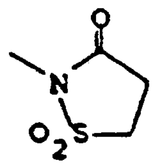 .

$Q_2$-79

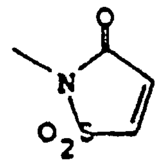 .

$Q_2$-80

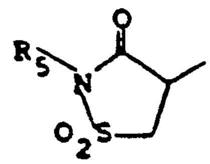 .

$Q_2$-81

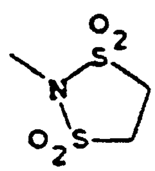 .

$Q_2$-82

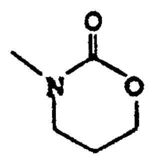 .

$Q_2$-83

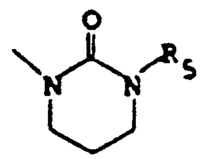 .

$Q_2$-84

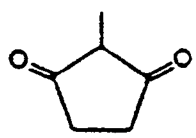 .

$Q_2$-85

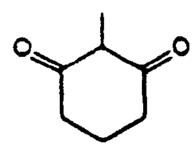 et

$Q_2$-86

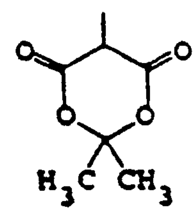 ;

$Q_2$-87

où

$Q_2$ -1 à $Q_2$-87 peuvent être facultativement substitués par 1 ou 2 groupes choisis parmi les groupes alkyles en $C_1$ -$C_2$ et halogénalkyles en $C_1$ -$C_2$ ;

$R_5$ et $R_6$ sont indépendamment H ou un groupe alkyle en $C_1$ -$C_3$ ;

$X_b$ est O ou $NR_5$ ; et

$X_c$ est O, S, SO, $SO_2$ ou $NR_5$.

**4.** Composés de la revendication 1, où

Q est $Q_1$ ;

E est une liaison simple ; et

Z est CH ou N.

**5.** Composés de la revendication 1, où

Q est $Q_1$ ;

E est $CH_2$ ;

W est O ; et

Z est CH ou N.

**6.** Composés de la revendication 1, où
Q est $Q_1$ ;
E est O ; et
Z est CH ou N.

**7.** Composés de la revendication 2, où E est une liaison simple et Z est CH ou N.

**8.** Composés de la revendication 7, où
W est O ;
R est H ;
$R_1$ est H, F, C1, Br, un groupe alkyle en $C_1$-$C_2$, alcoxy en $C_1$ -$C_3$, alkylthio en $C_1$ -$C_3$, ou alkyle en $C_1$ -$C_2$, alcoxy en $C_1$ -$C_3$ ou alkylthio en $C_1$ -$C_3$ substitué par 1 à 3 atomes de F, Cl ou Br ;
X est un groupe alkyle en $C_1$ -$C_2$, alcoxy en $C_1$ -$C_2$, Cl, F, Br, I, $OCF_2H$, $CH_2F$, $CF_3$ , $OCH_2CH_2F$, $OCH_2CHF_2$, $OCH_2CF_3$, $CH_2Cl$ ou $CH_2Br$ ; et
Y est H, un groupe alkyle en $C_1$ -$C_2$, alcoxy en $C_1$ -$C_2$, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$, $CH_2SCH_3$

, 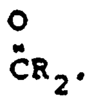 , 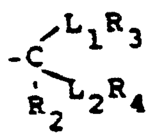 ,

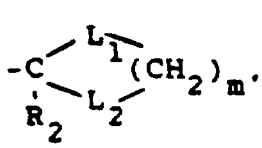 , 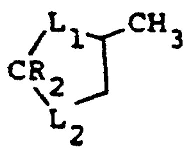 ,

$OCF_2H$, $SCF_2H$, cyclopropyle, $C\equiv CH$ ou $C\equiv CCH_3$.

**9.** Composés de la revendication 8, où A est A-1.

**10.** Composés de la revendication 9, où J est J-1 à J-5.

**11.** Composés de la revendication 9, où
J est J-1 ;
$R_1$ est H, Cl, $CH_3$ ou $OCH_3$ et n'est pas fixé en para par rapport au pont sulfonylurée ;
X est $CH_3$, $OCH_3$, Cl ou $OCF_2H$ ; et
Y est $CH_3$, $OCH_3$, $C_2H_5$, $CH_2$ $OCH_3$, $NHCH_3$, $CH(OCH_3)_2$ ou le groupe cyclopropyle.

**12.** Composés de la revendication 11, où $Q_1$ est $Q_1$ -1 à $Q_1$ -27.

**13.** Composés de la revendication 2, où
E est $CH_2$ ;
W est O ;
R est H ;
J est J-1 ;
$R_1$ est H ;
A est A-1 ;
X est $CH_3$, $OCH_3$, $OCH_2CH_3$, Cl ou $OCF_2H$ ;
Y est $CH_3$, $OCH_3$, $C_2H_5$, $CH_2OCH_3$, $NHCH_3$, $CH(OCH_3)_2$ ou le groupe cyclopropyle et $Q_1$ est $Q_1$ -1, $Q_1$ -7, $Q_1$ -10 ou $Q_1$ -15 ; et
Z est CH ou N.

**14.** Composés de la revendication 2, où

E est O ;
R est H ;
J est J-1 ;
$R_1$ est H ;
A est A-1 ;
X est $CH_3$, $OCH_3$, $OCH_2CH_3$, Cl ou $OCF_2H$ ;
Y est $CH_3$, $OCH_3$, $C_2H_5$, $CH_2OCH_3$, $NHCH_3$, $CH(OCH_3)_2$ ou le groupe cyclopropyle et $Q_1$ est $Q_1$ -1, $Q_1$ -7, $Q_1$ -10 ou $Q_1$ -15 ; et
Z est CH ou N.

**15.** Composés de la revendication 1, où
Q est $E_1X_aQ_2$ ;
E est une liaison simple ; et
Z est CH ou N.

**16.** Composés de la revendication 1, où
Q est $E_1X_aQ_2$ ;
E est $CH_2$ ;
W est O ;
Z est CH ou N ; et
$E_1$ est une liaison simple.

**17.** Composés de la revendication 1, où
Q est $E_1X_aQ_2$ ;
E est O ;
Z est CH ou N ; et
$E_1$ est une liaison simple.

**18.** Composés de la revendication 3, où
$Q_2$ -1 à $Q_2$ -87 peuvent être facultativement substitués par 1 ou 2 groupes choisis parmi les groupes alkyles en $C_1$ -$C_2$ ou halogénalkyles en $C_1$ -$C_2$ ;
$R_5$ et $R_6$ sont indépendamment H ou un groupe alkyle en $C_1$ -$C_3$ ;
$X_b$ est O ou $NR_5$ ;
$X_c$ est O, S, SO, SO2 ou $NR_5$ ;
E est une liaison simple ; et
Z est CH ou N.

**19.** Composés de la revendication 18, où
W est O ;
$E_1$ est une liaison simple ;
$X_a$ est $CH_2$ ou $CH_2CH_2$ ;
R est H ;
$R_1$ est H, F, Cl, Br, un groupe alkyle en $C_1$-$C_2$, alcoxy en $C_1$ -$C_3$, alkylthio en $C_1$ -$C_3$, ou alkyle en $C_1$ -$C_2$, alcoxy en $C_1$ -$C_3$ ou alkylthio $C_1$ -$C_3$ substitué par 1 à 3 atomes de F, Cl ou Br ;
X est un groupe alkyle en $C_1$ -$C_2$, alcoxy en $C_1$ -$C_2$, Cl, F, Br, I, $OCF_2H$, $CH_2F$, $CF_3$, $OCH_2CH_2F$, $OCH_2CHF_2$, $OCH_2CF_3$, $CH_2Cl$ ou $CH_2Br$ ; et
Y est H, un groupe alkyle en $C_1$ -$C_2$, alcoxy en $C_1$ -$C_2$, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C≡CH$, $OCH_2$ $CH_2OCH_3$, $CH_2SCH_3$,

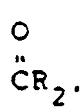,
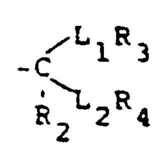.

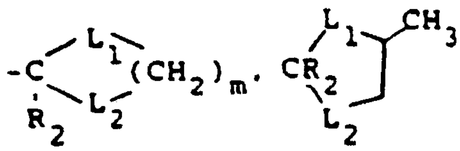 .

$OCF_2H$, $SCF_2H$, cyclopropyle, C≡CH ou $C{\equiv}CCH_3$.

**20.** Composés de la revendication 19, où A est A-1.

**21.** Composés de la revendication 20, où J est J-1 à J-5.

**22.** Composés de la revendication 21, où
J est J-1 ;
$R_1$ est H, Cl, $CH_3$ ou $OCH_3$ ;
X est $CH_3$, $OCH_3$, Cl ou $OCF_2H$ ; et
Y est $CH_3$, $OCH_3$, $C_2H_5$, $CH_2OCH_3$, $NHCH_3$, $CH(OCH_3)_2$ ou le groupe cyclopropyle.

**23.** Composés de la revendication 16, où
R est H ;
J est J-1 ;
$R_1$ est H ;
A est A-1 ;
X est $CH_3$, $OCH_3$, $OCH_2CH_3$, Cl ou $OCF_2H$ ;
Y est $CH_3$, $OCH_3$, $C_2H_5$, $CH_2OCH_3$, $NHCH_3$, $CH(OCH_3)_2$ ou le groupe cyclopropyle ;
Z est CH ou N ; et
$Q_2$ est $Q_2$ -1, $Q_2$ -4, $Q_2$ -5, $Q_2$ -7, $Q_2$ -10, $Q_2$ -11, $Q_2$ -12, $Q_2$ -17, $Q_2$ -19, $Q_2$ -20, $Q_2$ -24, $Q_2$ -25, $Q_2$ -27, $Q_2$ -28, $Q_2$ -36, $Q_2$ -38, $Q_2$ -46, $Q_2$ -47, $Q_2$ -54, $Q_2$ -56, $Q_2$ -59, $Q_2$ -60, $Q_2$ -63, $Q_2$ -71, $Q_2$ -74, $Q_2$ -76, $Q_2$ -78 et $Q_2$ -79.

**24.** Composés de la revendication 17, où
R est H ;
$R_1$ est H ;
A est A-1 ;
X est $CH_3$, $OCH_3$, $OCH_2CH_3$, Cl ou $OCF_2H$ ;
Y est $CH_3$, $OCH_3$, $C_2H_5$, $CH_2OCH_3$, $NHCH_3$, $CH(OCH_3)_2$ ou le groupe cyclopropyle ;
Z est CH ou N ; et
$Q_2$ est $Q_2$ -1, $Q_2$ -4, $Q_2$ -5, $Q_2$ -7, $Q_2$ -10, $Q_2$ -11, $Q_2$ -12, Q2 -17, $Q_2$ -19, $Q_2$ -20, $Q_2$ -24, $Q_2$ -25, $Q_2$ -27, $Q_2$ -28, $Q_2$ -36, $Q_2$ -38, $Q_2$ -46, $Q_2$ -47, $Q_2$ -54, $Q_2$ -56, $Q_2$ -59, $Q_2$ -60, $Q_2$ -63, $Q_2$ -71, $Q_2$ -74, $Q_2$ -76, $Q_2$ -78, et $Q_2$ 79.

**25.** Composés de la revendication I, où
Q est $E_1X_aQ_2$ ;
$E_1$ est une liaison simple ;
$X_a$ est $CH_2$, $CH(CH_3)$, $CH_2CH_2$ ou $CH_2CH_2CH_2$ ;
$Q_2$ est un carbocycle penta- ou hexagonal saturé ou partiellement saturé contenant un ou deux groupes carbonyle, ou un hétérocycle penta- ou hexagonal saturé ou partiellement saturé contenant 2 à 5 atomes de carbone et 1 à 3 hétéroatomes choisis dans le groupe formé par 0 à 2 atomes d'oxygène, 0 à 2 atomes de soufre ou 0 à 3 atomes d'azote, où le soufre peut prendre la forme de S, SO ou $SO_2$, et contenant un ou deux groupes carbonyle ou sulfonyle ($SO_2$), ou un groupe carbonyle et un groupe sulfonyle ; Q peut de plus être facultativement substitué par 1 ou 2 groupes substituants : les substituants fixés sur du carbone peuvent être choisis dans la classe formée par les groupes halogéno, alkyles en $C_1$ -$C_4$, halogénalkyles en $C_1$ -$C_4$, $CH_2$ (alcényle en $C_2$ -$C_3$), $CH_2$ -(alcynyle en $C_2$ -$C_3$), alcoxycarbonyle en $C_2$ -$C_4$, CN, OH, alcoxy en $C_1$ -$C_3$, alkylthio en $C_1$ -$C_3$, alkylsulfinyles en $C_1$ -$C_3$ , alkylsulfonyles en $C_1$ -$C_3$ ou alkylcarbonyles en $C_2$ -$C_3$, les substituants fixés sur de l'azote peuvent être choisis dans la classe formée par les groupes alkyles en $C_1$ -$C_4$, halogénalkyles en $C_1$ -$C_4$, $CH_2$ (alcényle en $C_2$ - $C_3$ ), $CH_2$ (alcynyle en $C_2$ - $C_3$ ), alcoxycarbonyles en $C_2$ -$C_4$ ou alkylcarbonyles en $C_2$-$C_4$ ;

$R_1$ est H, un groupe alkyle en $C_1$ -$C_3$, halogénalkyle en $C_1$ -$C_3$, halogéno, nitro, alcoxy en $C_1$ -$C_3$, $SO_2$ $NR_aR_b$, alkylthio en $C_1$ -$C_3$, alkylsulfinyle en $C_1$ -$C_3$, alkylsulfonyle en $C_1$ -$C_3$, $CH_2CN$, CN, $CO_2R_c$, halogénalcoxy en $C_1$ -$C_3$ ou halogénalkylthio en $C_1$ -$C_3$ ;

A est 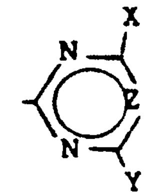 , 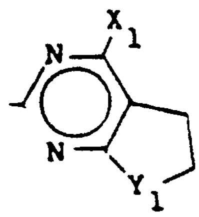 , 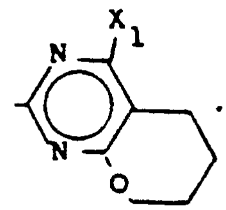 ,

A-1 A-2 A-3

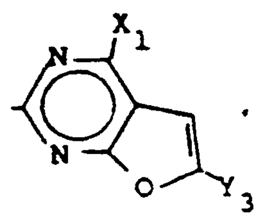 , 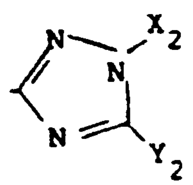 ou 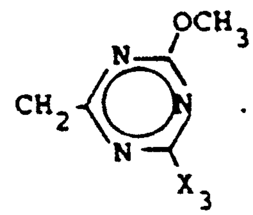 .

A-4 A-5 A-6

26. Un composé choisi parmi
- le 2-(4,5-dihydro-4-méthyl-5-oxo-l,3,4-oxadiazole-2-yl)-N-[(4,6-diméthoxypyrimidine-2-yl)-aminocarbonyl]benzène-sulfonamide,
- le 2-( 4, 5-dihydro-4-méthyl-5-oxo-1, 3, 4-oxadiazole-2-yl)-N-[(4-méthoxy-6-méthyl-1,3,5-triazine-2-yl)aminocarbonyl]benzène-sulfonamide,
- le N-[(4,6-diméthoxypyrimidine-2-yl)aminocarbonyl]-2-(2-oxo-1-pyrrolidinylméthyl)benzène-sulfonamide,
- le N- [ ( 4, 6-diméthoxy-1,3,5-triazine-2-y1) aminocarbonyl]-2-(2-oxo-1-pyrrolidinylméthyl)benzène-sulfonamide,
- le N-[(4,6-diméthoxypyrimidine-2-yl)aminocarbonyl]-2-(2-oxo-3-oxazolidinylméthyl)-3-thiophène-sulfonamide, et
- le N-[(4-méthoxy-6-méthyl-1,3,5-triazine-2-yl)aminocarbonyl]-2-(2-oxo-3-oxazolidinylméthyl)-3-thiophène-sulfonamide.

27. Une composition convenant pour lutter contre la croissance de végétation indésirable, qui comprend une quantité efficace d'un composé de l'une quelconque des revendications 1 à 26 et au moins l'un des ingrédients suivants: agents tensio-actif, diluant solide ou diluant liquide.

28. Une méthode pour lutter contre la croissance de végétation indésirable, qui consiste à appliquer à l'emplacement à protéger une quantité efficace d'un composé de l'une quelconque des revendications 1 à 26.

29. Une méthode pour réguler la croissance de plantes, qui consiste à appliquer à l'emplacement où se trouvent ces plantes une quantité efficace, mais sensiblement non phytotoxique, d'un régulateur de la croissance de plantes choisi parmi les composés de l'une quelconque des revendications 1 à 26.

30. Un procédé pour la préparation d'un composé de la revendication 1, qui comprend

(I) (a) la réaction de $JSO_2NCW$ avec A-NHR

(b) la réaction de $JSO_2NH_2$ avec $PhO\overset{W}{\overset{\|}{C}}NHA$

(c) la réaction de $JSO_2NH\underset{W}{\underset{\|}{C}}OPh$ avec A-NHR

où J, R, W et A sont tels que définis dans la revendication 1, à condition que E soit $CH_2$ ou une liaison simple ; ou

(II) (a) la réaction de $JSO_2NCW$ avec A-NHR

(b) la réaction de JH avec $ClSO_2NH\underset{O}{\underset{\|}{C}}NHA$

où J, W et A sont tels que définis dans la revendication 1, à condition que E soit O.

**31.** Composés des formules :
$JSO_2NCW$

$JSO_2NH_2$

$JSO_2NH\underset{W}{\underset{\|}{C}}OPh$

et JH

tels que définis dans la revendication 30.

**Ansprüche**

**1.** Verbindung, ausgewählt aus

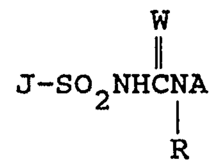 ,

worin

J 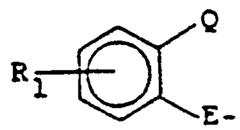 , 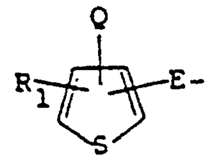 , 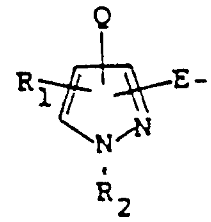 ,

J-1 J-2 J-3

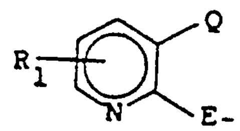 , 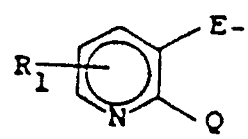

J-4 J-5

ist;

W O oder S ist;
R H oder $CH_3$ ist;
E eine Einfachbindung, $CH_2$ oder O ist;
Q $Q_1$ oder $E_1X_aQ_2$ ist;
$Q_1$ ein gesättigter 5- oder 6-gliedriger heterocyclischer, über Kohlenstoff oder Stickstoff gebundener Ring, der eine Carbonyl-Gruppe und 2 bis 3 Hetero-Atome enthält, die aus der aus 0-2 Sauerstoff, 0-2 Schwefel oder 0-2 Stickstoff bestehenden Gruppe ausgewählt sind;
ein 5-gliedriger heterocyclischer, über Kohlenstoff oder Stickstoff gebundener Ring, der eine Carbonyl-Gruppe und 2 bis 3 Hetero-Atome enthält, die aus der aus 0-2 Sauerstoff, 0-2 Schwefel oder 1-3 Stickstoff bestehenden Gruppe ausgewählt sind und eine endocyclische Doppelbindung enthält;
ein 6-gliedriger heterocyclischer, über Kohlenstoff oder Stickstoff gebundener Ring, der eine Carbonyl-Gruppe und 2 bis 3 Hetero-Atome enthält, die aus der aus 0-2 Sauerstoff, 0-2 Schwefel oder 1-3 Stickstoff bestehenden Gruppe ausgewählt sind, und eine oder zwei endocyclische Doppelbindung(en) enthält; oder
ein 5-gliedriger heterocyclischer, über Kohlenstoff oder Stickstoff gebundener Ring, der zwei benachbarte Carbonyl-Gruppen und 2 Hetero-Atome enthält, die aus der aus 0-1 Sauerstoff, 0-1 Schwefel oder 1-2 Stickstoff bestehenden Gruppe ausgewählt sind und eine endocyclische Doppelbindung enthält, ist,
wobei der Wert Q substituiert oder unsubstituiert sein kann, wobei die Substituenten-Gruppen aus der aus $C_1$-$C_4$-Alkyl, $C_1$-$C_4$-Halogenoalkyl, $C_3$-$C_4$-Alken-yl, $C_3$-$C_4$-Halogenoalkenyl, $C_3$-$C_4$-Alkinyl, $C_3$-$C_4$-Halogenoalkinyl, $C_1$-$C_3$-Cyanoalkyl, $C_2$-$C_4$-Alkoxy-alkyl, $C_2$-$C_4$-Alkylthioalkyl, $C_2$-$C_4$ -Alkylcarbonyl, $C_3$-$C_4$-Alkylcarbonylalkyl, $C_1$-$C_4$-Alkyl, das mit OH oder $NH_2$ substituiert ist, $C_2$-$C_4$-Alkylaminoalkyl, $C_3$-$C_4$-Dialkylaminoalkyl, $CH_2CH(OCH_3)_2$,

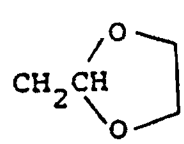 ,

$C(O)N(CH_3)_2$, $P(O)(OC_1$-$C_2$-$Alkyl)_2$,

$P(S)(OC_1-C_2-Alkyl)_2$ oder $C_1-C_2$-Alkyl, das mit $C_1-C_2$-Alkoxycarbonyl substituiert ist, bestehenden Gruppe ausgewählt sind;

$E_1$ O, S, SO, $SO_2$ oder eine Einfachbindung ist;

$Q_2$ ein 5- oder 6-gliedriger carbocyclischer Ring, der entweder eine oder zwei Carbonyl-Gruppen und 0-1 endocyclische Doppelbindungen enthält

ein 5-gliedriger heterocyclischer Ring, der 2-4 Kohlenstoff-Atome und 1-3 Hetero-Atome enthält, die aus der aus 0-2 Sauerstoff, 0-2 Schwefel oder 0-3 Stickstoff bestehenden Gruppe ausgewählt sind, wobei Schwefel die Form S, SO oder $SO_2$ annehmen kann, und der eine oder zwei Carbonyl- oder Sulfonyl-($SO_2$-)Gruppen oder eine Carbonyl- und eine Sulfonyl-Gruppe und 0-1 endocyclische Doppelbindungen enthält; oder

ein 6-gliedriger heterocyclischer Ring, der 2-5 Kohlenstoff-Atome und 1-3 Hetero-Atome enthält, die aus der aus 0-2 Sauerstoff, 0-2 Schwefel oder 0-3 Stickstoff bestehenden Gruppe ausgewählt sind, wobei Schwefel die Form S, SO oder $SO_2$ annehmen kann, und der eine oder zwei Carbonyl- oder Sulfonyl-($SO_2$-)Gruppen oder eine Carbonyl- und eine Sulfonyl-Gruppe und 0-2 endocyclische Doppelbindungen enthält, ist,

wobei der Wert Q gegebenenfalls mit 1-2 Substituenten-Gruppen substituiert sein kann, wobei die Substituenten am Kohlenstoff aus der aus Halogen, $C_1-C_4$-Alkyl, $C_1-C_4$-Halogenoalkyl, $CH_2(C_2-C_3$-Alkenyl), $CH_2(C_2-C_3$-Alkinyl), $C_2-C_4$-Alkoxycarbonyl, CN, OH, $C_1-C_3$-Alkoxy, $C_1-C_3$-Alkylthio, $C_1-C_3$-Alkyl-sulfinyl, $C_1-C_3$-Alkylsulfonyl oder $C_2-C_4$-Alkyl-carbonyl bestehenden Gruppe ausgewählt sein können und die Substituenten am Stickstoff aus der aus $C_1-C_4$-Alkyl, $C_1-C_4$-Halogenoalkyl, $CH_2(C_2-C_3$-Alkenyl), $CH_2(C_2-C_3$-Alkinyl), $C_2-C_4$-Alkoxycarbonyl oder $C_2-C_4$-Alkylcarbonyl bestehenden Gruppe ausgewählt sein können;

$X_a$ $CH_2$, $CH(CH_3)$, $CH_2CH_2$, $CH_2CH_2CH_2$ oder CO ist;

$R_1$ H, $C_1-C_3$-Alkyl, $C_1-C_3$-Halogenoalkyl, Halogen, Nitro, $C_1-C_3$-Alkoxy, $SO_2NR_aR_b$, $C_1-C_3$-Alkylthio, $C_1-C_3$-Alkylsulfinyl, $C_1-C_3$-Alkylsulfonyl, $CH_2CN$, CN, $CO_2R_c$, $C_1-C_3$-Halogenoalkoxy, $C_1-C_3$-Halogenoalkylthio, $C_2-C_4$-Alkoxyalkyl, $C_2-C_4$-Alkylthio-alkyl, $CH_2N_3$ oder $NR_dR_e$ ist;

$R_a$ H, $C_1-C_4$-Alkyl, $C_2-C_3$-Cyanoalkyl, Methoxy oder Ethoxy ist,

$R_b$ H, $C_1-C_4$-Alkyl oder $C_3-C_4$-Alkenyl ist, oder

$R_a$ und $R_b$ zusammen genommen $-(CH_2)_3-$, $-(CH_2)_4-$, $-(CH_2)_5-$ oder $-CH_2CH_2OCH_2CH_2-$ sein können;

$R_c$ $C_1-C_4$-Alkyl, $C_3-C_4$-Alkenyl, $C_3-C_4$-Alkinyl, $C_2-C_4$-Halogenoalkyl, $C_2-C_3$-Cyanoalkyl, $C_5-C_6$-Cycloalkyl, $C_4-C_7$-Cycloalkylalkyl oder $C_2-C_4$-Alkoxyalkyl ist;

$R_d$ und $R_e$ unabhängig voneinander H oder $C_1-C_2$-Alkyl sind;

A

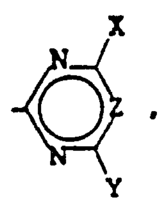
,

A-1

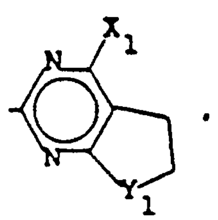
,

A-2

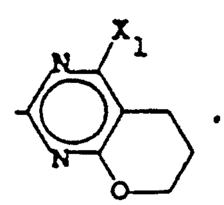
,

A-3

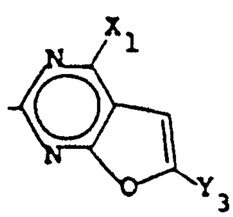
,

A-4

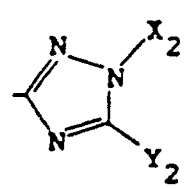
,

A-5

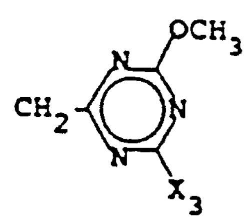

A-6

oder 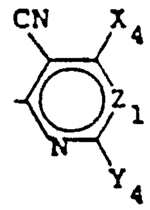 ist;

A-7

X H, $C_1$-$C_4$-Alkyl, $C_1$-$C_4$-Alkoxy, $C_1$-$C_4$-Halogenoalkoxy, $C_1$-$C_4$-Halogenoalkyl, $C_1$-$C_4$-Halogenoalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di-($C_1$-$C_3$)alkylamino oder $C_3$-$C_5$-Cycloalkyl ist;
Y H, $C_1$-$C_4$-Alkyl, $C_1$-$C_4$-Alkoxy, $C_1$-$C_4$-Halogenoalkoxy, $C_1$-$C_4$-Halogenoalkylthio, $C_1$-$C_4$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$)alkylamino, $C_3$-$C_4$-Alkenyloxy, $C_3$-$C_4$-Alkinyloxy, $C_2$-$C_5$-Alkylthioalkyl, $C_1$-$C_4$-Halogenoalkyl, $C_2$-$C_4$-Alkinyl, Azido, Cyano, $C_2$-$C_5$-Alkylsulfinylalkyl, $C_2$-$C_5$-Alkylsulfonylalkyl,

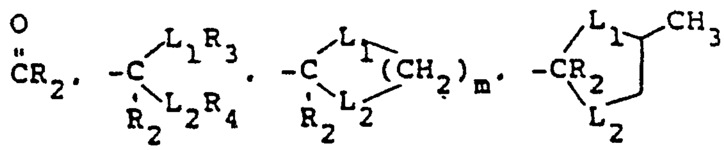

oder $N(OCH_3)CH_3$ ist;
m 2 oder 3 ist,
$L_1$ und $L_2$ unabhängig voneinander O oder S sind;
$R_2$ H oder $C_1$-$C_3$-Alkyl ist;
$R_3$ und $R_4$ unabhängig voneinander $C_1$-$C_3$-Alkyl sind;
Z CH, N, $CCH_3$, $CC_2H_5$, CCl oder CBr ist;
$Z_1$ CH oder N ist;
$Y_1$ O oder $CH_2$ ist;
$X_1$ $CH_3$, $OCH_3$, $OC_2H_5$ oder $OCF_2H$ ist;
$X_2$ $CH_3$, $C_2H_5$ oder $CH_2CF_3$ ist;
$Y_2$ $OCH_3$, $OC_2H_5$, $SCH_3$, $SC_2H_5$, $CH_3$ oder $CH_2CH_3$ ist;
$X_3$ $CH_3$ oder $OCH_3$ ist;
$Y_3$ H oder $CH_3$ ist;
$X_4$ $CH_3$, $OCH_3$, $OC_2H_5$, $CH_2OCH_3$ oder Cl ist; und
$Y_4$ $CH_3$, $OCH_3$, $OC_2H_5$ oder Cl ist; und ihre landwirtschaftlich geeigneten Salze,
mit den Maßgaben, daß

a) wenn Q 2 Hetero-Atome enthält, die aus 0-2 Sauerstoff und 0-2 Schwefel ausgewählt sind, diese Hetero-Atome nicht direkt aneinander gebunden sind, und wenn Q 3 Hetero-Atome enthält, nur zwei von diesen direkt aneinander gebunden sein können;

b) wenn X C1, F, Br oder I ist, dann Z CH ist und Y $OCH_3$, $OC_2H_5$, $N(OCH_3)CH_3$, $NHCH_3$, $N(CH_3)_2$ oder $OCF_2H$ ist;

c) wenn X oder Y $C_1$-Halogenalkoxy ist, dann Z CH ist;

d) wenn J J-2 oder J-3 ist, der Substiuent Q und die Sulfonylharnstoff-Brucke sich an benachbarten Kohlenstoff-Atomen befinden;

e) wenn E O ist, dann J J-1 ist und W O ist;

f) wenn W S ist, dann R H ist, A A-1 ist, Z CH oder N ist und Y $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$ oder 1,3-Dioxolan-2-yl ist;

g) wenn die Gesamtzahl der Kohlenstoff-Atome von X und Y größer als 4 ist, dann die Zahl der Kohlenstoff-Atome von $R_1$ kleiner als oder gleich zwei sein muß und die Zahl der Kohlenstoff-Atome des Substituenten an Q kleiner als oder gleich zwei sein muß;

h) $X_4$ und $Y_4$ nicht gleichzeitig C1 sind;

i) wenn $Q_1$ über Stickstoff gebunden ist und 2 Hetero-Atome und eine Carbonyl-Gruppe enthält und diese Hetero-Atome über das Carbonyl gebunden sind, dann J von J-1 verschieden ist;
j) wenn A A-1 ist und J J-1 ist, worin E eine Einfachbindung ist, $X_a$ $CH_2$, $CH(CH_3)$ oder $CH_2CH_2$ ist und Q ein 5-gliedriger heterocyclischer Rest mit einer endocyclischen Doppelbindung oder ein 6-gliedriger heterocyclischer Rest mit einer oder zwei endocyclischen Doppelbindung(en) ist, der unsubstituiert oder mit einer oder mehreren $C_1$-$C_4$-Alkyl-Gruppen substituiert ist, dann dieser Heterocyclus wenigstens einen Stickstoff enthalten und über Stickstoff an $X_a$ gebunden sein muß;
k) wenn $X_a$ CO ist, dann $E_1$ eine Einfachbindung ist; und
l) wenn J J-1 ist, dann $E_1$ eine Einfachbindung ist.

2. Verbindung nach Anspruch 1, worin $Q_1$ ausgewählt ist aus

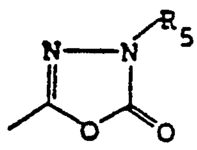
$Q_1$-1
,
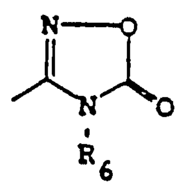
$Q_1$-2
,
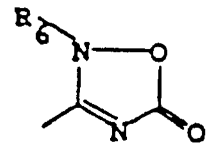
$Q_1$-3
,
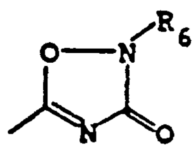
$Q_1$-4
,
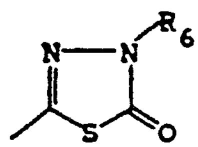
$Q_1$-5
,
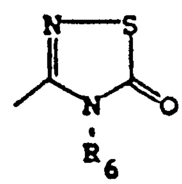
$Q_1$-6
,

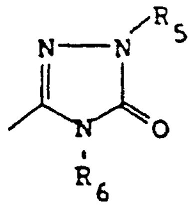

$Q_1$-7 ,

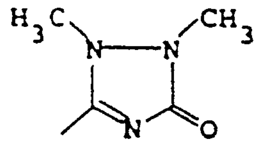

$Q_1$-8 ,

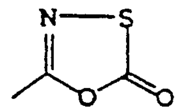

$Q_1$-9 ,

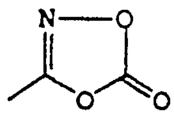

$Q_1$-10 ,

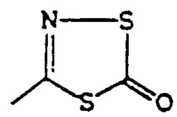

$Q_1$-11 ,

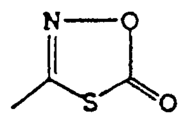

$Q_1$-12 ,

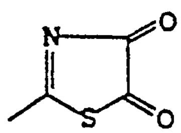

$Q_1$-13 ,

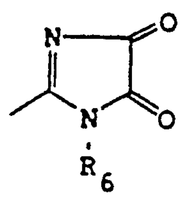

$Q_1$-14 ,

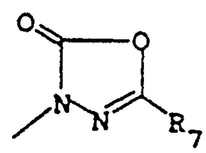

$Q_1$-15 ,

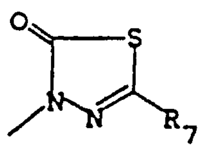

$Q_1$-16 ,

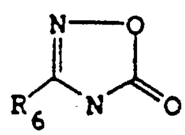

$Q_1$-17 ,

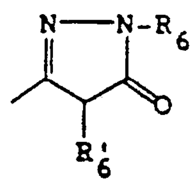

$Q_1$-18 ,

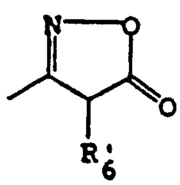

$Q_1$-19 ,

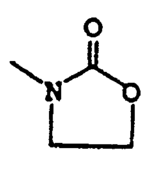

$Q_1$-20 ,

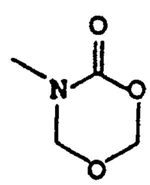

$Q_1$-21 ,

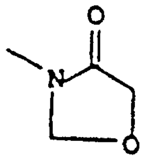 , 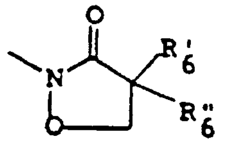 , 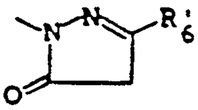

$Q_1$-22 $Q_1$-23 $Q_1$-24

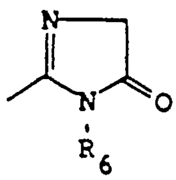 , 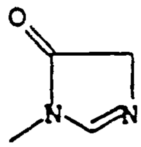 , 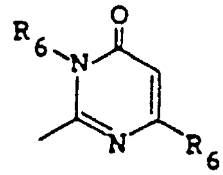 ,

$Q_1$-25 $Q_1$-26 $Q_1$-27

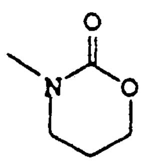 , 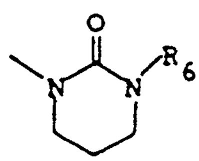 oder 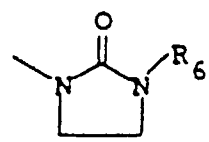 ;

$Q_1$-28 $Q_1$-29 $Q_1$-30

worin

$R_5$ H, $C_1$-$C_4$-Alkyl, $CH_2CH=CH_2$, $CH_2CH=CHCH_3$, $CH_2C \equiv CH$, $CH_2C \equiv CCH_3$, $CH_2CN$, $CH_2CO_2(C_1$-$C_2$-alkyl), $CH(CH_3)CO_2(C_1$-$C_2$-alkyl), $CF_2H$, mit 1 bis 3 F- oder Cl-Atomen substituiertes $C_2$-$C_3$-Alkyl, $CH_2OCH_3$, $CH_2OCH_2CH_3$, $CH_2CH_2OCH_3$, oder $CH_2CH_2OCH_2CH_3$ ist;

$R_6$ $C_1$-$C_3$-Alkyl ist; $R_6'$ und $R_6''$ unabhängig voneinander H oder $C_1$-$C_2$-Alkyl sind; und

$R_7$ H, $C_1$-$C_4$-Alkyl, $C_1$-$C_3$-Halogenoalkyl oder $CH_2CH=CH_2$ ist.

**3.** Verbindung nach Anspruch 1, worin $Q_2$ ausgewählt ist aus

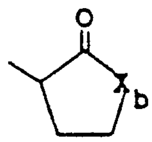 .

$Q_2$-1

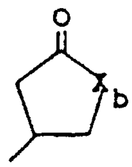 .

$Q_2$-2

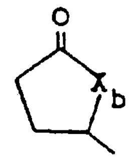 .

$Q_2$-3

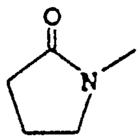 .

$Q_2$-4

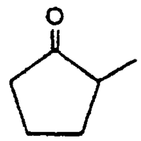 .

$Q_2$-5

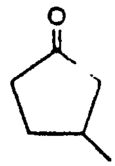 .

$Q_2$-6

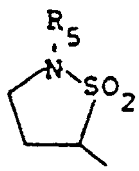 .

$Q_2$-7

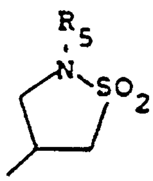 .

$Q_2$-8

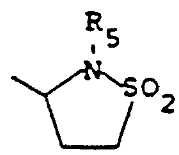 .

$Q_2$-9

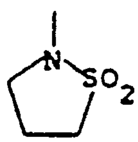 .

$Q_2$-10

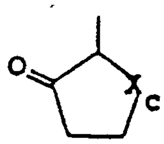 .

$Q_2$-11

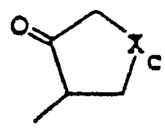 .

$Q_2$-12

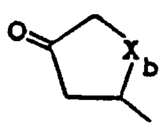 .

$Q_2$-13

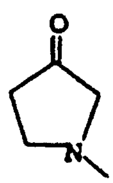 .

$Q_2$-14

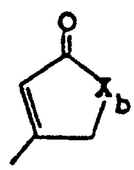 .

$Q_2$-15

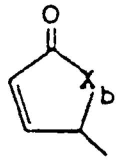 .

$Q_2$-16

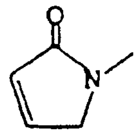 .

$Q_2$-17

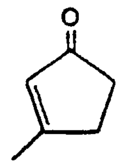 .

$Q_2$-18

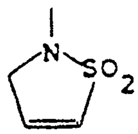 .

$Q_2$-19

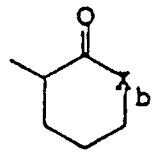 .

$Q_2$-20

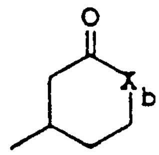 .

$Q_2$-21

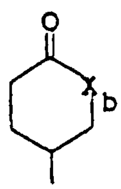 .

$Q_2$-22

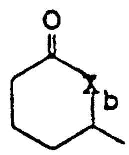 .

$Q_2$-23

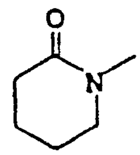 .

$Q_2$-24

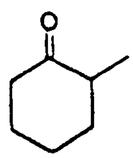 .

$Q_2$-25

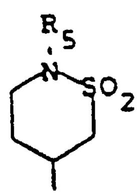 .

$Q_2$-26

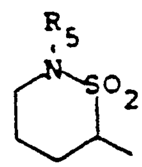 .

$Q_2$-27

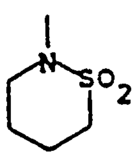 .

$Q_2$-28

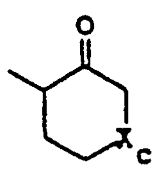 .

$Q_2$-29

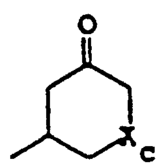 .

$Q_2$-30

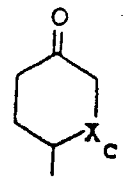 .

$Q_2$-31

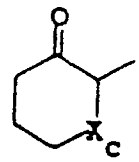 .

$Q_2$-32

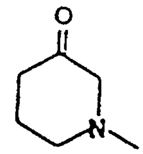 .

$Q_2$-33

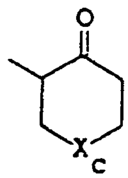 .

$Q_2$-34

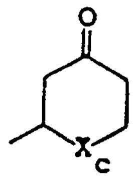 .

$Q_2$-35

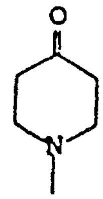 .

$Q_2$-36

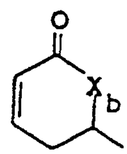 .

$Q_2$-37

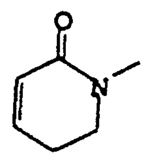 .

$Q_2$-38

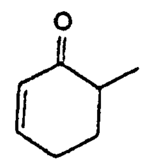 .

$Q_2$-39

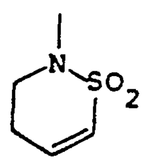 .

$Q_2$-40

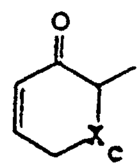 .

$Q_2$-41

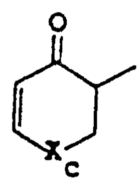 .

$Q_2$-42

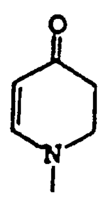 .

$Q_2$-43

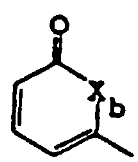 .

$Q_2$-44

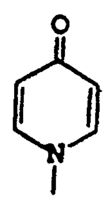 .

$Q_2$-45

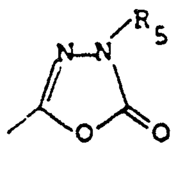

.

$Q_2$-46

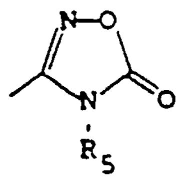

.

$Q_2$-47

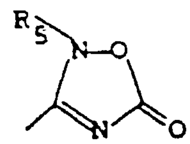

.

$Q_2$-48

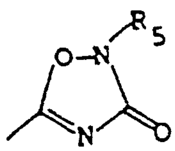

.

$Q_2$-49

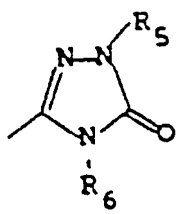

.

$Q_2$-50

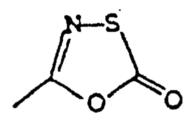

.

$Q_2$-51

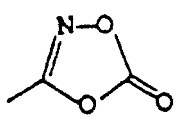

.

$Q_2$-52

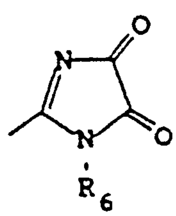

.

$Q_2$-53

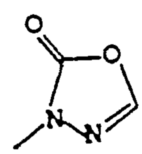

.

$Q_2$-54

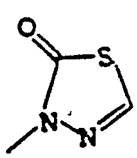

.

$Q_2$-55

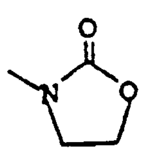

.

$Q_2$-56

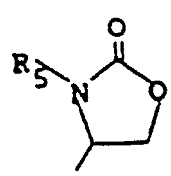

.

$Q_2$-57

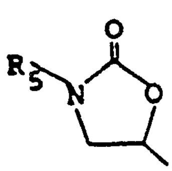

.

$Q_2$-58

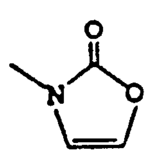

.

$Q_2$-59

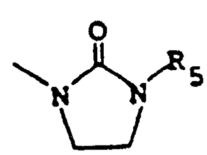

.

$Q_2$-60

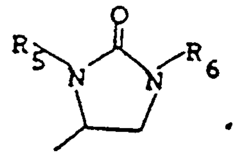

$Q_2$-61

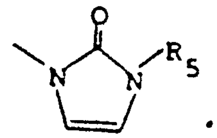

$Q_2$-62

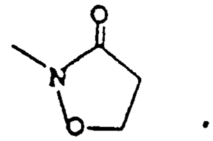

$Q_2$-63

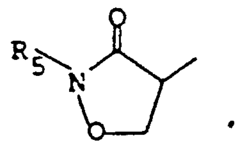

$Q_2$-64

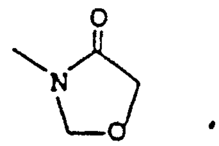

$Q_2$-65

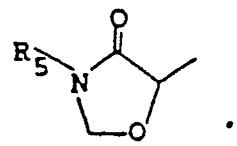

$Q_2$-66

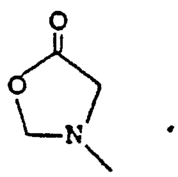

$Q_2$-67

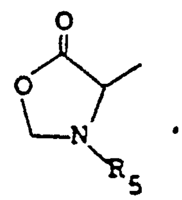

$Q_2$-68

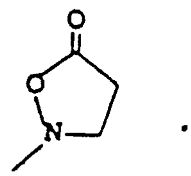

$Q_2$-69

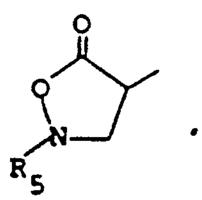

$Q_2$-70

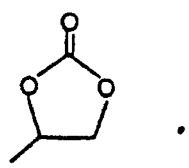

$Q_2$-71

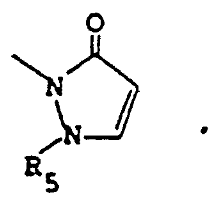

$Q_2$-72

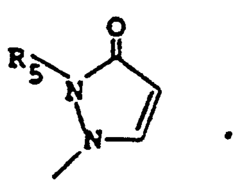

$Q_2$-73

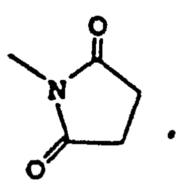

$Q_2$-74

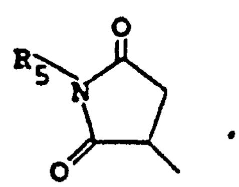

$Q_2$-75

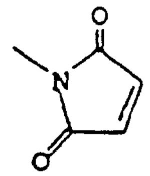 ,

$Q_2$-76

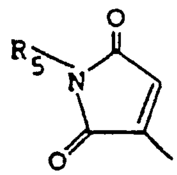 ,

$Q_2$-77

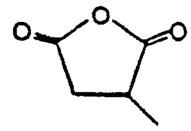 ,

$Q_2$-78

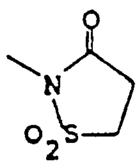 ,

$Q_2$-79

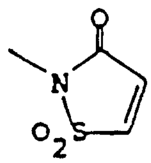 ,

$Q_2$-80

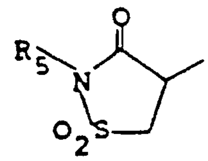 ,

$Q_2$-81

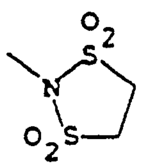 ,

$Q_2$-82

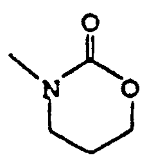 ,

$Q_2$-83

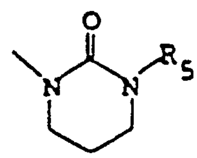 ,

$Q_2$-84

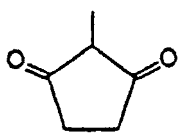 ,

$Q_2$-85

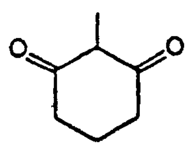 und

$Q_2$-86

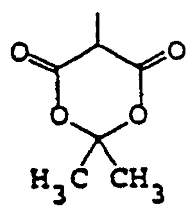 ;

$Q_2$-87

worin
$Q_2$-1 bis $Q_2$-87 gegebenenfalls mit 1 oder 2 Gruppen substituiert sein können, die aus $C_1$-$C_2$-Alkyl und $C_1$-$C_2$-Halogenalkyl ausgewählt sind;
$R_5$ und $R_6$ unabhängig voneinander H oder $C_1$-$C_3$-Alkyl sind;
$X_b$ O oder $NR_5$ ist; und
$X_c$ O, S, SO, $SO_2$ oder $NR_5$ ist.

**4.** Verbindungen nach Anspruch 1, worin
Q $Q_1$ ist;
E eine Einfachbindung ist; und
Z CH oder N ist.

**5.** Verbindungen nach Anspruch 1, worin
Q $Q_1$ ist;
E $CH_2$ ist;
W O ist; und
Z CH oder N ist.

**6.** Verbindungen nach Anspruch 1, worin
Q $Q_1$ ist;
E O ist; und
Z CH oder N ist.

**7.** Verbindungen nach Anspruch 2, worin E eine Einfachbindung ist und Z CH oder N ist.

**8.** Verbindungen nach Anspruch 7, worin
W O ist;
R H ist;
$R_1$ H, F, Cl, Br, $C_1$-$C_2$-Alkyl, $C_1$-$C_3$-Alkoxy, $C_1$-$C_3$-Alkylthio oder mit 1 bis 3 F-, Cl- oder Br-Atomen substituiertes $C_1$-$C_2$-Alkyl, $C_1$-$C_3$-Alkoxy oder $C_1$-$C_3$-Alkylthio ist;
X $C_1$-$C_2$-Alkyl, $C_1$-$C_2$-Alkoxy, C1, F, Br, I, $OCF_2H$, $CH_2F$, $CF_3$, $OCH_2CH_2F$, $OCH_2CHF_2$, $OCH_2CF_3$, $CH_2Cl$ oder $CH_2Br$ ist; und
Y $C_1$-$C_2$-Alkyl, $C_1$-$C_2$-Alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C{\equiv}CH$, $OCH_2CH_2OCH_3$, $CH_2SCH_3$,

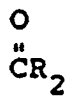, 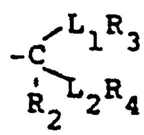, 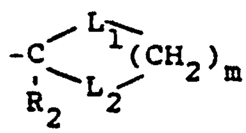, 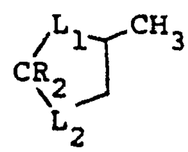,

$OCF_2H$, $SCF_2H$, Cyclopropyl, $C{\equiv}CH$ oder $C{\equiv}CCH_3$ ist.

**9.** Verbindungen nach Anspruch 8, worin A A-1 ist.

**10.** Verbindungen nach Anspruch 9, worin J J-1 bis J-5 ist.

**11.** Verbindungen nach Anspruch 9, worin
J J-1 ist;
$R_1$ H, Cl, $CH_3$ oder $OCH_3$ ist und sich nicht in der para-Position zu der Sulfonylharnstoff-Brücke befindet;
X $CH_3$, $OCH_3$, Cl oder $OCF_2H$ ist; und
Y $CH_3$, $OCH_3$, $C_2H_5$, $CH_2OCH_3$, $NHCH_3$, $CH(OCH_3)_2$ oder Cyclopropyl ist.

**12.** Verbindungen nach Anspruch 11, worin $Q_1$ $Q_1$-1 bis $Q_1$-27 ist.

**13.** Verbindungen nach Anspruch 2, worin
E $CH_2$ ist;
W O ist;
R H ist;
J J-1 ist;
$R_1$ H ist;
A A-1 ist;
X $CH_3$, $OCH_3$, $OCH_2CH_3$, Cl oder $OCF_2H$ ist;
Y $CH_3$, $OCH_3$, $C_2H_5$, $CH_2OCH_3$, $NHCH_3$, $CH(OCH_3)_2$ oder Cyclopropyl ist und $Q_1$ $Q_1$-1, $Q_1$-7, $Q_1$-10 oder $Q_1$-15 ist; und
Z CH oder N ist.

**14.** Verbindungen nach Anspruch 2, worin
E O ist;
R H ist;
J J-1 ist;
$R_1$ H ist;
A A-1 ist;
X $CH_3$, $OCH_3$, $OCH_2CH_3$, Cl oder $OCF_2H$ ist;

Y $CH_3$, $OCH_3$, $C_2H_5$, $CH_2OCH_3$, $NHCH_3$, $CH(OCH_3)_2$ oder Cyclopropyl ist und $Q_1$ $Q_1$-1, $Q_1$-7, $Q_1$-10 oder $Q_1$-15 ist; und
Z CH oder N ist.

**15.** Verbindungen nach Anspruch 1, worin
Q $E_1X_aQ_2$ ist;
E eine Einfachbindung ist; und
Z CH oder N ist.

**16.** Verbindungen nach Anspruch 1, worin
Q $E_1X_aQ_2$ ist;
E $CH_2$ ist;
W O ist;
Z CH oder N ist; und
$E_1$ eine Einfachbindung ist.

**17.** Verbindungen nach Anspruch 1, worin
Q $E_1X_aQ_2$ ist;
E O ist;
Z CH oder N ist; und
$E_1$ eine Einfachbindung ist.

**18.** Verbindungen nach Anspruch 3, worin
$Q_2$-1 bis $O_2$-87 gegebenenfalls mit 1 oder 2 Gruppen substituiert sein können, die aus $C_1$-$C_2$-Alkyl und $C_1$-$C_2$-Halogenalkyl ausgewählt sind;
$R_5$ und $R_6$ unabhängig voneinander H oder $C_1$-$C_3$-Alkyl sind;
$X_b$ O oder $NR_5$ ist;
$X_c$ O, S, SO, $SO_2$ oder $NR_5$ ist;
E eine Einfachbindung ist; und
Z CH oder N ist.

**19.** Verbindungen nach Anspruch 18, worin
W O ist;
$E_1$ eine Einfachbindung ist;
$X_a$ $CH_2$ oder $CH_2CH_2$ ist;
R H ist;
$R_1$ H, F, Cl, Br, $C_1$-$C_2$-Alkyl, $C_1$-$C_3$-Alkoxy, $C_1$-$C_3$-Alkylthio oder mit 1 bis 3 F-, Cl- oder Br-Atomen substituiertes $C_1$-$C_2$-Alkyl, $C_1$-$C_3$-Alkoxy oder $C_1$-$C_3$-Alkylthio ist;
X $C_1$-$C_2$-Alkyl, $C_1$-$C_2$-Alkoxy, Cl, F, Br, I, $OCF_2H$, $CH_2F$, $CF_3$, $OCH_2CH_2F$, $OCH_2CHF_2$, $OCH_2CF_3$, $CH_2Cl$ oder $CH_2Br$ ist; und
Y H, $C_1$-$C_2$-Alkyl, $C_1$-$C_2$-Alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C{\equiv}CH$, $OCH_2CH_2OCH_3$, $CH_2SCH_3$,

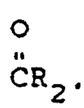, 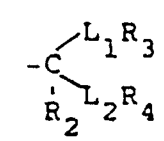, 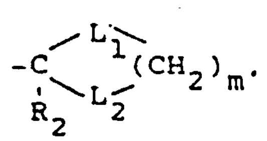, 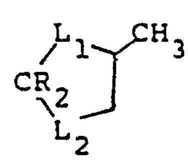,

$OCF_2H$, $SCF_2H$, Cyclopropyl, $C{\equiv}CH$ oder $C{\equiv}CCH_3$ ist.

**20.** Verbindungen nach Anspruch 19, worin A A-1 ist.

**21.** Verbindungen nach Anspruch 20, worin J J-1 bis J-5 ist.

**22.** Verbindungen nach Anspruch 21, worin
J J-1 ist;

$R_1$ H, Cl, $CH_3$ oder $OCH_3$ ist;
X $CH_3$, $OCH_3$, Cl oder $OCF_2H$ ist; und
Y $CH_3$, $OCH_3$, $C_2H_5$, $CH_2OCH_3$, $NHCH_3$, $CH(OCH_3)_2$ oder Cyclopropyl ist.

**23.** Verbindungen nach Anspruch 16, worin
R H ist;
J J-1 ist;
$R_1$ H ist;
A A-1 ist;
X $CH_3$, $OCH_3$, $OCH_2CH_3$, Cl oder $OCF_2H$ ist;
Y $CH_3$, $OCH_3$, $C_2H_5$, $CH_2OCH_3$, $NHCH_3$, $CH(OCH_3)_2$ oder Cyclopropyl ist;
Z CH oder N ist; und
$Q_2$ $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24, $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$-6O, $Q_2$-63, $Q_2$-71, $Q_2$-74, $Q_2$-76, $Q_2$-78 und $Q_2$-79 ist.

**24.** Verbindungen nach Anspruch 17, worin
R H ist;
$R_1$ H ist;
A A-1 ist;
X $CH_3$, $OCH_3$, $OCH_2CH_3$, Cl oder $OCF_2H$ ist;
Y $CH_3$, $OCH_3$, $C_2H_5$, $CH_2OCH_3$, $NHCH_3$, $CH(OCH_3)_2$ oder Cyclopropyl ist;
Z CH oder N ist; und
$Q_2$ $Q_2$-1, $Q_2$-4, $Q_2$-5, $Q_2$-7, $Q_2$-10, $Q_2$-11, $Q_2$-12, $Q_2$-17, $Q_2$-19, $Q_2$-20, $Q_2$-24, $Q_2$-25, $Q_2$-27, $Q_2$-28, $Q_2$-36, $Q_2$-38, $Q_2$-46, $Q_2$-47, $Q_2$-54, $Q_2$-56, $Q_2$-59, $Q_2$-60, $Q_2$-63, $Q_2$-71, $Q_2$-74, $Q_2$-76, $Q_2$-78 und $Q_2$-79 ist.

**25.** Verbindungen nach Anspruch 1, worin
Q $E_1X_aQ_2$ ist;
E eine Einfachbindung ist;
$X_a$ $CH_2$, $CH(CH_3)$, $CH_2CH_2$ oder $CH_2CH_2CH_2$ ist;
$Q_2$ ein gesättigter oder teilweise gesättigter 5- oder 6-gliedriger carbocyclischer Ring, der entweder eine oder zwei Carbonyl-Gruppen enthält, oder ein gesättigter oder teilweise gesättigter 5- oder 6-gliedriger heterocyclischer Ring, der 2-5 Kohlenstoff-Atome und 1-3 Hetero-Atome enthält, die aus der aus 0-2 Sauerstoff, 0-2 Schwefel oder 0-3 Stickstoff bestehenden Gruppe ausgewählt sind, wobei Schwefel die Form S, SO oder $SO_2$ annehmen kann, und der eine oder zwei Carbonyl- oder Sulfonyl-($SO_2$-)Gruppen oder eine Carbonyl- und eine Sulfonyl-Gruppe enthält, ist, wobei Q gegebenenfalls mit 1-2 Substituenten-Gruppen substituiert sein kann, wobei die Substituenten am Kohlenstoff aus der aus Halogen, $C_1$-$C_4$-Alkyl, $C_1$-$C_4$-Halogenoalkyl, $CH_2$($C_2$-$C_3$-Alkenyl), $CH_2$($C_2$-$C_3$-Alkinyl), $C_2$-$C_4$-Alkoxy-carbonyl, CN, OH, $C_1$-$C_3$-Alkoxy, $C_1$-$C_3$-Alkylthio, $C_1$-$C_3$-Alkylsulfinyl, $C_1$-$C_3$-Alkylsulfonyl oder $C_2$-$C_4$-Alkylcarbonyl bestehenden Gruppe ausgewählt sein können und die Substituenten am Stickstoff aus der aus $C_1$-$C_4$-Alkyl, $C_1$-$C_4$-Halogenoalkyl, $CH_2$($C_2$-$C_3$-Alkenyl), $CH_2$($C_2$-$C_3$-Alkinyl), $C_2$-$C_4$-Alkoxycarbonyl oder $C_2$-$C_4$-Alkylcarbonyl bestehenden Gruppe ausgewählt sein können;
$R_1$ H, $C_1$-$C_3$-Alkyl, $C_1$-$C_3$-Halogenoalkyl, Halogen, Nitro, $C_1$-$C_3$-Alkoxy, $SO_2NR_aR_b$, $C_1$-$C_3$-Alkylthio, $C_1$-$C_3$-Alkylsulfinyl, $C_1$-$C_3$-Alkylsulfonyl, $CH_2CN$, CN, $CO_2R_c$' $C_1$-$C_3$-Halogenoalkoxy oder $C_1$-$C_3$-Halo-genoalkylthio ist; und

A

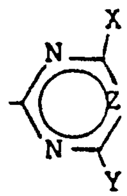 , 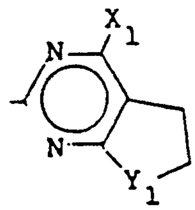 , 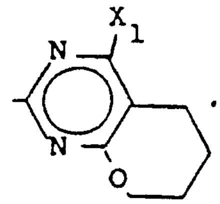 ,

A-1 A-2 A-3

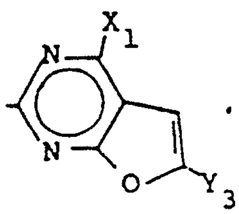 , 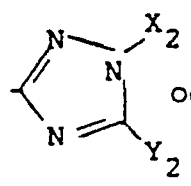 oder 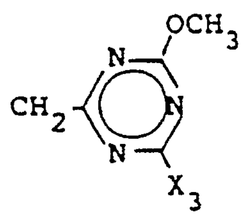

A-4 A-5 A-6

ist.

26. Verbindung ausgewählt aus
   - o 2-(4,5-Dihydro-4-methyl-5-oxo-1,3,4-oxadiazol-2-yl)-N-[ (4,6-dimethoxypyrimidin-2-yl)-aminocarbonyl]-benzolsulfonamid,
   - o 2-(4,5-Dihydro-4-methyl-5-oxo-1,3,4-oxadiazol-2-yl)-N-[ (4-methoxy-6-methyl-1,3,5-triazin-2-yl)-aminocarbonyl]benzolsulfonamid,
   - o N-[(4,6-Dimethoxypyrimidin-2-yl)aminocarbonyl]-2-(2-oxo-l-pyrrolidinylmethyl)benzolsulfonamid,
   - o N-[(4,6-Dimethoxy-1,3,5-triazin-2-yl)amino-carbonyl]2-(2-oxo-1-pyrrolidinylmethyl)-benzolsulfonamid,
   - o N-[(4,6-Dimethoxypyrimidin-2-yl)aminocarbonyl]-2-(2-oxo-3-oxazolidinylmethyl)-3-thiophensulfonamid und
   - o N-[(4-methoxy-6-methyl-1,3,5-triazin-2-yl)amino-carbonyl]-2-(2-oxo-3-oxazolidinylmethyl)-3-thiophensulfonamid.

27. Zusammensetzung, die zur Steuerung des Wachstums unerwünschter Vegetation geeignet ist, umfassend eine wirksame Menge einer Verbindung nach irgendeinem der Ansprüche 1 bis 26 und wenigstens einen der folgendenden Stoffe: Tensid, festes oder flüssiges Verdünnungsmittel.

28. Verfahren zur Steuerung des Wachstums unerwünschter Vegetation, umfassend das Aufbringen einer wirksamen Menge einer Verbindung nach irgendeinem der Ansprüche 1 bis 26 auf den zu schützenden Ort.

29. Verfahren zur Regulierung des Wachstums von Pflanzen, umfassend das Aufbringen einer wirksamen, jedoch im wesentlichen nicht phytotoxischen Menge eines das Pflanzenwachstum regulierenden Mittels, das aus den Verbindungen nach irgendeinem der Ansprüche 1 bis 26 ausgewählt ist, auf den Standort solcher Pflanzen.

30. Verfahren zur Herstellung einer Verbindung nach Anspruch 1, umfassend

(I) (a) die Umsetzung von $JSO_2NCW$ mit A-NHR,

(b) die Umsetzung von $JSO_2NH_2$ mit $PhO\overset{\overset{W}{\|}}{C}NHA$,

(c) die Umsetzung von $JSO_2NH\underset{\underset{W}{\|}}{C}OPh$ mit A-NHR ,

worin J, R, W und A die in Anspruch 1 angegebenen Bedeutungen haben, vorausgesetzt, daß E $CH_2$ oder eine Einfachbindung ist; oder

(II) (a) die Umsetzung von $JSO_2NCW$ mit A-NHR,

(b) die Umsetzung von JH mit $ClSO_2NH\underset{\underset{O}{\|}}{C}NHA$ ,

worin J, W und A die Anspruch 1 angegebenen Bedeutungen haben, vorausgesetzt, daß E O ist.

**31.** Verbindungen der Formeln

$JSO_2NCW$,

$JSO_2NH_2$,

$JSO_2NH\underset{\underset{W}{\|}}{C}OPh$

und

JH,

wie sie in Anspruch 30 definiert sind.